# EUROPÄISCHE PATENTSCHRIFT

(11) **EP 0 966 436 B1**
(45) Veröffentlichungstag und Bekanntmachung des Hinweises auf die Patenterteilung: **11.12.2002**
(21) Anmeldenummer: 98909427.1
(22) Anmeldetag: 10.02.1998
(51) Int. Cl.: C07C 311/08, C07D 217/02, C07D 217/04, A61K 31/18, C07D 209/44, C07D 213/76, C07D 215/26, C07C 311/10, C07C 309/80, C07C 309/82, C07C 43/275, C07C 217/90, C07D 275/02, A61K 31/47, A61K 31/40

(54) **ARYLSULFONAMIDE UND ANALOGA UND IHRE VERWENDUNG ZUR BEHANDLUNG VON NEURODEGENERATIVEN ERKRANKUNGEN**
ARYL SULFONAMIDES AND ANALOGUES THEREOF AND THEIR USE IN THE TREATMENT OF NEURODEGENERATIVE DISEASES
ARYLSULFONAMIDES ET LEURS ANALOGUES, ET PROCEDES D'UTILISATION DESDITES SUBSTANCES POUR TRAITER LES MALADIES NEURODEGENERATIVES

(30) Priorität: 21.02.1997 DE 19706902; 17.09.1997 DE 19740785
(43) Veröffentlichungstag der Anmeldung: 29.12.1999
(73) Patentinhaber: Bayer Aktiengesellschaft, 51368 Leverkusen (DE)
(72) Erfinder: MITTENDORF, Joachim, D-42113 Wuppertal (DE); DRESSEL, Jürgen, D-42477 Radevormwald (DE); MATZKE, Michael, D-42113 Wuppertal (DE); KELDENICH, Jörg, D-42113 Wuppertal (DE); MOHRS, Klaus-Helmut, D-42113 Wuppertal (DE); RADDATZ, Siegfried, D-51065 Köln (DE); FRANZ, Jürgen, D-42781 Haan (DE); SPREYER, Peter, D-40225 Düsseldorf (DE); VÖHRINGER, Verena, D-42113 Wuppertal (DE); SCHUHMACHER, Joachim, D-42113 Wuppertal (DE); ROCK, Michael-Harold, D-51065 Köln (DE); HORVATH, Ervin, D-51373 Leverkusen (DE); FRIEDL, Arno, D-51427 Bergisch Gladbach (DE); MAULER, Frank, D-51491 Overath (DE); DE VRY, Jean-Marie-Viktor, D-51503 Rösrath (DE); JORK, Reinhard, D-51491 Overath (DE)
(86) Internationale Anmeldenummer: EP9800716
(87) Internationale Veröffentlichungsnummer: WO98037061

(56) Entgegenhaltungen:
- EP-A- 0 261 539
- DE-A- 1 942 264
- DE-A- 2 136 828
- US-A- 3 462 473
- US-A- 4 931 457
- US-A- 5 532 237
- CHEMICAL ABSTRACTS, vol. 78, no. 21, 28.Mai 1973 Columbus, Ohio, US; abstract no. 135804w, S.M. SHEIN ET AL: "Preparation of phenyl ethers by condensation of aromatic bromo derivatives with phenols" Seite 341; XP002068305 & IZV.SIB.OTD.AKAD.NAUK.SSSR,SER. KHIM NAUK.., Nr. 1, 1973, Seiten 104-107,
- British Medical Associaction: "Therapeutic uses of cannabis", harwood academic publisher, 1997, pp. v-vii

## Beschreibung

Die vorliegende Erfindung betrifft neue Arylsulfonamide und Analoga, Verfahren zu ihrer Herstellung und ihre Verwendung zur Prophylaxe und Behandlung von neurodegenerativen Erkrankungen, insbesondere zur Behandlung von Apoplexia Cerebri und Schädel-Hirn-Trauma

Δ⁹-Tetrahydrocannabinol (Δ⁹-THC) und in geringem Maße auch Δ⁸-THC sind die biologisch aktiven Bestandteile in Extrakten der Pflanze Cannabis sativa (Marihuana, Haschisch) und sind verantwortlich für die Effekte auf das menschliche Zentrale Nervensystem (ZNS). Potentielle historische und kontemporäre therapeutische Anwendungen von Cannabis-Präparaten umfassen u.a. Analgesie, Emesis, Anorexie, Glaukom und Bewegungsstörungen.

Bislang wurden zwei Subtypen von Cannabinoid-Rezeptoren und eine Spleiß-Variante identifiziert. Der CB1-Rezeptor (Nature 1990, 346, 561) und eine Spleiß-Variante CB1a (J. Biol. Chem. 1995, 270, 3726) sind überwiegend im Zentralen Nervensystem lokalisiert. Der CB2-Rezeptor wurde überwiegend im peripheren Gewebe, insbesondere in Leukozyten, Milz und Makrophagen gefunden (Eur. J. Biochem. 1995, 232, 54).

CB1 und CB2-Rezeptoren besitzen sieben Transmembranregionen und gehören zur Familie der G-Protein-Rezeptoren. Beide Rezeptoren sind negativ gekoppelt via Gᵢ/Gₒ-Protein zur Adenylatcyclase und möglicherweise negativ gekoppelt zur präsynaptischen Freisetzung von Glutamat (J. Neurosci. 1996, 16, 4322). CB1-Rezeptoren sind darüberhinaus positiv gekoppelt mit Kalium-Kanälen sowie negativ gekoppelt mit N- und Q-Typ Calcium-Kanälen.

Vier Klassen von CB1-Rezeptor-Agonisten sind bisher bekannt: klassische Cannabinoide, wie beispielsweise Δ⁹-THC, nichtklassische Cannabinoide, Aminoalkylindole und Eicosanoide. Zu den letzten gehört der allgemein akzeptierte endogene CB1-Rezeptor-Agonist Anandamid.

Außerdem ist bekannt, daß Apoplexia Cerebri eine Folge einer plötzlichen Durchblutungsstörung eines menschlichen Gehirnbereichs mit nachfolgenden Funktionsausfällen, mit entsprechenden neurologischen und/oder psychischen Symptomen ist. Die Ursachen für Apoplexia Cerebri können in Hirnblutungen (z.B nach einem Gefäßriß bei Hypertonie, Arteriosklerose und Aneurysma) und Ischämien (z.B. durch eine Blutdruckabfallkrise oder Embolie) liegen. Die Funktionsausfälle im Gehirn führen zu einer Degeneration oder Abtötung der Gehirnzellen (Journal of Cerebral Blood Flow and Metabolism 1981, 1, 155; Chem. Eng. News 1996 (May 13), 41; Trends Pharmacol, Sci. 1996, 17, 227). Unter Schädel-Hirn-Trauma versteht man gedeckte und offene Schädelverletzungen mit Gehirnbeteiligung. Die EP-A-0 261 539 offenbart N-(Chinolyl-oxy-phenyl)sulfonamide zur Behandlung von Ischämien.

Aus dem US-A-3,462,473 sind Phenoxyphenyl Alkansulfonsäureesther sowie deren hypocholersterolämische und hypotriglyceridämische Wirkung bekannt.

Das ÜS-A-4,931,457 beschreibt Aryloxypyridinamine zur Behandlung von Hauterkrankungen.

In der DE-A-2 136 828 werden Phenoxyphenole als Zwischenstufen für Lipidsenker beschrieben. Aus Chem. Abstr. 1973, 78: 135804w sind bestimmte Naphthoxy- und Phenoxyphenole sowie bis-Naphthoxy- und bis-Phenoxybenzole bekannt.

Die vorliegende Erfindung betrifft Verbindungen der allgemeinen Formel (I)

R¹-A-D-E-G-L-R² (I)

in welcher
- R¹: für Naphthyl oder für einen Rest der Formel 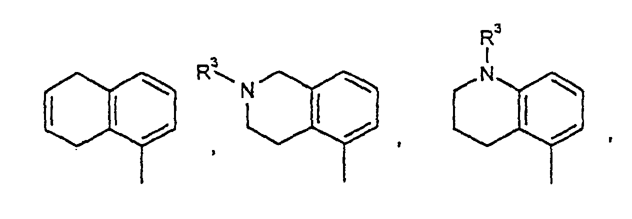 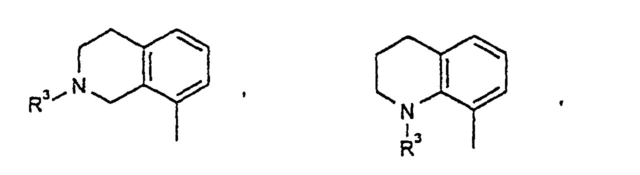 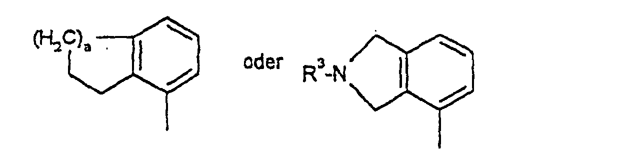 steht,
worin
a eine Zahl 1 oder 2 bedeutet,
R³ Wasserstoff, (C₂-C₆)-Alkenyl, (C₁-C₆)-Alkyl oder (C₁-C₆)-Acyl bedeutet, und wobei alle oben aufgeführten Ringsysteme und Reste gegebenenfalls mit einem oder mehreren, gleichen oder verschiedenen Substituenten gegebenenfalls geminal substituiert sind, die aus der Gruppe ausgewählt sind, die besteht aus:
Halogen, Carboxyl, Hydroxy, Phenyl, (C₁-C₆)-Alkoxy, (C₁-C₆)-Alkoxycarbonyl, (C₁-C₈)-Alkyl, das seinerseits durch Halogen, C₁-C₆-Alkylsulfonyloxy, Azid, Amino, Mono(C₁-C₆)-Alkylamino, Di(C₁-C₆)-Alkylamino oder Hydroxy substituiert sein kann,
einer Gruppe der Formel -(CO)_{b}-NR⁴R⁵,
worin
b eine Zahl 0 oder 1 bedeutet,
R⁴ und R⁵ gleich oder verschieden sind und unabhängig voneinander Wasserstoff, Phenyl, (C₁-C₆)-Acyl, cyclo(C₄-C₇)-Acyl, Benzoyl oder (C₁-C₆)-Alkyl, das gegebenenfalls durch Amino, Mono(C₁-C₆)-Alkylamino, Di(C₁-C₆)-Alkylamino substituiert ist, bedeuten, oder
R⁴ und R⁵ gemeinsam mit dem Stickstoffatom einen 5- oder 6-gliedrigen gesättigten Heterocyclus bilden, der gegebenenfalls ein oder mehrere weitere(s) Heteroatom(e) aus der Reihe S, O und/oder einen oder mehrere Rest(e) der Formel -NR⁸ enthalten kann,
worin
R⁸ Wasserstoff, (C₁-C₆)-Alkyl oder (C₁-C₆)-Acyl bedeutet, und
einer Gruppe der Formel -NR⁶-SO₂-R⁷
worin
R⁶ Wasserstoff, Phenyl, (C₁-C₆)-Alkyl oder (C₁-C₆)-Acyl bedeutet,
R⁷ Phenyl oder (C₁-C₆)-Alkyl bedeutet,
- A und E: gleich oder verschieden sind und für eine Bindung oder für (C₁-C₄)-Alkylen stehen,
- D: für ein Sauerstoffatom oder für einen Rest der Formel -S(O)_{c}- oder -N(R⁹)- steht,
worin
c eine Zahl 0, 1 oder 2 bedeutet,
R⁹ Wasserstoff, (C₁-C₆)-Alkyl oder (C₁-C₆)-Acyl bedeutet,
- G: für zweifach gebundenes (C₆-C₁₀)-Aryl oder für einen zweifach gebundenen 5- bis 7-gliedrigen aromatischen Heterocyclus mit bis zu 3 Heteroatomen aus der Reihe S, N und/oder O steht, die gegebenenfalls mit einem oder mehreren, gleichen oder verschiedenen Substituenten substituiert sind, die aus der Gruppe ausgewählt sind, die besteht aus:
Hydroxy, Trifluormethyl, Carboxyl, Halogen, (C₁-C₆)-Alkyl, Hydroxy(C₁-C₆)alkyl, (C₁-C₆)-Alkoxy, (C₁-C₆)-Alkoxycarbonyl,
sowie Gruppen der Formeln

-CO-O-(CH₂)_{d}-NR¹⁰R¹¹, -NR¹²-SO₂R¹³,

-(CH₂)ₑ-(CO)_{f}-NR¹⁴R¹⁵ und -OR¹⁶,

worin
d eine Zahl 1, 2, 3 oder 4 bedeutet,
e und f gleich oder verschieden sind und eine Zahl 0 oder 1 bedeuten,
R¹⁰ und R¹¹ die oben angegebene Bedeutung von R⁴ und R⁵ haben und mit dieser gleich oder verschieden sind,
R¹² die oben angegebene Bedeutung von R⁶ hat und mit dieser gleich oder verschieden ist,
R¹³ die oben angegebene Bedeutung von R⁷ hat und mit dieser gleich oder verschieden ist,
R¹⁴ und R¹⁵ die oben angegebene Bedeutung von R⁴ und R⁵ haben und mit dieser gleich oder verschieden sind,
oder unabhängig voneinander einen Rest der Formel

-(CH₂)_{g}-NR¹⁷R¹⁸

darstellen,
worin
g eine Zahl 1, 2, 3 oder 4 bedeutet, und
R¹⁷ und R¹⁸ die oben angegebene Bedeutung von R⁴ und R⁵ haben und mit dieser gleich oder verschieden sind,
R¹⁶ (C₆-C₁₀)-Aryl bedeutet,
- L: für einen Rest der Formel

-O-, -NH-,

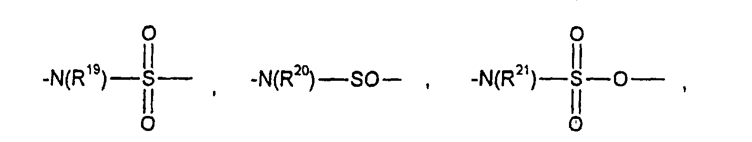

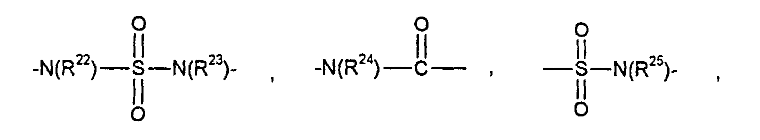

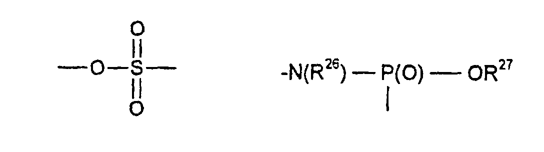

oder 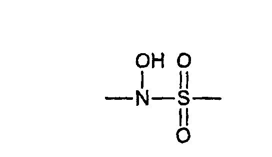 steht,
wobei die Anbindung der Reste an G linksbündig erfolgt,
und worin R¹⁹, R²⁰, R²¹, R²², R²³, R²⁴, R²⁵, R²⁶ und R²⁷ gleich oder verschieden sind und Wasserstoff oder (C₁-C₄)-Alkyl bedeuten,
oder
R¹⁹ einen Rest der Formel -SO₂R² bedeutet,
- R²: für (C₆-C₁₀)-Aryl oder für einen 5- bis 7-gliedrigen gesättigten oder aromatischen Heterocyclus mit bis zu 3 Heteroatomen aus der Reihe S, N und/oder O steht, die gegebenenfalls mit einem oder mehreren, gleichen oder verschiedenen Substituenten substituiert sind, die aus der Gruppe ausgewählt sind, die besteht aus:
Halogen, Trifluormethyl, Nitro, Amino und (C₁-C₆)-Alkyl,
oder
für den Rest der Formel 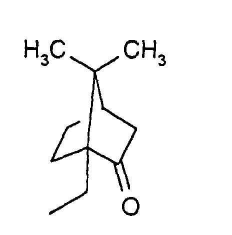 oder Morpholin steht, oder
für C₃-C₈-Cycloalkyl steht, oder
für (C₁-C₁₂)-Alkyl, (C₂-C₁₂)-Alkenyl oder (C₂-C₁₂)-Alkinyl steht, die gegebenenfalls mit einem oder mehreren, gleichen oder verschiedenen Substituenten substituiert sind, die aus der Gruppe ausgewählt sind, die besteht aus:
Halogen, Trifluormethyl, Hydroxy, Cyano, Azido, (C₁-C₆)-Alkoxy, (C₁-C₆)-Perfluoralkoxy, partiell fluoriertem (C₁-C₆)-Alkoxy, einem Rest der Formel 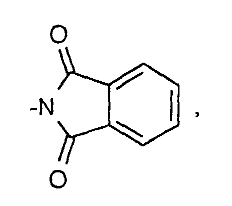 -NR²⁸R²⁹,
worin R²⁸ und R²⁹ die oben angegebene Bedeutung von R⁴ und R⁵ haben und mit dieser gleich oder verschieden sind,
Phenyl, gegebenenfalls substituiert mit einem oder mehreren, gleichen oder verschiedenen Substituenten, die aus der Gruppe ausgewählt sind, die besteht aus:
Halogen, Nitro, Hydroxy, (C₁-C₆)-Alkyl, (C₁-C₆)-Alkoxy und einer Gruppe der Formel -NR³⁰R³¹,
worin R³⁰ und R³¹ gleich oder verschieden sind und Wasserstoff oder (C₁-C₆)-Alkyl oder (C₁-C₆)-Acyl bedeuten,
und einem 5- bis 6-gliedrigen aromatischen Heterocyclus mit bis zu drei Heteroatomen aus der Reihe S, N und/oder O, gegebenenfalls substituiert mit einem oder. mehreren, gleichen oder verschiedenen Substituenten, die aus der Gruppe ausgewählt sind, die besteht aus:
Halogen, Nitro, Hydroxy, (C₁-C₆)-Alkyl, (C₁-C₆)-Alkoxy und einer Gruppe der Formel -NR³⁰R³¹,
worin R³⁰ und R³¹ wie oben definiert sind,
oder
- L und R²: gemeinsam für einen Rest der Formel 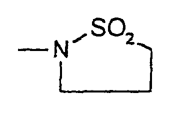 stehen,
und deren Salze,
zur Verwendung als Medikament in der Behandlung von Menschen und Tieren.
Bevorzugt sind Verbindungen der Formel (I), worin
- R¹: für Naphthyl oder für einen Rest der Formel 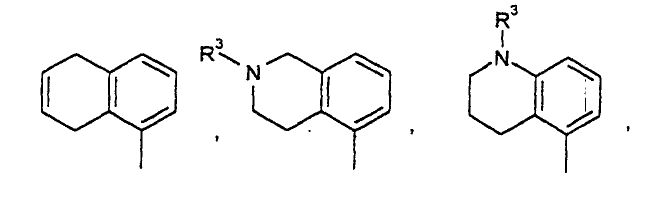 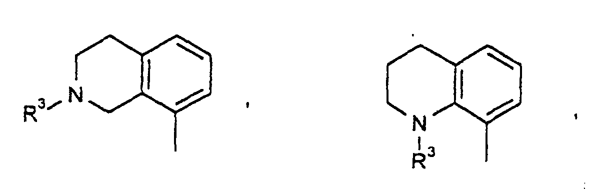 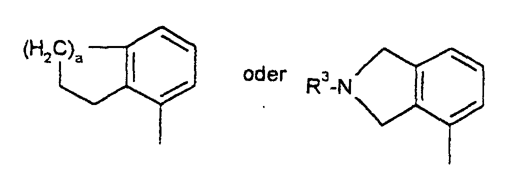 steht,
worin
a eine Zahl 1 oder 2 bedeutet,
R³ Wasserstoff, (C₂-C₄)-Alkenyl, (C₁-C₄)-Alkyl oder (C₁-C₄)-Acyl bedeutet, und wobei alle oben aufgeführten Ringsysteme und Reste gegebenenfalls mit einem oder mehreren, gleichen oder verschiedenen Substituenten gegebenenfalls geminal substituiert sind, die aus der Gruppe ausgewählt sind, die besteht aus:
Halogen, Carboxyl, Hydroxyl, Phenyl, (C₁-C₄)-Alkoxy, (C₁-C₅)-Alkoxycarbonyl, (C₁-C₆)-Alkyl, das seinerseits durch Halogen, (C₁-C₄)-Alkylsulfonyloxy, Azid, Amino, Mono(C₁-C₄)-Alkylamino, Di(C₁-C₄)-Alkylamino oder Hydroxy substituiert sein kann,
einer Gruppe der Formel -(CO)_{b}-NR⁴R⁵
worin
b eine Zahl 0 oder 1 bedeutet,
R⁴ und R⁵ gleich oder verschieden sind und unabhängig voneinander Wasserstoff, Phenyl, (C₁-C₄)-Acyl, cyclo(C₄-C₇)-Acyl, Benzoyl oder (C₁-C₄)-Alkyl, das gegebenenfalls durch Amino, Mono(C₁-C₄)-Alkylamino, Di(C₁-C₄)-Alkyl substituiert ist, bedeuten, oder
R⁴ und R⁵ gemeinsam mit dem Stickstoffatom einen Morpholin-, Piperidin- oder N-Methylpiperazinring bilden, und
einer Gruppe der Formel -NR⁶-SO₂-R⁷
worin
R⁶ Wasserstoff, Phenyl, (C₁-C₄)-Alkyl oder (C₁-C₄)-Acyl bedeutet und
R⁷ Phenyl oder (C₁-C₅)-Alkyl bedeutet,
- A und E: gleich oder verschieden sind und für eine Bindung oder für (C₁-C₄)-Alkylen stehen,
- D: für ein Sauerstoffatom oder für einen Rest der Formel -S(O)_{c}- oder -NR⁹- steht,
worin
c eine Zahl 0, 1 oder 2 bedeutet,
R⁹ Wasserstoff oder (C₁-C₄)-Alkyl oder (C₁-C₄)-Acyl bedeutet,
- G: für zweifach gebundenes Phenyl, Naphthyl, Pyrimidyl, Pyridazinyl oder Pyridyl steht, die gegebenenfalls mit einem oder mehreren, gleichen oder verschiedenen Substituenten substituiert sind, die aus der Gruppe ausgewählt sind, die besteht aus:
Hydroxy, Trifluormethyl, Carboxyl, Halogen, (C₁-C₄)-Alkyl, Hydroxy(C₁-C₄)alkyl, (C₁-C₄)-Alkoxy, (C₁-C₄)-Alkoxycarbonyl, sowie Gruppen der Formeln

-CO-O-(CH₂)_{d}-NR¹⁰R¹¹, -NR¹²-SO₂R¹³,

-(CH₂)ₑ-(CO)_{f}-NR¹⁴R¹⁵ und -OR¹⁶,

worin
d eine Zahl 1, 2, 3 oder 4 bedeutet,
e und f gleich oder verschieden sind und eine Zahl 0 oder 1 bedeuten,
R¹⁰ und R¹¹ die oben angegebene Bedeutung von R⁴ und R⁵ haben und mit dieser gleich oder verschieden sind,
R¹² die oben angegebene Bedeutung von R⁶ hat und mit dieser gleich oder verschieden ist,
R¹³ die oben angegebene Bedeutung von R⁷ hat und mit dieser gleich oder verschieden ist,
R¹⁴ und R¹⁵ die oben angegebene Bedeutung von R⁴ und R⁵ haben und mit dieser gleich oder verschieden sind, oder unabhängig voneinander einen Rest der Formel

-(CH₂)_{g}-NR¹⁷R¹⁸

darstellen,
worin
g eine Zahl 1, 2 oder 3 bedeutet, und
R¹⁷ und R¹⁸ die oben angegebene Bedeutung von R¹⁰ und R¹¹ haben und mit dieser gleich oder verschieden sind,
R¹⁶ Phenyl oder Naphthyl bedeutet,
- L: für einen Rest der Formel

-O-, -NH-,

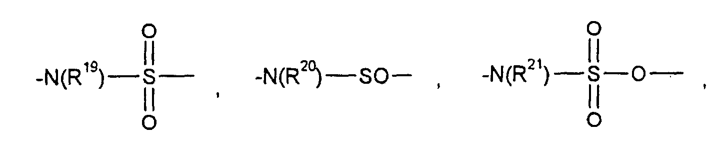

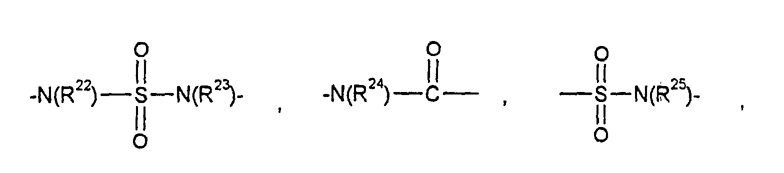

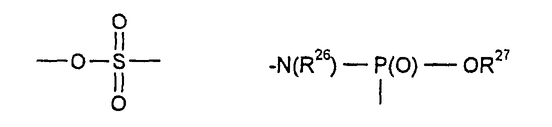

oder 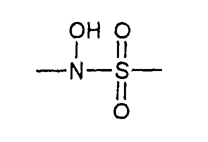 steht,
wobei die Anbindung der Reste an G linksbündig erfolgt,
und
worin
R¹⁹, R²⁰, R²¹, R²², R²³, R²⁴, R²⁵, R²⁶ und R²⁷ gleich oder verschieden sind und Wasserstoff oder (C₁-C₃)-Alkyl bedeuten, oder
R¹⁹ einen Rest der Formel -SO₂R² bedeutet,
- R²: für Phenyl, Naphthyl, Pyridyl, Furyl, Thienyl oder Pyrimidyl steht, die gegebenenfalls mit einem oder mehreren, gleichen oder verschiedenen Substituenten substituiert sind, die aus der Gruppe ausgewählt sind, die besteht aus:
Halogen, Amino, Trifluormethyl, Nitro und (C₁-C₄)-Alkyl,
oder
für den Rest der Formel 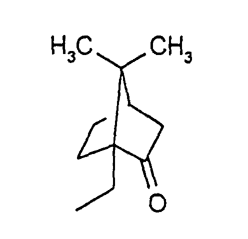 oder für Morpholin steht,
oder
für Cyclopropyl, Cyclohexyl oder Cyclopentyl steht, oder
für (C₁-C₁₀)-Alkyl, (C₂-C₁₀)-Alkenyl oder (C₂-C₁₀)-Alkinyl steht, die gegebenenfalls mit einem oder mehreren, gleichen oder verschiedenen Substitutenen substituiert sind, die aus der Gruppe ausgewählt sind die besteht aus:
Halogen, Trifluormethyl, Hydroxy, Azido, (C₁-C₄)-Alkoxy, (C₁-C₅)-Perfluoralkoxy, partiell fluoriertem (C₁-C₄)-Alkoxy, einem Rest der Formel 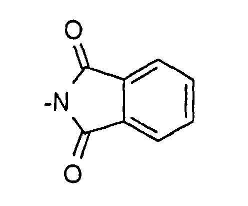 und -NR²⁸R²⁹,
worin
R²⁸ und R²⁹ die oben angegebene Bedeutung von R⁴ und R⁵ haben und mit dieser gleich oder verschieden sind, Phenyl, gegebenenfalls substituiert mit einem oder mehreren, gleichen oder verschiedenen Substituenten, die aus der Gruppe ausgewählt sind, die besteht aus:
Halogen, Nitro, Hydroxy, (C₁-C₄)-Alkyl, (C₁-C₄)-Alkoxy und einer Gruppe der Formel -NR³⁰R³¹,
worin R³⁰ und R³¹ gleich oder verschieden sind und Wasserstoff oder (C₁-C₄)-Alkyl oder (C₁-C₄)-Acyl bedeuten,
Pyridyl und Pyrimidyl, gegebenenfalls substituiert mit einem oder mehreren, gleichen oder verschiedenen Substituenten, die aus der Gruppe ausgewählt sind, die besteht aus:
Halogen, Nitro, Hydroxy, (C₁-C₄)-Alkyl, (C₁-C₄)-Alkoxy und einer Gruppe der Formel -NR³⁰R³¹,
worin R³⁰ und R³¹ wie oben definiert sind,
oder
- L und R²: gemeinsam für einen Rest der Formel 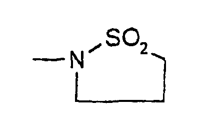 stehen,
und deren Salze,
zur Verwendung als Medikament in der Behandlung von Menschen und Tieren. Besonders bevorzugt sind Verbindungen der Formel (I), worin
- R¹: für Naphthyl oder für einen Rest der Formel 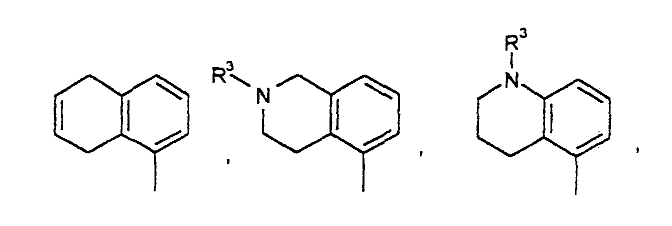 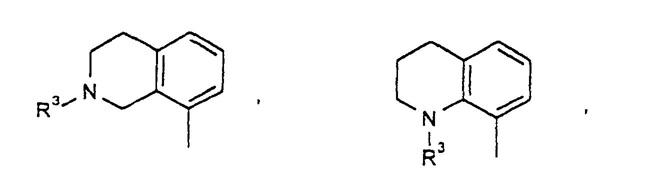 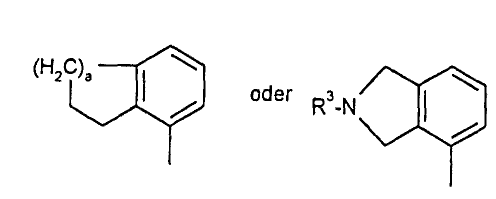 steht,
worin
a eine Zahl 1 oder 2 bedeutet,
R³ Wasserstoff, (C₂-C₃)-Alkenyl, (C₁-C₃)-Alkyl oder (C₁-C₃)-Acyl bedeutet, und wobei alle oben aufgeführten Ringsysteme gegebenenfalls mit einem oder mehreren, gleichen oder verschiedenen Substituenten gegebenenfalls geminal substituiert sind, die aus der Gruppe ausgewählt sind, die besteht aus:
Chlor, Fluor, Carboxyl, Hydroxyl, Phenyl, (C₁-C₃)-Alkoxy, (C₁-C₄)-Alkoxycarbonyl, (C₁-C₄)-Alkyl, das seinerseits durch Chlor, Methylsulfonyloxy oder Hydroxy substituiert sein kann,
einer Gruppe der Formel -(CO)_{b}-NR⁴R⁵
worin
b eine Zahl 0 oder 1 bedeutet,
R⁴ und R⁵ gleich oder verschieden sind und unabhängig voneinander Wasserstoff, (C₁-C₃)-Acyl, cyclo(C₄-C₆)-Acyl, Benzoyl oder (C₁-C₃)-Alkyl, das gegebenenfalls durch Amino, Mono(C₁-C₃)-Alkylamino, Di(C₁-C₃)-Alkylamino substituiert ist, bedeuten, oder
R⁴ und R⁵ gemeinsam mit dem Stickstoffatom einen Morpholin-, Piperidin- oder N-Methylpiperazinring bilden, und
einer Gruppe der Formel -NR⁶-SO₂-R⁷
worin
R⁶ Wasserstoff, (C₁-C₃)-Alkyl oder (C₁-C₃)-Acyl bedeutet, und
R⁷ Phenyl oder (C₁-C₄)-Alkyl bedeutet,
- A und E: gleich oder verschieden sind und für eine Bindung oder für (C₁-C₃)-Alkyl stehen,
- D: für ein Sauerstoffatom oder für einen Rest der Formel -S(O)_{c}- oder -NR⁹- steht,
worin
c eine Zahl 0, 1 oder 2 bedeutet,
R⁹ Wasserstoff oder (C₁-C₃)-Alkyl oder (C₁-C₃)-Acyl bedeutet,
- G: für zweifach gebundenes Phenyl, Naphthyl, Pyrimidyl, Pyridazinyl oder Pyridyl steht, die gegebenenfalls mit einem oder mehreren, gleichen oder verschiedenen Substituenten substituiert sind, die aus der Gruppe ausgewählt sind, die besteht aus:
Hydroxy, Trifluormethyl, Carboxyl, Fluor, Chlor, Brom, (C₁-C₃)-Alkyl, Hydroxy(C₁-C₃)alkyl, (C₁-C₃)-Alkoxy, (C₁-C₃)-Alkoxycarbonyl, sowie Gruppen der Formeln

-CO-O-(CH₂)_{d}-NR¹⁰R¹¹, -NR¹²-SO₂R¹³,

-(CH₂)ₑ-(CO)_{f}-NR¹⁴R¹⁵, -CH₂OH und -OR¹⁶,

worin
d eine Zahl 1, 2 oder 3 bedeutet,
e und f gleich oder verschieden sind und eine Zahl 0 oder 1 bedeuten,
R¹⁰ und R¹¹ Wasserstoff oder Methyl bedeuten,
R¹² Wasserstoff bedeutet,
R¹³ (C₁-C₄)-Alkyl bedeutet,
R¹⁴ und R¹⁵ die oben angegebene Bedeutung von R⁴ und R⁵ haben und mit dieser gleich oder verschieden sind oder einen Rest der Formel -(CH₂)_{g}-NR¹⁷R¹⁸ bedeuten,
worin
g eine Zahl 1, 2 oder 3 bedeutet, und
R¹⁷ und R¹⁸ Wasserstoff oder Methyl bedeuten, oder
R¹⁴ und R¹⁵ gemeinsam mit dem Stickstoffatom einen Rest der Formel 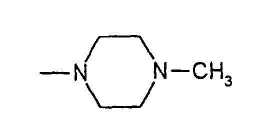 bilden,
R¹⁶ Phenyl oder Naphthyl bedeutet,
- L: für einen Rest der Formel

-O-, -NH-,

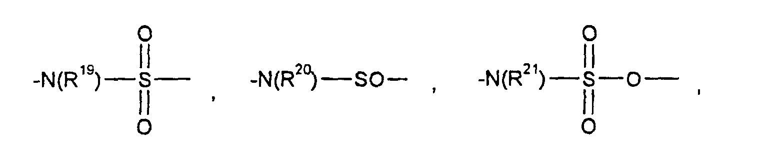

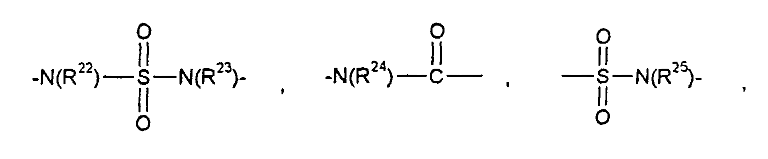

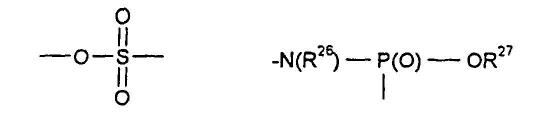

oder 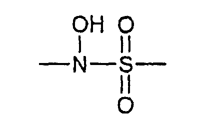 steht,
wobei die Anbindung der Reste an G linksbündig erfolgt,
und
worin
R¹⁹, R²⁰, R²¹, R²², R²³, R²⁴, R²⁵, R²⁶ und R²⁷ gleich oder verschieden sind und Wasserstoff, Methyl oder Ethyl bedeuten, oder
R¹⁹ einen Rest der Formel -SO₂R² bedeutet,
- R²: für Phenyl, Furyl oder Pyridyl steht, die gegebenenfalls mit einem oder mehreren, gleichen oder verschiedenen Substituenten substituiert sind, die aus der Gruppe ausgewählt sind, die besteht aus: Fluor, Chlor, Brom oder Trifluormethyl,
oder
für den Rest der Formel 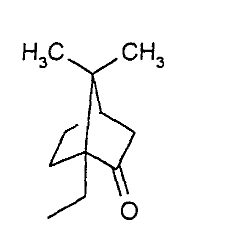 oder Morpholin steht,
oder
für Cyclopentyl oder Cyclohexyl steht, oder
für (C₁-C₈)-Alkyl, (C₂-C₈)-Alkenyl oder (C₂-C₈)-Alkinyl steht, die gegebenenfalls mit einem oder mehreren, gleichen oder verschiedenen Substitutenen substituiert sind, die aus der Gruppe ausgewählt sind, die besteht aus:
Fluor, Chlor, Brom, Trifluormethyl, Hydroxy, Azido, (C₁-C₃)-Alkoxy, (C₁-C₄)-Perfluoralkoxy, Trifluormethyl-substituiertem (C₁-C₄)-Alkoxy, einem Rest der Formel 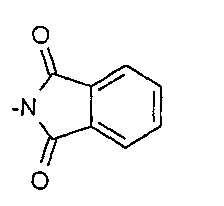 und -NR²⁸R²⁹,
worin
R²⁸ und R²⁹ Wasserstoff oder Methyl bedeuten, Phenyl, Pyridyl und Pyrimidyl, gegebenenfalls substituiert mit einem oder mehreren, gleichen oder verschiedenen Substituenten, die aus der Gruppe ausgewählt sind, die besteht aus:
Fluor, Chlor, Brom, Mitro, Hydroxy, (C₁-C₃)-Alkyl, (C₁-C₃)-Alkoxy und einer Gruppe der Formel -NR³⁰R³¹,
worin R³⁰ und R³¹ gleich oder verschieden sind und Wasserstoff, Methyl oder Methylcarbonyl bedeuten,
oder
- L und R²: gemeinsam für einen Rest der Formel 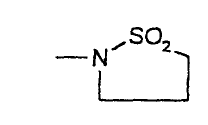 stehen,
und deren Salze,
zur Verwendung als Medikament in der Behandlung von Menschen und Tieren.

Außerdem betrifft dit Erfindung.

Verbindungen der allgemeinen Formel (I),

R¹-A-D-E-G-L-R² (I)

in welcher R¹, A, D, E, G, L und R² die oben angegebene Bedeutung haben,
mit der Ausnahme von Verbindungen der allgemeinen Formel (I),
in welchen
- R¹: für Naphth-1-yl steht, wobei die 3-Position des Naphth-1-yl-Rests gegebenenfalls mit Chlor oder (C₁-C₄)-Alkyl substituiert ist und die 4-Position des Naphth-1-yl-Rests gegebenenfalls mit Chlor oder Phenyl substituiert ist,
- A und E: für eine Bindung stehen,
- D: für ein Sauerstoffatom steht,
- G: für 1,4-Phenylen, das gegebenenfalls durch (C₁-C₄)-Alkyl substituiert ist, steht,
- L: für ein Sauerstoffatom steht, und
- R²: für Methyl steht,
und mit der Ausnahme von m-bis-(1-Naphthyloxy)benzol.

Die vorliegende Erfindung betrifft auch Verbindungen der Formel (I),
worin
- R¹: für Naphthyl oder für einen Rest der Formel 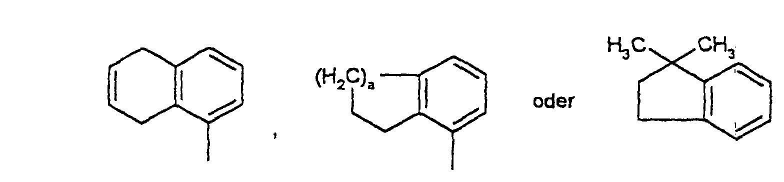 steht,
worin
a eine Zahl 1 oder 2 bedeutet, und wobei alle oben aufgeführten Ringsysteme und Reste gegebenenfalls mit 1 bis 3, gleichen oder verschiedenen Substituenten, gegebenenfalls geminal, substituiert sind, die aus der Gruppe ausgewählt sind, die besteht aus:
Halogen, Carboxyl, Hydroxy, (C₁-C₆)-Alkoxy, (C₁-C₆)-Alkoxycarbonyl, (C₁-C₈)-Alkyl, das seinerseits durch Halogen oder Hydroxy substituiert sein kann,
einer Gruppe der Formel -(CO)_{b}-NR⁴R⁵,
worin
b eine Zahl 0 oder 1 bedeutet,
R⁴ und R⁵ gleich oder verschieden sind und Wasserstoff, Phenyl oder (C₁-C₆)-Alkyl bedeuten, und
einer Gruppe der Formel -NR⁶-SO₂-R⁷
worin
R⁶ Wasserstoff, Phenyl oder (C₁-C₆)-Alkyl bedeutet,
R⁷ Phenyl oder (C₁-C₆)-Alkyl bedeutet,
- A und E: gleich oder verschieden sind und für eine Bindung oder für (C₁-C₄)-Alkylen stehen,
- D: für ein Sauerstoffatom oder für einen Rest der Formel -S(O)_{c}- oder -NH- steht,
worin
c eine Zahl 0, 1 oder 2 bedeutet,
- G: für zweifach gebundenes (C₆-C₁₀)-Aryl oder für einen zweifach gebundenen 5- bis 7-gliedrigen aromatischen Heterocyclus mit bis zu 3 Heteroatomen aus der Reihe S, N und/oder O steht, die gegebenenfalls mit einem bis drei, gleichen oder verschiedenen Substituenten substituiert sind, die aus der Gruppe ausgewählt sind, die besteht aus:
Hydroxy, Carboxyl, Halogen, (C₁-C₆)-Alkyl, Hydroxy(C₁-C₆)alkyl, (C₁-C₆)-Alkoxy, (C₁-C₆)-Alkoxycarbonyl, sowie Gruppen der Formeln

-CO-O-(CH₂)_{d}-NR¹⁰R¹¹, -NR¹²-SO₂R¹³ und -CO-NR¹⁴R¹⁵

worin
d eine Zahl 1, 2, 3 oder 4 bedeutet,
R¹⁰ und R¹¹ die oben angegebene Bedeutung von R⁴ und R⁵ haben und mit dieser gleich oder verschieden sind,
R¹² die oben angegebene Bedeutung von R⁶ hat und mit dieser gleich oder verschieden ist,
R¹³ die oben angegebene Bedeutung von R⁷ hat und mit dieser gleich oder verschieden ist,
R¹⁴ und R¹³ die oben angegebene Bedeutung von R⁴ und R⁵ haben und mit dieser gleich oder verschieden sind, oder gemeinsam mit dem Stickstoffatom einen 5- bis 6-gliedrigen gesättigten Heterocyclus bilden, der gegebenenfalls noch ein weiteres Heteroatom aus der Reihe S und O oder eine Gruppe der Formel -NH- enthalten kann,
- L: für einen Rest der Formel 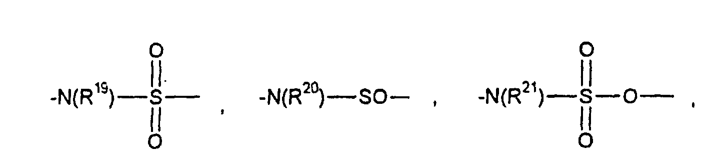 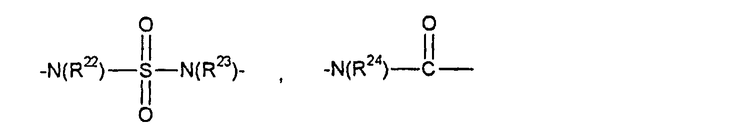 oder 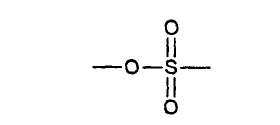 steht,
wobei die Anbindung der Reste an G linksbündig erfolgt,
und worin R¹⁹, R²⁰, R²¹, R²², R²³ und R²⁴ gleich oder verschieden sind und Wasserstoff oder (C₁-C₄)-Alkyl bedeuten,
- R²: für Phenyl steht, das gegebenenfalls mit Halogen, Trifluormethyl, Nitro, Amino, oder (C₁-C₆)-Alkyl substituiert ist,
- R²: für den Rest der Formel 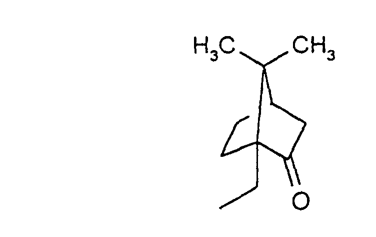 oder Morpholin steht,
oder
für Perfluoralkyl mit bis zu 12 Fluoratomen steht, oder
für (C₁-C₁₂)-Alkyl oder (C₂-C₁₂)-Alkinyl steht, die gegebenenfalls mit Halogen, Trifluormethyl, Hydroxy, Azido oder durch einen Rest der Formel 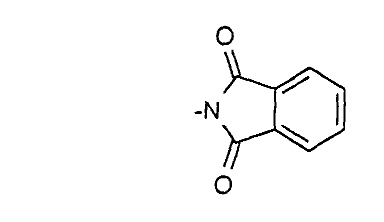 oder -NR²⁸R²⁹ substituiert sind,
worin R²⁸ und R²⁹ die oben angegebene Bedeutung von R⁴ und R⁵ haben und mit dieser gleich oder verschieden sind,
und/oder gegebenenfalls durch Phenyl oder durch einen 5- bis 6-gliedrigen aromatischen Heterocyclus mit bis zu 3 Heteroatomen aus der Reihe S, N und/oder O substituiert sind, die ihrerseits bis zu 2-fach gleich oder verschieden durch Halogen, Nitro, Hydroxy, (C₁-C₆)-Alkyl, (C₁-C₆)-Alkoxy oder durch eine Gruppe der Formel -NR³⁰R³¹ substituiert sein können,
worin R³⁰ und R³¹ gleich oder verschieden sind und Wasserstoff, (C₁-C₆)-Alkyl oder (C₁-C₆)-Acyl bedeuten,
- L und R²: gemeinsam für einen Rest der Formel 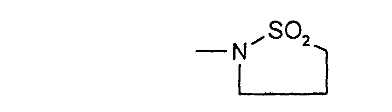 stehen, und deren Salze.

Ganz besonders bevorzugt sind erfindungsgemäße Verbindungen der Formel (I),
worin
- R¹: für Naphth-1-yl, gegebenenfalls substituiert durch (C₁-C₆)-Alkyl substituiert mit Hydroxy, (C₁-C₆)-Acylamino, Amino oder (C₁-C₆)-Alkoxy, Indan-4-yl, substituiert durch Hydroxy(C₁-C₆)-Alkyl,
für einen Rest der Formel 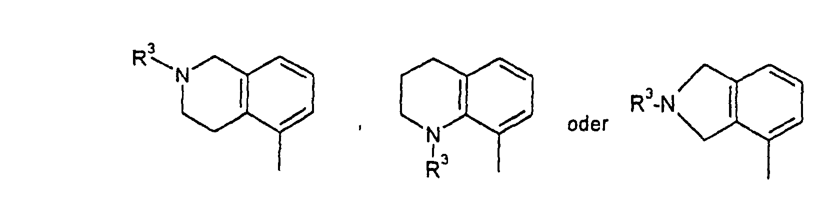 steht,
worin
R³ (C₁-C₆)-Alkyl ist,
- E und A: für eine Bindung stehen,
- D: für ein Sauerstoffatom stehen,
- G: für 1,3-Phenylen, 1,4-Phenylen oder 2,5-Pyridylen steht, die gegebenenfalls durch Halogen substituiert sind,
- L: für einen Rest der Formel -NH-SO₂- oder -O-SO₂- steht und
- R²: für (C₁-C₆)-Alkyl steht, das gegebenenfalls durch Chlor, Trifluormethyl, durch einen Rest der Formel -O-CH₂-CF₃ oder durch Phenyl oder durch Pyridyl substituiert ist, die ihrerseits durch Brom oder Chlor substituiert sein können,
und deren Salze.

Insbesondere seien folgende ganz besonders bevorzugte Verbindungen genannt:

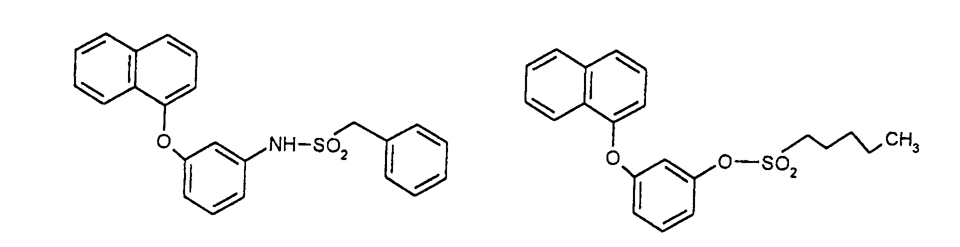

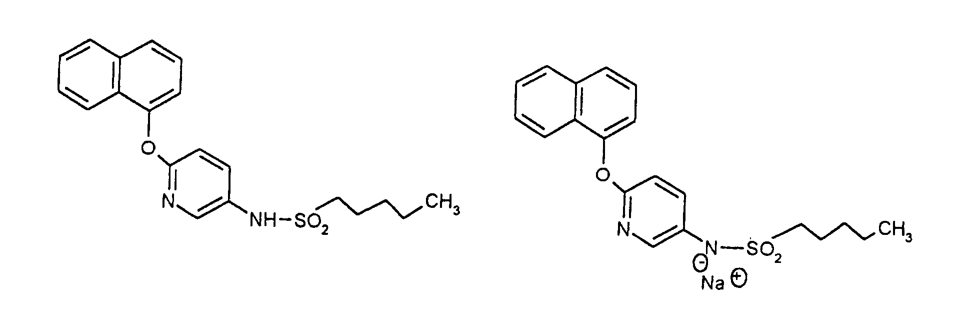

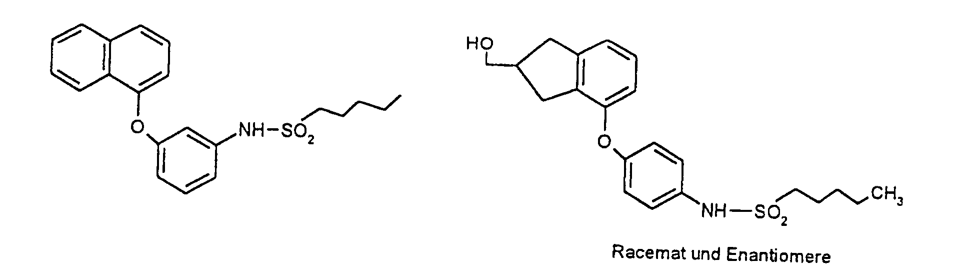

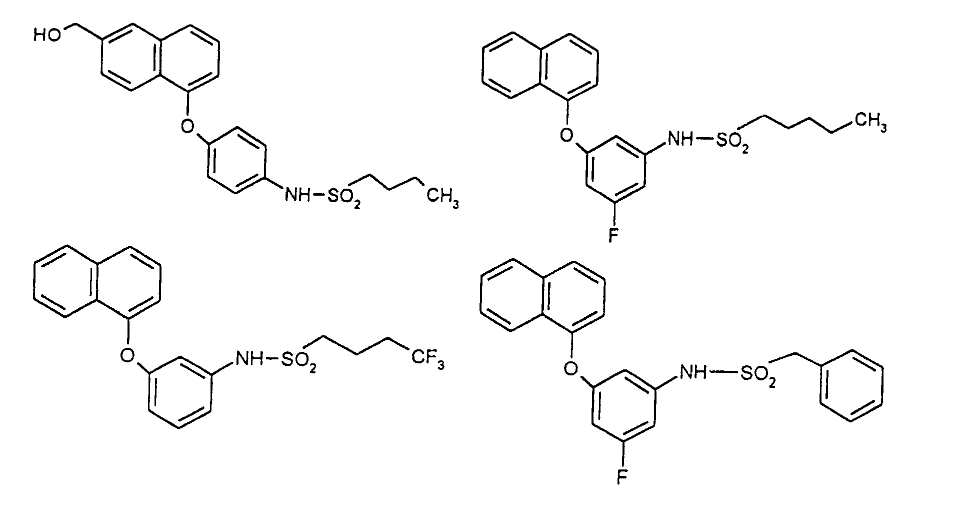

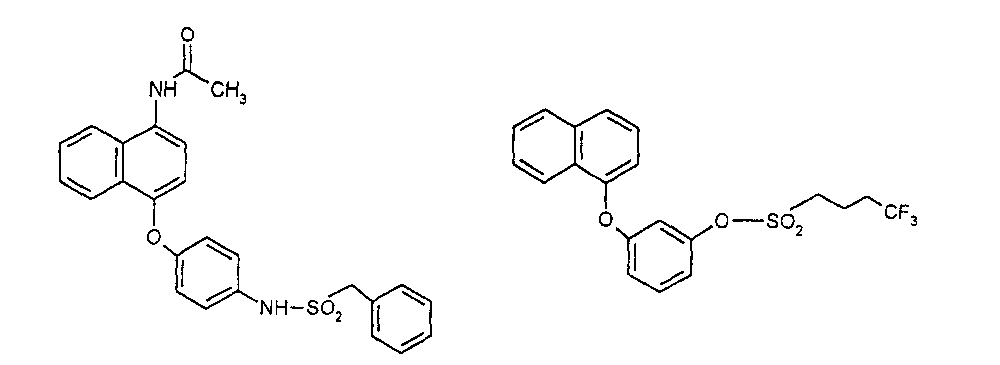

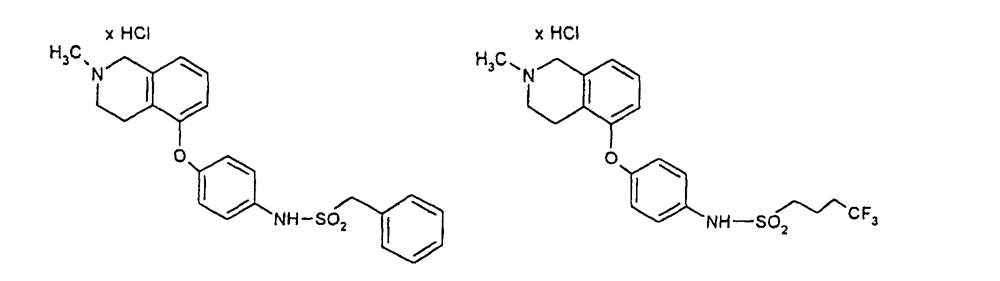

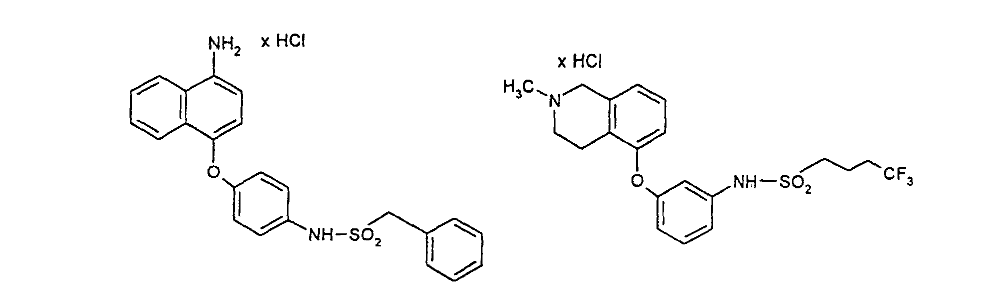

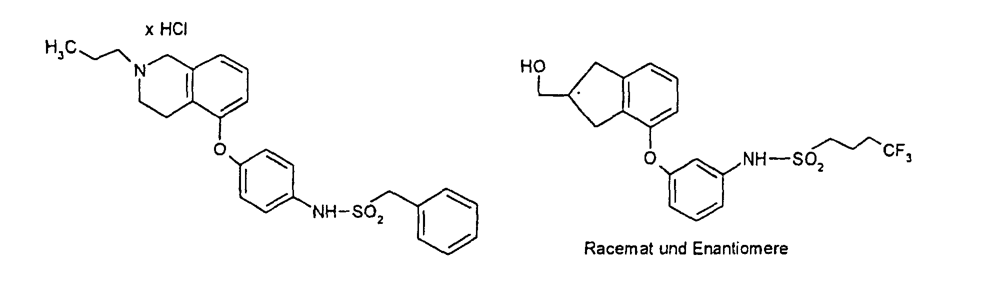

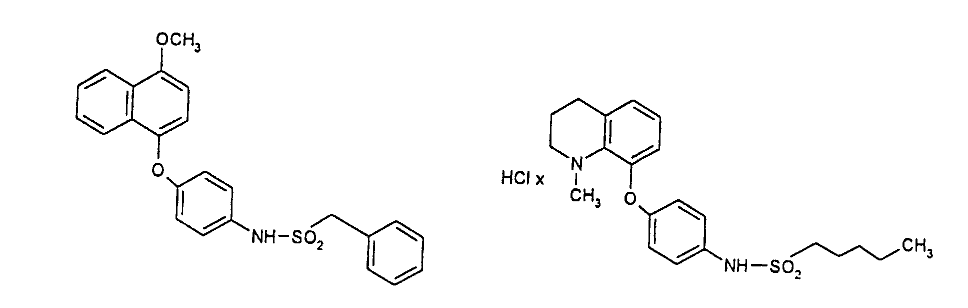

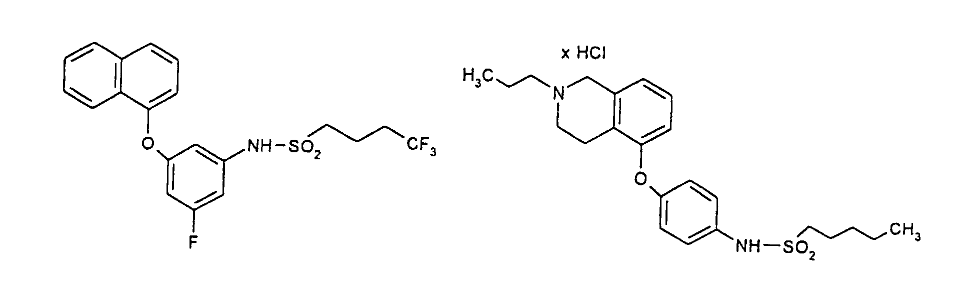

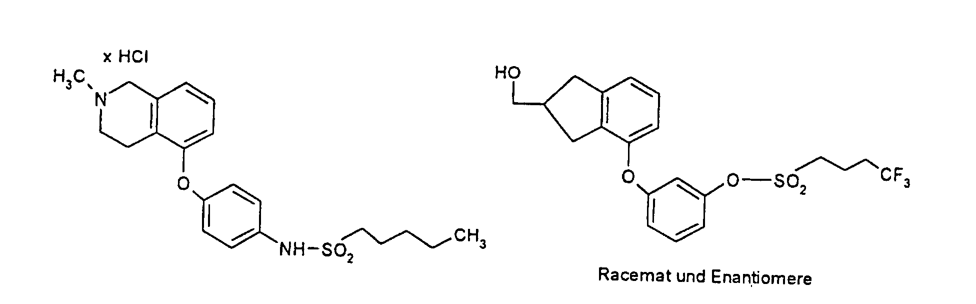

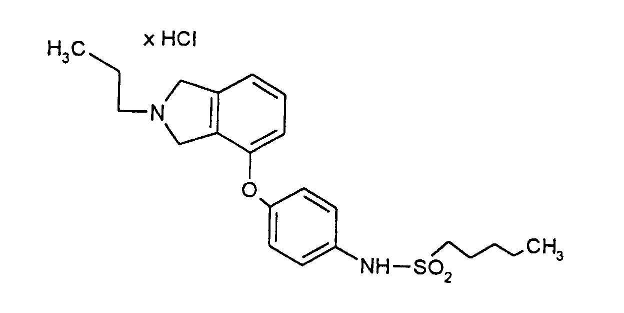

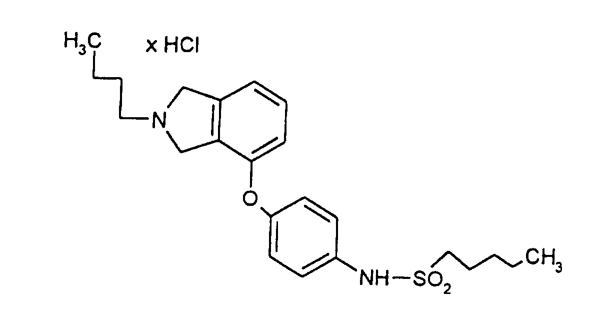

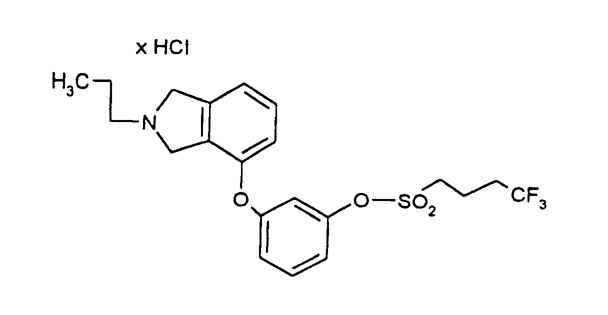

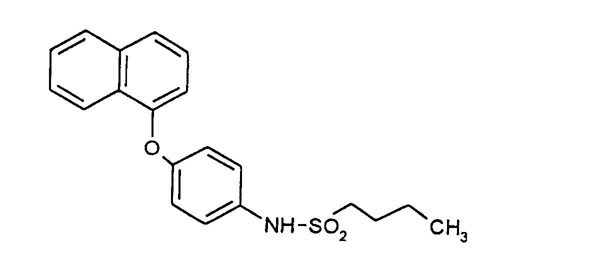

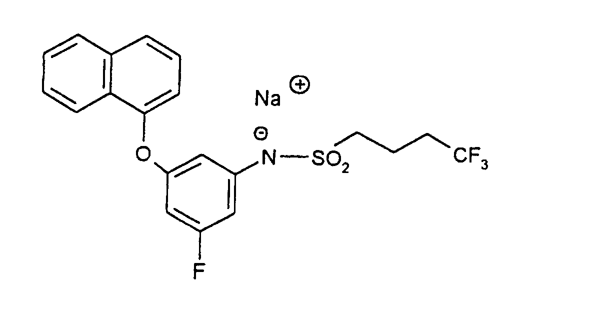

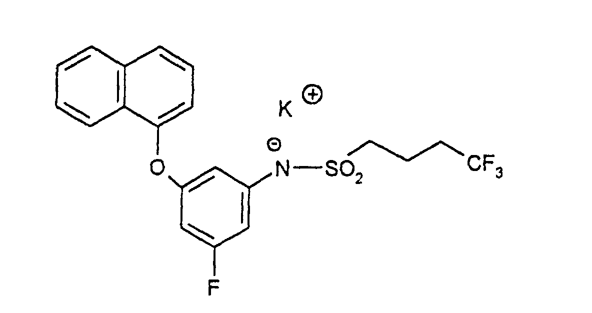

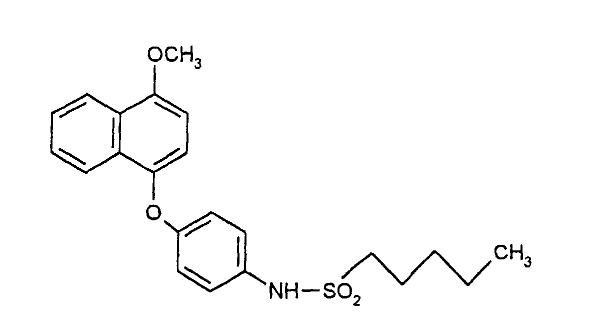

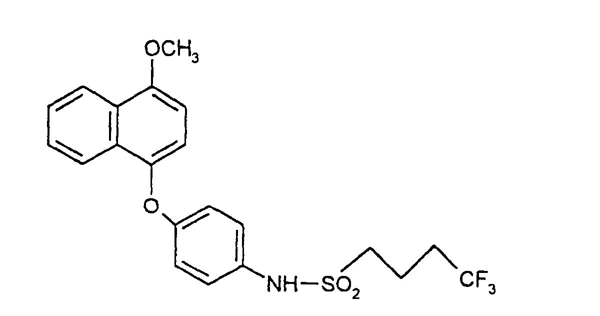

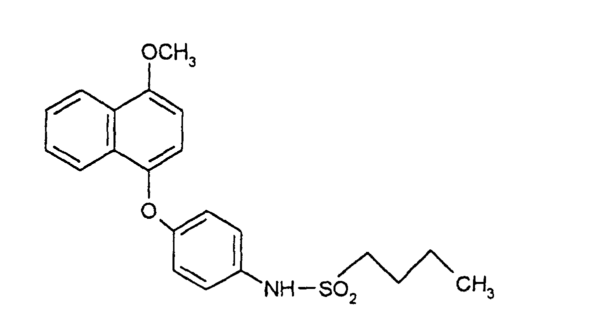

Die erfindungsgemäßen Verbindungen können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Zur vorliegenden Erfindung gehören auch Ammoniumverbindungen, die durch Überführung der freien Amine mittels Alkylierung hergestellt werden können.

Im Rahmen der vorliegenden Erfindung haben die Substituenten im allgemeinen die folgende Bedeutung:

(C₁-C₁₂)-Alkyl steht im allgemeinen in Abhängigkeit von den oben aufgeführten Substituenten für einen geradkettigen oder verzweigten Kohlenwassserstoffrest mit 1 bis 12 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Bevorzugt ist (C₁-C₈)-Alkyl mit 1 bis 8 Kohlenstoffatomen, z.B. Nonyl, Decyl, Undecyl, Dodecyl.

(C₂-C₁₂)-Alkenyl stehen im allgemeinen in Abhängigkeit von den oben aufgeführten Substitutenten für einen geradkettigen oder verzweigten Kohlenwasserstoffrest mit 2 bis 6 und 2 bis 20 Kohlenstoffatomen und einer oder mehreren, bevorzugt mit einer oder zwei Doppelbindungen. Bevorzugt ist der Niederalkylrest mit 2 bis 4 und 2 bis 10 Kohlenstoffatomen und einer Doppelbindung. Besonders bevorzugt ist ein Alkenylrest mit 2 bis 3 und 2 bis 8 Kohlenstoffatomen und einer Doppelbindung. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl und Isooctenyl genannt.

(C₂-C₁₂)-Alkinyl steht im allgemeinen in Abhängigkeit von den oben aufgeführten Substituenten für einen geradkettigen oder verzweigten Kohlenwasserstoffrest mit 2 bis 12 Kohlenstoffatomen und einer oder mehreren, bevorzugt mit einer oder zwei Dreifachbindungen. Bevorzugt ist der Niederalkylrest mit 12 bis etwa 10 Kohlenstoffatomen und einer Dreifachbindung. Besonders bevorzugt ist ein Alkylrest mit 2 bis 8 Kohlenstoffatomen und einer Dreifachbindung. Beispielsweise seien Acetylen, 2-Butin, 2-Pentin und 2-Hexin genannt.

(C₁-C₆)-Acyl steht im allgemeinen in Abhängigkeit von den oben aufgeführten Substituenten für geradkettiges oder verzweigtes Niedrigalkyl mit 1 bis 6 Kohlenstoffatomen, die über eine Carbonylgruppe gebunden sind. Bevorzugt sind Alkylreste mit bis zu 4 Kohlenstoffatomen. Ganz besonders bevorzugt sind beispielsweise Alkylreste mit bis zu 3 Kohlenstoffatomen. Beispielsweise seien genannt: Acetyl, Ethylcarbonyl, Propylcarbonyl, Isopropylcarbonyl, Butylcarbonyl und Isobutylcarbonyl.

(C₁-C₆)-Alkoxy steht im allgemeinen in Abhängigkeit von den oben aufgeführten Substituenten für einen über ein Sauerstoffatom gebundenen geradkettigen oder verzweigten Kohlenwasserstoffrest mit 1 bis 6 Kohlenstoffatomen. Bevorzugt ist Niederalkoxy mit 1 bis 4 Kohlenstoffatomen. Besonders bevorzugt ist ein Alkoxyrest mit 1 bis 3 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

(C₁-C₆)- Alkoxycarbonyl kann beispielsweise durch die Formel 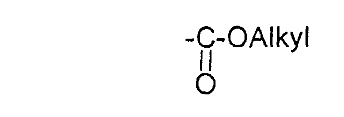 dargestellt werden.

Alkyl steht hierbei für einen geradkettigen oder verzweigten Kohlenwasserstoffrest mit 1 bis 6 Kohlenstoffatomen. Bevorzugt wird Niederalkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

(C₃-C₈)-Cycloalkcyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.

cyclo(C₄-C₇)Acyl steht im allgemeinen in Abhängigkeit von den oben aufgeführten Substituenten für Cyclopropylcarbonyl, Cyclobutylcarbonyl, Cyclopentylcarbonyl oder Cyclohexylcarbonyl.

(C₆-C₁₀)-Aryl steht im allgemeinen für einen aromatischen Rest mit 6 bis 10 Kohlenstoffatomen. Bevorzugte Arylreste sind Phenyl und Naphthyl.

(C₁-C₆)-Perfluoralkoxy steht im Rahmen der Erfindung für einen Alkoxyrest mit 1 bis 6 Kohlenstoffatomen und 3 bis 13 Fluoratomen. Bevorzugt ist ein Alkoxyrest mit 1 bis 5 Kohlenstoffatomen und 3 bis 9 Fluoratomen.

(C₁-C₆)-partiell fluoriertes Alkoxy steht im Rahmen der Erfindung für einen Alkoxyrest mit 1 bis 6 Kohlenstoffatomen und 3 bis 5 Fluoratomen. Bevorzugt ist ein Alkoxyrest mit 1 bis 4 Kohlenstoffatomen und 3 Fluoratomen. Besonders bevorzugt ist ein Alkoxyrest mit 1 bis 3 Kohlenstoffatomen, der durch Trifluormethyl substituiert ist.

Halogen steht im Rahmen der Erfindung für Fluor, Chlor, Brom und Jod.

Aromatische, gesättigte und ungesättigte Heterocyclen stehen im Rahmen der Erfindung in Abhängigkeit von den oben aufgeführten Substituenten im allgemeinen für einen 5- bis 7-gliedrigen oder 5- bis 6-gliedrigen, vorzugsweise 5- bis 6-gliedrigen Heterocyclus, der bis zu 3 Heteroatome aus der Reihe S, N und/oder O enthalten und der gegebenenfalls auch über ein Stickstoffatom gebunden sein kann. Beispielsweise seien genannt: Pyridyl, Thienyl, Furyl, Pyrrolyl, Pyrrolidinyl, Piperazinyl, Pyrimidyl, Thiazolyl, Oxazolyl, Imidazolyl, Morpholinyl oder Piperidyl. Bevorzugt sind Pyridyl, Furyl, Morpholinyl, Piperidyl und Piperazinyl.

Abgangsgruppen im Sinne der Erfindung sind Gruppen, die in einer nukleophilen Substitution durch ein Nukleophil ersetzt werden können (Streitwieser, A., Jr.; Heathcock, C.H. Organische Chemie, Verlag Chemie, 1980, S. 169ff.). Bevorzugte Abgangsgruppen sind Halogenide und Sulfonsäureester/-anhydride. Eine besonders bevorzugte Abgangsgruppe ist Chlorid.

(C₃-C₆)-Keton steht im Rahmen der Erfindung für ein gesättigtes oder ungesättigtes Keton mit 3 bis 6 Kohlenstoffatomen. Beispielsweise seien genannt: Aceton, Butanon, But-1-en-3-on, But-1-in-3-on, Pentan-3-on, Pentan-2-on, Pent-1-en-3-on, Pent-1-in-3-on, Penta-1,4-dien-3-on, 3-Methylbutan-2-on, Cyclopropylmethylketon, Cyclopentanon, Hexan-2-on, Hexan-3-on, Cyclohexanon, 2-Methylcyclopentanon, 2-Ethylcyclobutanon.

(C₁-C₆)-Aldehyd steht im Rahmen der Erfindung für einen gesättigten oder ungesättigten Aldehyd mit 1 bis 6 Kohlenstoffatomen. Beispielsweise seien genannt: Formaldehyd, Acetaldehyd, Propionaldehyd, Butyraldehyd, Isobutyraldehyd, Cyclopropylcarbaldehyd, But-2-enal, But-2-inal, Pentanal, Isopentanal, Pivaldehyd, Cyclobutylcarbaldehyd, 2-Methylcyclopropylcarbaldehyd, Pent-2-enal, Pent-4-enal, Hexanal, 2-Cyclobutylacetaldehyd.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Diese Mischungen der Enantiomeren und Diastereomeren lassen sich in bekannter Weise in die stereoisomer einheitlichen Bestandteile trennen.

Außerdem wurden Verfahren zur Herstellung der erfindungsgemäßen Verbindungen der allgemeinen Formel (I) gefunden, dadurch gekennzeichnet, daß man
[A] Verbindungen der allgemeinen Formel (II)

   R¹-A-D-E-G-M-H (II)

   in welcher
   - R¹, A, D, E und G: oben angegebene Bedeutung haben und
   - M: für Sauerstoff oder -N(R³²)- steht und
   R³² Wasserstoff oder (C₁-C₄)-Alkyl ist,
   mit Verbindungen der allgemeinen Formel (III)

   R³³-Q-R² (III)

   in welcher
   - R²: die oben angegebene Bedeutung hat,
   - R³³: für Halogen, vorzugsweise Chlor oder Iod steht,
   - Q: für einen Rest der Formel -SO₂-, -SO-, -CO- , -P(O)(OR²⁷)- oder eine Einfachbindung steht,
   worin
   R²⁷ die oben angegebene Bedeutung hat,
   zu Verbindungen der allgemeinen Formel (Ia)

   R¹-A-D-E-G-M-Q-R² (Ia)

   in welcher
   - R¹, A, D, E, G, M, Q und R²: die oben angegebene Bedeutung haben,
   in inerten Lösemitteln, gegebenenfalls in Anwesenheit einer Base, umgesetzt werden
   oder
[B] Verbindungen der allgemeinen Formel (II)
   zunächst mit Chlorsulfonsäuretrialkylsilylester, vorzugsweise Chlorsulfonsäuretrimethylsilylester, umgesetzt werden, mit einer Säure versetzt werden und dann mit einem Chlorierungsmittel, vorzugsweise Phosphorpentachlorid, zu einer Verbindung der allgemeinen Formel (IV)

   R¹-A-D-E-G-M-SO₂-Cl (IV)

   in welcher
   - R¹, A, D, E, G und M: die oben angegebene Bedeutung haben,
   umgesetzt werden und anschließend mit Verbindungen der allgemeinen Formel (V)

   H-T-R² (V)

   in welcher
   - R²: die oben angegebene Bedeutung hat und
   - T: für Sauerstoff oder Stickstoff steht,
   zu Verbindungen der allgemeinen Formel (Ib)

   R¹-A-D-E-G-M-SO₂-T-R² (Ib)

   in welcher
   - R¹, A, D, E, G, M, T und R²: die oben angegebene Bedeutung haben,
   in inerten Lösemitteln in Anwesenheit von Bzl-NEt₃⁺Cl⁻ und einer Base, umgesetzt werden
   oder
[C] Verbindungen der allgemeinen Formel (VI)

   R¹-A-D'-H (VI)

   in welcher
   - R¹ und A: die oben angegebene Bedeutung haben und
   - D': für Sauerstoff, Schwefel oder -N(R⁹)- steht und
   R⁹ die oben angegebene Bedeutung hat,
   mit Verbindungen der allgemeinen Formel (VII)

   R³⁴-E-G-SO₂-NH-R² (VII)

   in welcher
   - E, G und R²: die oben angegebene Bedeutung haben und
   - R³⁴: für eine Abgangsgruppe, bevorzugt Halogen, besonders bevorzugt Fluor, Chlor oder Brom steht,
   zu Verbindungen der allgemeinen Formel (Ic)

   R¹-A-D'-E-G-SO₂-NH-R² (Ic)

   in welcher
   - R¹, A, D', E, G und R²: die oben angegebene Bedeutung haben,
   umgesetzt werden,
   oder
[D] Verbindungen der allgemeinen Formel (Id)

   R³⁷-A-D-E-G-L-R² (Id)

   in welcher
   - A, D, E, G, L und R²: die oben angegebene Bedeutung haben und
   - R³⁷: für einen Rest der Formel 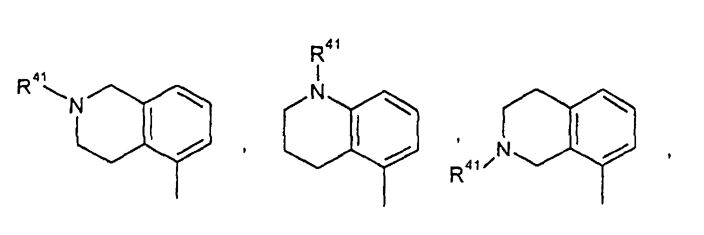 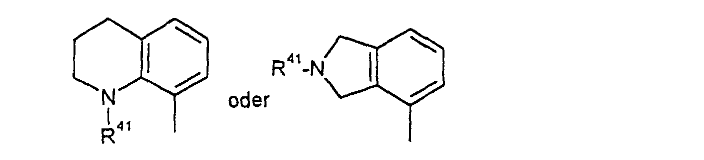 steht,
   worin
   R⁴¹ für (C₁-C₆)-Alkyl steht,
   mit Chlorameisensäureester, vorzugsweise Chlorameisensäure-1-(1-chlor)ethylester oder Chlorameisensäuremethylester, und anschließend mit Alkoholen, bevorzugt Methanol, gegebenenfalls in Anwesenheit einer Base, zu Verbindungen der allgemeinen Formel (Ie)

   R³⁸-A-D-E-G-L-R² (Ie)

   in welcher
   - A, D, E, G, L und R²: die oben angegebene Bedeutung haben und
   - R³⁸: für einen Rest der Formel 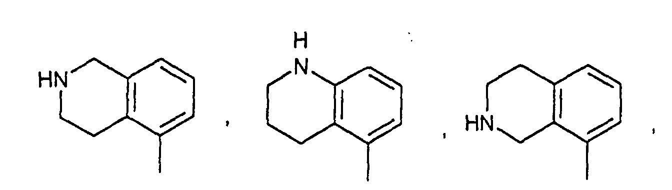 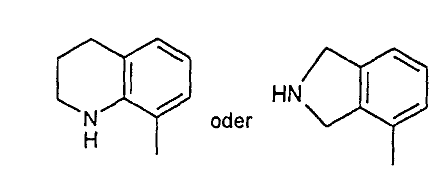 steht,
   umgesetzt werden
   oder
[E] Verbindungen der allgemeinen Formel (Ie)
   mit (C₁-C₆)-Ketonen oder (C₁-C₆)-Aldehyden in Gegenwart eines Reduktionsmittels, vorzugsweise Natriumcyanoborhydrid, gegebenenfalls in der Gegenwart einer Säure zu Verbindungen der allgemeinen Formel (If)

   R³⁹-A-D-E-G-L-R² (If)

   in welcher
   - A, D, E, G, L und R²: die oben angegebene Bedeutung haben und
   - R³⁹: für (C₃-C₆)-Alkenyl oder (C₁-C₆)-Alkyl steht,
   umgesetzt werden
   oder
[F] Verbindungen der allgemeinen Formel (Ie) mit Verbindungen der allgemeinen Formel (VIII)

   R³⁵-R³ (VIII)

   in welcher
   - R³: die oben genannte Bedeutung hat,
   - R³⁵: für eine Abgangsgruppe, bevorzugt Halogen steht,
   in inerten Lösemitteln, gegebenenfalls in Anwesenheit einer Base, zu Verbindungen der allgemeinen Formel (Ig)

   R⁴⁰-A-D-E-G-L-R² (Ig)

   in welcher
   - A, D, E, G, L und R²: die oben angegebene Bedeutung haben und
   - R⁴⁰: für einen Rest der Formel 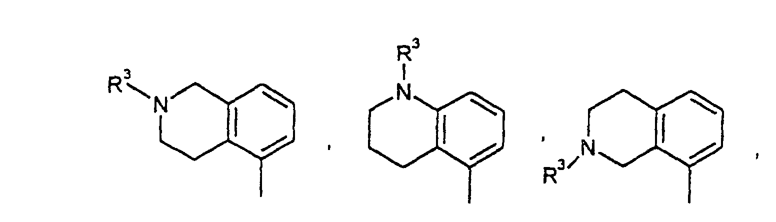 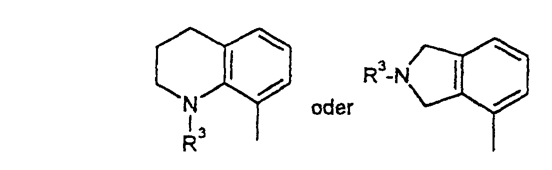 steht,
   worin
   - R³: die oben angegebene Bedeutung hat,
   umgesetzt werden,
   oder
[G] Verbindungen der allgemeinen Formel (Ih) 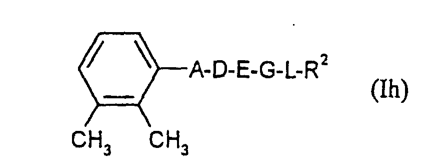 in welcher
   - A,D,E,G,L und R²: die oben angegebene Bedeutung haben,
   durch radikalische Bromierung, beispielsweise mit N-Bromsuccinimid, in einem inerten Lösungsmittel in Verbindungen der allgemeinen Formel (Ii) 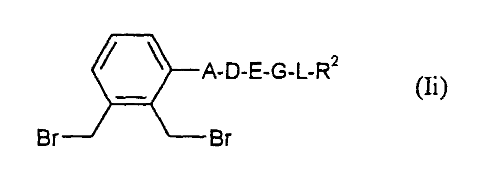 in welcher
   - A, D, E, G, L und R²: die oben angegebene Bedeutung haben,
   übergeführt werden,
   und anschließend mit Verbindungen der allgemeinen Formeln (IX) oder (X)

   CH₂(CO₂R⁴²)₂ (IX)

   H₂N-R³ (X)

   in welchen
   - R⁴²: für (C₁-C₆)-Alkyl steht und
   - R³: die oben angegebene Bedeutung hat,
   in inerten Lösemitteln, gegebenenfalls in Anwesenheit einer Base, zu Verbindungen der allgemeinen Formel (Ij)

   R⁴³-A-D-E-G-L-R² (Ij)

   in welcher
   - A, D, E, G, L und R²: die oben genannte Bedeutung haben und
   - R⁴³: für 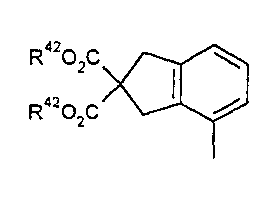 oder 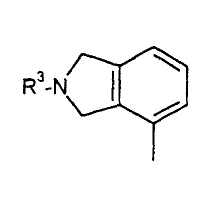 steht,
   worin
   R⁴² und R³ die oben genannte Bedeutung haben,
umgesetzt werden,
und gegebenenfalls die oben aufgeführten Substituenten nach üblichen Methoden eingeführt und derivatisiert werden,
und im Fall D ist = -SO- oder -SO₂- ausgehend von den entsprechenden Thioethern (D = S) eine Oxidation nach üblichen Methoden durchgeführt wird,
und im Fall der Ammoniumverbindungen ausgehend von den entsprechenden Aminen eine Alkylierung durchgeführt wird.

Die erfindungsgemäßen Verfahren können durch folgende Formelschemata beispielhaft erläutert werden:

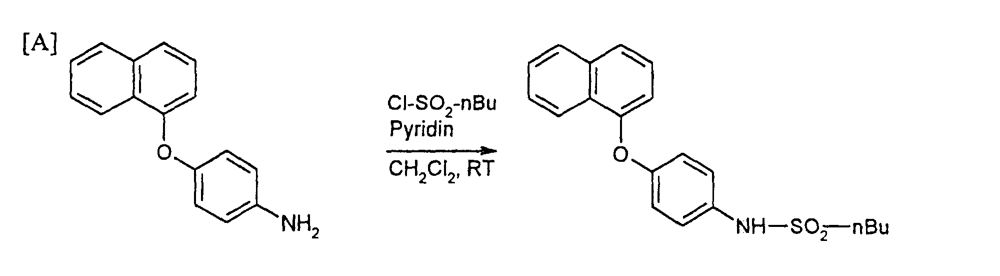

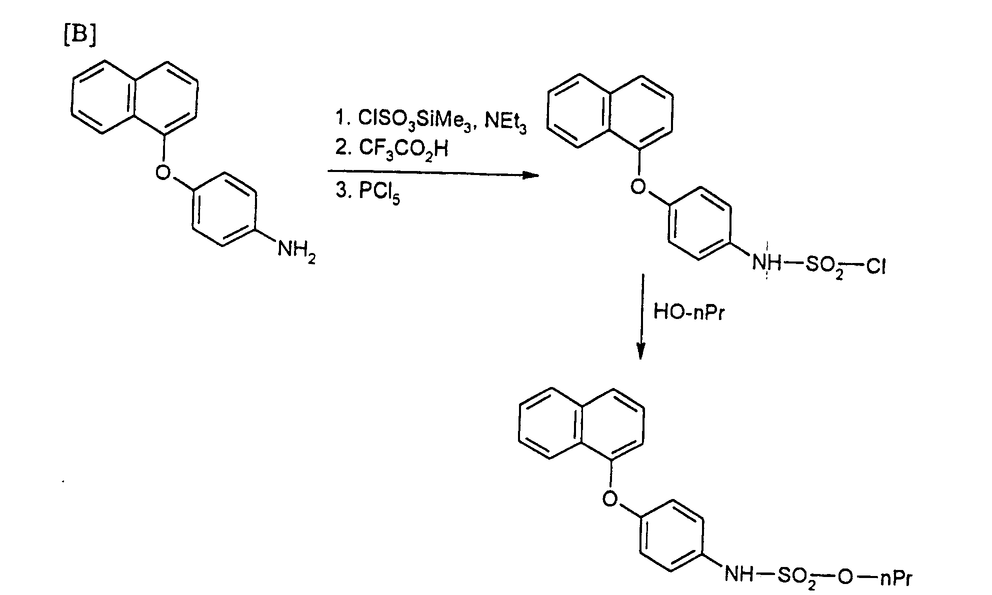

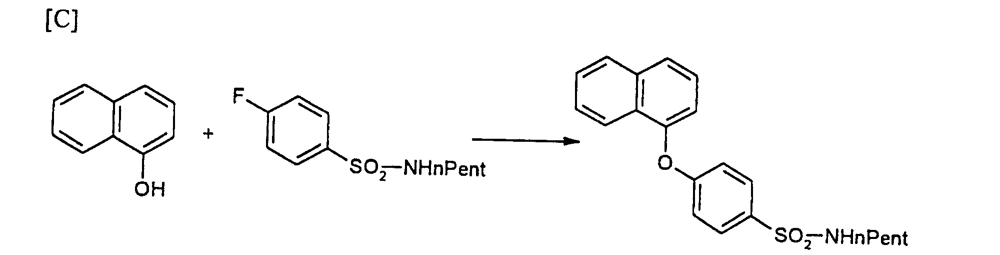

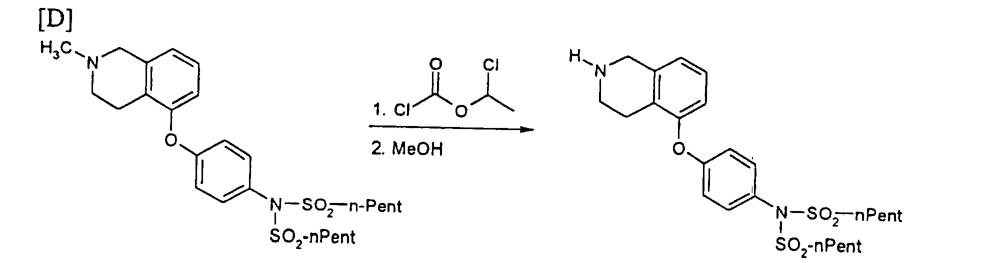

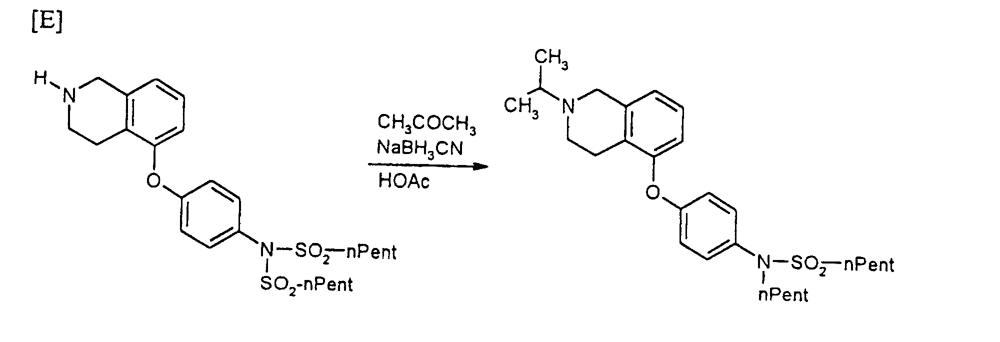

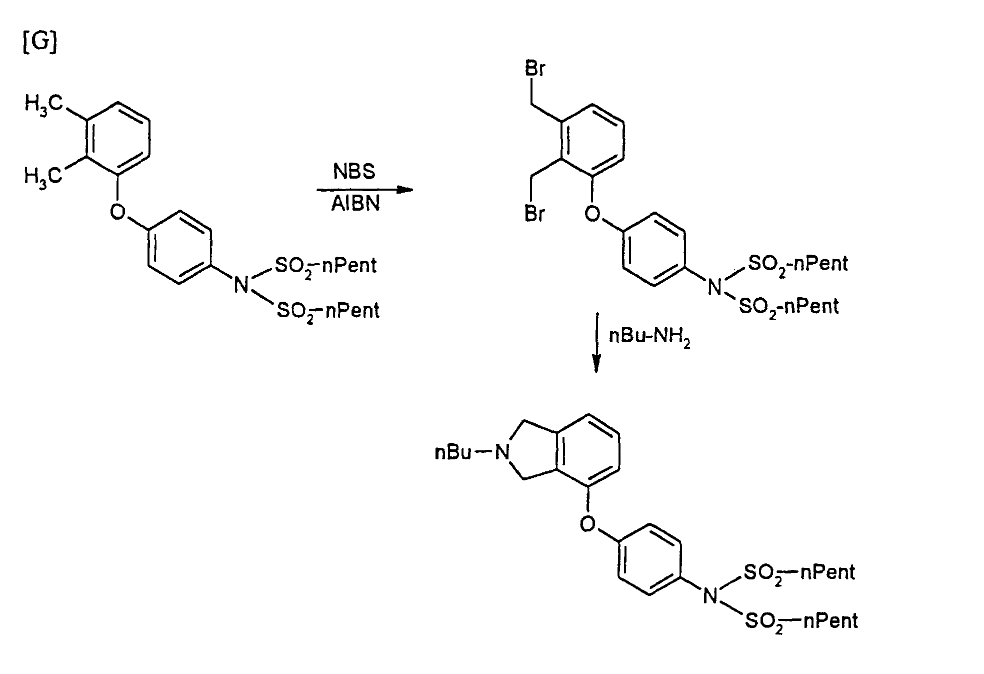

Als Lösemittel eignen sich Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Basen eignen sich im allgemeine Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Triethylamin, Natriumhydrid, Pyridin und/oder Dimethylaminopyridin.

Als Basen eignen sich außerdem üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natriumoder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt sind Kaliumcarbonat und Natriumhydroxid.

In einer Variante wird die Umsetzung in Pyridin, dem eine katalytische Menge DMAP zugesetzt wird, durchgeführt. Gegebenenfalls kann noch Toluol zugefügt werden.

Die Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Die vorliegende Erfindung betrifft außerdem Verbindungen der allgemeinen Formel (II)

R¹-A-D-E-G-M-H (II)

in welcher
- R¹: für einen Rest der Formel 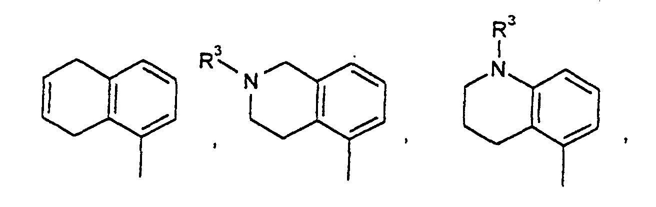 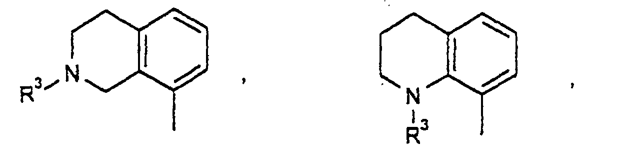 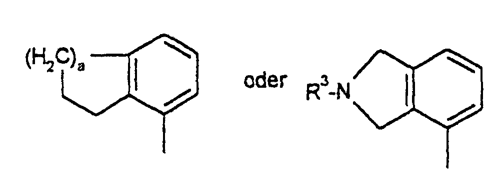 steht,
worin
a eine Zahl 1 oder 2 bedeutet,
R³ Wasserstoff, (C₂-C₆)-Alkenyl, (C₁-C₆)-Alkyl oder (C₁-C₆)-Acyl bedeutet, und wobei alle oben aufgeführten Ringsysteme und Reste gegebenenfalls
wie oben angegeben substituiert sind, und
A, D, E, G und M die oben angegebenen Bedeutungen haben.

Bevorzugt sind Verbindungen der allgemeinen Formel (II),
in welcher
- R¹: für Indan-4-yl, substituiert durch Hydroxy(C₁-C₆)-Alkyl, oder
für einen Rest der Formel 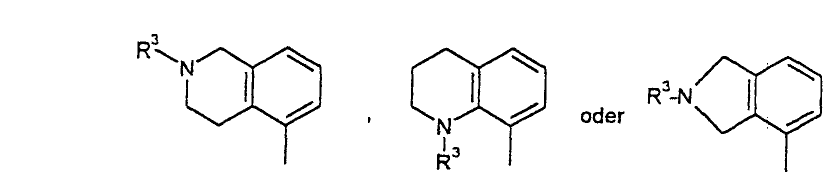 steht,
worin
R³ (C₁-C₆)-Alkyl ist,
- E und A: für eine Bindung stehen,
- D: für ein Sauerstoffatom steht,
- G: für 1,3-Phenylen, 1,4-Phenylen oder 2,5-Pyridylen steht, die gegebenenfalls durch Halogen substituiert sind,
- L: für einen Rest der Formel -NH-SO₂-, oder -O-SO₂- steht,
- R²: für (C₁-C₆)-Alkyl steht, das gegebenenfalls durch Chlor, Trifluormethyl, durcheinen Rest der Formel -O-CH₂-CF₃ oder durch Phenyl oder durch Pyridyl substituiert ist, die ihrerseits durch Brom oder Chlor substituiert sein können, und
- M: für Sauerstoff oder -N(R³²)- steht,
worin R³² Wasserstoff oder (C₁-C₄)-Alkyl ist.

Die Verbindungen der allgemeinen Formel (II) können beispielsweise hergestellt werden, indem man
[A] Verbindungen der allgemeinen Formel (VI)

   R¹-A-D'-H (VI)

   in welcher
   - R¹, A und D': die oben angegebene Bedeutung haben,
   mit Verbindungen der allgemeinen Formel (XI)

   R⁴⁴-E-G-NO₂ (XI)

   in welcher
   - E und G: die oben angegebene Bedeutung haben und
   - R⁴⁴: eine Abgangsgruppe, bevorzugt Halogen, ist,
   in inerten Lösemitteln, gegebenenfalls in Anwesenheit einer Base, umgesetzt werden und anschließend mit üblichen Reduktionsmitteln, vorzugsweise H₂/Pd/C in einem inerten Lösemittel oder mit Hydrazinhydrat, Pd/C, gegebenenfalls unter gleichzeitiger Hydrogenierung von (C-C)-Mehrfachbindungen, zu Verbindungen der allgemeinen Formel (IIa)

   R¹-A-D'-E-G-NH₂ (IIa)

   in welcher
   - R¹, A, D', E und G: die oben angegebene Bedeutung haben, umgesetzt werden,
   oder
[B] Verbindungen der allgemeinen Formel (IIb)

   R¹-A-D-E-G-NH₂ (IIb)

   in welcher
   R¹, A, D, E und G die oben angegebene Bedeutung haben, mit einem Nitrosierungsmittel, bevorzugt einer wäßrigen Lösung von Schwefelsäure und Natriumnitrit und anschließender Erwärmung, bevorzugt auf 60 bis 100°C, zu Verbindungen der allgemeinen Formel (IIc)

   R¹-A-D-E-G-OH (IIc)

   in welcher
   - R¹, A, D, E und G: die oben genannte Bedeutung haben,
   umgesetzt werden,
   oder
[C] Verbindungen der allgemeinen Formel (XII)

   R¹-R³⁶ (XII)

   in welcher
   - R¹: die oben angegebene Bedeutung hat und
   - R³⁶: für eine Abgangsgruppe, bevorzugt Halogen, besonders bevorzugt Brom, steht,
   mit Verbindungen der allgemeinen Formel (XIII)

   HO-G-O-R⁴⁵ (XIII)

   in welcher
   - G: die oben angegebene Bedeutung hat und
   - R⁴⁵: für (C₁-C₆)-Alkyl, bevorzugt Methyl, steht,
   in einem inerten Lösungsmittel, bevorzugt Dimethylformamid oder Pyridin, gegebenenfalls in Anwesenheit einer Base, bevorzugt Kaliumcarbonat, und gegebenenfalls in Anwesenheit von Kupfer (I/II)-Salzen, bevorzugt Kupfer (II)-Oxid oder Kupfer (I)-Iodid, in einem Temperaturbereich von 0°C bis 200°C, bevorzugt 80 bis 150°C und Normaldruck zu Verbindungen der allgemeinen Formel (Ik)

   R¹-O-G-O-R⁴⁵ (Ik)

   in welcher
   - R¹, G und R⁴⁵: die oben genannte Bedeutung haben,
   umgesetzt werden und anschließend in Gegenwart einer Säure, bevorzugt Bromwasserstoffsäure, zu Verbindungen der allgemeinen Formel (IId)

   R¹-O-G-OH (IId)

   reagiert werden
   oder
[D] Verbindungen der allgemeinen Formel (VI)

   R¹-A-D'-H (VI)
in welcher
- R¹, A und D': die oben angegebene Bedeutung haben,
mit Verbindungen der allgemeinen Formel (XIV)

R⁴⁶-E-G'-R⁴⁷ (XIV)

in welcher
- R⁴⁶: die für R³⁶ angegebene Bedeutung hat und mit dieser gleich oder verschieden ist,
- E: die oben genannte Bedeutung hat,
- G': für einen zweifach gebundenen 5- bis 7-gliedrigen aromatischen Heterocyclus mit bis zu 3 Heteroatomen aus der Reihe Schwefel, Stickstoff und/oder Sauerstoff steht, der gegebenenfalls mit einem oder mehreren, gleichen, oder verschiedenen Substituenten wie im Anspruch 1 für G definiert substituiert sein kann und
- R⁴⁷: für Halogen, bevorzugt für Chlor oder Brom, steht,
zu einer Verbindung der allgemeinen Formel (XV)

R¹-A-D'-E-G'-R⁴⁷ (XV)

in welcher
- R¹, A, D', E, G' und R⁴⁷: die oben genannte Bedeutung haben,
in inerten Lösungsmitteln, gegebenenfalls in Anwesenheit einer Base, umgesetzt werden und anschließend mit Kaliumamid in flüssigem Ammoniak in die entsprechenden freien Amine der allgemeinen Formel (IIe)

R¹-A-D'-E-G'-NH₂ (IIe)

in welcher
- R¹, A, D', E und G': die oben genannte Bedeutung haben,
transformiert wird.

In DOS 1 942 264 wird die Herstellung von fluorierten Alkansulfonsäurechloriden beschrieben, in US 5 149 357 u.a. die Herstellung eines 4,4,4-Trifluorbutansulfonsäureamids, ohne jedoch die Herstellung des entsprechenden Sulfonsäurechlorids zu offenbaren.

Die fluorierten Sulfonsäurechloride wurden analog DOS 1 942 264 hergestellt.

Außerdem betrifft die vorliegende Erfindung Verbindungen der Formeln

Cl -SO₂-CH₂-CH₂-CH₂-CF₃

oder

Cl-SO₂CH₂-CH₂-CH₂-CF₂-CF₃.

Als Lösemittel eignen sich Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlörethylen oder Chlorbenzol, oder Essigester, oder Tristhylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Basen eignen sich im allgemeine Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Natriumhydrid, Pyridin und/oder Dimethylaminopyridin.

Als Basen eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium oder Kaliumcarbonat oder Natriumhydrogencarbonat, oder Alkalialkoholate wie Natriummethanolat, Natriumethanolat, Kaliummethanolati Kaliumethanolat oder Kalium-tert.butanolat. Besonders bevorzugt sind Kaliumcarbonat und Natriumhydroxid.

Die Basen werden in einer Menge von 1 - 20 Äquivalenten, bevorzugt von 2 bis 10 Äquivalenten, jeweils bezogen auf 1 Äquivalent der Verbindungen der allgemeinen Formeln (X) und (XII) eingesetzt.

Die Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Die Verfahren werden im allgemeinen in einem Temperaurbereich von 0°C bis 200°C, bevorzugt von Raumtemperatur bis 140°C durchgeführt.

Die Verbindungen der allgemeinen Formeln (III), (V), (VIII), (IX), (X) und (XII) sind an sich bekannt oder nach üblichen Methoden herstellbar.

Die Alkylierung zur Herstellung der Ammoniumverbindungen erfolgt im allgemeinen mit Alkylierungsmitteln wie beispielsweise Alkylhalogenide, Sulfonsäureester oder substituierte oder unsubstituierte Dialkyl- oder Diarylsulfate, vorzugsweise mit Methyljodid oder Dimethylsulfat.

Die Alkylierung erfolgt im allgemeinen in einem der oben aufgeführten Lösemitteln, vorzugweise in Dimethylformamid in einem Temperaturbereich von 0°C bis +70°C, vorzugsweise von 0°C bis +30°C und Normaldruck.

Überraschenderweise zeigen die neuen Arylsulfonamide und ihre Analoga ein nicht vorhersehbares, wertvolles pharmakologisches Wirkspektrum.

Sie zeichnen sich als hochwirksame Agonisten des CB1-Rezeptors und teilweise des CB2-Rezeptors aus. Sie können allein oder in Kombination mit anderen Arzneimitteln eingesetzt werden zur Behandlung und/oder Prävention von neuronalen Schädigungen unterschiedlicher Ursache wie beispielsweise durch ischämischen, thromb- und/oder thrombembolischen, und hämorrhagischen Schlaganfall, Zuständen nach direkten und indirekten Verletzungen im Bereich des Gehirnes und des Schädels, ferner zur Behandlung und/oder Prävention von cerebralen Ischämien nach sämtlichen operativen Eingriffen am Gehirn oder peripheren Organen bzw. Körperteilen und damit einhergehenden oder vorausgehenden Zuständen krankhafter bzw. allergischer Natur, die primär und/oder sekundär zu einer neuronalen Schädigung führen können. Gleichfalls eignen sich die erfindungsgemäßen Verbindungen auch zur Therapie von primären und/oder sekundären krankhaften Zuständen des Gehirnes, beispielsweise während oder nach cerebralen Vasospasmen, Hypoxie und/oder Anoxie nicht vorher genannter Genese, perinataler Asphyxie, Autoimmunerkrankungen, Stoffwechsel- und Organerkrankungen, die mit einer Schädigung des Gehirnes einhergehen können sowie Schädigungen des Gehirnes infolge primärer Gehirnerkrankungen beispielsweise Krampfleiden und athero- und/oder arteriosklerotischer Veränderungen, zur Behandlung chronischer oder psychiatrischer Leiden wie beispielsweise Depression, neurodegenerativer Erkrankungen wie beispielsweise Alzheimersche, Parkinsonsche oder Huntingtonsche Erkrankung, Multiple Sklerose, amyotrophische laterale Sklerose, Neurodegeneration durch akute und/oder chronische virale oder bakterielle Infektionen und Multiinfarktdemenz.

Darüber hinaus können sie in Arzneimitteln eingesetzt werden zur Behandlung von Schmerzzuständen, Emesis, Übelkeit, Glaukom, Asthma, Anorexie, Konvulsionen, Rheuma, Sedation und Bewegungsstörungen.

Die erfindungsgemäßen Substanzen eignen sich auch zur Behandlung von Erkrankungen, die durch bakterielle und/oder virale Infektion verursacht werden, die auf direkte und/oder indirekte Veränderungen des Immunsystems bzw. auf Fehlsteuerungen unter Mitwirkung des Immunsystems beruhen, wie z.B. bei lokalen oder systemischen Autoimmunerkrankungen (z.B. Lupus erythematodes in allen seinen Varianten), entzündlichen und/oder autoimmunologisch bedingten Erkrankungen der Gelenke (z.B. primär chronische Polyarthritis, traumatisch bedingten Entzündungen), entzündlichen und/oder autoimmunologisch bedingten Erkrankungen des Knochen- und Muskelapparates, entzündlichen und/oder autoimmunologisch bedingten krankhaften Prozessen der inneren Organe (z.B. Morbus Crohn, Glomerulonephritis) und der äußeren Organe (z.B. allergische Reaktionen durch aerogene Aufnahme von Antigenen) und des zentralen Nervensystems (z.B. Multiple Sklerose, Morbus Alzheimer, psychiatrische Erkrankungen) sowie der Sinnesorgane, primären und/oder sekundären und/oder autoimmunologischen Erkrankungen des blutbildenden Systems und des Immunsystems (z.B. Abstoßungsreaktionen, AIDS) selbst, sowie bei Hauterkrankungen entzündlicher und/oder immunologischer Genese bei Mensch und Tier. Ferner wirken diese Substanzen bei den indirekten Symptomen dieser Erkrankungen wie z.B. Schmerz.

Bevorzugt ist ihre Verwendung zur Behandlung von cerebralen Ischämien und Schädel/Hirn-Trauma.

### CB1-Luciferase Reportergen Test

### 1. Klonierung des Ratten Cannabinoid Rezeptors CB1

Gesamt-RNA aus Ratten-Hirn (das Gewebe wurde frisch getöteten Tieren entnommen und in flüssigem Stickstoff schockgefroren) wurde durch saure Guanidinium-Thiocyanat/Phenol/Chloroform-Extraktion (J. Biol. Chem. 1979, 18, 5294) isoliert und mittels reverser Transkriptase und Random-Primern (jeweils von Invitrogen) in cDNA überführt. Die Polymerase Ketten Reaktion (PCR, Bedingungen: 4 min 94°C, 1x; 1 min 94°C; 2 min 53°C; 1 min 72°C, 50 Zyklen; 1 min 94°C, 2 min 53°C, 4 min 72°C, 1x) wurde in einem Perkin Elmer Thermocycler mit dem Enzym Taq Polymerase (Perkin Elmer) durchgeführt; die eingesetzten Oligonukleotid-Primer (Basen 99 bis 122: 5'→3', "down"; 1556-1575: 3'←5', "up") waren von der publizierten Sequenz des Ratten Cannabinoid- Rezeptors (Nature 1990, 346, 561) abgeleitet und wurden auf einem DNA Synthesizer, Modell 1380 der Fa. Applied Biosystems, synthetisiert. Ein Teil der PCR-Reaktion wurde in einem 1 %igen Agarose-Gel in 1x TBE-Puffer aufgetrennt und anschließend mit Ethidium-Bromid angefärbt, wobei nur eine Bande mit der erwarteten Länge sichtbar war (etwa 1,5 kb). Dieses PCR-Produkt wurde in den TA-Cloning Vektor (Invitrogen) subkloniert und die Nukleotid-Sequenz des Inserts mit T7DNA Polymerase (Sequenase, USA/Amersham) durch die Dideoxynukleotid-Kettenabbruch-Reaktion bestimmt. Das Insert besitzt eine Länge von 1477 Basenpaaren und enthält ein offenes Leseraster von 1419 Basenpaaren was einem Protein von 473 Aminosäuren entspricht. Die Anzahl der Basenpaare, die Position des offenen Leserasters und die Anzahl der Aminosäuren stimmen mit der publizierten Sequenz überein. Computer-Analysen wurden mit Hilfe der GCG Software Suite (Genetic Computer Group) durchgeführt. Das cDNA Insert wurde nach Partialverdauung mit HindIII und NotI (Biolabs) in den Expressionsvektor pRc/CMV (Invitrogen) subkloniert. Dieses Konstrukt (Plasmid CMV-RH) wurde für Transfektions-Experimente eingesetzt.

### 2. Stabile Transfektion der CHOluc9 Reporter Zellen

CHOluc9 Zellen wurden in 50 % Dulbecco's modifiziertem Eagle Medium / 50 % F-12 (DMEM/F12) gezüchtet, das 10 % foetales Kälberserum (FCS) enthielt. Transfektionen wurden in 6-well Platten angesetzt. 7,5 µg Qiagen-gereinigte CMV-RH Plasmid DNA wurde pro 105 Zellen mit dem DOTAP Transfektions System zugegeben, entsprechend dem Versuchsprotokoll des Herstellers (Boehringer Mannheim). Transfizierte Zellen wurden mit 1 mg/ml G418 selektioniert und Einzelklone wurden durch Limiting Dilution auf 96-well Platten erhalten. Zelllinien, die den Cannabinoid-Rezeptor exprimieren, wurden nach Inkubation mit dem Cannabinoid-Rezeptor Agonisten, WIN-55,212-2, in Gegenwart von Forskolin an der Hemmung der Reportergen-Expression identifiziert. Mehrere stabil transfizierte und subklonierte Zellinien wurden mittels RT-PCR, wie unter 1. beschrieben, weiter charakterisiert.

### 3. Test-Optimierung und pharmakologische Charakterisierung der CHOCB1 Reporter-Zellinie

Der Luciferase-Test wurde mit dem Ziel hoher Sensitivität und Reproduzierbarkeit, geringer Varianz und guter Eignung für die Durchführung auf dem Robotersystem optimiert durch Variation mehrerer Testparameter, wie z.B. Zelldichte, Dauer der Anzuchtphase und der Testinkubation, Forskolin-Konzentration, Medium-Zusammensetzung. Zur pharmakologischen Charakterisierung der Zellen und zum Roboter-gestützten Substanz-Screening wurde das folgende Testprotokoll verwendet: Die Stammkulturen wurden in 50 % Dulbecco's modifiziertem Eagle Medium /50 % F-12 (DMEM/F12) mit 10 % FCS bei 37°C unter 10 % CO₂ gezüchtet und jeweils nach 2 bis 3 Tagen 1:10 gesplittet. Testkulturen wurden mit 5000 Zellen pro Napf in 96-well Platten ausgesät und 70 Stunden bei 37°C angezogen. Dann wurden die Kulturen vorsichtig mit Phosphat-gepufferter Saline gewaschen und mit serumfreiem Ultra-CHO Medium (Bio-Whittaker) rekonstituiert Die in DMSO gelösten Substanzen wurden 1 x in Medium verdünnt und zu den Testkulturen pipettiert (maximale DMSO-Endkonzentration im Testansatz: 0,5 %). 20 Minuten später wurde Forskolin zugegeben und die Kulturen anschließend 3 Stunden im Brutschrank bei 37°C inkubiert. Danach wurden die Überstände entfernt und die Zellen durch Zugabe von 25 µl Lysereagens (25 mM Triphosphat, pH 7,8 mit 2mM DTT, 10 % Glycerin, 3 % TritonX100) lysiert. Direkt danach wurde Luciferase Substrat Lösung (2,5mM ATP, 0,5 mM Luciferin, 0,1 mM Coenzym A, 10mM Tricin, 1,35mM MgSO₄, 15mM DTT, pH 7,8) zugegeben, kurz geschüttelt, und die Luciferase-Aktivität mit einem Hamamatzu Kamerasystem gemessen.

Zur Inaktivierung von Gᵢ-Proteinen wurden die Testkulturen vor dem Test für 16 Stunden mit 5 ng/ml (Endkonz.) Pertussis Toxin behandelt.

Die IC₅₀-Werte wurden mit dem Programm GraphPadPrism berechnet (Hill-Gleichung, speziell: one-site competition).

| Aktivität im Ratten CB1-Rezeptor-Luciferase Rezeptorgen Test | |
|---|---|
| **Beispiel** | **IC**_{**50**} **(nmol/l)** |
| 1 | 15 |
| 33 | 10 |
| 51 | 0,9 |
| 65 | 13 |
| 99 | 2,9 |

### hCB2-Luciferase Reportergen Test

CHOluc9 Zellen wurden mit dem humanen CB2-Rezeptor stabil transfiziert. Transfektion, Klonselektion und Testentwicklung wurden analog zu den Arbeiten mit dem Ratten CBI-Rezeptor durchgeführt. Das folgende Testprotokoll wurde zur pharmakologischen Charakterisierung der Zellen und zur Substanz-Testung verwendet:

Die Stammkulturen wurden in 50% Dulbecco's modifizierten Eagle Medium/50% F-12 (DMEM/F12) mit 10% FCS bei 37°C unter 10% CO₂ gezüchtet und jeweils nach 2 bis 3 Tagen 1:10 gesplittet. Testkulturen wurden mit 5000 Zellen pro Napf in 96-well-Platten in DMEM/F12 Medium mit 5 % FCS ausgesät und 70 Stunden bei 37°C angezogen. Dann wurde das Medium von den Kulturen entfernt und durch serumfreies Ultra-CHO Medium (Bio-Whittaker) ersetzt. Die in DMSO gelösten Substanzen (200x Endkonzentration) wurden zu den Testkulturen pipettiert (maximale DMSO-Endkonz. im Testansatz: 0,5%) und 20 min später wurde Forskolin zugegeben. Anschließend wurden die Kulturen 3,5 Stunden im Brutschrank bei 37°C inkubiert. Danach wurden die Überstände entfernt und die Zellen durch Zugabe von 25 µl Lysereagens (25 mM Trisphosphat, pH 7,8 mit 2 mM DTT, 10 % Glycerin, 3 % Triton X100) lysiert. Direkt anschließend wurden 50 µl Luciferase Substrat Lösung, doppelt konzentriert (5 mM ATP, 1 mM Luciferin, 0,2 mM Coenzym A, 10 mM Tricin, 1,35 mM MgSO₄, 15 mM DTT, pH 7,8) zugegeben, kurz geschüttelt, und die Luciferase-Aktivität mit einem Photomultiplier-Kamera-Meßsystem (Hamamatzu) bestimmt.

Die IC₅₀-Werte wurden mit dem Program GraphPad Prism™ berechnet (Hill-Gleichung; speziell: one site competition).

### Bindungsstudien an Ratten Cortex Membranen

Membranprotein wird nach Standardmethoden aus unterschiedlichen Geweben bzw. von Zellen präpariert. Puffer, markierter Ligand, DMSO oder Teststubstanz werden zusammenpipettiert, anschließend werden 100 µg Protein hinzugegeben, die Mischung gut vermischt und 60 min bei 30°C im Wasserbad inkubiert. Nach Ablauf der Inkubationszeit wird die Reaktion durch Zugabe von eiskaltem Inkubationspuffer in jedes Röhrchen gestoppt. Nach Abfiltrieren wird mit 3/4 ml Inkubationspuffer nachgewaschen. Die Filter werden in Minivials überführt, die Radioaktivität wird in einem Flüssigszintillationszähler bestimmt.

| Affinintät zum CB1-Rezeptor (Ratten Cortex Membranen) | |
|---|---|
| **Beispiel** | **K**_{**i**} **(nmol/l)** |
| 1 | 590 |
| 33 | 420 |
| 51 | 41 |
| 65 | 250 |

### Inhibition der Glutamat-Freisetzung

Nach Dekapitieren einer Ratte wird der Schädel eröffnet, das Gehirn herausgehoben und entlang der Mittelfurche durchschnitten. Der Hippocampus wird freipräpariert, vom restlichen Gewebe getrennt, in 350 µm dicke Schnitte geschnitten und für 60 min in Siebgefäßen bei 37°C inkubiert. Gefolgt von Basalwert und Stimulation 1 mit 75 mM KCl (S1) werden die Schnitte mit Testsubstanz inkubiert und dann die Stimulation mit KCl und Testsubstanz (S2) wiederholt. Die Glutamat-Konzentration der zu untersuchenden Proben wird dann über eine enzymatische Reaktion (GLDH) und fluorometrischer Messung von NADH gemessen. Anhand einer Eichkurve wird der Glutamatgehalt der Probe bestimmt, und unter Kenntnis des Proteingehaltes kann der Glutamatgehalt/mg Protein errechnet werden. Verglichen wird das Verhältnis S2/S1, Glutamat-Freisetzungsinhibitoren reduzieren dieses Verhältnis konzentrationsabhängig.

### Hypothermie

### 1. Agonismus Prüfung:

Fünf Minuten nach Bestimmung der Basal-Körpertemperatur via Oesophagus Temperatursonde wird die Prüfsubstanz (i.v.) appliziert. Eine Kontrollgruppe erhält, ebenfalls i.v., nur das Lösungsmittel der Prüfsubstanzen. Die Körpertemperatur wird 7,5, 15, 30 und 60 Minuten nach i.v.-Applikation gemessen. Die Gruppengröße pro Dosis beträgt 5-7 Tiere (Ratten).

| Ratten Hypothermie - Agonismus Prüfung | |
|---|---|
| **Beispiel** | **ED**_{**-1°C**} ^{**a)**} **[mg/kg]** |
| 1 | 1,0 ^{b)} |
| 33 | 0,6 ^{b)} |
| 51 | 0,1 ^{b)} |
| 65 | 1,0 ^{b)} |
| 99 | 0,6 ^{b)} |

| | |
|---|---|
| a) Effektive Dosis für 1°C Körpertemperatur-Reduktion | |
| b) Die Hypothermie wird durch Applikation des spezifischen CB1-Antagonisten SR 141716 A signifikant reduziert (siehe Methode "Antagonismus Prüfung") | |

### 2. Antagonismus Prüfung:

60 Minuten vor Prüfsubstanz Applikation wird der spezifische CB1 Antagonist *SR 141716A*, der Kontrollgruppe nur das Lösemittel (Solutol/0,9% NaCl) intra-peritoneal appliziert. Die basale Körpertemperatur wird fünf Minuten vor Applikation von *SR 141716A* via Oesophagus Temperatursonde gemessen. Das weitere Vorgehen entspricht der Methode "Agonismus Prüfung". Die Gruppengröße pro Dosis beträgt 5-7 Tiere (Ratten).

### Permanente focale cerebrale Ischämie bei der Ratte (MCA-O)

Unter Isofluran Anästhesie wird die Arteria cerebri media einseitig freipräpariert mittels Elektrokoagulation diese und deren Nebenäste irreversibel verschlossen. Als Folge des Eingriffs entsteht ein cerebraler Infarkt. Während der Operation wird die Körpertemperatur des Tieres auf 37°C gehalten. Nach Wundverschluß und Abklingen der Narkose werden die Tiere wieder in ihren Käfig entlassen. Die Substanzapplikation erfolgt nach unterschiedlichen zeitlichen Schemata und über unterschiedliche Applikationswege (i.v. i.p.) nach der Okklusion. Die Infarktgröße wird nach 7 Tagen bestimmt. Dazu wird das Gehirn entnommen, histologisch aufgearbeitet und mit Hilfe eines computergestützten Auswertsystemes das Infarktvolumen bestimmt.

| Wirksamkeit in dem Modell der permanenten focalen cerebralen Ischämie (MCA-O) | | |
|---|---|---|
| **Beispiel** | **% Reduktion des Infarktvolumens** | **Dosis** |
| 1 | 35 | 0,03 mg/kg/h ^{b)} |
| 33 | 33 | 0,1 mg/kg ^{a)} |
| 51 | 24 | 0,1 mg/kg ^{a)} |
| 65 | 37 | 0,03 mg/kg/h ^{b)} |
| | (47) | (0,01 mg/kg/h) |

| | | |
|---|---|---|
| a) Substanzgabe als intravenöse Bolusinjektionen jeweils direkt, 2 und 4 Stunden nach der Okklusion | | |
| b) Substanzgabe als intravenöse, kontinuierliche Infusion direkt bis 4 Stunden nach der Okklusion | | |

### Subdurales Hämaton bei der Ratte (SDH)

Unter Anästhesie wird den Tieren einseitig subdural Eigenblut injiziert. Unter dem Hämatom bildet sich ein Infarkt. Die Substanzapplikation erfolgt nach unterschiedlichen zeitlichen Schemata und über unterschiedliche Applikationswege (i.v., i.p.).

Die Bestimmung der Infarktgröße erfolgt wie beim Modell der Permanenten focalen Ischämie bei der Ratte (MCA-O) beschrieben.

| Wirksamkeit in dem Modell "Subdurales Hämatom bei der Ratte (SDH)" | | |
|---|---|---|
| **Beispiel** | **% Reduktion des Infarktvolumens** | **Dosis** |
| 1 | 54 | 0,1 mg/kg ^{a)} |
| | (84) | (1,0 mg/kg ^{a)}) |
| 33 | 42 | 0,1 mg/kg ^{a)} |
| 51 | 54 | 0,01 mg/kg/h ^{b)} |
| 65 | 53 | 0,1 mg/kg ^{a)} |
| | (65) | (0,3 mg/kg/h ^{b)}) |

| | | |
|---|---|---|
| a) Substanzgabe als intravenöse Bolusinjektionen jeweils direkt, 2 und 4 Stunden nach der Okklusion | | |
| b) Substanzgabe als intravenöse, kontinuierliche Infusion direkt bis 4 Stunden post-Trauma | | |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 10 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Verwendete Abkürzungen

- Me =: CH₃
- Et =: C₂H₅
- nPr =: n-(CH₂)₂CH₃
- nBu =: n-(CH₂)₃CH₃
- nPent =: n-(CH₂)₄CH₃
- nHex =: n-(CH₂)₅CH₃
- nOkt =: n-(CH₂)₇CH₃
- PE =: Petrolether
- Tol =: Toluol
- EE =: Essigester
- Et₂O =: Diethylether

### Lösemittel

| | | |
|---|---|---|
| I | PE : Et₂O10:1 | |
| II | PE : Et₂O 5:1 | |
| III | PE : Dichlormethan 5:1 | |
| IV | Tol : EE10:1 | |
| V | Cyclohexan : Dichlormethan 5:1 | |
| VI | Tol : EE 5:1 | |
| VII | Tol : EE 1:1 | |
| VIII | Tol : EE 5:3 | |
| IX | PE : Dichlormethan 1:1 | |
| X | Tol : EE20:1 | |
| XI | PE : EE 5:1 | |
| XII | Tol : EE 8:1 | |
| XIII | EE : Aceton20:1 | |
| XIV | PE : EE10:1 | |
| XV | Dichlormethan : Ameisensäure40:1 | |
| XVI | Tol : EE 3:1 | |
| XVII | Dichlormethan : Et₂O10:1 | |
| XVIII | Tol : EE 1:2 | |
| XIX | EE : Aceton20:3 | |
| XX | EE : Aceton 10:1 | |
| XXI | Dichlormethan : Ameisensäure 10:1 | |
| XXII | Tol : EE : Ameisensäure10:1:0,05 | |
| XXIII | Dichlormethan : Methanol : konz.NH₃10:1:0,5 | |
| XXIV | Dichlormethan : Ethanol20:1 | |
| XXV | Dichlormethan : Methanol10:1 | |
| XXVI | Dichlormethan : Methanol5:1 | |
| XXVII | Tol : EE2:1 | |
| XXVIII | Hexan : EE4:1 | |
| XXIX | Tol : EE15:1 | |
| XXX | Toluol | |
| XXXI | Toluol : EE30:1 | |
| XXXII | Dichlormethan : Methanol19:1 | |
| XXXIII | Dichlormethan : Methanol9: | |
| XXXIV | Dichlormethan : Methanol4:1 | |
| XXXV | Essigester | |
| XXXVI | Cyclohexan : Essigester3:1 | |
| XXXVII | Cyclohexan : Essigester : Methanol 10:2:1 | |
| XXXVIII | n-Hexan : Essigester | 2:1 |
| XXXIX | Dichlormethan : Methanol | 3:1 |
| XL | Essigester : Methanol | 4:1 |
| XLI | Dichlormethan : Methanol | 95:5 |
| XLII | EE : Isooctan | 1:1 |
| XLIII | EE : Cyclohexan | 8:2 |
| XLIV | EE : Cyclohexan | 3:7 |
| XLV | Dichlormethan : Methanol : Triethylamin | 9:1:0,1 |
| XLVI | Dichlormethan : Methanol | 98 : 2 |

### Methoden der Massenspektroskopie

- A: EI
- B: DCI, NH₃
- C: ESI
- D: FAB
- E: DCI, Isobutan

### Ausgangsverbindungen

### Beispiel 1 A

### 1-(Naphthyl-1-oxy)-4-nitrobenzol

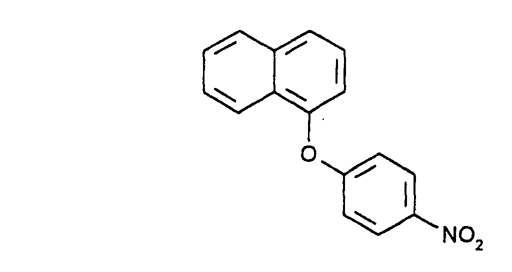

Eine Lösung von 1-Naphthol (102 g, 0,709 mol) in DMF (800 ml) wird mit K₂CO₃ (97,9 g, 0,709 mol) versetzt und 2 h bei RT gerührt. Nach tropfenweiser Zugabe einer Lösung von 4-Fluor-1-nitrobenzol (100 g, 0,709 mol) in DMF (200 ml) wird die Reaktionsmischung über Nacht bei RT gerührt. Anschließend wird das Lösemittel im Vakuum abdestilliert und der Rückstand mit Ethylacetat (600 ml) versetzt. Nach Filtration wird der größte Teil des Lösemittels im Vakuum abdestilliert. Ausgefallenes Produkt wird abgesaugt, mit wenig Ethylacetat gewaschen und i.V. getrocknet.
Ausbeute: 107 g
Durch weiteres Eindampfen der Mutterlauge werden zusätzlich noch 25 g Produkt erhalten.
Gesamtausbeute: 132 g (69% d.Th.)
Smp.: 143°C
MS (EI): m/z 265 (M)

In Analogie zu Beispiel 1A werden die in der Tabelle I dargestellten Verbindungen hergestellt:

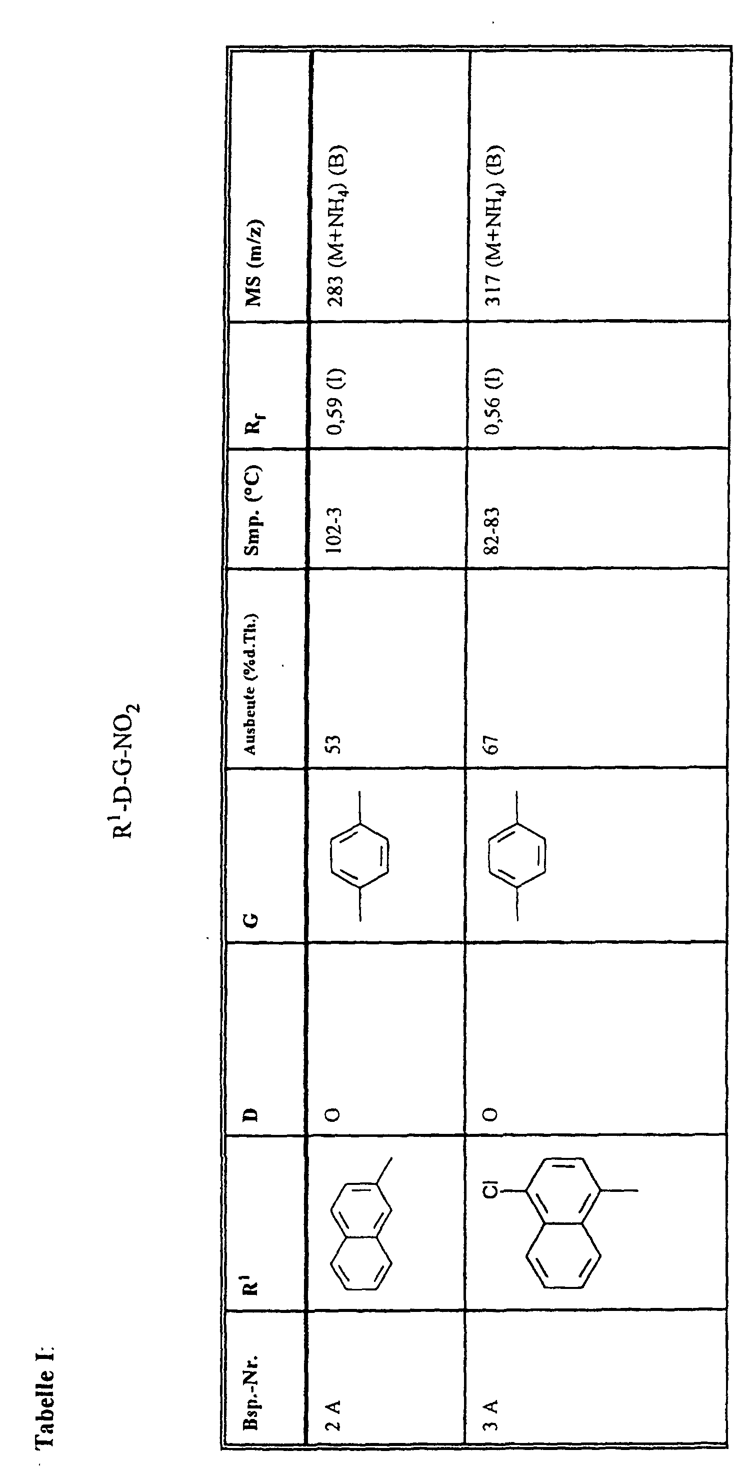

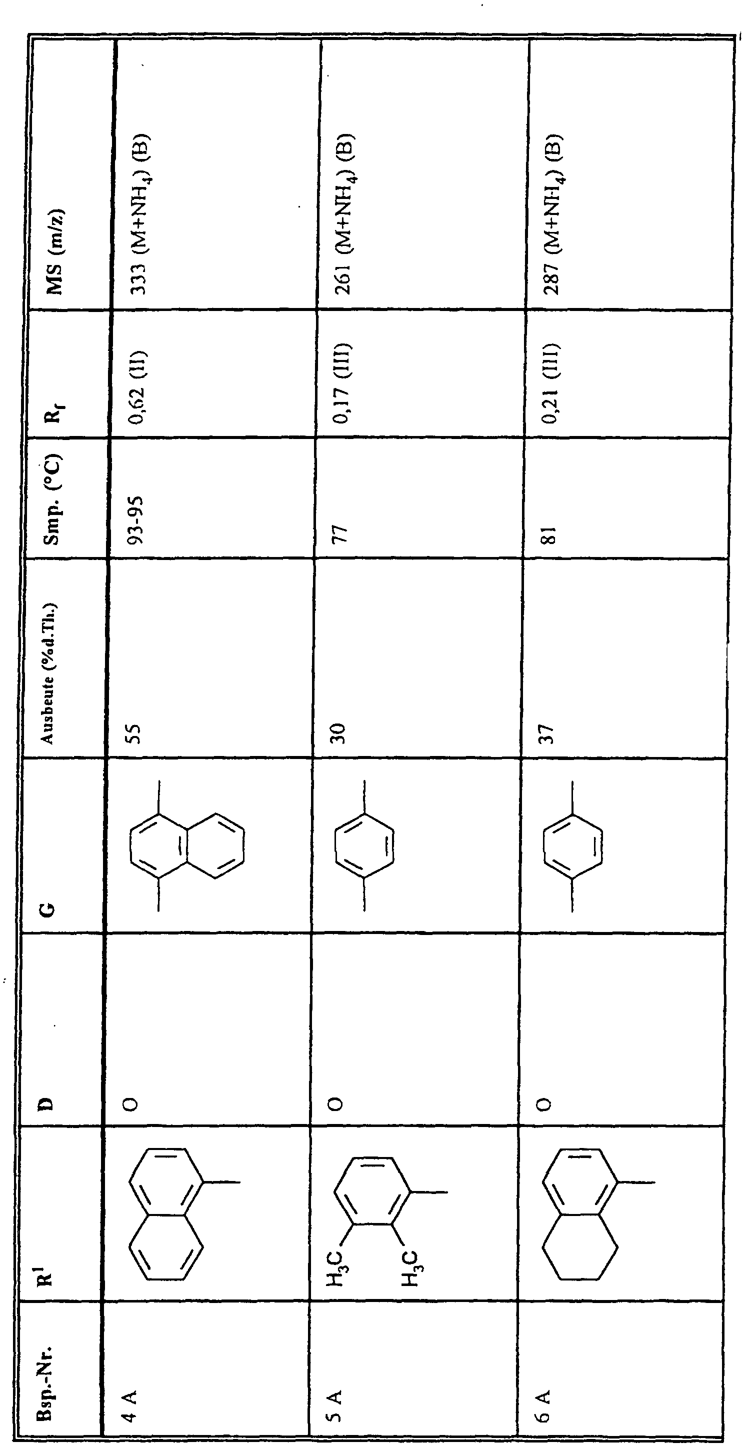

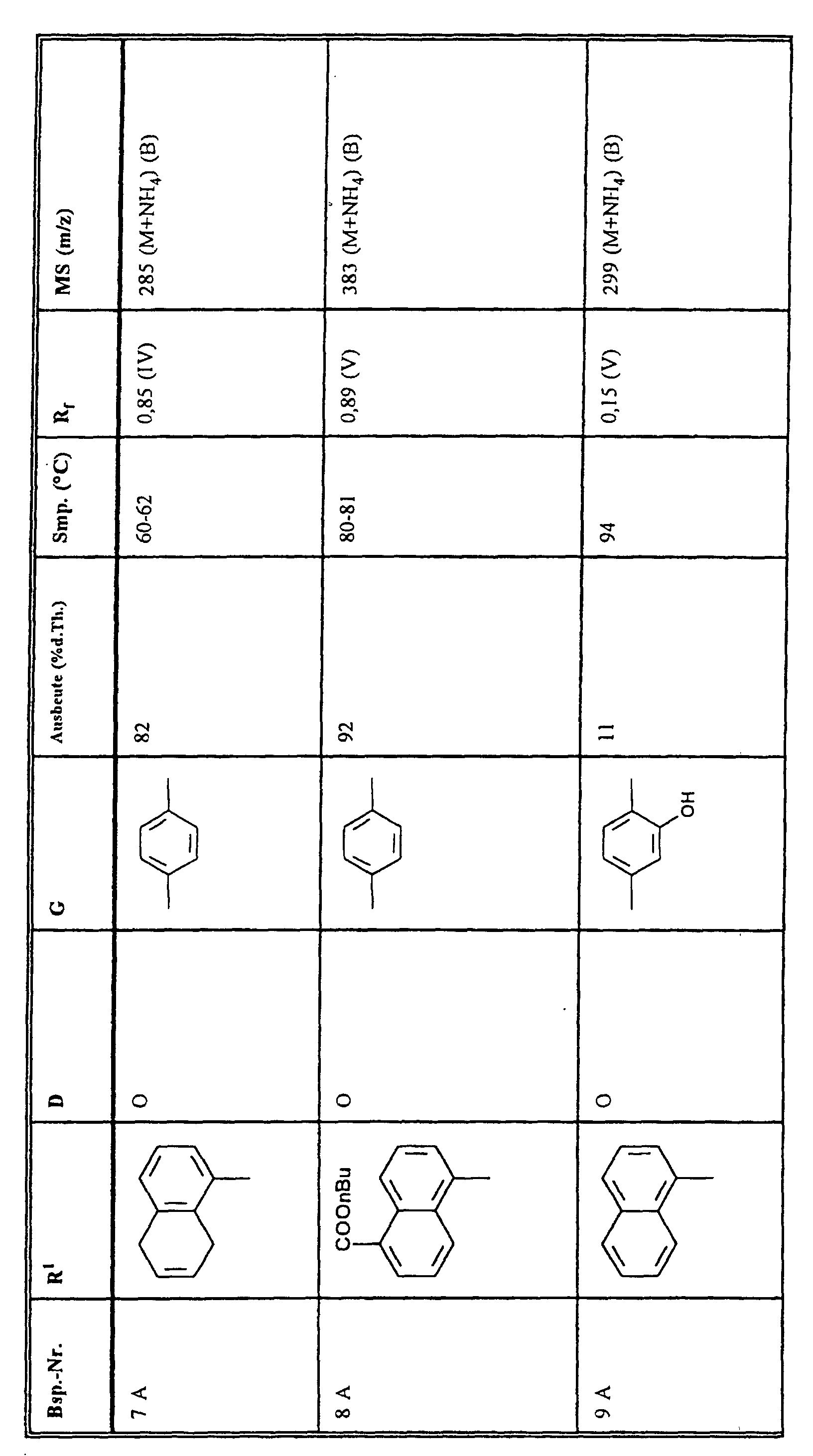

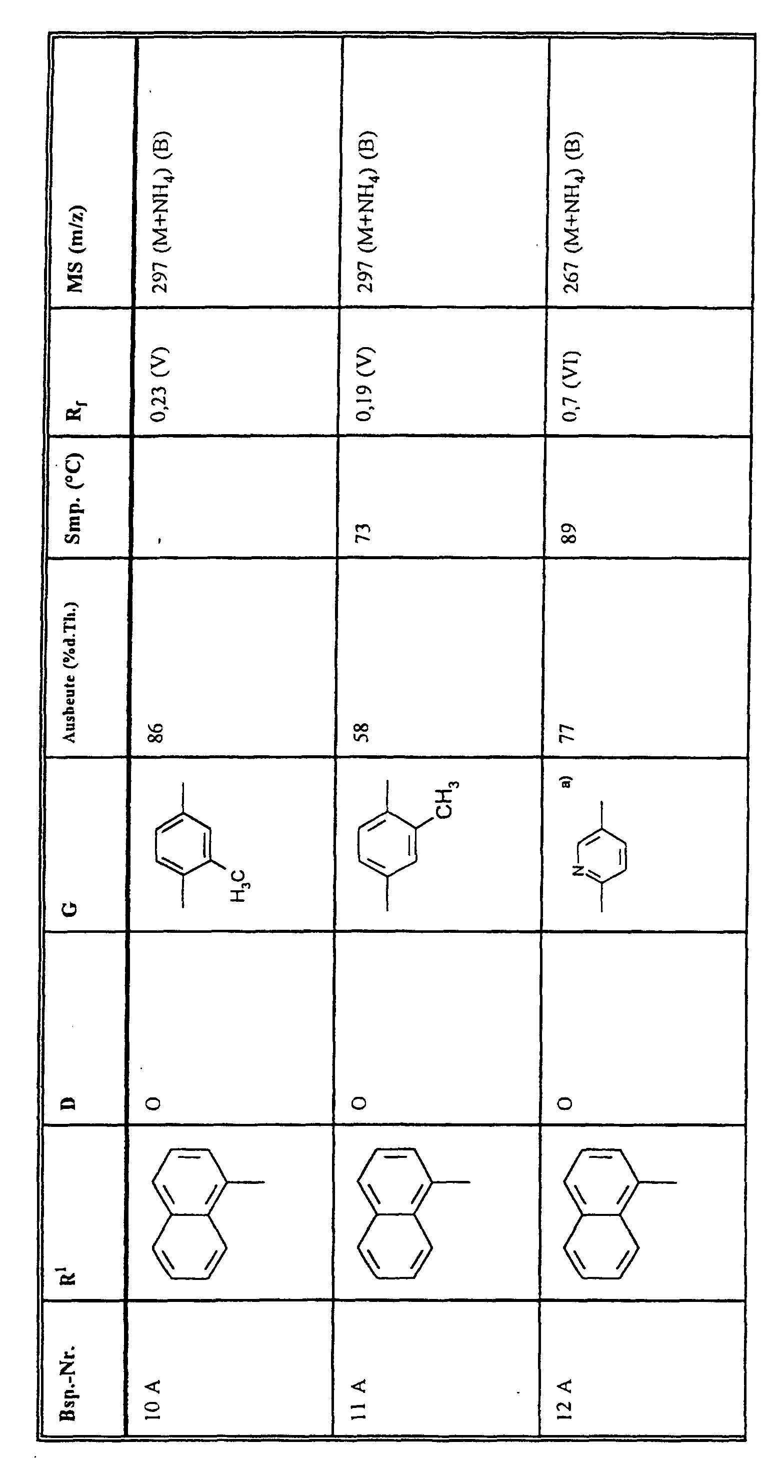

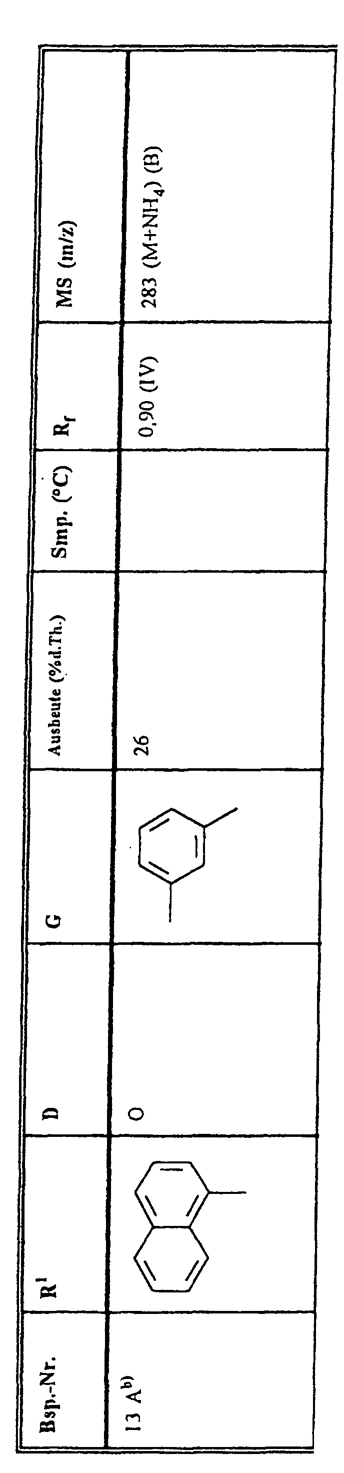

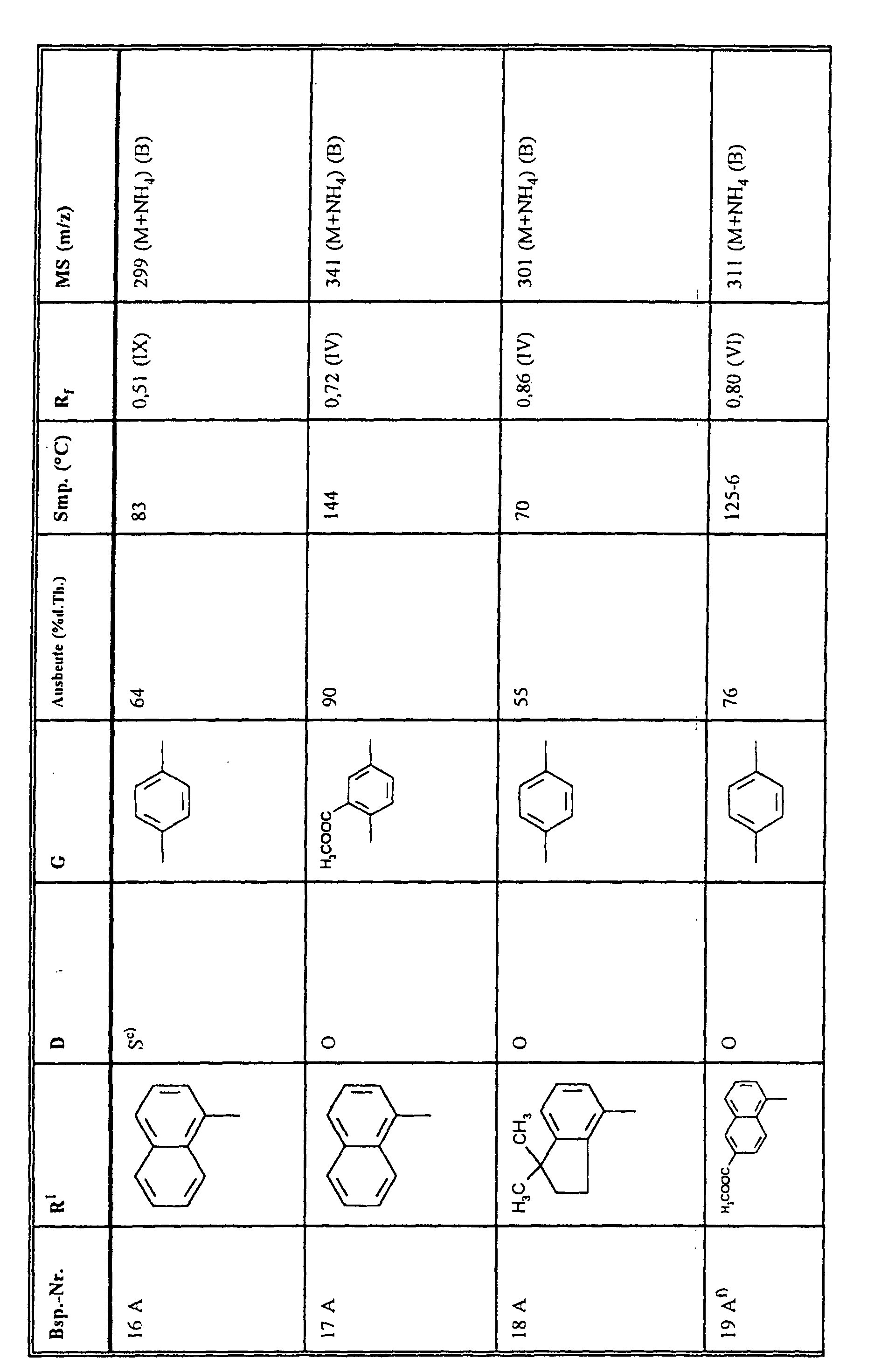

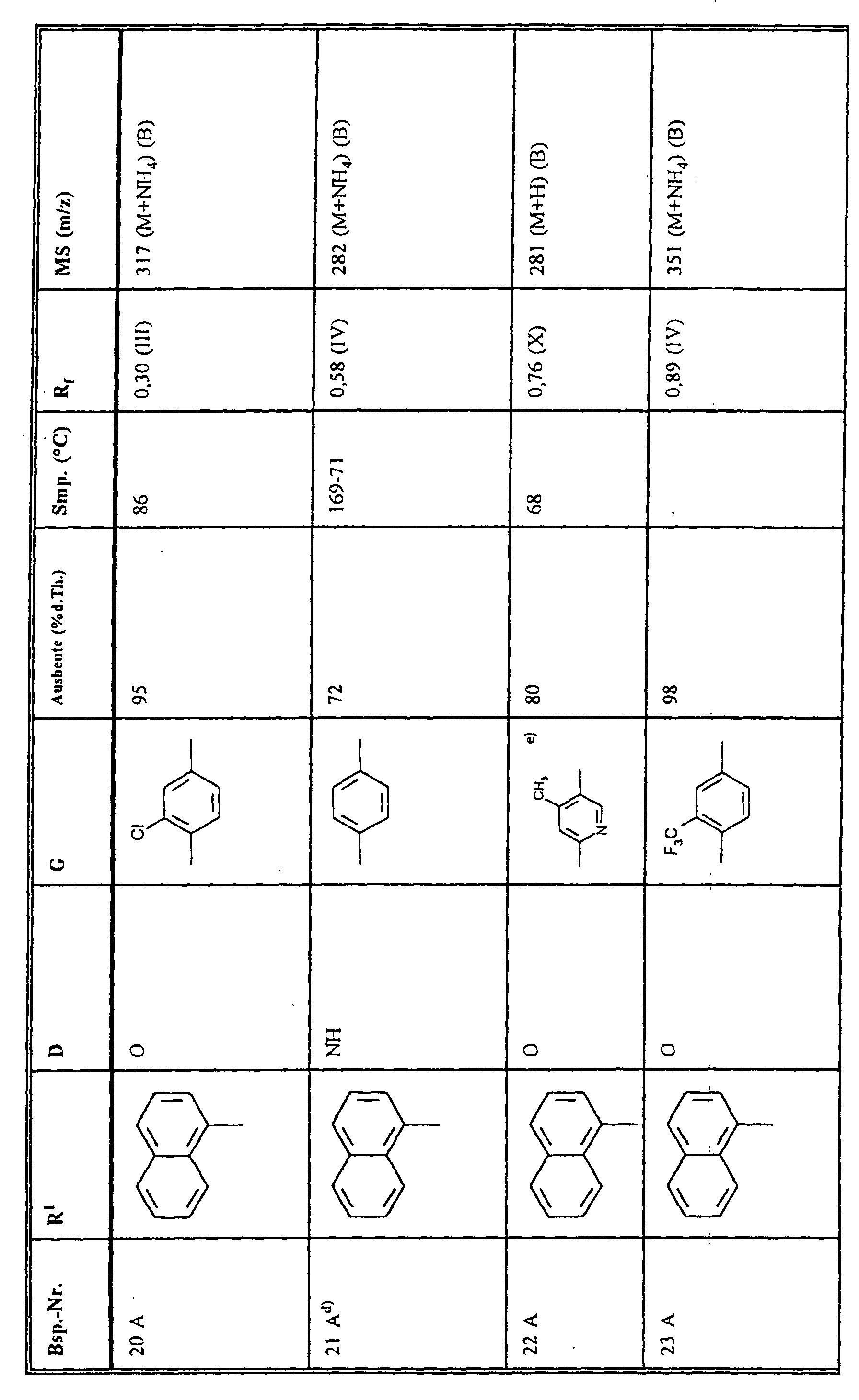

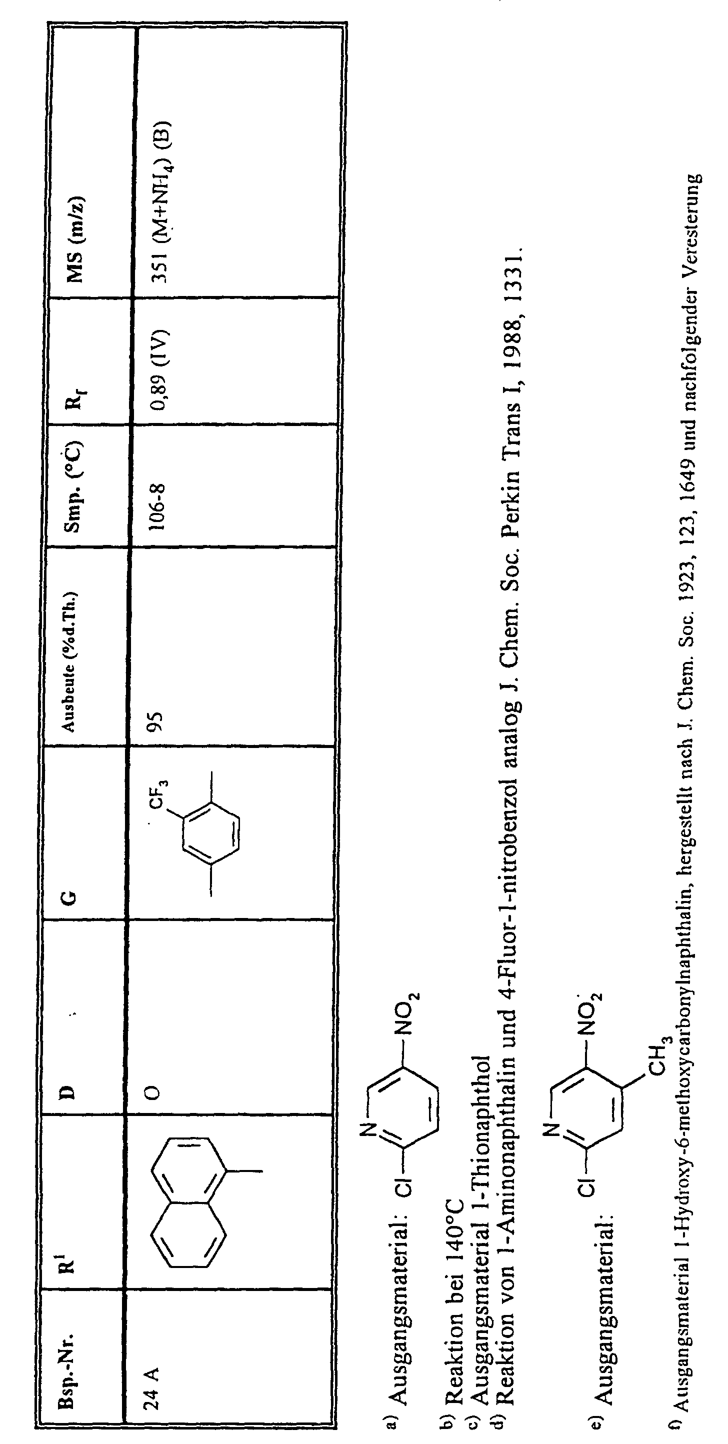

### Beispiel 25 A

### 1-(Naphthyl-1-methyloxy)-4-nitrobenzol

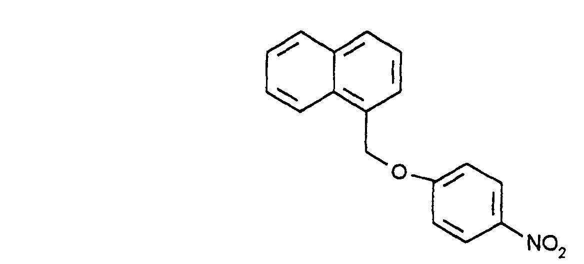

Eine Lösung von 4-Nitrophenol (15,7 g; 113 mmol) in DMF (300 ml) wird mit K₂CO₃ (30,8 g, 223 mmol) versetzt und 1 h bei RT gerührt. Nach Zugabe von 1-Naphthylmethylbromid (25,0 g; 113 mmol) wird die Reaktionsmischung über Nacht bei 50°C gerührt. Das Lösemittel wird im Vakuum abdestilliert und der Rückstand wird mit Ethylacetat (600 ml) und Wasser (250 ml) aufgenommen. Nach Filtration werden die Phasen getrennt und die wäßrige Phase mit Ethylacetat (3 x 300 ml) extrahiert. Die vereinigten organischen Phasen werden mit Wasser (200 ml) gewaschen, über MgSO₄ getrocknet und im Vakuum weitgehend eingeengt. Ausgefallenes Rohprodukt wird abgesaugt, in Essigester / Petrolether verrührt, erneut abgesaugt und getrocknet. Die Reinigung des Produkts erfolgt durch Umkristallisation aus CH₂Cl₂ / Methanol.
Ausbeute: 15,7 g (50% d.Th.)
Smp.: 145-146°C
MS (DCI, NH₃): m/z = 297 (M+NH₄)
R_{f} = 0,83 (IV)

In Analogie zur Vorschrift des Beispiels 25 A werden die in Tabelle II aufgeführten Beispiele hergestellt:

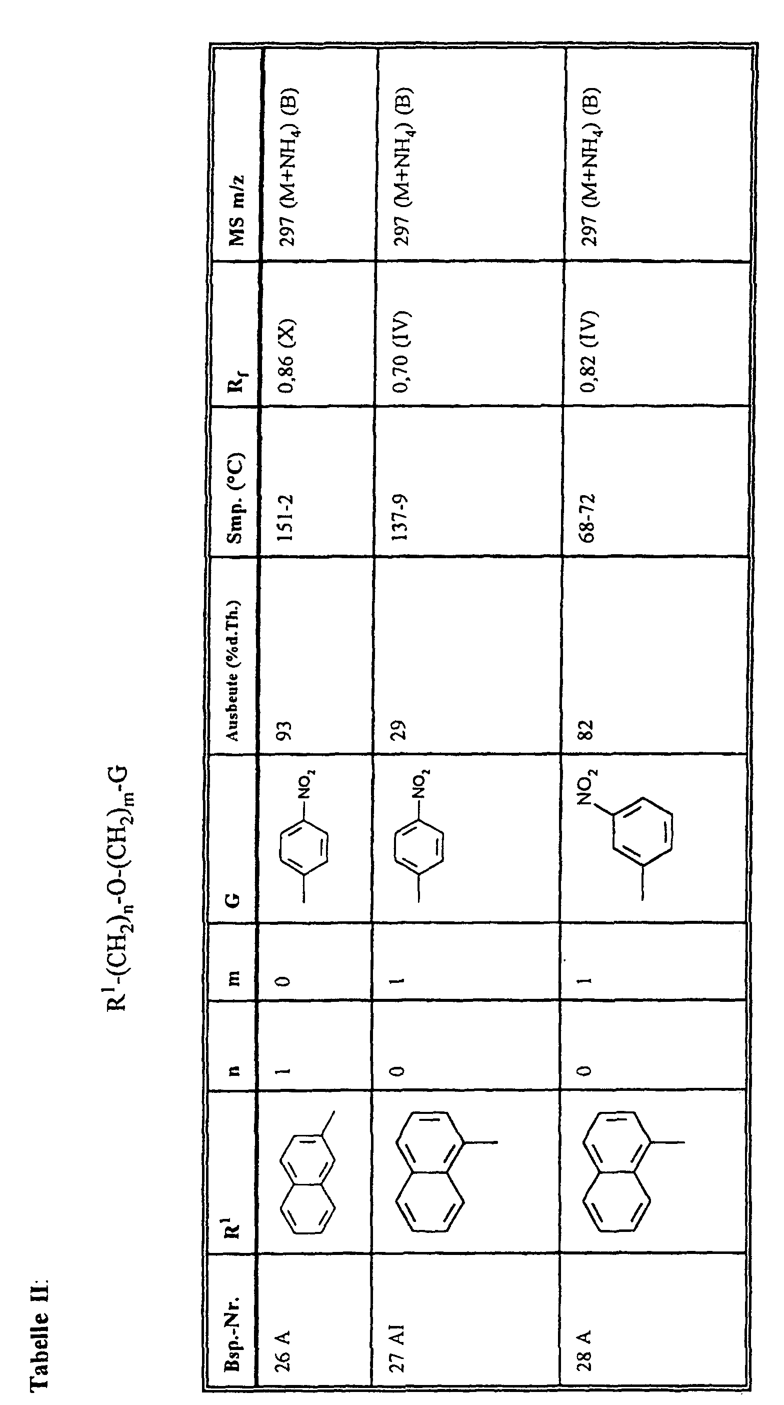

### Reduktion der Nitrogruppen der Beispiele 1 A - 29 A

### Methode A

### Beispiel 29 A

### 1-Amino-4-(2,3-dimethylphenyl-1-oxy)benzol

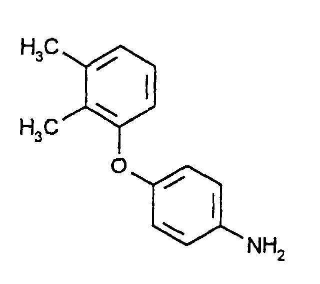

Eine Suspension von Beispiel 5 A (13,5 g, 55,6 mmol) und 10% Palladium auf Aktivkohle (1,45 g) in Methanol (132 ml) wird unter Argon zum Rückfluß erhitzt. Nach tropfenweise Zugabe von Hydrazin-Hydrat (5,4 ml, 111 mmol) wird noch 2 h unter Rückfluß gerührt. Die Reaktionsmischung wird über Kieselgur filtriert, mit Methanol gewaschen und anschließend im Vakuum eingeengt. Der Rückstand wird über Kieselgel mit Toluol : Ethylacetat (10:1) chromatographiert.
Ausbeute: 0,33 (IV)
MS (DCI, NH₃): m/z = 231 (M+NH₄)

### Methode B

### Beispiel 30 A

### 5-(4-Aminophenyl-1-oxy)-naphthalin-1-carbonsäure-n-butylester

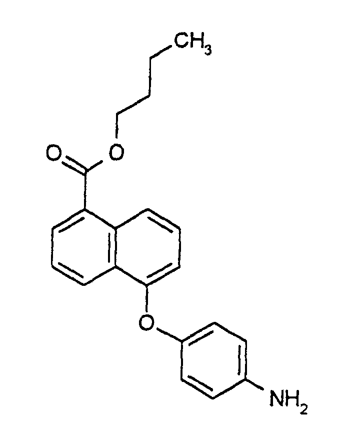

Eine Lösung von Beispiel 8 A (10,96 g, 30,0 mmol) in THF (100 ml) wird mit 10% Palladium auf Aktivkohle (0,25 g) versetzt und 5 h bei Normaldruck hydriert. Die Reaktionsmischung wird über Kieselgel filtriert, mit THF gewaschen und im Vakuum eingeengt. Der Rückstand wird in Diethylether verrührt, abgesaugt und im Vakuum getrocknet.
Ausbeute: 8,38 g (83% d.Th.)
Smp.: 104-105°C
R_{f} = 0,31 (IV)
MS (ESI): m/z = 336 (M+H)

### Methode C

### Beispiel 31 A

### 1-Amino-4-(5,8-dihydro-naphthyl-1-oxy)benzol

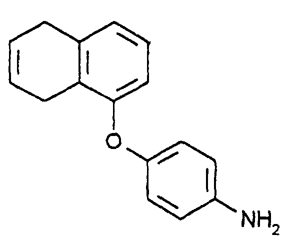

Zur Lösung der Verbindung aus Beispiel 7 A (10,7 g; 40,0 mmol) in Eisessig (380 ml) und Wasser (80 ml) tropft man eine 15%ige Lösung von Titan-(III)-chlorid in 10% Salzsäure (212 ml, 243 mmol) und läßt über Nacht rühren. Die Lösemittel werden im Vakuum abdestilliert und der Rückstand in Ethylacetat / Wasser aufgenommen. Durch Zugabe von 3 N Natronlauge wird pH 9-10 eingestellt und nach Phasentrennung wird die wäßrige Phase 3 x mit Ethylacetat extrahiert. Die vereinten organischen Phasen werden 2 x mit Wasser gewaschen, über MgSO₄ getrocknet und im Vakuum eingeengt. Der Rückstand wird über Kieselgel mit Toluol / Ethylacetat (20:1) chromatographiert.
Ausbeute: 2,1 g (22% d.Th.)
R_{f} = 0,25 (X)
MS (DCI, NH₃): m/z = 238 (M+H)

In Analogie zu den Beispielen 29 A - 31 A werden die in Tabelle III aufgeführten Beispiele hergestellt:

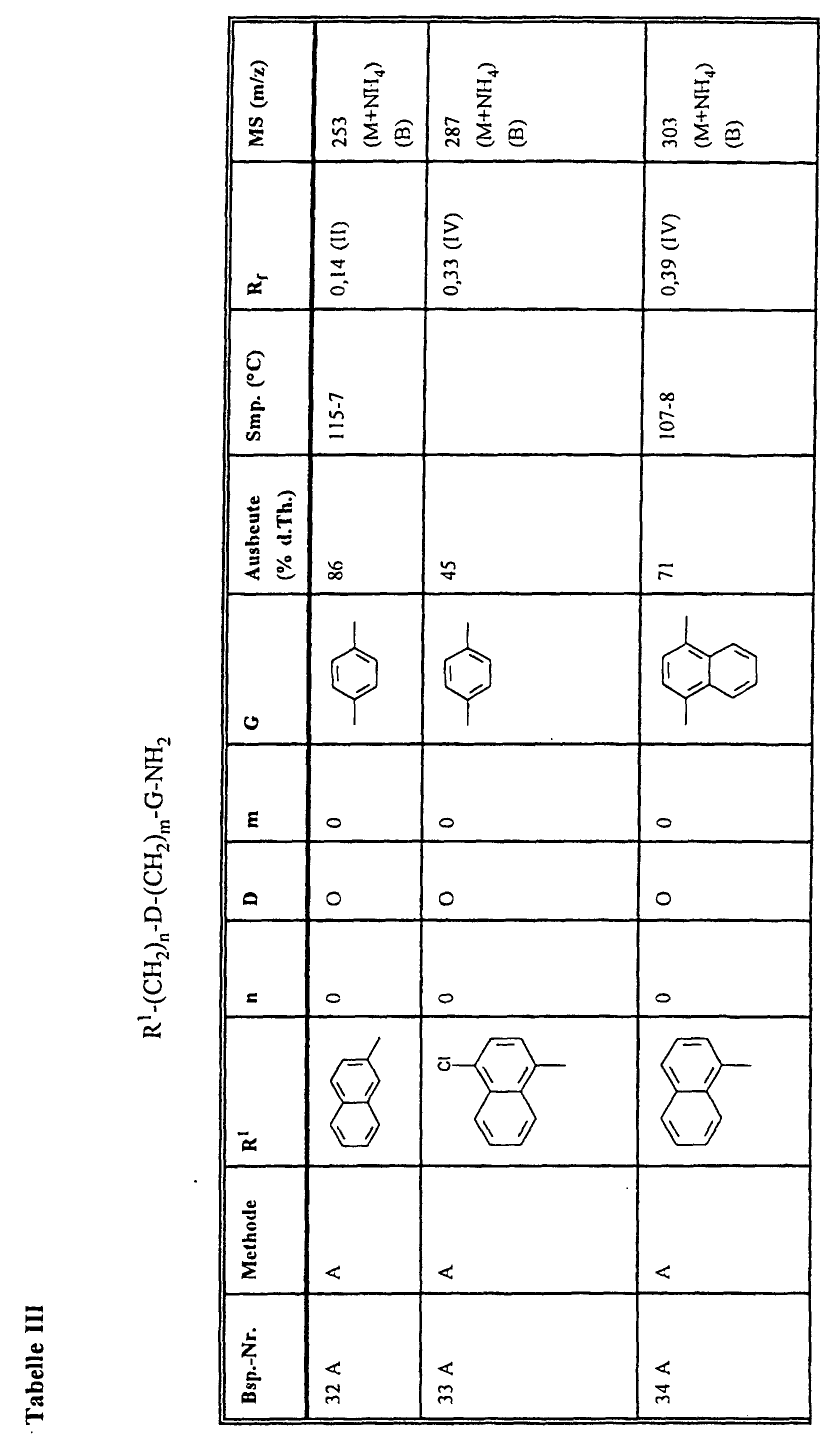

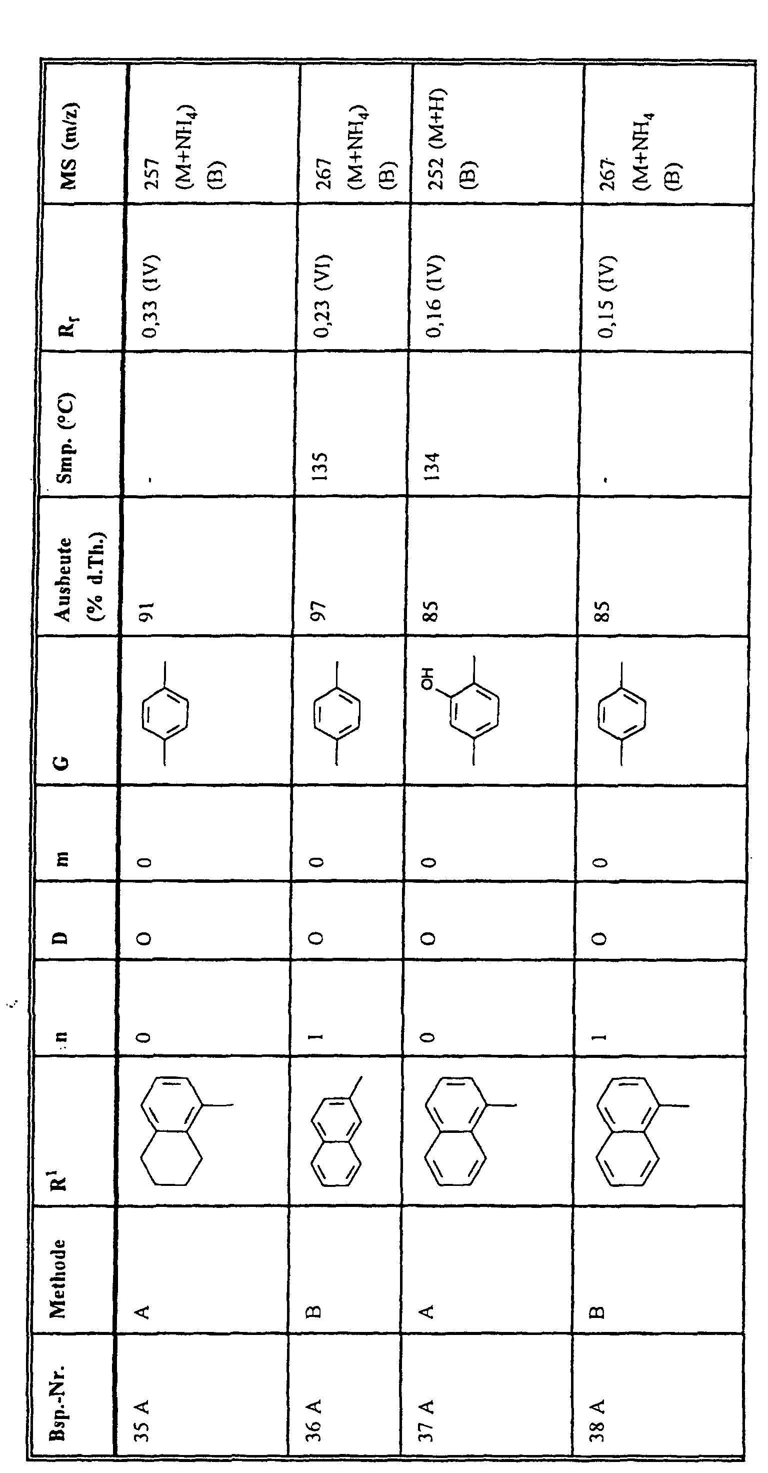

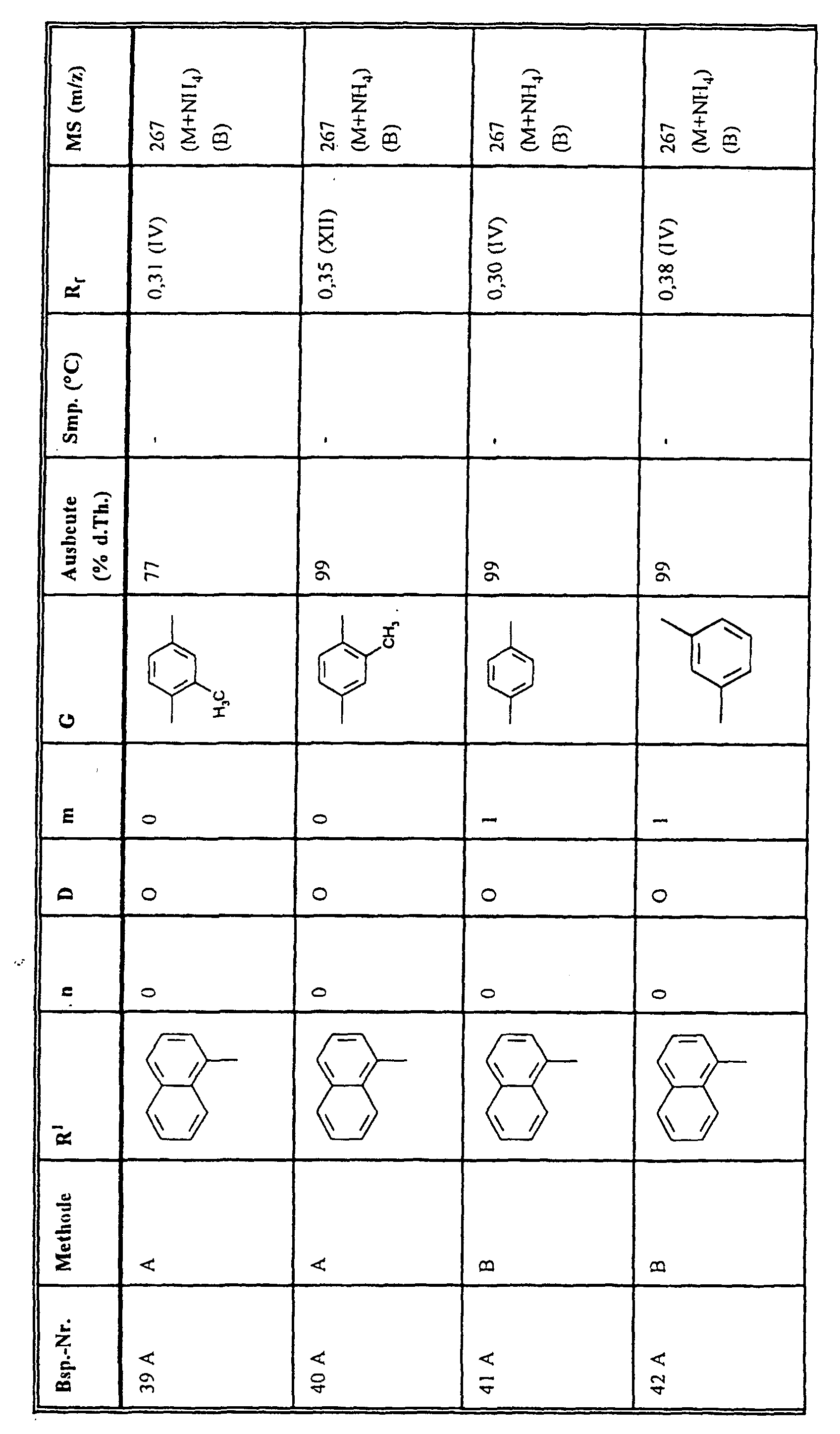

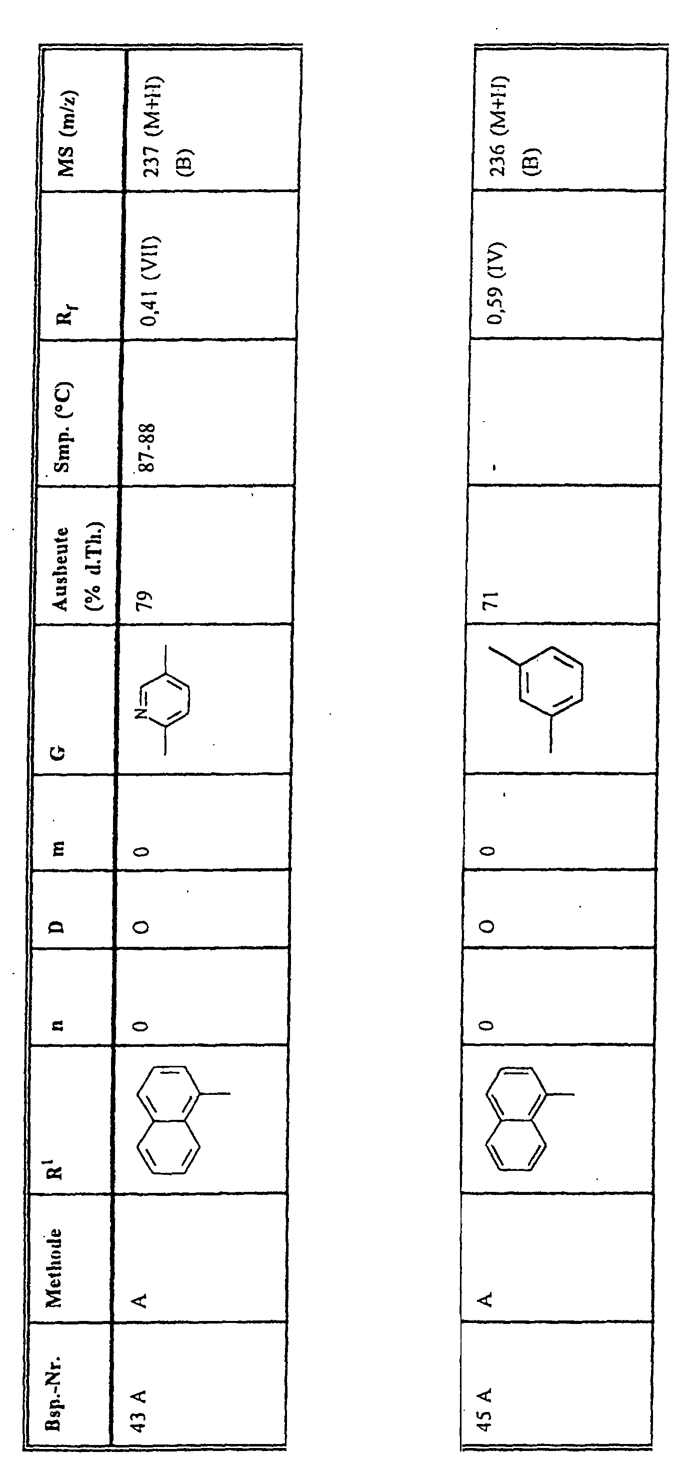

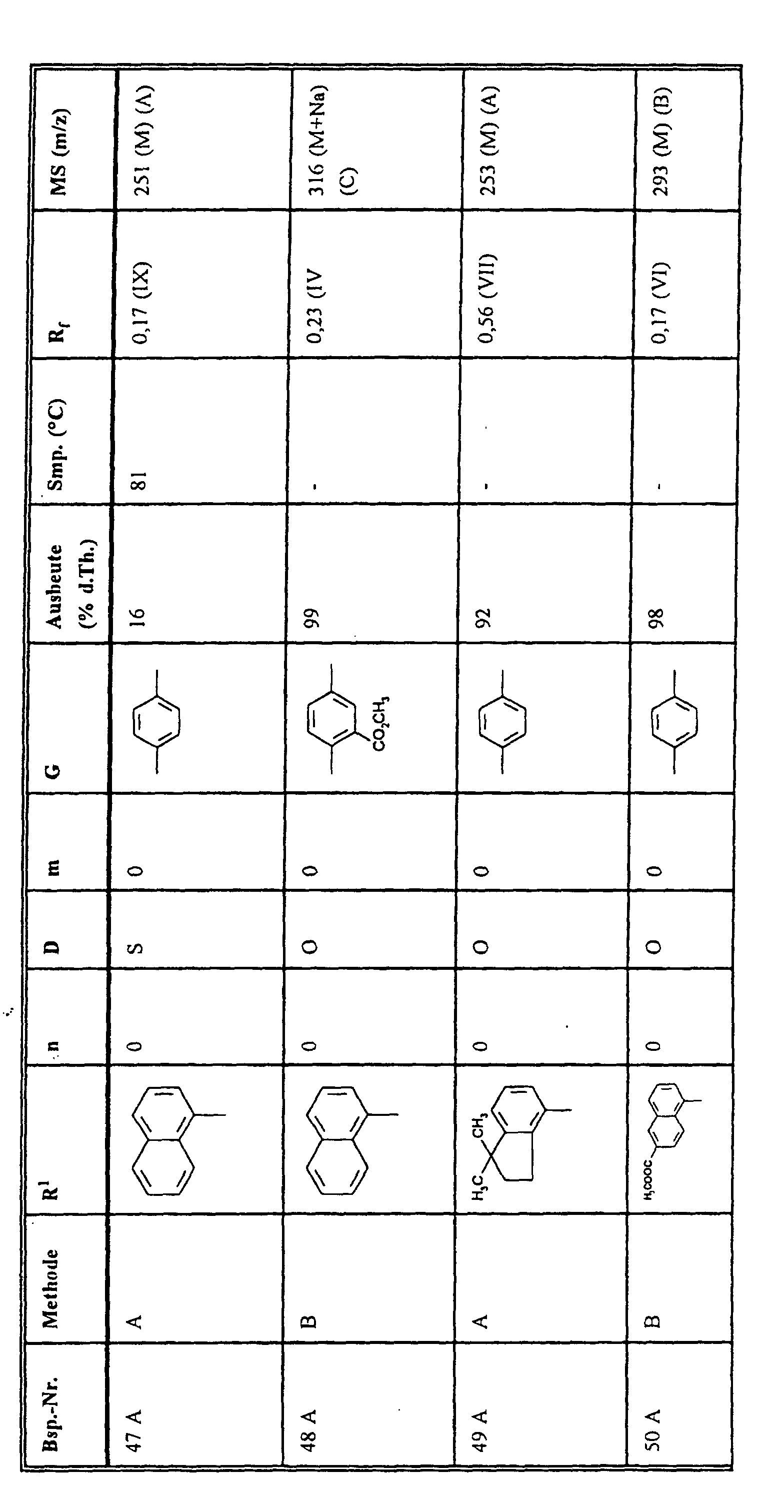

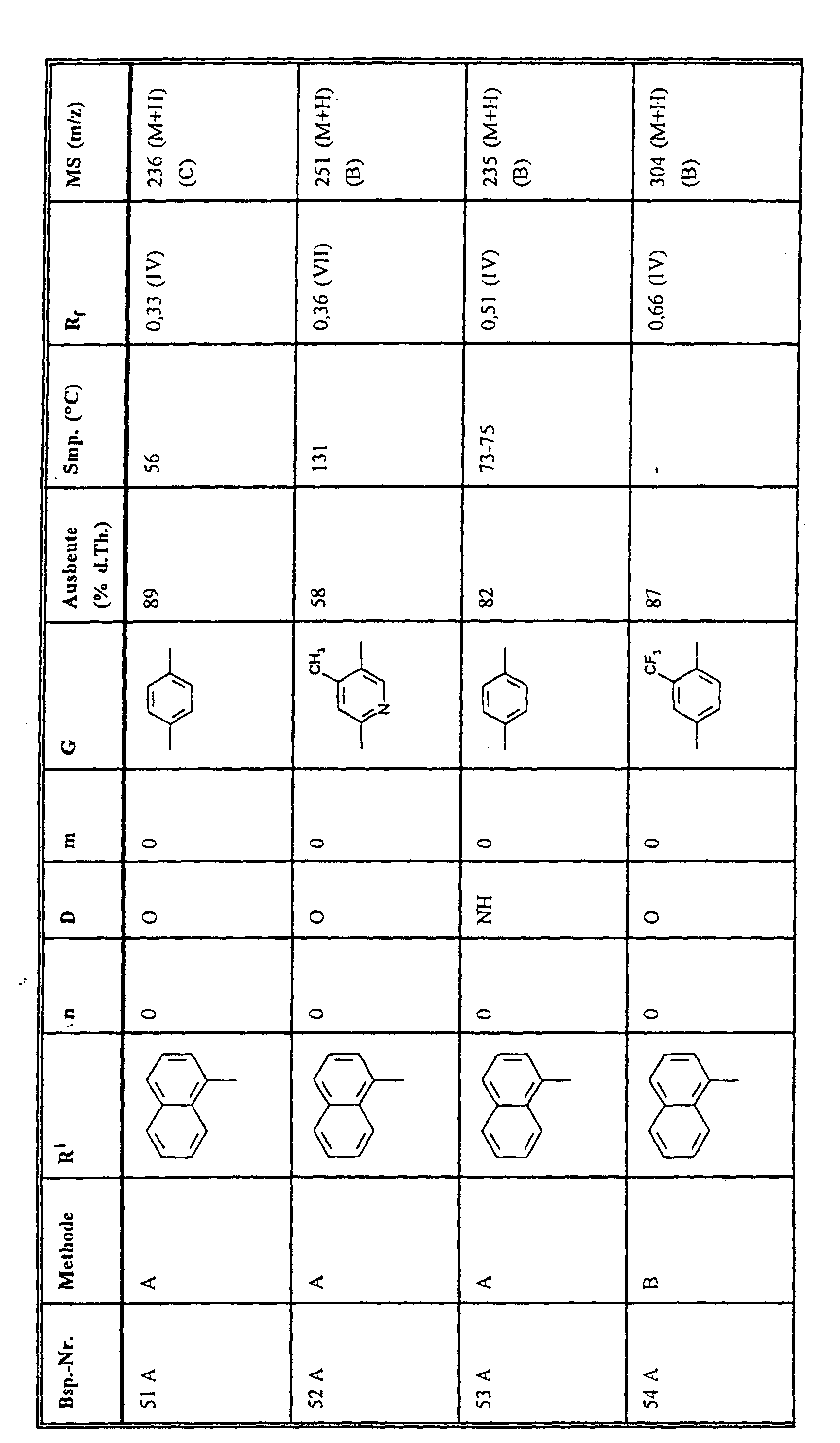

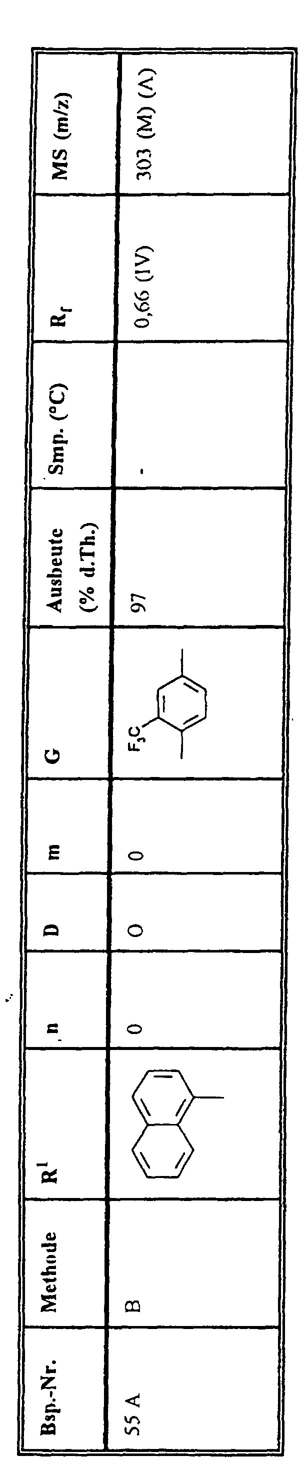

### Beispiel 56 A

### 4-(Naphthyl-1-oxy)phenol

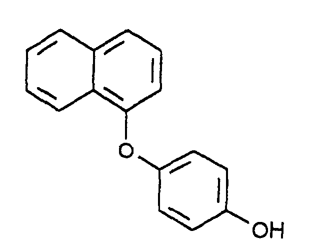

Zur Suspension der Verbindung aus 51 A (25,8 g; 110 mmol) in 50%iger wäßriger H₂SO₄ (400 ml) tropft man bei 0°C eine Lösung von NaNO₂ (7,6 g; 110 mmol) in Wasser (45 ml) und läßt 10 Minuten nachrühren. Anschließend wird die Reaktionsmischung 2,5 h auf 100°C erwärmt und nach dem Abkühlen mit Dichlormethan (3 x 150 ml) extrahiert. Die vereinten organischen Phasen werden mit Wasser (1 x 100 ml) gewaschen, getrocknet (Na₂SO₄) und im Vakuum eingeengt. Der Rückstand wird an Kieselgel mit Dichlormethan chromatographiert. Ausbeute: 6,1g (24% d.Th.)
R_{f} = 0,39 (IV)
MS (DCI, NH₃): m/z = 237 (M+H)

### Beispiel 57 A

### 3-Methyl-4-(naphthyl-1-oxy)phenol

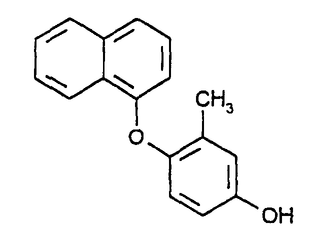

Die Herstellung erfolgt in Analogie zur Synthese des Beispiels 56 A ausgehend von Beispiel 39 A (5,0 g; 20 mmol).
Ausbeute: 2,1 g (42% d.Th.)
R_{f} = 0,36 (IV)
MS (DCI, NH₃): 251 (M+H)

### Beispiel 58 A

### [4-(Naphthyl-1-oxy)phenyl]aminosulfonsäure

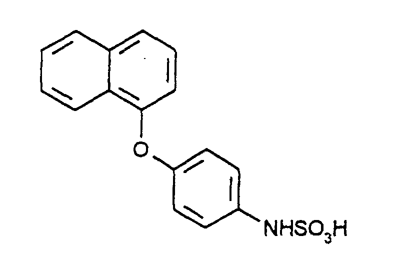

Zur Lösung von Chlortrimethylsilan (6,93 g; 63,8 mmol) in Cyclohexan tropft man bei 5°C unter Argon Triethylamin (6,44 g; 63,8 mmol) zu und läßt 1 h bei Eiskühlung rühren. Die Verbindung aus Beispiel 51 A (15,0 g; 63,8 mmol) wird in Cyclohexan (350 ml) heiß gelöst und bei 5°C zur Lösung von Chlortrimethylsilan / Triethylamin getropft. Die Reaktionsmischung wird über Nacht bei RT gerührt und ausgefallenes Triethylammoniumchlorid wird abfiltriert. Man wäscht mit Cyclohexan nach und engt das Filtrat im Vakuum ein. Der Rückstand wird in Dichlormethan (120 ml) aufgenommen und unter Argon bei -15°C über einen Zeitraum von 40 min tropft man Chlorsulfonsäure(trimethylsilyl)ester (12,0 g; 63,8 mmol) zu. Die Reaktionsmischung wird über Nacht bei -15°C gerührt, anschließend unter Argon filtriert, bei -15°C tropfenweise mit Trifluoressigsäure (7,3 g; 63,8 mmol) versetzt und noch 3 h bei -15C° nachgerührt. Ausgefallenes Produkt wird abgesaugt, mit Dichlormethan gewaschen und im Vakuum getrocknet.
Ausbeute: 5,6 g (28% d.Th.)
Smp.: 220°C
MS (FAB): m/z = 316 (M+H)

### Beispiel 59 A

### 4-Amino-2-(naphthyl-2-oxy)-pyridin

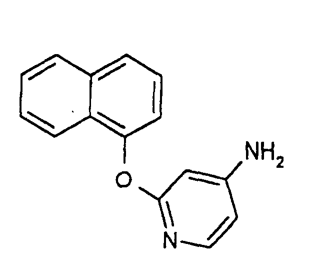

Eine Suspension von 4-Amino-2-chlorpyridin (4,20 g; 32,7 mmol), 1-Naphthol (7,06 g; 49,0 mmol) und Kaliumcarbonat (6,77 g; 49,0 mmol) in Pyridin (50 ml) wird zum Rückfluß erhitzt, mit Kupfer-(II)-oxid (5,8 g; 73,5 mmol) versetzt, und noch 18 Stunden bei Rückflußtemperatur nachgerührt.
Anschließend wird Pyridin i. Vak. abkondensiert, der Rückstand wird in Dichlormethan (100 ml) aufgenommen und über Kieselgur filtriert. Das Filtrat wird mit Wasser gewaschen und die wäßrige Phase wird zweimal mit Dichlormethan extrahiert. Die vereinten Dichlormethanphasen werden getrocknet (Na₂SO₄) und i. Vak. eingeengt. Der Rückstand wird an Kieselgel mit Toluol:Ethylacetat (10:1) chromatographiert.
Ausbeute: 4,63 g (60 % der Theorie)
Smp: 156°C
R_{f} = 0,12 (VI)
MS (CDCI, NH₃): m/z = 237 (M+H)

### Beispiel 60 A

### 6-Amino-2-(naphthyl-1-oxy)-pyridin

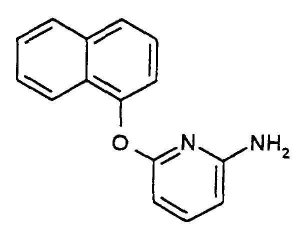

In Analogie zu Beispiel 59 A wurden 6-Amino-2-chlorpyridin (6,60 g; 51,3 mmol) und 1-Naphthol (11,1 g; 77,0 mmol) umgesetzt.
Ausbeute: 4,04 g (33 % der Theorie)
R_{f} = 0,59 (IV)
MS (ESI): m/z = 237 (M+H)

### Beispiele 61 A und 62 A

### 4-Amino-2-Chlor-6-(naphthyl-1-oxy)pyridin (Beispiel 61 A)

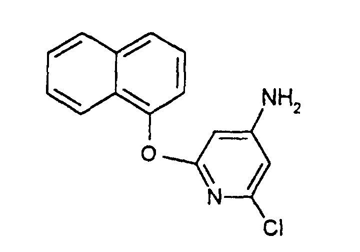

### 4-Amino-2,6-[bis(naphthyl)-1-oxy]pyridin (Beispiel 62 A)

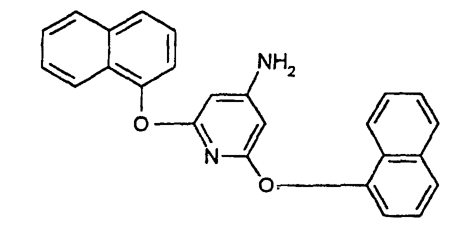

In Analogie zu Beispiel 59 A wurden 4-Amino-2,6-dichlorpyridin (4,96g; 30,4 mmol) und 1-Naphthol (6,58g; 45,6mmol) umgesetzt.

Ausbeute: (Beispiel 61 A): 0,14 g (1,8% d.Th.)
Smp.: 174°C
R_{f} = 0,37 (IV)
MS (DCI/NH₃): m/z = 271 (M+H)

Ausbeute: (Beispiel 62 A): 3,59 g (44% d.Th.)
Smp.: 169°C
R_{f} = 0,48 (IV)
MS (DCI/NH₃): m/z = 379 (M+H)

### Beispiel 63 A

### 3-(Naphthyl-1-oxy)phenol

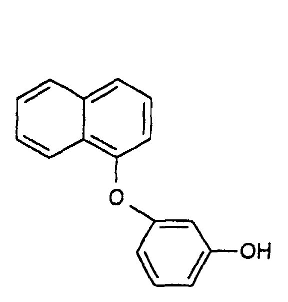

Die Herstellung erfolgt in Analogie zur Synthese des Beispiel 56 A ausgehend von Beispiel 45 A (9,40 g; 40,0mmol).
Ausbeute: 3,08 g (33% d.Th.)
R_{f} = 0,41 (CH₂Cl₂)
MS (DCI/NH₃): m/z = 237 (M+H)

### Beispiel 64 A

### 3-Brom-5-(naphthyl-1-oxy)pyridin

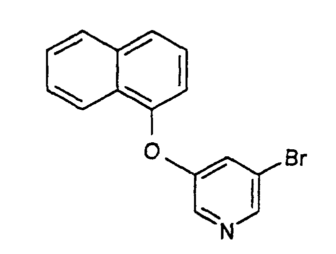

3,5-Dibrompyridin (24,9 g; 105 mmol), 1-Naphthol (15,1 g; 105 mmol) und Kaliumcarbonat (21,8 g; 158 mmol) werden in Pyridin (200 ml) unter Argon vorgelegt. Die Reaktionsmischung wird zum Rückfluß erhitzt, nach 15 min bei Kupfer-(II)-oxid (0,8 g; 10 mmol) versetzt und anschließend weitere 10 h zum Rückfluß erhitzt.

Nach dem Abkühlen auf Raumtemperatur wird die Reaktionsmischung filtriert und der Rückstand mit Dichlormethan nachgewaschen. Das Filtrat wird im Vakuum eingeengt. Der Rückstand wird in Dichlormethan aufgenommen, und nach erneuter Filtration wird die Dichlormethan-Lösung mit Wasser gewaschen. Die wäßrige Phase wird mit Dichlormethan extrahiert und die vereinten Dichlormethan-Phasen werden getrocknet (MgSO₄) und im Vakuum eingeengt. Der Rückstand wird an Kieselgel mit Toluol : Essigester (10:1) chromatographiert. Das so erhaltene Produkt wird aus Diethylether / Petrolether umkristallisiert.
Ausbeute: 2,9 g (10 % d.Th.)
Smp.: 59-61°C
R_{f} = 0,54 (IV)
MS (DCI/NH₃): m/z = 300, 302 (M+H)

### Beispiel 65 A

### 3-Amino-5-(naphthyl-1-oxy)pyridin

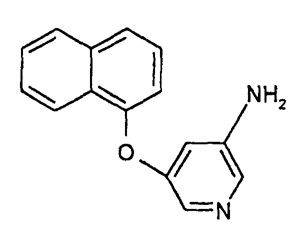

In Kaliumamid [26,4 mmol, hergestellt aus Kalium, (1.03 g) und kat. Mengen FeCl₃] in flüssigem Ammoniak (50 ml) wird bei -33°C eine Lösung des Beispiels 64 A (1,98 g; 6,6 mmol) in THF (15 ml) getropft.

Nach 10 min wird NH₄Cl (2,0 g) zugegeben und man läßt den Ammoniak abdampfen. Der Rückstand wird mit einer konz. wäßrigen NH₄Cl-Lösung (25 ml) und Wasser (25 ml) versetzt und mit Dichlormethan extrahiert (5 x 25 ml). Die vereinten org. Phasen werden mit Wasser (1 x 25. ml) gewaschen, getrocknet und im Vakuum eingeengt.
Ausbeute: 1,40 g (90 % d.Th.)
Smp.: 91-92°C
R_{f} = 0,22 (VII)
MS (ESI): m/z = 237 (M+H)

In Analogie zu Beispiel 1 A werden die in der Tabelle IV dargestellten Verbindungen hergestellt:

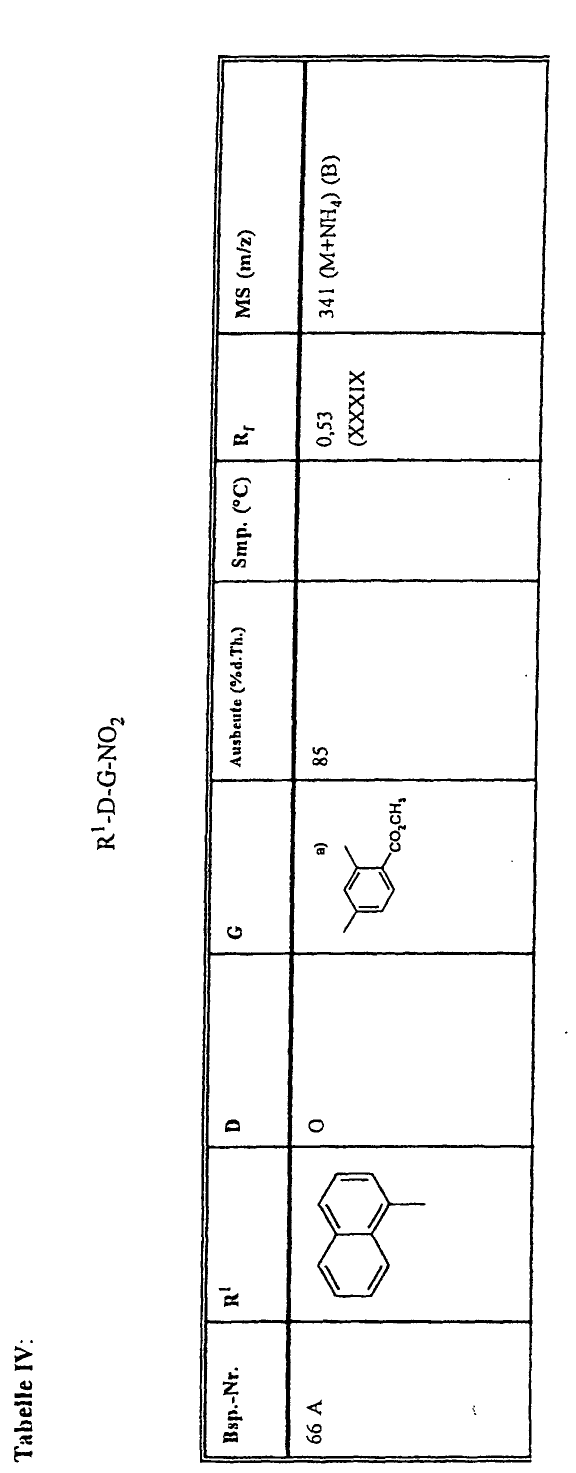

### Beispiel 81 A

### 4-Fluoro-2-nitrobenzoesäuremethylester

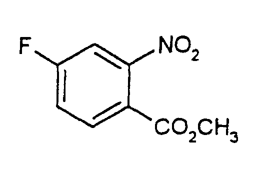

Thionylchlorid (31,5 ml; 0,432 mol) wurde bei 0°C langsam zu einer Lösung von 4-Fluoro-2-nitrobenzoesäure (16,0 g; 86,4 mmol) in Methanol (240 ml) getropft. Nach Aufwärmen auf RT, Rühren über Nacht und 4h Kochen unter Rückfluß wurde die Reaktionslösung im Vakuum eingeengt und zwischen Essigester und Kaliumhydrogencarbonatlösung verteilt. Trocknen und Einengen der organischen Phase ergaben gelbes Öl.
Ausbeute: 15,7 g (85 % d.Th.)
R_{f} = 0,53 (XXIX)
MS (EI): m/z = 199 (M)

In Analogie zu den Beispielen 29 A (Methode A) und 30 A (Methode B) werden die in Tabelle V aufgeführten Beispiele hergestellt:

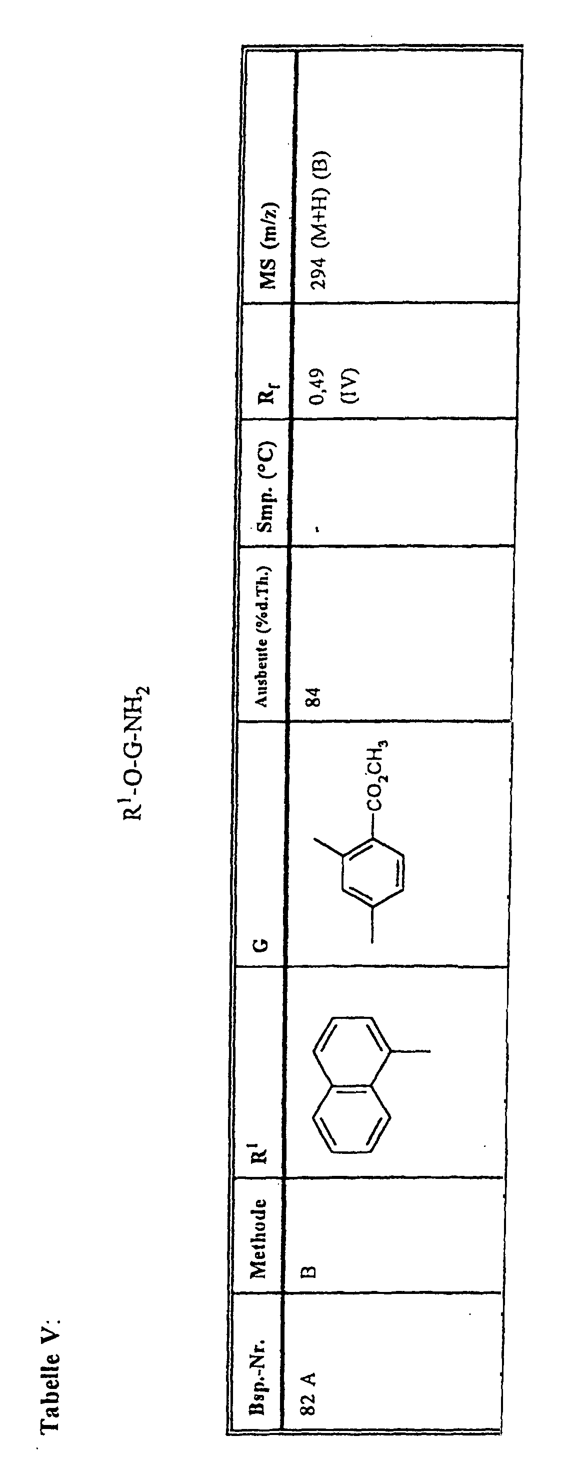

### Beispiel 97 A

### 4-(2-Ethoxycarbonylindan-4-oxy)-1-nitrobenzol

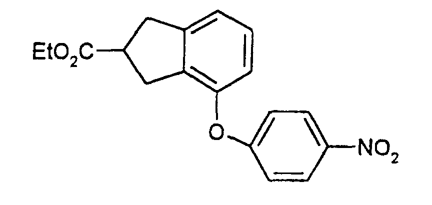

Die Herstellung erfolgte in Analogie zur Herstellung des Beispiels 1 A ausgehend von 4-Fluor-1-nitrobenzol (3,76 g; 26,7 mmol) und 4-Hydroxy-indan-2-carbonsäureethylester (5,50 g; 26,7 mmol; EP 425 946).
Ausbeute: 0,70 g (7,5 % d.Th.)
R_{f} = 0,37 (X)
MS (DCI, NH₃): m/z = 345 (M+NH₄)

### Beispiel 98 A

### 4-(2-Ethoxycarbonyl-indan-4-oxy)-anilin

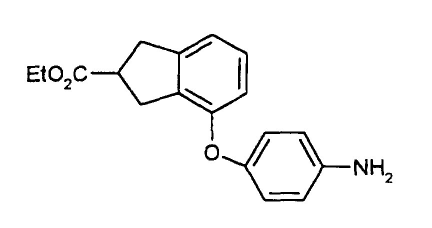

Die Herstellung erfolgte in Analogie zur Herstellung des Beispiels 30 A ausgehend von Beispiel 97 A (0,70 g; 2,14 mmol).
Ausbeute: 0,616 g (94 % d.Th.)
R_{f}= 0,12 (XXXI)
MS (DCI, NH₃): m/z = 315 (M+NH₄)

### Beispiel 99 A

### 3-Fluor-5-(naphthyl-1-oxy)-1-nitrobenzol

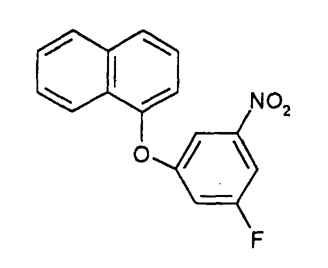

Die Herstellung erfolgte in Analogie zur Herstellung des Beispiel 13 A ausgehend von 1-Naphthol (13,59 g; 94,3 mmol) und 3,5-Difluornitrobenzol (15,00 g; 94,3 mmol).
Ausbeute: 17,9 g (67 % d.Th.)
R_{f} = 0,32 (III)
MS (DCI, NH₃): m/z = 425 (M+NH₄)

### Beispiele 100 A und 101 A

### 3-Fluor-5-(naphthyl-1-oxy)-anilin (Beispiel 100 A)

### N-[3-Fluor-5-(naphthyl-1-oxy)-phenyl]hydroxylamin (Beispiel 101 A)

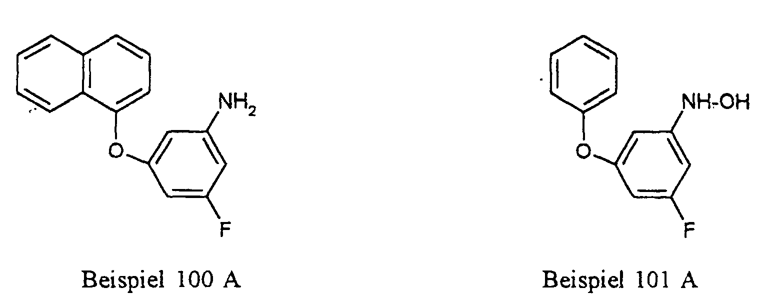

Eine Lösung des Beispiels 99A in Methanol (200 ml) und THF (15 ml) wird mit Palladium, 10 % auf Aktivkohle (0,2 g) versetzt und bei 1 atm bis zur Aufnahme von 1,8 1 Wasserstoff hydriert. Die Reaktionsmischung wird über Kieselgur filtriert und das Filtrat wird im Vakuum eingeengt. Der Rückstand wird an Kieselgel mit Toluol : Ethylacetat (10:1) chromatographiert.

Ausbeute (Beispiel 100 A): 3,92 g (44 % d.Th.)
R_{f} = 0,55 (IV)
MS (DCI, NH₃): m/z = 254 (M+H)

Ausbeute (Beispiel 101 A): 5,2 g (47 % d.Th.)
R_{f} = 0,33 (IV)
MS (DCI, NH₃): m/z = 270 (M+H)

In Analogie zur Herstellung von Beispiel 1A werden die in Tabelle VI aufgeführten Beispiele hergestellt:

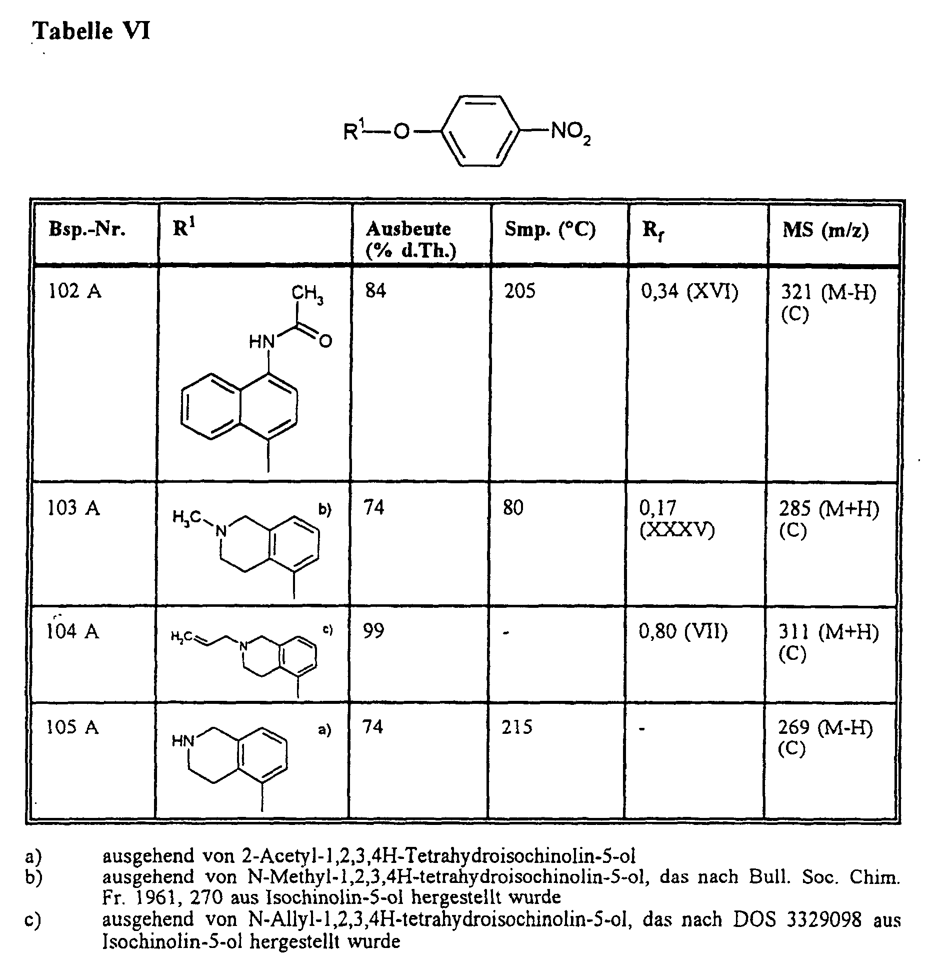

### Beispiel 106 A

### 1-(2-Acetyl-1,2,3,4H-tetrahydroisochinolin-5-oxy)-4-nitrobenzol

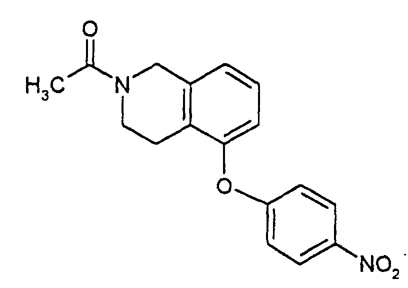

Eine Lösung von Beispiel 105 A (12 g; 45 mmol), Acetanhydrid (4,3 ml; 45 mmol) und Pyridin (3,6 ml; 45 mmol) in Dichlormethan wurde 4 h unter Rückfluß gekocht. Nach Abkühlen auf RT wurde der Reaktionsansatz auf Eis gegeben, viermal mit Wasser gewaschen und eingeengt. Der Rückstand wurde aus Dichlormethan / Petrolether umkristallisiert.
Ausbeute: 11,1 g (79% d.Th.)
Smp.: 137°C
MS (ESI): m/z = 313 (M+H)

In Analogie zur Herstellung von Beispiel 29 A (Methode A) und Beispiel 30 A (Methode B) wurden die in der Tabelle VII aufgeführten Beispiele hergestellt:

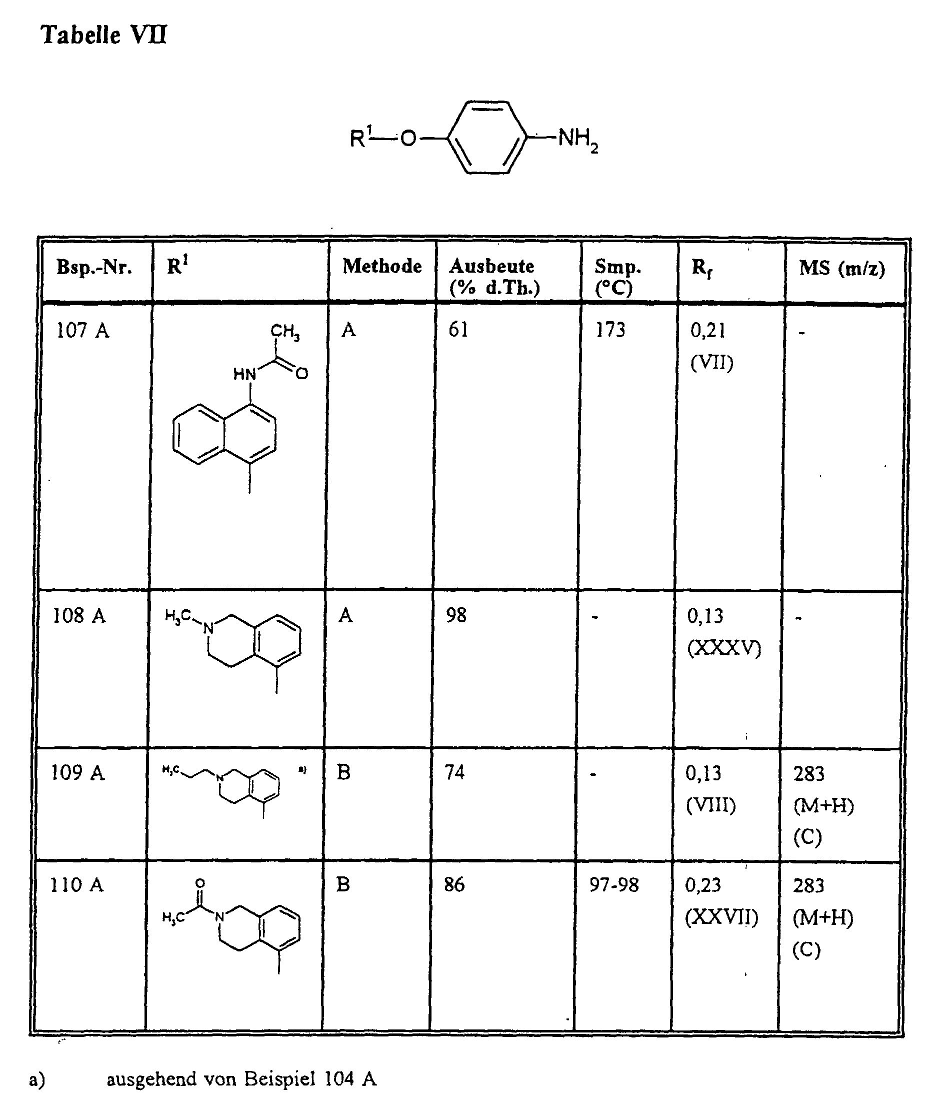

### Beispiel 111 A

### 2-Fluoro-6-nitrobenzoesäure

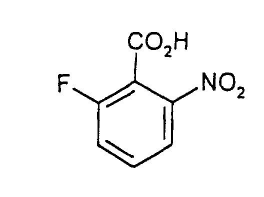

In Analogie zu Kaminski et al. J. Med. Chem. 1987, 30, 2047 wurde Beispiel 111 A hergestellt.

Ausbeute: 70 % d.Th.
Fp.: 149-51°C
R_{f} = 0,35 (XXXIX)
MS 185 (M) (A)

### Beispiel 112 A

### 2-Fluoro-6-nitrobenzoesäuremethylester

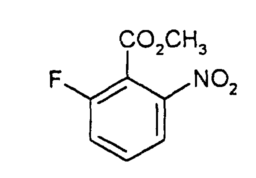

In Analogie zur Vorschrift des Beispiels 81 A wurde Beispiel 112 A hergestellt.

Ausbeute: 93 % d.Th.
Fp: 60-1°C
R_{f} = 0,83 (XXVII)
MS 199 (M) (A)

In Analogie zur Vorschrift des Beispiels 1A wurden die Beispiele der Tabelle VIII hergestellt.

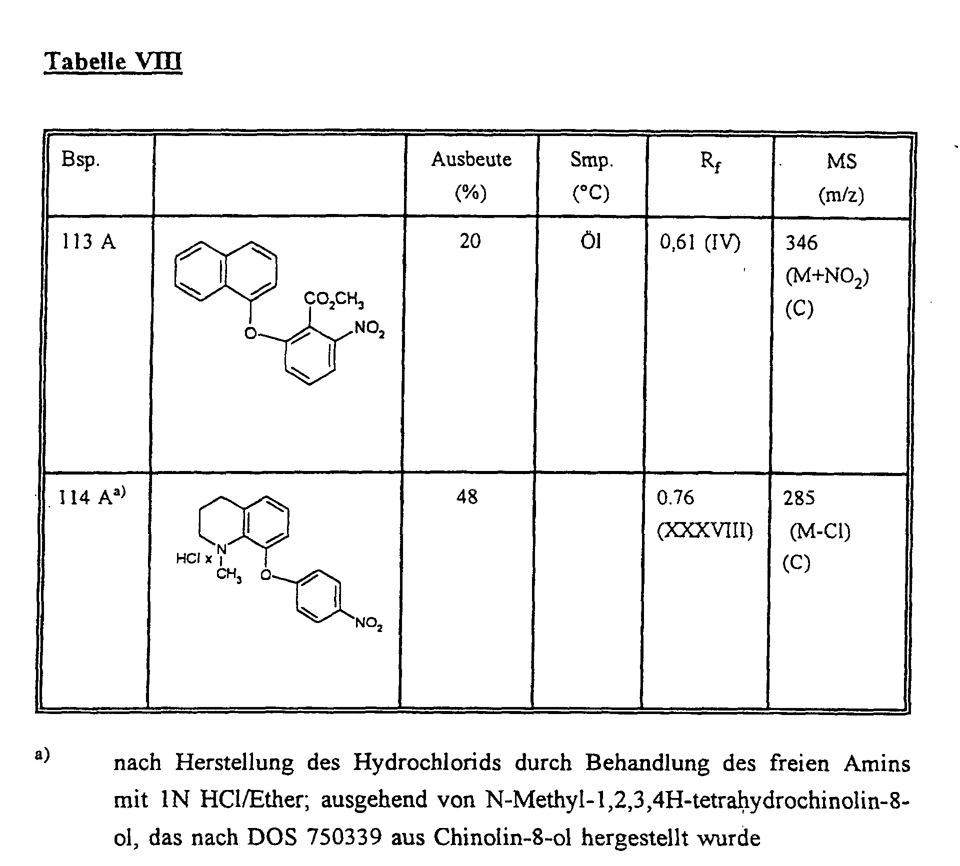

In Analogie zur Vorschrift des Beispiels 30A wurden die Beispiele der Tabelle IX hergestellt.

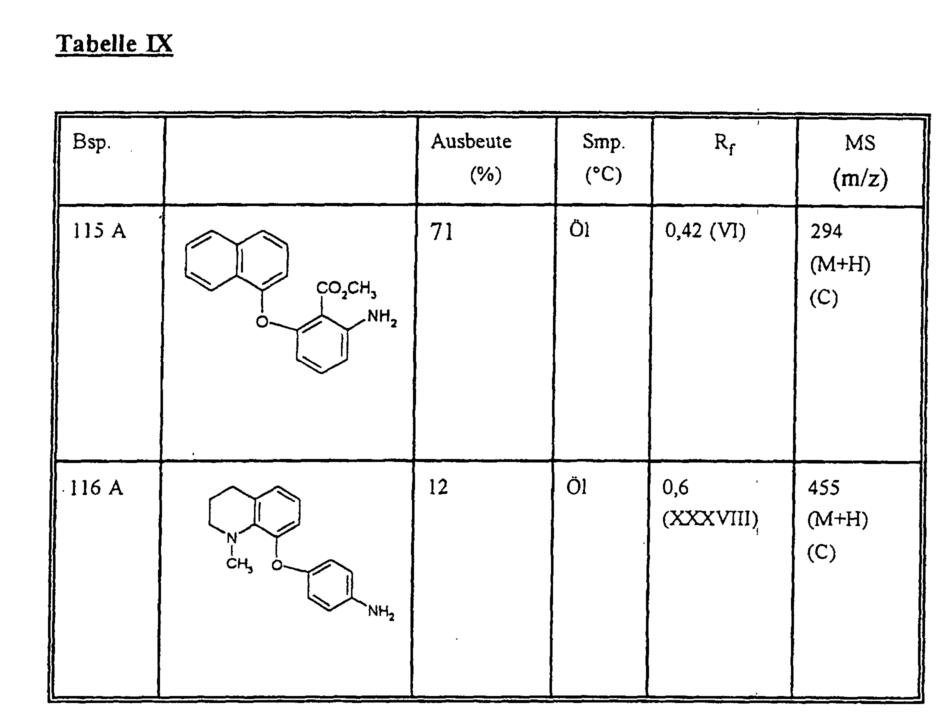

### Beispiel 117 A

### 2-Propyl-5-(4-hydroxyphenoxy)-[1,2,3,4H]-Tetrahydroisochinolin

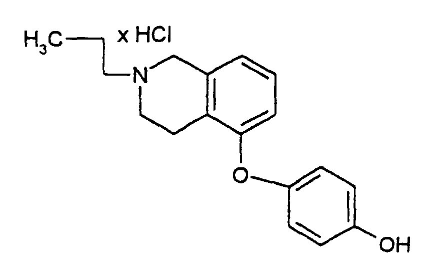

In Analogie zu Beispiel 56 A und Fällung mit 1N HCl/Ether wurde Beispiel 117 A hergestellt.

Ausbeute: 47 % d.Th.
Fp: 239-40°C
R_{f} = 0,58 (XL)
MS 284 (M+H) (C)

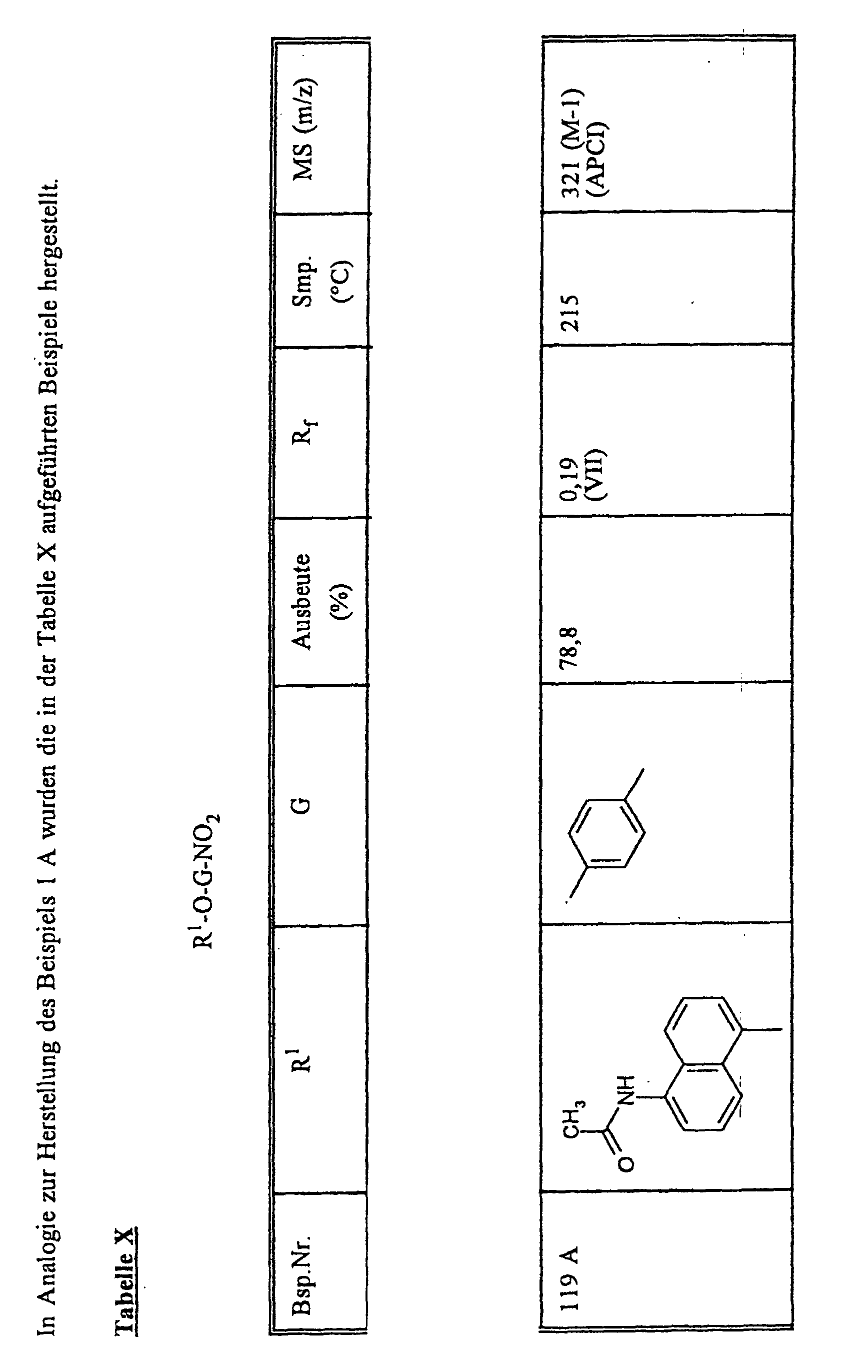

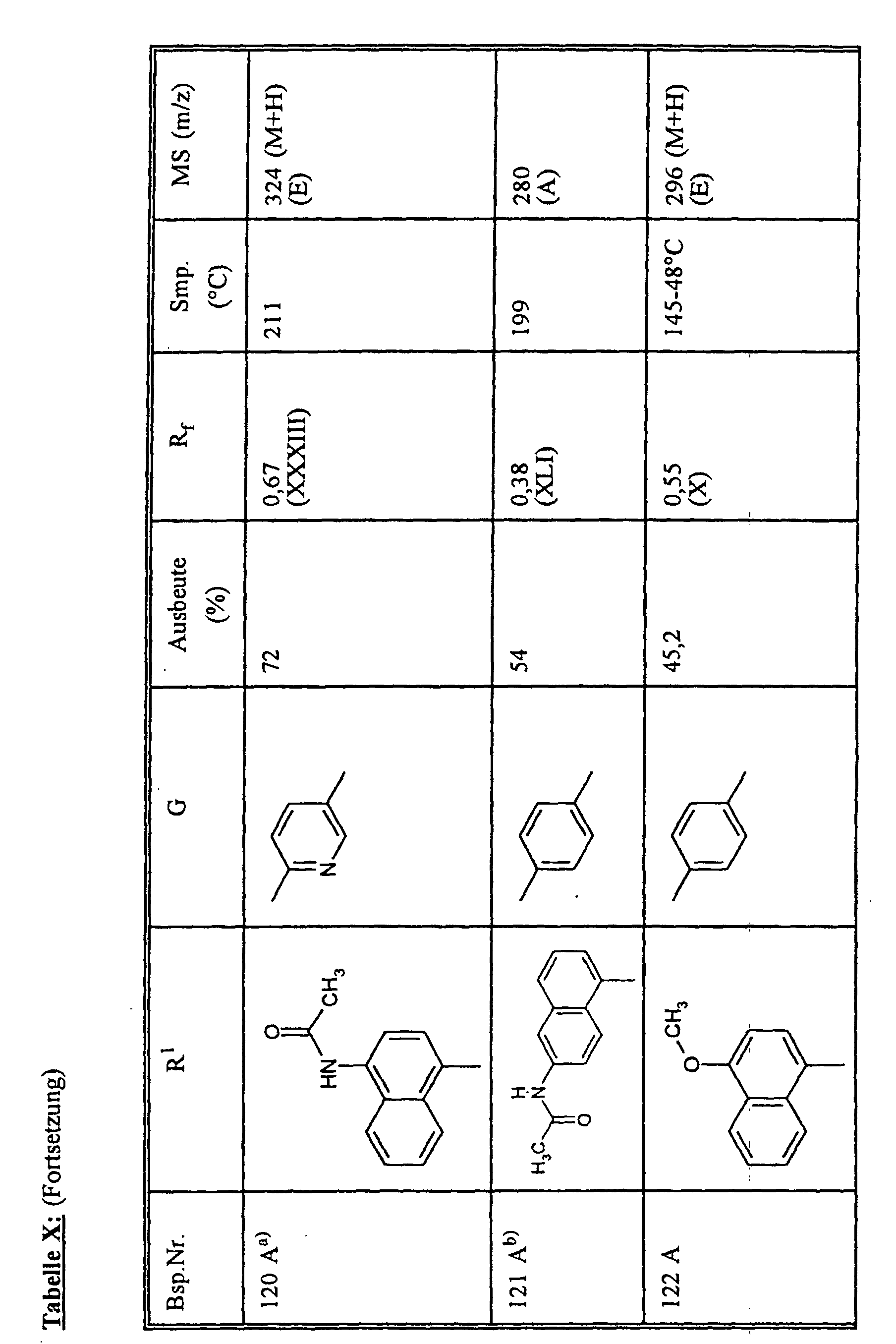

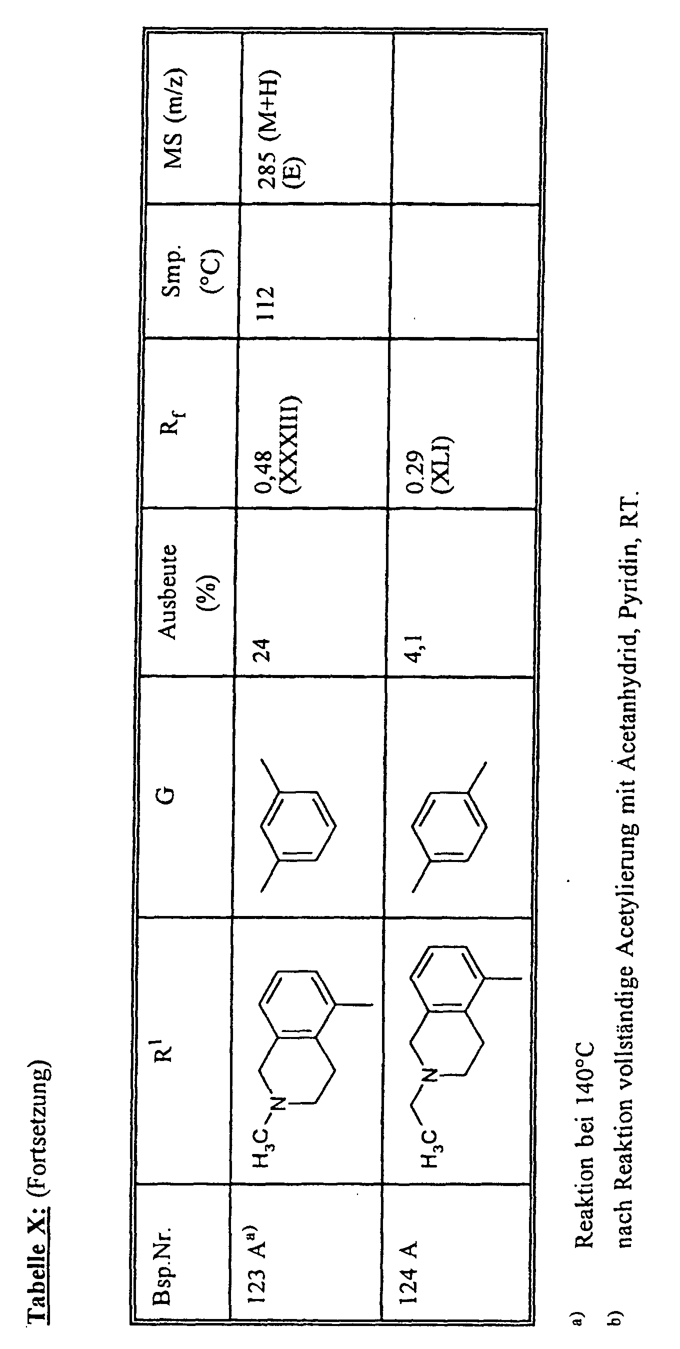

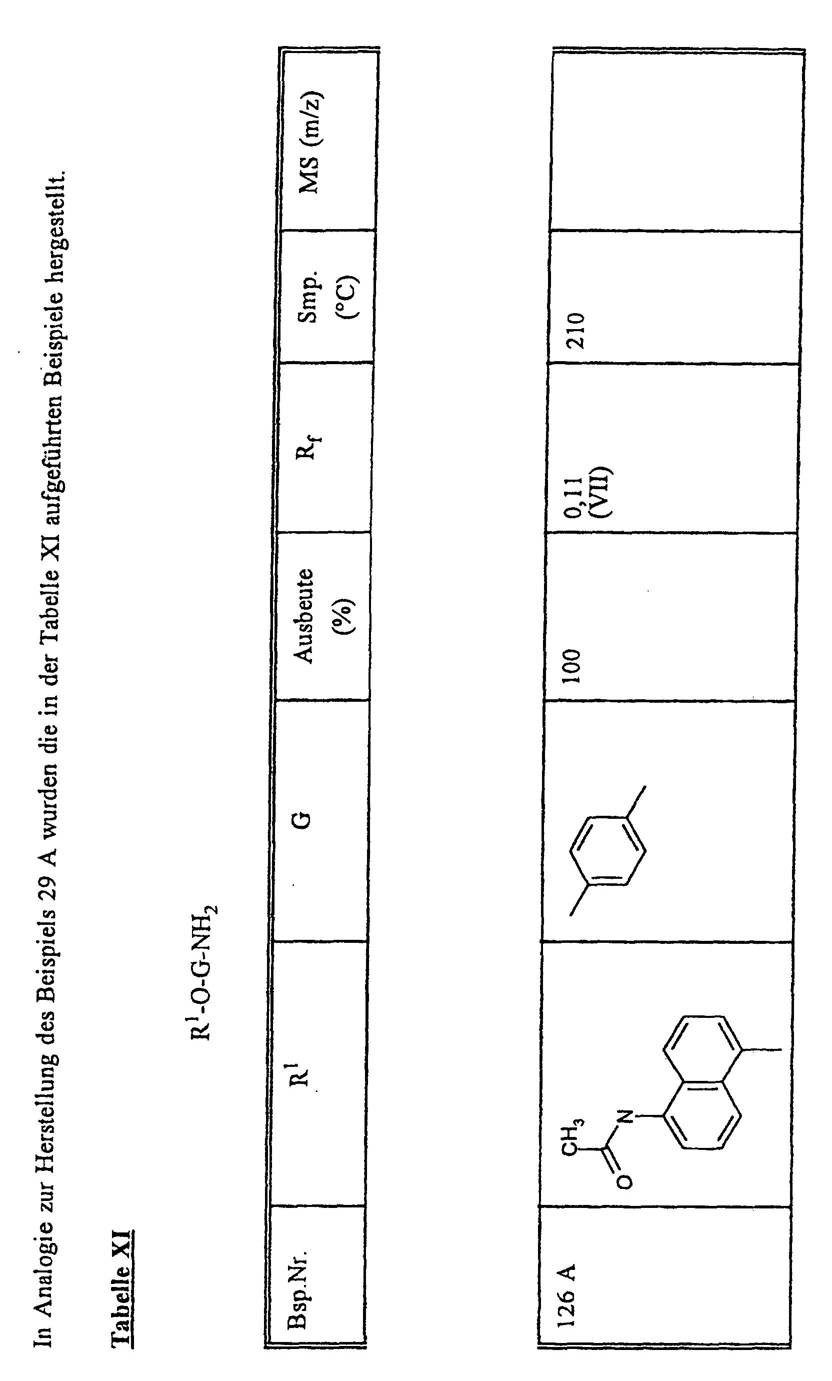

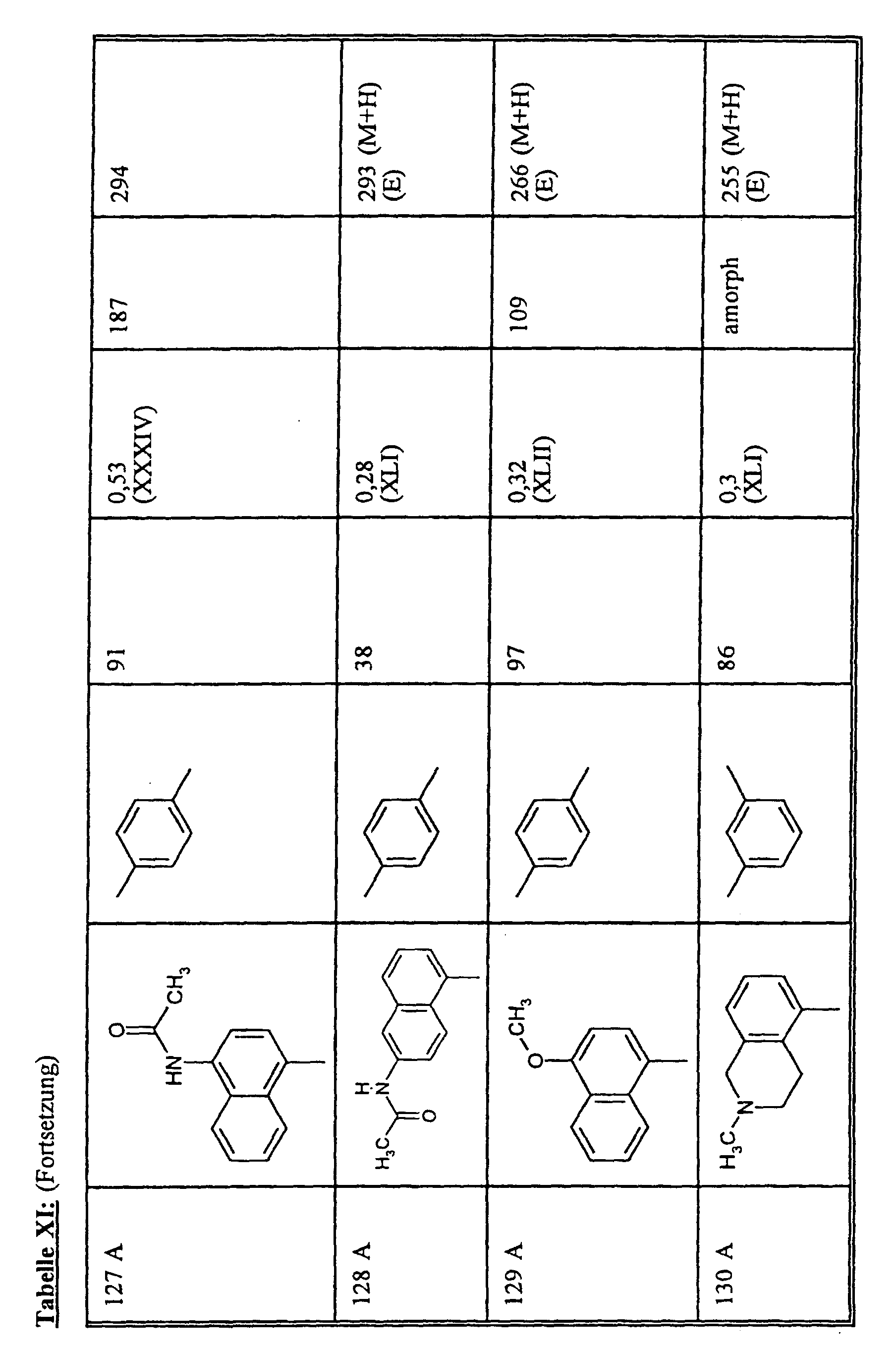

### Beispiel 131 A

### 4-(2-Methyl-1,2,3,4-tetrahydroisochinolin-5-yl-oxy)-phenol-semi-hydrosulfat

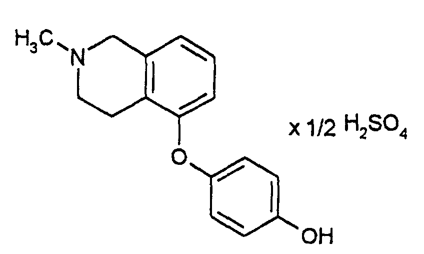

Zu einer Suspension der Verbindung aus Beispiel 108 A (5 g, 19,7 mmol) in 20 %iger Schwefelsäure (200 g) wird eine 5 %ige wäßrige NaNO₂-Lösung (30 ml, 21,7 mmol) innerhalb von 60 min bei einer Temperatur von 3-4°C zugetropft. Anschließend wird überschüssiges Nitrit durch Zugabe von 200 mg Amidschwefelsäure zerstört und der Ansatz 4 h auf 100°C erhitzt. Das Reaktionsgemisch wird auf 3°C abgekühlt, der ausgefallene Niederschlag abfiltriert und mit Isopropanol gewaschen.

Ausbeute: 4,1 g (68 % d.Th.)
R_{f} = 0,28 (XXXIII)
Smp.: 207°C
MS (DCI, Isobutan): m/z = 256 (M+H)

### Beispiel 132 A

### 5-Hydroxy-naphthalin-2-carbonsäuremethylester

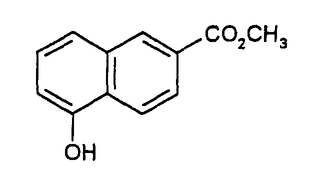

5-Methoxy-2-naphthoesäure (49,7 g; 0,246 mol, J. Med. Chem. 1993, 36, 2485) in Eisessig (450 ml) und in 48 %iger wäßriger Bromwasserstofflösung (450 ml) wird 15 h zum Rückfluß erhitzt. Nach dem Abkühlen wird die Reaktionsmischung im Vakuum eingeengt und nach Zugabe in Wasser wird mit Dichlormethan extrahiert.

Die organische Phase wird mit Wasser gewaschen, über MgSO₄ getrocknet und im Vakuum eingeengt. Der Rückstand wird in MeOH (1,61) gelöst. Die Lösung wird mit Chlorwasserstoff gesättigt (ca. 1 h), wobei sich die Reaktionsmischung auf Rückflußtemperatur erwärmt. Anschließend wird das Lösungsmittel im Vakuum abgezogen, der Rückstand wird in Ethylacetat aufgenommen, mit ges. NaCl-Lösung gewaschen, getrocknet (MgSO₄) und im Vakuum eingeengt. Der Rückstand wird an Kieselgel mit Dichlormethan:Ethylacetat (20:1) chromatographiert. Das so erhaltene Produkt wird mit Dichlormethan/Petrolether verrührt, abgesaugt und im Vakuum getrocknet.

Ausbeute: 31,5 g (63 % d. Th.)
Smp.: 116-117°C
R_{f} = 0,33 (IV)
MS (ESI): m/z = 220 (M + NH₄)

### Beispiel 133 A

### 6-Hydroxymethyl-1-naphthol

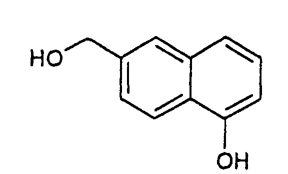

Zur Lösung des Beispiels 132 A (18,2 g; 90 mmol) in THF (500 ml) tropft man eine 1N Lösung von Lithiumaluminiumhydrid in THF (112,5 ml; 112,5 mmol) bei 20-25°C. Nach 3 h versetzt man mit konz. wäßriger NH₄Cl-Lösung (250 ml) und extrahiert mit Ethylacetat (3x). Die vereinten organischen Phasen werden mit konz. wäßriger NH₄Cl-Lösung gewaschen (2x), getrocknet (MgSO₄) und im Vakuum eingeengt. Der Rückstand wird aus Ethylacetat umkristallisiert.

Ausbeute: 11,7 g (75 % d.Th.)
Smp.: 169-170°C
R_{f} = 0,22 (Dichlormethan:Ethylacetat = 10:1
MS (DCI): m/z = 192 (M+NH₄)

### Beispiel 134 A

### 1-Brom-6-hydroxymethyl-naphthalin

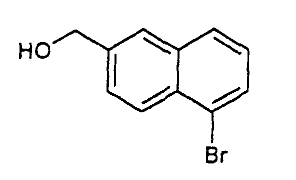

Die Herstellung erfolgte in Analogie zur Herstellung des Beispiels 133 A ausgehend von 5-Brom-naphthalin-2-carbonsäuremethylester (104,7 g, 395 mmol; Aust. J. Chem. 1965, 18, 1351).

Ausbeute: 78,7 g (84 % d.Th.)
R_{f} = 0,52 (VII)
MS (DCI/NH₃): m/z = 254 (M+NH₄)

### Beispiel 135 A

### 4-Hydroxy-2-hydroxymethyl-indan

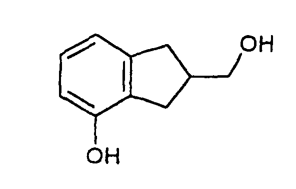

Die Herstellung erfolgte in Analogie zur Herstellung des Beispiels 133 A ausgehend von 4-Hydroxyindan-2-carbonsäureethylester (10,0 g; 48,5 mmol; EP 425 946).

Ausbeute: 7,0 g (84 % d.Th.)
Smp.: 101°C
R_{f} = 0,33 (VII)
MS (DCI/NH₃: m/z = 224 (M+NH₄)

### Beispiel 136 A

### 4-(1-Naphthyloxy)-pyridin

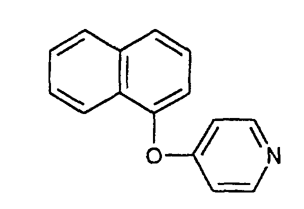

Eine Suspension von 1-Naphthol (24,00 g; 166,5 mmol), 4-Chlorpyridin-Hydrochiorid (24,97 g; 166,5 mmol) und Kaliumcarbonat (46,02 g; 332,9 mmol) wird in Pyridin (200 ml) mit Argon deoxygeniert. Anschließend wird Kupfer-(II)-oxid (26,48 g; 332,9 mmol) zugegeben und die Reaktionsmischung wird über Nacht unter Rückfluß unter Argon gerührt. Anschließend wird das Pyridin im Vakuum abgezogen, der Rückstand wird in Dichlormethan aufgenommen, mit Wasser gewaschen, getrocknet (Na₂SO₄) und im Vakuum eingeengt. Der Rückstand wird an Kieselgel mit Toluol: EE (10:1) chromatographiert. Das so erhaltene Produkt wird in Diethylether verrührt, abgesaugt und im Vakuum getrocknet.

Ausbeute: 6,80 g (18 % d.Th.)
Smp.: 85-86°C
R_{f} = 0,29 (VII)
MS (DCI/NH₃): m/z = 222 (M+H)

### Beispiel 137 A

### 4-(1-Naphthyloxy)-pyridin-N-oxid

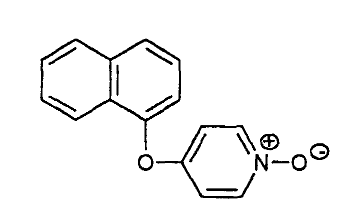

Eine Lösung von Beispiel 136 A (6,62 g; 29,9 mmol) in Dichlormethan (40 ml) wird mit m-Chlorperbenzoesäure, 80 %ig (7,10 g; 32,9 mmol), versetzt, 24 h bei Raumtemperatur gerührt und anschließend noch 2 h zum Rückfluß erhitzt. Die Reaktionsmischung wird zweimal mit ges. wäßriger NaHCO₃-Lösung gewaschen. Die vereinten wäßrigen Phasen werden mit Dichlormethan extrahiert und die vereinten Dichlormethan-Phasen werden getrocknet (Na₂SO₄) und im Vakuum eingeengt. Der Rückstand wird aus Dichlormethan/Petrolether kristallisiert.

Ausbeute: 3,85 g (54 % d.Th.)
Smp.: 128°C
MS (ESI): m/z = 260 (M+Na)

### Beispiel 138 A

### 2-Chlor-4-(1-naphthyloxy)-pyridin

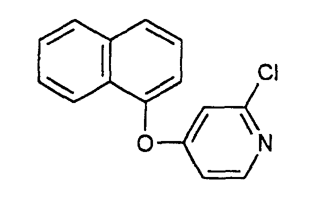

Eine Suspension von Beispiel 137 A (4,50 g; 19,0 mmol) in Phosphorylchlorid (50 ml) wird innerhalb von 1,5 h auf Rückflußtemperatur erhitzt und über Nacht bei dieser Temperatur gerührt. Das Phosphorylchlorid wird im Vakuum abgezogen, der Rückstand wird mit Eiswasser versetzt und mit Dichlormethan extrahiert. Die organische Phase wird mit ges. NaHCO₃-Lösung gewaschen, getrocknet (Na₂SO₄) und im Vakuum eingeengt. Der Rückstand wird an Kiegelgel mit Toluol:EE (5:1) chromatographiert.

Ausbeute: 2,99 g (60 % d.Th.)
R_{f} = 0,58 (IV)
MS (ESI): m/z = 256 (M+H)

### Beispiel 139 A

### 2-Amino-4-(1-naphthyloxy)-pyridin

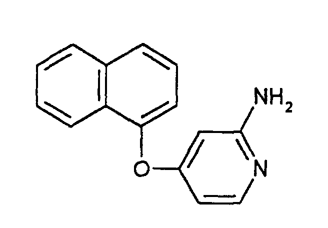

Die Herstellung erfolgte in Analogie zur Herstellung des Beispiels 65 A ausgehend von Beispiel 138 A (2,08 g; 8,13 mmol).

Ausbeute: 1,32 g (69 % d.Th.)
Smp.: 97-99°C
R_{f} = 0,23 (VII)
MS (ESI): m/z = 237 (M+H)

### Beispiel 140 A

### 1-(6-Hydroxymethyl-naphthyl-2-oxy)-3-nitrobenzol

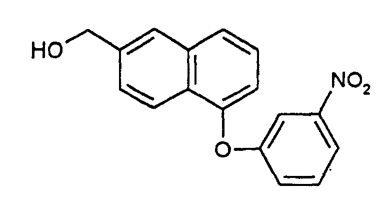

Eine Lösung von Beispiel 133 A (9,40 g; 54,0 mmol) in DMF (200 ml) wird mit Kaliumcarbonat (7,50 g; 54,0 mmol) versetzt, 1 h bei Raumtemperatur gerührt. Nach Zugabe von 3-Fluor-1-nitrobenzol (7,60 g; 54,0 mmol) wird die Reaktionsmischung über Nacht unter Argon bei 155°C (Badtemperatur) gerührt. Anschließend wird das DMF im Vakuum abkondensiert, der Rückstand wird mit Wasser und Ethylacetat (1:1) aufgenommen und filtriert. Nach Phasentrennung wird die wäßrige Phase noch dreimal mit Ethylacetat extrahiert. Die vereinten organischen Phasen werden zweimal mit ges. wäßriger NaCl-Lösung gewaschen, getrocknet (MgSO₄) und im Vakuum einrotiert. Der Rückstand wird an Kieselgel mit Dichlormethan: EE (20:1) chromatographiert.

Ausbeute: 1,75 g (11 % d.Th.)
R_{f} = 0,56 (Dichlormethan:EE = 20:3)
MS (DCI/NH₃): m/z = 313 (M+NH₄)

### Beispiel 141 A

### 3-(6-Methyl-naphthyl-l-oxy)-anilin

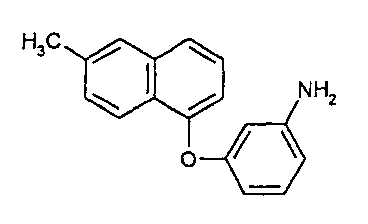

Eine Suspension des Beispiels 140 A (1,94 g; 6,60 mmol) und Palladium auf Aktivkohle, 10 %ig (0,6 g) in THF:MeOH (1:1, 50 ml) wird 3 h bei 3 bar Wasserstoff hydriert. Die Reaktionsmischung wird über Kieselgel filtriert. Das Filtrat wird im Vakuum eingeengt und der Rückstand wird an Kieselgel mit Dichlormethan chromatographiert.

Ausbeute: 1,05 g (64 % d.Th.)
R_{f} = 0,60 (Dichlormethan)
MS (ESI): m/z = 250 (M+H)

### Beispiel 142 A

### 2-(6-Hydroxymethyl-naphthyl-1-oxy)-5-nitro-pyridin

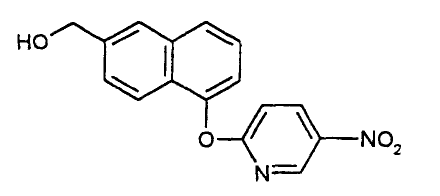

Die Herstellung erfolgte in Analogie zur Herstellung des Beispiels 12 A ausgehend von Beispiel 133 A (10,0 g; 57,4 mmol).

Ausbeute: 15,2 g (88 % d.Th.)
Smp.: 94°C
R_{f} = 0,12 (IV)
MS (ESI): m/z = 297 (M+H)

### Beispiel 143 A

### 5-Amino-2-(6-hydroxymethyl-naphthyl-1-oxy)-pyridin

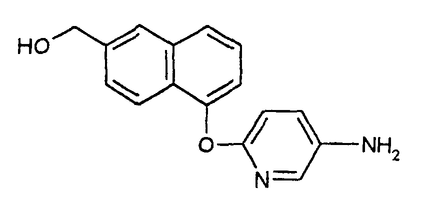

Eine Suspension des Beispiels 142 A (10,3 g; 34,8 mmol) und Platin auf Aktivkohle, 10 %ig (1,0 g) in THF (80 ml) wird 4 h bei Raumtemperatur und 1 bar Wasserstoff hydriert. Die Reaktionsmischung wird über Kieselgur filtriert und im Vakuum eingeengt.

Ausbeute: 9,2 g (89 % d.Th.)
Smp.: 163°C
R_{f} = 0,09 (VII)
MS (ESI): m/z = 267 (M+H)

### Beispiel 144 A

### 3-(6-Methoxymethyl-naphthyl-1-oxy)-anilin

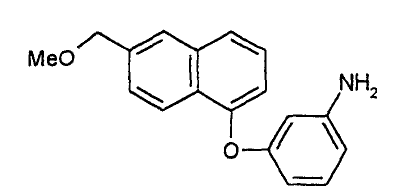

Zu Natriumhydrid, 60 %ig in Paraffinöl (0,152 g; 3,80 mmol) in THF (5 ml) gibt man bei 50°C (Badtemperatur) Jodmethan (0,853 g; 6,01 mmol), tropft dann eine Lösung des Beispiels 140 A (0,901 g; 3,05 mmol) in THF (10 ml) innerhalb von 15 min zu und läßt noch 10 min bei 50°C rühren. Nach Zugabe von Wasser wird mit Ethylacetat extrahiert. Die organischen Phasen werden zweimal mit ges. wäßriger NaCl-Lösung gewaschen, getrocknet (MgSO₄) und im Vakuum eingeengt. Der Rückstand wird an Kieselgel mit Dichlormethan chromatographiert. Das so erhaltene 1-(6-Methoxymethylnaphthyl-1-oxy)-3-nitrobenzol (0,43 g) wird ohne weitere Reinigung mit Platin auf Aktivkohle, 10 %ig (0,1 g) in THF (15 ml) 3 h bei Raumtemperatur und 1 bar Wasserstoff hydriert. Die Reaktionsmischung wird über Kieselgur filtriert und im Vakuum eingeengt. Der Rückstand wird an Kieselgel mit Dichlormethan:EE (20:1) chromatographiert.

Ausbeute: 0,070 g (7 % d.Th.)
R_{f} = 0,50 (Dichlormethan:EE = 10:1)
MS (EI): m/z = 279 (M)

### Beispiel 145 A

### (R,S)-1-(2-Hydroxymethyl-indanyl-4-oxy)-3-nitrobenzol

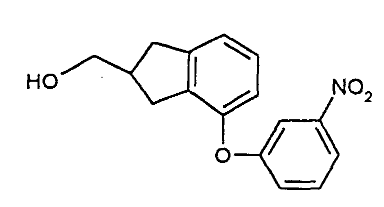

Die Herstellung erfolgte in Analogie zur Herstellung des Beispiels 140 A ausgehend von Beispiel 135 A (60,0 g; 365,4 mmol).

Ausbeute: 34,4 g (32 % d.Th.)
Smp.: 77-79°C
R_{f} = 0,24 (VI)
MS (ESI): m/z = 286 (M+H)

### Beispiel 146 A

### (R,S)-3-(2-Hydroxymethyl-indanyl-4-oxy)-anilin

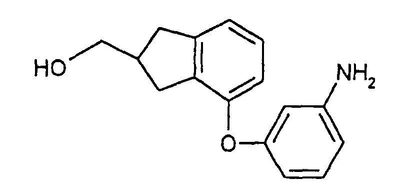

Die Herstellung erfolgte in Analogie zur Herstellung des Beispiels 30 A ausgehend von Beispiel 145 A (4,45 g; 15,60 mmol).

Ausbeute: 3,93 g (97 % d.Th.), Öl
R_{f} = 0,42 (VII)
MS (ESI): m/z = 256 (M+H)

### Beispiel 147 A

### (R,S)-3-(2-Hydroxymethyl-indanyl-4-oxy)-phenol

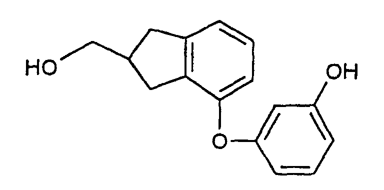

Die Herstellung erfolgte in Analogie zur Herstellung des Beispiels 56 A ausgehend von Beispiel 146 A (3,07 g; 12,0 mmol).

Ausbeute: 1,17 g (38 % d.Th.)
R_{f} = 0,49 (VII)
MS (DCI, NH₃): m/z = 274 (M+NH₄)

### Beispiel 148 A

### 3-(6-Hydroxymethyl-naphthyl-1-oxy)-phenol

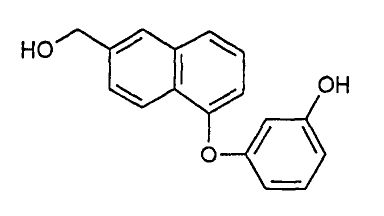

Eine Lösung des Beispiels 134 A (88,9 g; 375 mmol) und 3-Methoxyphenol (88,3 g; 651 mmol) in Pyridin (1000 ml) wird mit Kaliumcarbonat (89,9 g; 651 mmol) versetzt, mit Argon deoxygeniert und unter Argon auf Rückflußtemperatur erhitzt. Nach Zugabe von Kupfer-(II)-oxid (38,8 g; 488 mmol) wird die Reaktionsmischung über Nacht zum Rückfluß erhitzt. Nach dem Abkühlen auf Raumtemperatur wird die Reaktionsmischung filtriert und das Filtrat wird im Vakuum eingeengt. Der Rückstand wird in Ethylacetat aufgenommen, erneut filtriert und das Filtrat wird dreimal mit Wasser gewaschen, getrocknet (MgSO₄) und im Vakuum einrotiert. Der Rückstand wird an Kieselgel mit Dichlormethan:EE (5:2) chromatographiert. Das so erhaltene Gemisch von 3-(6-Hydroxymethylnaphthyl-1-oxy)-anisol (R_{f} = 0,56 (VII)), Beispiel 134 A (R_{f} = 0,51 (VII)) und 3-Methoxyphenol (R_{f} = 0,6 (VII)) im Verhältnis 49 %:32 %:5 % (HPLC) wird in N-Methylpyrrolidon (470 ml) vorgelegt, mit wasserfreiem Natriumsulfid (111,2 g; 1,42 mol) versetzt und 3 h bei 140°C gerührt. Das Reaktionsgemisch wird anschließend in 2N HCl (1000 ml) eingetragen und mit 20 %iger Salzsäure auf pH 2-3 gestellt. Die Mischung wird dann dreimal mit Ethylacetat extrahiert und die vereinten organischen Phasen werden zweimal mit Wasser gewaschen, über MgSO₄ getrocknet und im Vakuum eingeengt.

Der Rückstand wird an Kieselgel mit Toluol:EE (10:3) chromatographiert.

Ausbeute: 8,7 g (9 % d.Th.)
R_{f} = 0,54 (Tol:EE = 5:4)
MS (DCI/NH₃): m/z = 284 (M+NH₄)

### Beispiel 149 A

### 3-(2,3-Dimethylphenyloxy)-anisol

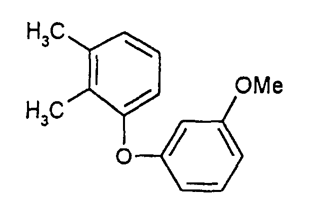

2,3-Dimethyl-1-brombenzol (80,0 g; 0,432 mol), 3-Methoxyphenol (107,3 g; 0,865 mol) und Kaliumcarbonat (119,5 g; 0,865 mol) werden unter Argon in Pyridin (350 ml) vorgelegt und auf 100°C erhitzt. Nach Zugabe von Kupfer-(II)-oxid (51,6 g; 0,648 mol) wird der Ansatz bei 140°C gerührt. Nach 15 h und 40 h wird nochmals 2,3-Dimethyl-1-brombenzol (80,0 g; 0,432 mol nach 15 h und 66,0 g; 0,357 mol nach 40 h) zugegeben. Nach 64 h wird der Ansatz im Vakuum eingeengt, der Rückstand in Ethylacetat aufgenommen und mit halbkonz. Salzsäure auf pH 2-3 eingestellt. Nach Phasentrennung wird die organische Phase mit ges. NaCl-Lösung gewaschen, getrocknet (Na₂SO₄) und im Vakuum einrotiert. Der Rückstand wird an Kieselgel mit Tol:EE = 5:1 chromatographiert.

Ausbeute: 94,9 g (36 % d.Th.)
R_{f} = 0,76 (Toluol)
MS (DCI, NH₃): m/z = 246 (M+NH₄)

### Beispiel 150 A

### 3-(2,3-Dimethylphenyloxy)-phenol

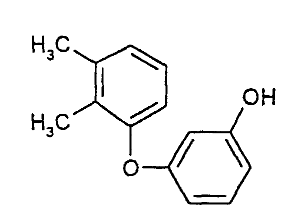

Beispiel 149 A (109,6 g; 480 mmol) wird in 48 % wäßrigem Bromwasserstoff (900 ml) und Essigsäure (1500 ml) vorgelegt und über Nacht unter Rückfluß gerührt. Anschließend wird der Ansatz im Vakuum eingeengt, der Rückstand in rührt. Anschließend wird der Ansatz im Vakuum eingeengt, der Rückstand in Wasser aufgenommen und dreimal mit Ethylacetat extrahiert. Die vereinten organischen Phasen werden zweimal mit Wasser gewaschen, getrocknet (MgSO₄) und im Vakuum eingeengt. Der Rückstand wird an Kieselgel mit Toluol:EE (10:1) chromatographiert.

Ausbeute: 86,5 g (83 % d.Th.)
R_{f} = 0,15 (Toluol)
MS (ESI): m/z = 215 (M+H)

### Beispiel 151 A

### Thiocyansäure-4,4,4-trifluorbutylester

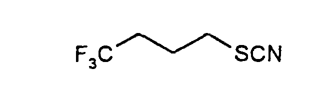

Eine gerührte Lösung von 4,4,4-Trifluorbutanol (35 g; 0,027 mol) und Triethylamin (28,3 g; 0,280 mol) in 200 ml Dichlormethan wurde bei 0°C tropfenweise mit einer Lösung von Methansulfonsäurechlorid (32,1 g; 0,280 mol) in 100 ml Dichlormethan versetzt. Nach Ende der Zugabe wurde weitere 30 min gerührt, dann auf Eis gegossen und anschließend die Phasen getrennt. Die organische Phase wurde über Magnesiumsulfat getrocknet und unter vermindertem Druck aufkonzentriert. Es wurden 55 g rohes 4,4,4-Trifluorbutyl-methansulfonat als oranges Öl erhalten.

Das Mesylat (55 g) wurde mit Natriumthiocyanat (30,6 g; 0,30 mol) in Aceton (300 ml) 6 h unter Rückfluß gekocht. Nach Abkühlung auf Raumtemperatur wurde die Mischung auf Eis gegossen, die Phasen getrennt und die organische über Magnesiumsulfat getrocknet. Nach Filtration und Aufkonzentrieren unter vermindertem Druck wurden 41 g (89 % d.Th.) Thiocyansäure-4,4,4-trifluörbutylester als Öl erhalten.
¹⁹F-NMR (376 MHz, CDCl₃; CFCl₃) δ [ppm]: -66,3
¹H-NMR (400 MHz, CDCl₃, TMS) δ [ppm]: 2,15 (m, 2H); 2,3 (m, 2H); 3,05 (t, J = 7,1 Hz, 2H)

Analog Beispiel 151A wurden die in der Tabelle XII aufgeführten Verbindungen hergestellt.

**Tabelle XII**

| R⁵¹-CF₂-CR⁴⁹R⁵⁰-U-CH₂-CH₂-SCN | | | | | |
|---|---|---|---|---|---|
| Bsp.Nr. | U | R⁴⁹ | R⁵⁰ | R⁵¹ | Ausbeute [%] |
| 152 A | O | H | H | F | 91,5 |
| 153 A | O | CF₃ | H | F | 94 |
| 154 A | CH₂ | F | F | F | 93 |
| 155 A | - | Cl | F | Cl | 55 |

### Beispiel 156 A

### 4,4,4-Trifluorbutansulfonsäurechlorid

F₃C-CH₂-CH₂-CH₂-SO₂Cl

In eine Lösung von Beispiel 151 A (40 g; 0,236 mol) in wäßriger Essigsäure (150 ml Essigsäure und 70 ml Wasser) wurde bei 20 bis 40°C Chlor eingeleitet und der Fortschritt der Reaktion gaschromatografisch verfolgt. Als die Chlorierung vollständig war, wurde der Überschuß Chlor mittels Durchleitung eines Stickstoffstromes verdrängt, 200 ml Wasser zugefügt und die Reaktionsmisehung mit Dichlormethan mehrfach extrahiert. Die vereinigten organischen Phasen wurden über Magnesiumsulfat getrocknet, davon abfiltriert und unter vermindertem Druck aufkonzentriert. Man erhielt 44 g (89 % d.Th.) 4,4,4-Trifluorbutansulfonsäurechlorid als gelbes Öl.
¹⁹F-NMR (376 MHz, CDCl₃; CFCl₃) δ [ppm]: -66,65 (t, J = 10 Hz)
¹H-NMR (400 MHz, CDCl₃, TMS) δ [ppm]: 3,8 (m, 2H); 2,35 (m, 4H)

Analog Beispiel 156 A wurden die in Tabelle XIII aufgeführten Verbindungen hergestellt.

**Tabelle XIII**

| R⁵¹-CF₂-CR⁴⁹R⁵⁰-U-CH₂-CH₂-SO₂-Cl | | | | | | |
|---|---|---|---|---|---|---|
| Bsp. Nr. | U | R⁴⁹ | R⁵⁰ | R⁵¹ | NMR-Daten (CDCl₃) ¹⁹F: CFCl₃/¹H: TMS: δ [ppm] | Ausbeute [%] |
| 157 A | O | H | H | F | -74.5 (t, 8Hz)/4,2 (m, 2H); 3,95 (m, 4H) | 87 |
| 158 A | O | CF₃ | H | F | -74,2/4,45 (m, 2H); 4,2 (m, 1H); 3,95 (m, 2H) | 75 |
| 159 A | CH₂ | F | F | F | -74,2 (CF₃); -118 (CF₂)/ 3,8 (m, 2H); 2,4 (m, 4H) | 91 |
| 160 A | - | Cl | F | Cl | -68,5 (2F); -120 (1F) | 60 |

### Herstellungsbeispiele

### Beispiel 1 (Methode A)

### 1-N-(1-Butylsulfonyl)amino-4-(naphthyl-1-oxy)benzol

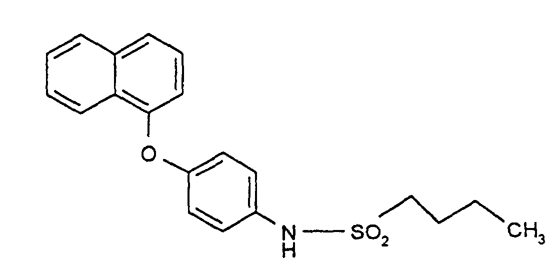

Zur Lösung des Beispiels 51 A (17,0 g; 72,3 mmol) in Dichlormethan (300 ml) tropft man bei RT unter Argon eine Lösung von n-Butylsulfonylchlorid (9,5 ml; 72,0 mmol) in Dichlormethan (100 ml) und läßt 1 h bei RT rühren. Nach Zugabe von Pyridin (11,7 ml, 140 mmol) läßt man über Nacht bei RT rühren. Die Reaktionsmischung wird nacheinander gewaschen mit Wasser, 1 N Salzsäure (2x), Wasser (2x), getrocknet (Na₂SO₄) und im Vakuum eingeengt. Der Rückstand wird heiß aus Ethanol umkristallisiert und anschließend in Dichlormethan gelöst. Nach Zugabe von Aktivkohle wird filtriert, im Vakuum eingeengt und aus Methanol umkristallisiert.
Ausbeute: 12,7 g (49% d.Th.)
Smp.: 108-109°C
R_{f} = 0,32 (IV)
M_{S} (DCI, NH₃): m/z = 373 (M+NH₄)

### Beispiele 2 und 3 (Methode B)

### 3-(Naphthyl-1-oxy)-1-N-(1-propylsulfonyl)-aminobenzol (Beispiel 2)

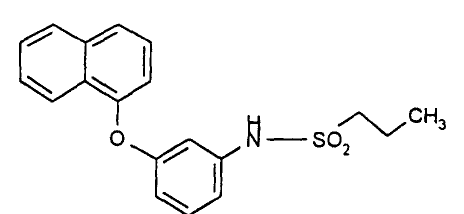

### 3-(Naphthyl-1-oxy)-1-Bis-N-(1-propylsulfonyl)aminobenzol (Beispiel 3)

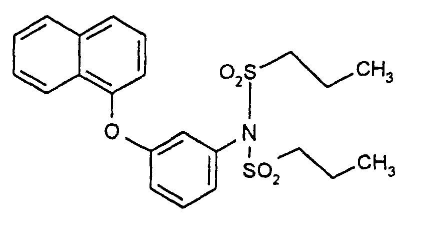

Zur Lösung von Beispiel 45 A (353 mg; 1,50 mmol) in Dichlormethan (10 ml) tropft man bei RT unter Argon 1-Propansulfonylchlorid (224 mg; 1,57 mmol) und Triethylamin (304 mg; 3,00 mmol) und läßt über Nacht bei RT rühren. Nach Zugabe von Dichlormethan (40 ml) wird mit Wasser (50 ml), 2 N Salzsäure (2 x 50 ml), 5%ige Schwefelsäure (70 ml) und Wasser (50 ml) gewaschen. Die organische Phase wird über Na₂SO₄ getrocknet und im Vakuum eingeengt. Der Rückstand wird an Kieselgel mit Dichlormethan : Ameisensäure (200:1) chromatographiert.
Ausbeute (Beispiel 2): 259 mg (51% d.Th.)
R_{f} = 0,40 (XV)
MS (DCI, NH₃): m/z = 359 (M+NH₄)
Ausbeute (Beispiel 3): 111 mg (16% d.Th.)
Smp.: 112°C
R_{f} = 0,48 (XV)
MS (DCI, NH₃): m/z = 465 (M+NH₄)

In Analogie zur Herstellung des Beispiels 1 (Methode A) und der Beispiele 2 und 3 (Methode B) werden die in Tabelle 1 aufgeführten Beispiele hergestellt:

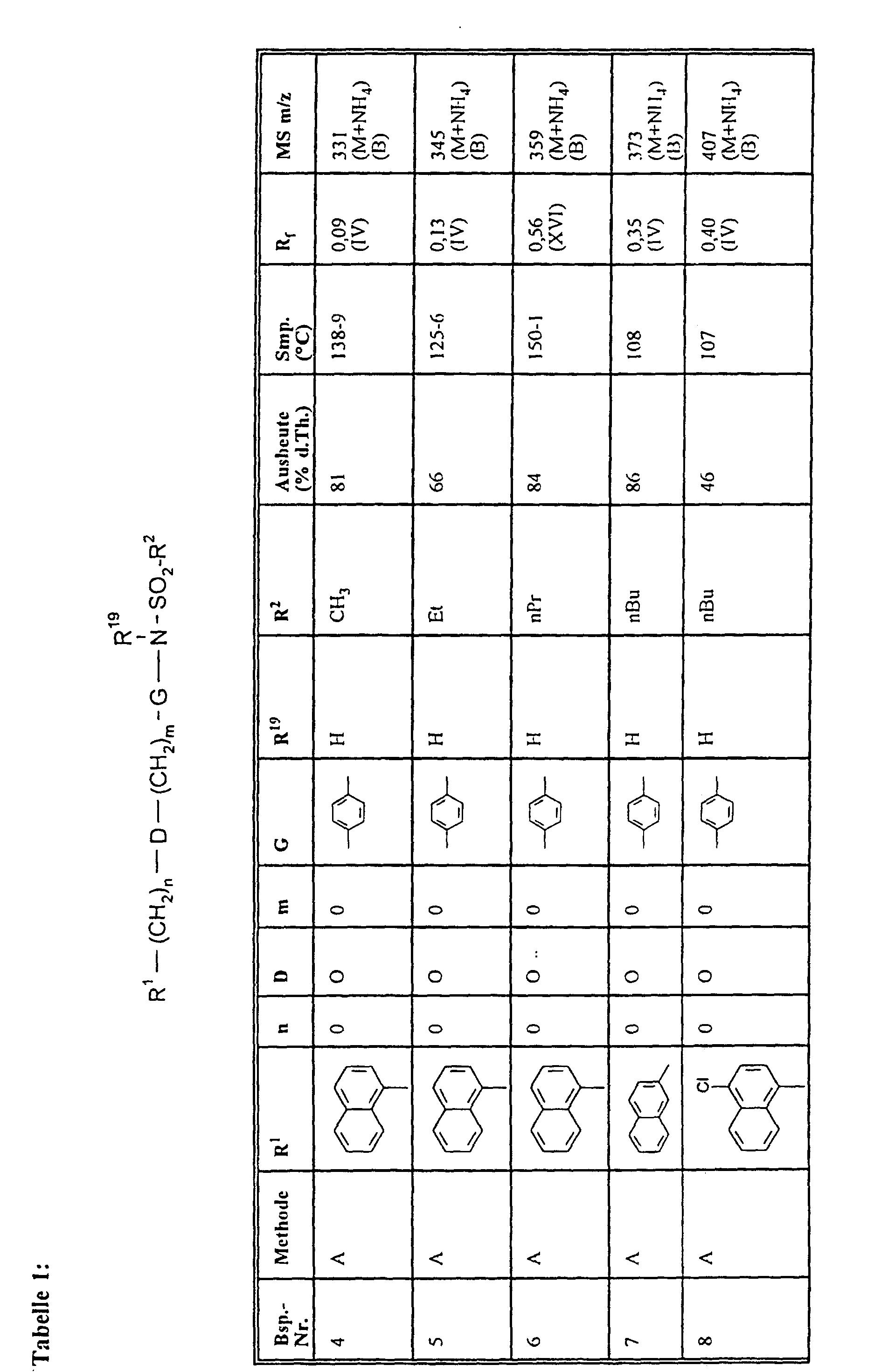

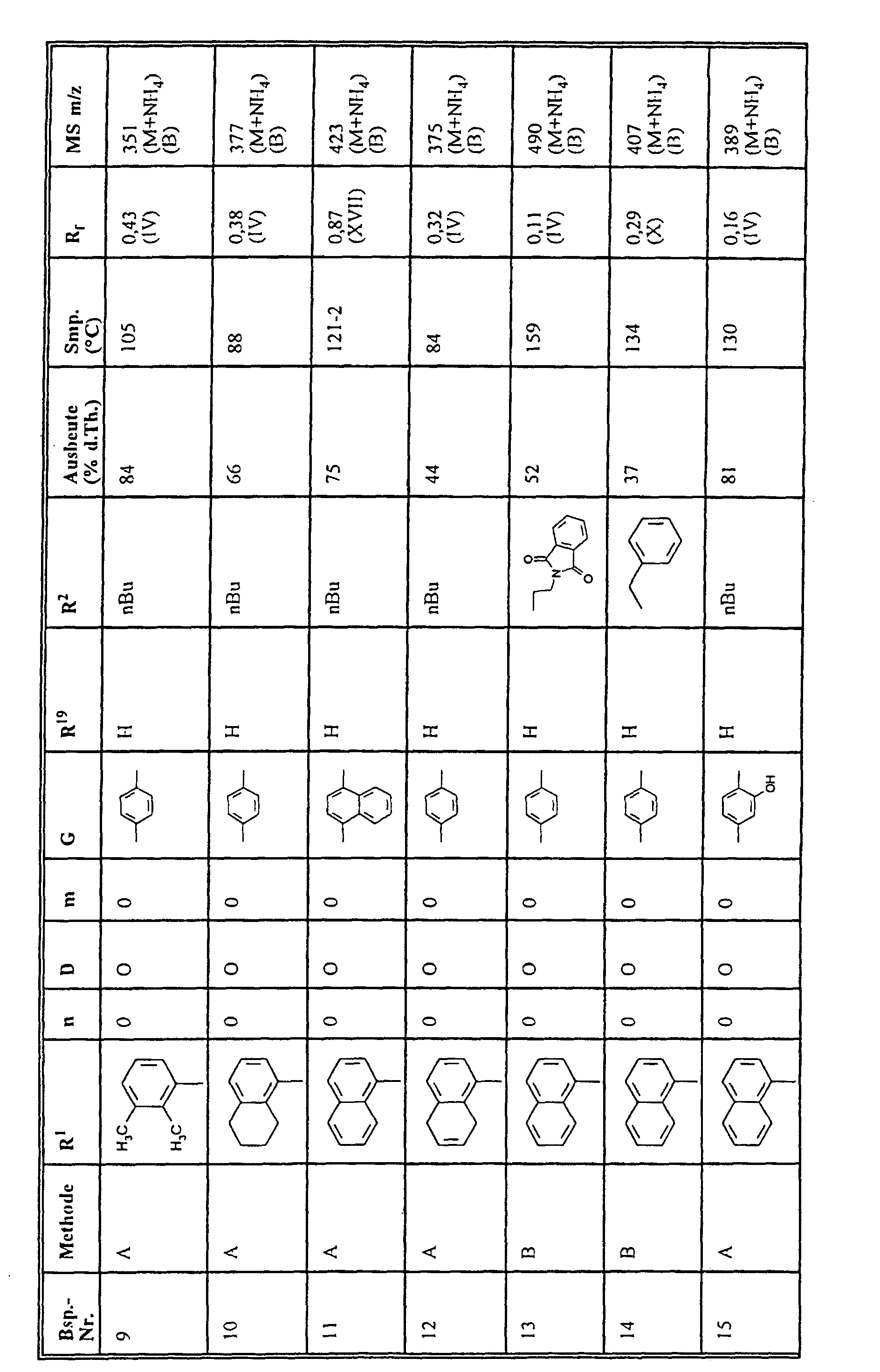

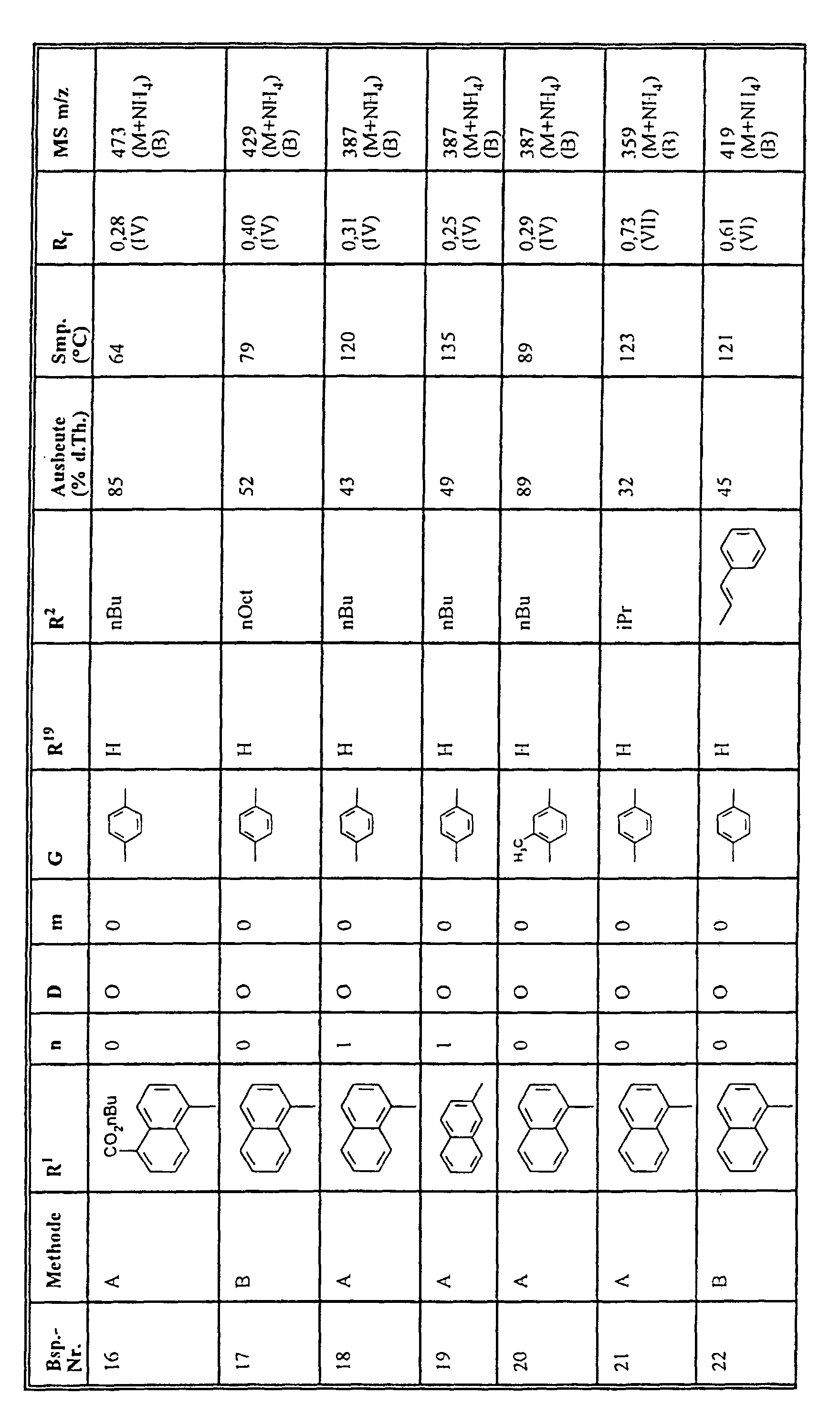

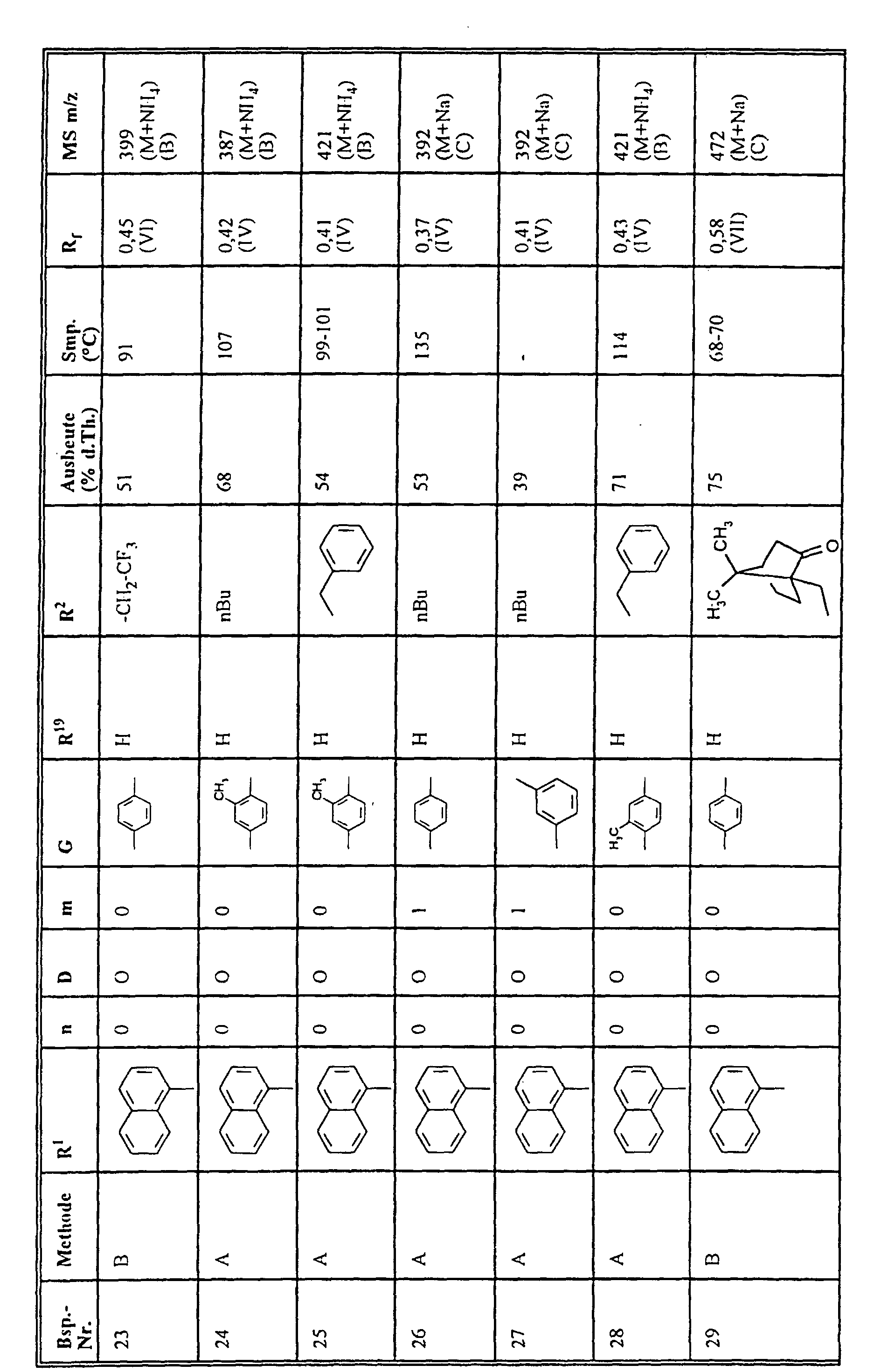

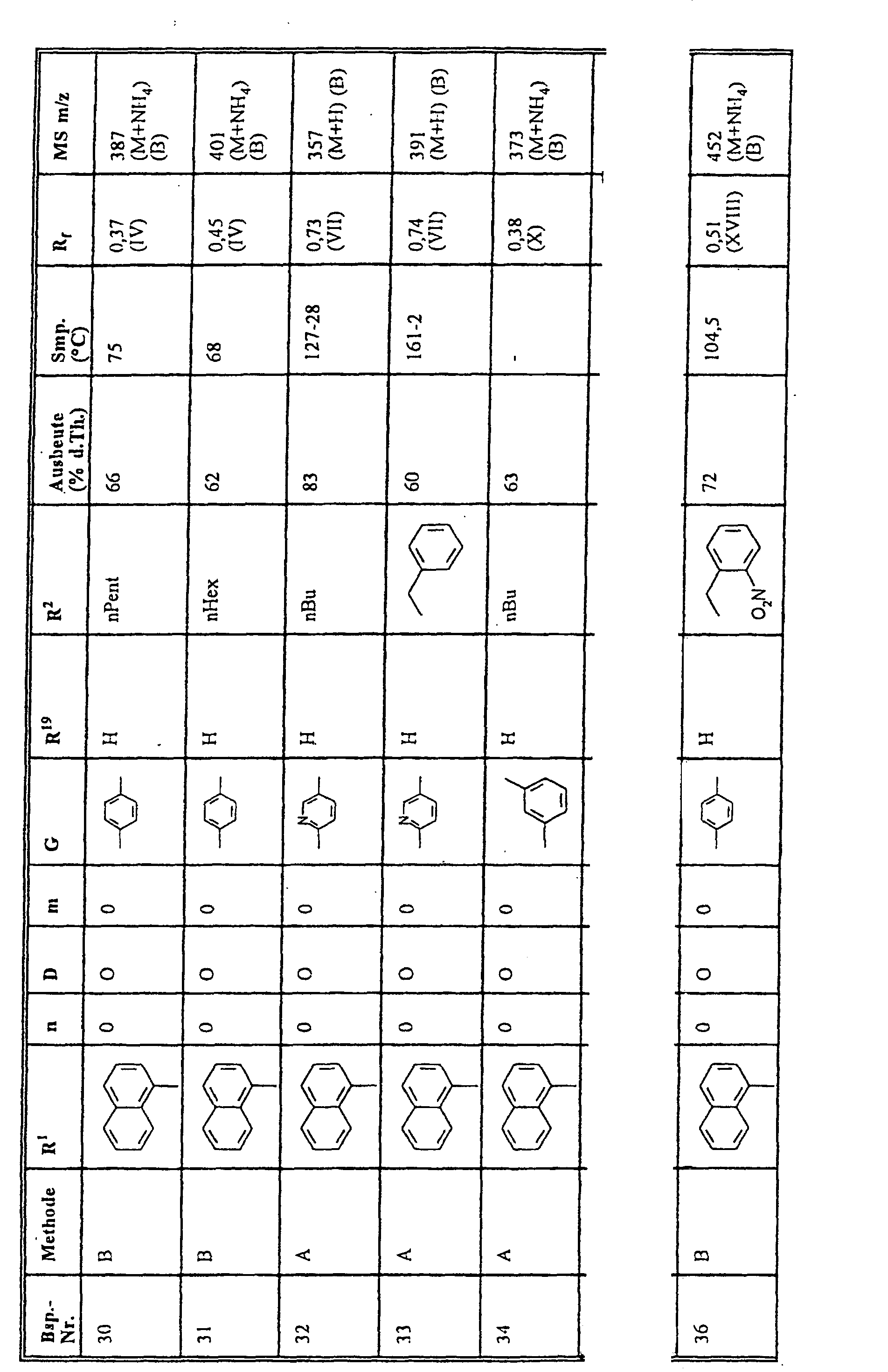

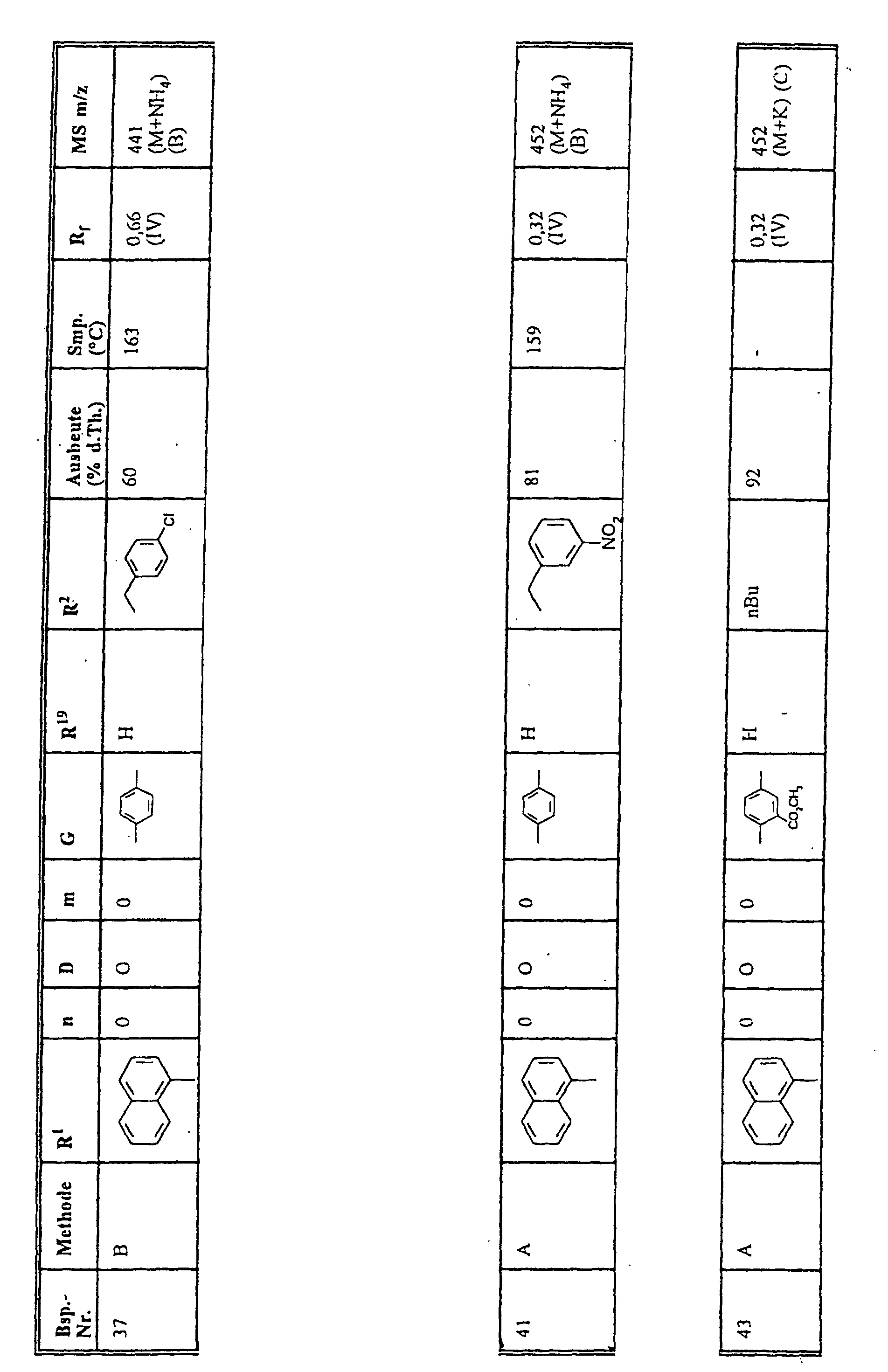

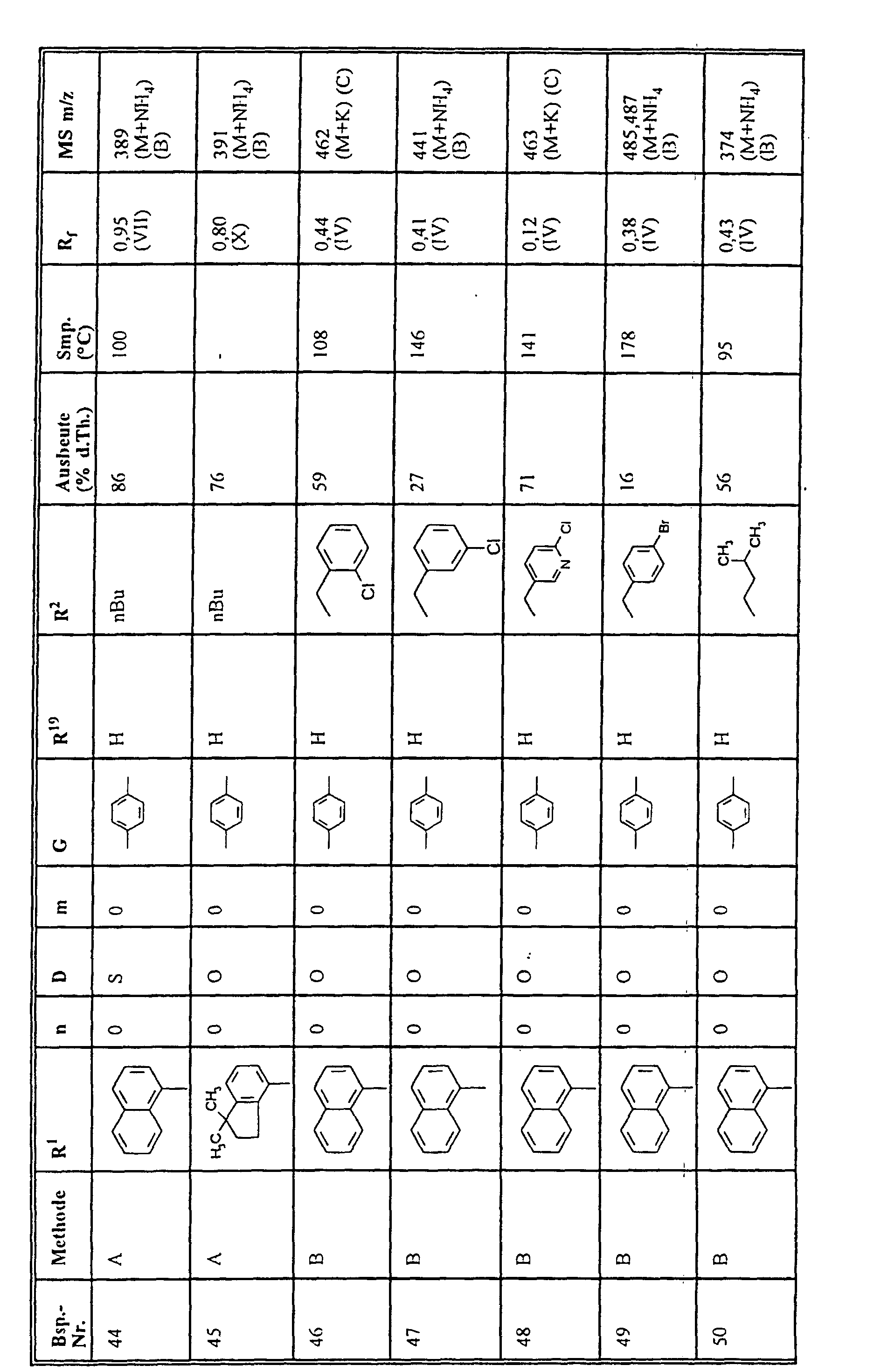

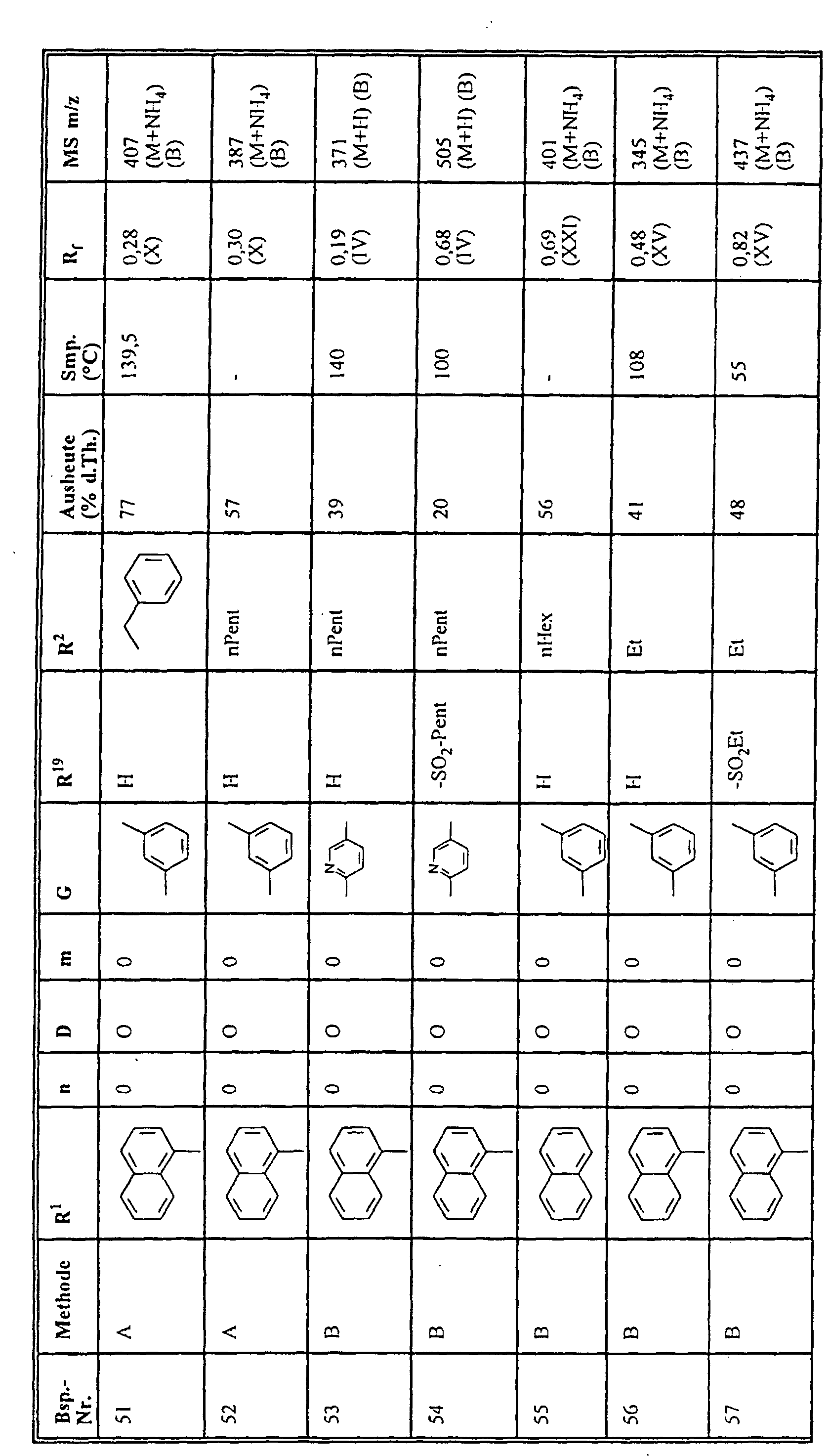

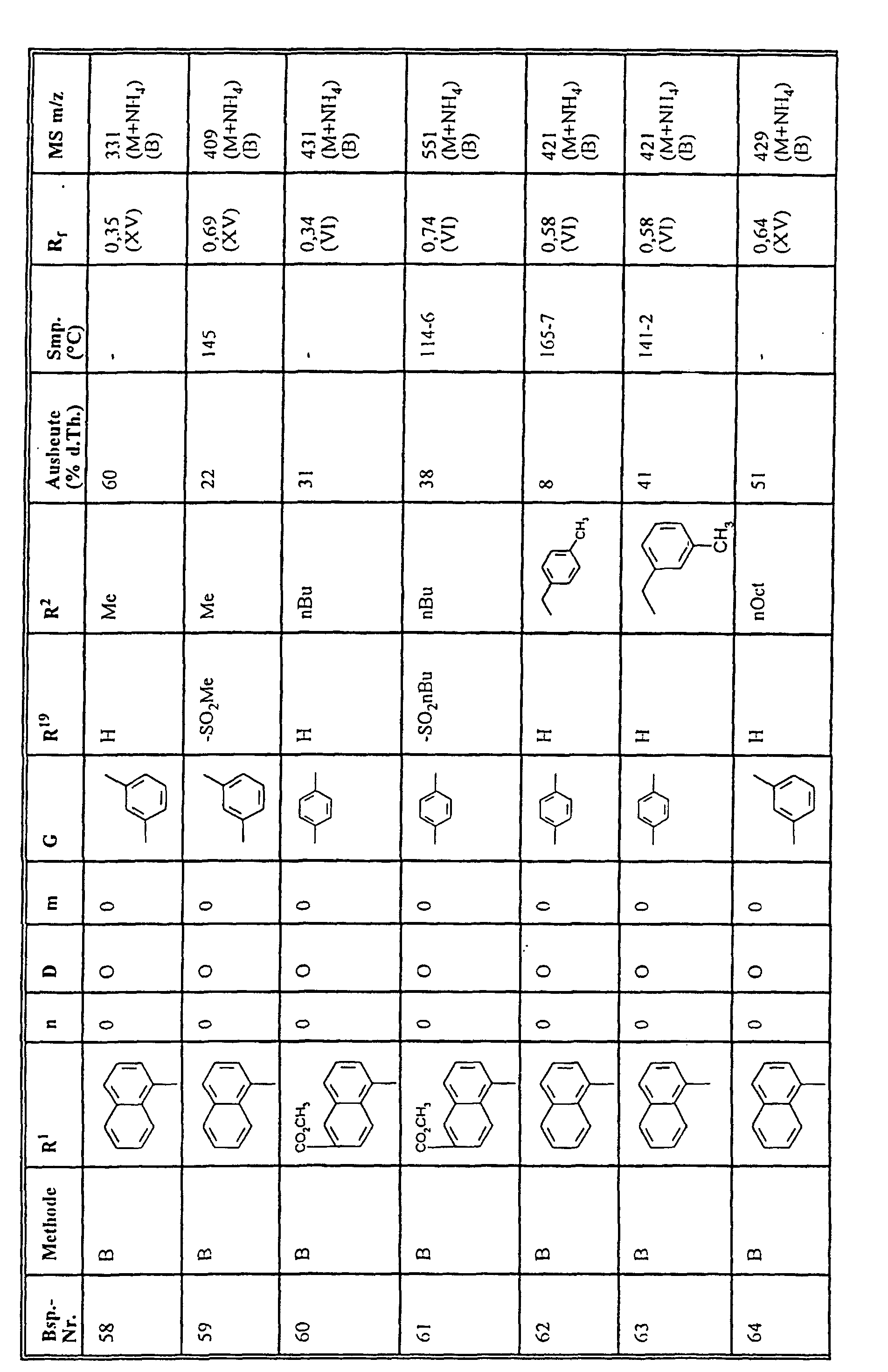

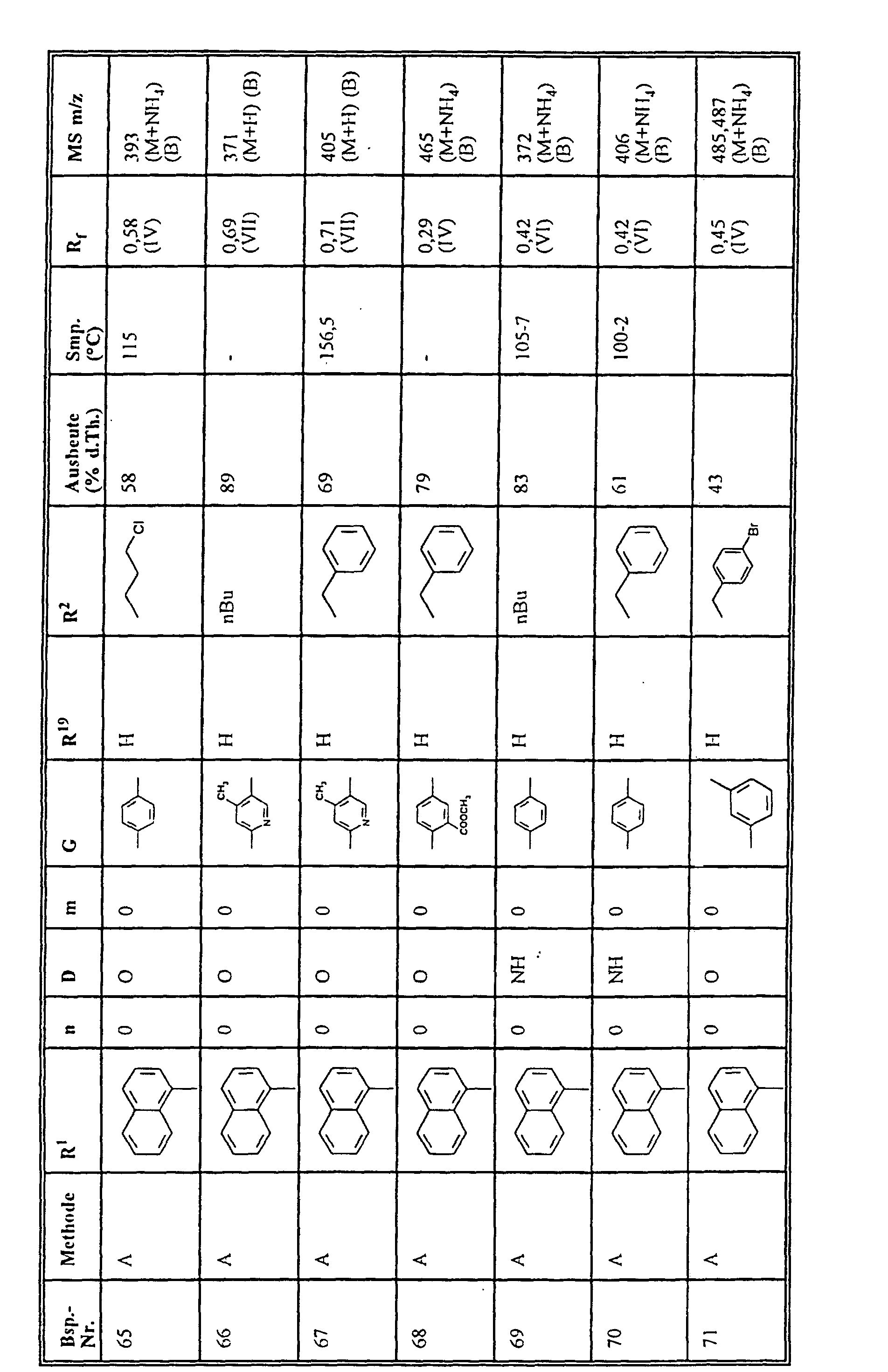

### Beispiele 72 und 73

### 1-N-[1-(Methyl)butylsulfonyl]amino-4-(naphthyl-1-oxy)benzol (Beispiel 72)

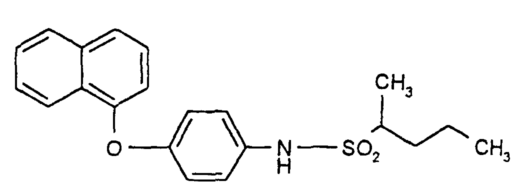

### 1-N-[1-(1,1-Dimethyl)butylsulfonyl]amino-4-(naphthyl-1-oxy)benzol (Beispiel 73)

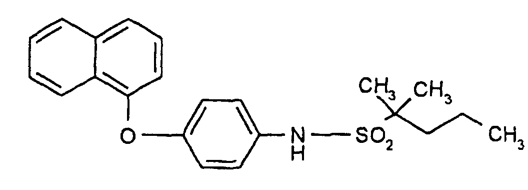

Zur Lösung des Beispiels 1 (500 mg, 1,40 mmol) in THF (15 ml) tropft man unter Argon bei -70°C bis -78°C n-Butyllithium, 1,6 N in Hexan (1,84 ml; 2,94 mmol) und läßt 2 h bei -20°C bis -30°C rühren. Das Reaktionsgemisch wird auf -70°C bis -78°C abgeühlt und eine Lösung von Jodmethan (199 mg; 1,40 mmol) in THF (5 ml) wird bei dieser Temperatur zugetropft. Man läßt 1 h bei -70°C bis -78°C nachrühren und läßt den Ansatz auf RT erwärmen. Nach Zugabe von 1 N Salzsäure (10 ml) wird mit Ethylacetat (30 ml) verdünnt und geschüttelt. Nach Phasentrennung wird die wäßrige Phase mit Ethylacetat (2 x 20 ml) extrahiert. Die vereinigten organischen Phasen werden mit 5%iger wäßriger Natriumthiosulfatlösung (2 x 20 ml) und mit Wasser (3 x 40 ml) gewaschen, getrocknet (MgSO₄) und im Vakuum eingeengt. Der Rückstand (442 mg) wird in THF (10 ml) gelöst und nach Zugabe von Beispiel 1 (60,0 mg, 0,17 mol) wird bei -70°C bis -78°C unter Argon n-Butyllithium, 1,6 N in Hexan (1,8 ml; 2,94 mmol) zugetropft. Anschließend wird das Reaktionsgemisch 2 h bei 0°C gerührt, auf -70°C bis -78°C abgekühlt und eine Lösung von Jodmethan (199 mg; 1,40 mmol) in THF (5 ml) wird zugetropft. Nach einer Nachrührzeit von 1 h bei -70°C bis -78°C wird der Ansatz auf RT erwärmt und wie oben beschrieben aufgearbeitet. Das Rohprodukt (523 mg) besteht aus einem Gemisch der Beispiele 72, 73 und 1 im Verhältnis 66:18:16. Die Abtrennung der Verbindungen 72 und 73 aus diesem Gemisch gelingt durch präparative HPLC (Säule: 250 x 20 mm gefüllt mit Kromasil 100, C-18, 5 µm; Fluß: 15 ml/Min; Fließmittel: 25% Wasser, 75% Methanol; T = 40°C).
Ausbeute (Beispiel 72): 222 mg (38% d.Th.)
Retentionszeit (HPLC): 7,07 min
MS (DCI, NH₃): m/z = 387 (M+NH₄).
Ausbeute (Beispiel 73): 59 mg (10% d.Th.)
Smp.: 97-98°C
Retentionszeit (HPLC): 8,45 min
MS (DCI, NH₃):m/z = 401 (M+NH₄).

### Beispiel 74

### 5-[4-(n-Butylsulfonyl)aminophenyl-1-oxy]-naphthalin-1-carbonsäure

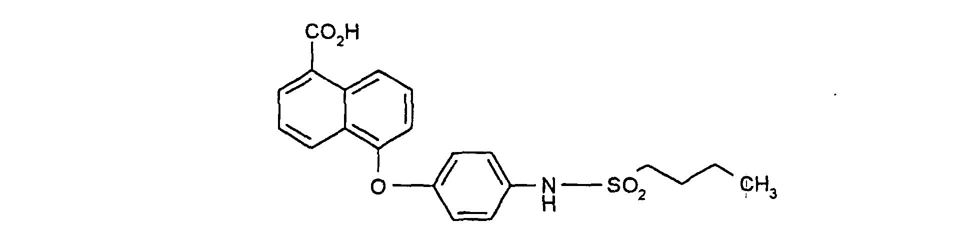

Zur Lösung des Beispiels 16 (4,10 g; 9,0 mmol) in Dioxan (20 ml) tropft man bei RT eine Lösung von Kaliumhydroxid (1,51 g; 27,0 mmol) in Wasser (10 ml) und läßt über Nacht bei RT rühren. Nach Zugabe von Wasser (100 ml) wird mit Ethylacetat (100 ml) extrahiert. Die organische Phase wird verworfen und die wäßrige Phase wird mit 2 N Salzsäure auf pH 3 gestellt. Ausgefallenes Produkt wird abgesaugt, mit Wasser (50 ml) gewaschen und im Vakuum getrocknet. Ausbeute: 3,16 g (88% d.Th.)
Smp.: 193°C
R_{f} = 0,24 (XXII)
MS (DCI, NH₃): m/z = 417 (M+NH₄)

### Beispiel 75

### 5-[N-(n-Butylsulfonyl)amino]-2-(naphthyl-1-oxy)benzoesäure

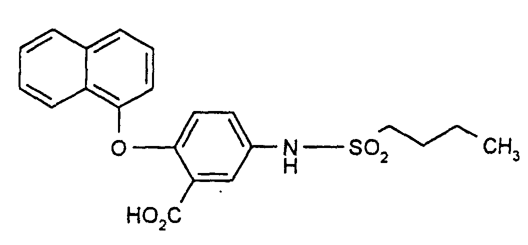

In Analogie zur Herstellung des Beispiels 74 wurde die Titelverbindung ausgehend von Beispiel 43 (3,74 g; 9,4 mmol) hergestellt.
Ausbeute: 3,49 g (93% d.Th.)
Smp.: 162°C
R_{f} = 0,22 (XXII)
MS (DCI, NH₃): m/z = 417 (M+NH₄)

### Beispiel 76

### 1-[N-(n-Butylsulfonyl)amino]-2-methoxy-4-(naphthyl-1-oxy)benzol

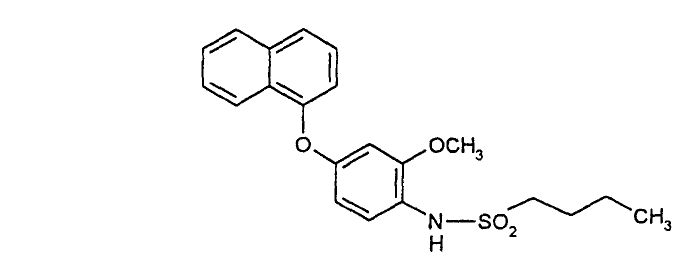

Eine Lösung des Beispiels 15 (463 mg; 1,25 mmol) in Aceton (10 ml) wird bei RT mit K₂CO₃ (345 mg; 2,50 mmol) und nach 10 min mit Jodmethan (177 mg; 1,25 mmol) versetzt. Das Reaktionsgemisch läßt man 48 h bei RT rühren und destilliert das Lösemittel danach im Vakuum ab. Der Rückstand wird in Wasser (50 ml) aufgenommen und mit Ethylacetat (3 x 50 ml) extrahiert. Die vereinten organischen Phasen werden getrocknet (Na₂SO₄) und im Vakuum eingeengt. Das Rohprodukt wird an Kieselgel mit Toluol : Ethylacetat (10:1) chromatographiert. Ausbeute: 180 mg (39% d.Th.)
Smp.: 119°C
R_{f} = 0,35 (IV)
MS (ESI): 424 (M+K)

### Beispiel 77

### 1-[N-(Nonafluorbutylsulfonyl)amino]-4-(naphthyl-1-oxy)benzol

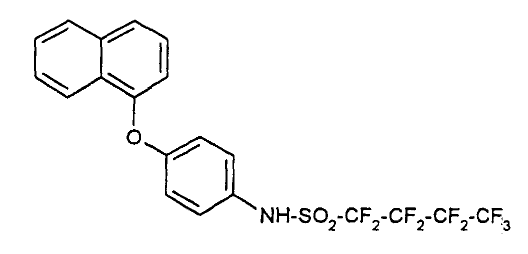

Zur Lösung der Verbindung aus Beispiel 51 A (1,20 g; 5,10 mmol) in THF (20 ml) tropft man unter Argon bei -70°C bis -75°C n-Butyllithium, 1,6 N in Hexan (3,50 ml; 5,61 mmol) und läßt 30 min nachrühren. Die entstandene Reaktionsmischung wird bei -70°C bis -75°C zu einer Lösung von Perfluorbutan-1-sulfofluorid (1,54 g; 5,10 mmol) in THF (20 ml) getropft. Man läßt den Ansatz auf RT erwärmen, zieht das Lösemittel im Vakuum ab und nimmt den Rückstand in Dichlormethan (40 ml) auf. Man wäscht mit 1 N Salzsäure (2 x 40 ml), filtriert über Kieselgur, wäscht mit Wasser (40 ml), trocknet über Na₂SO₄ und zieht das Lösemittel im Vakuum ab. Der Rückstand wird an Kieselgel mit Toluol : Ethylacetat (20:1) chromatographiert.
Ausbeute: 665 mg (25% d.Th.)
Smp.: 75°C
R_{f} = 0,38(X)
MS (FAB): m/z = 517 (M)

### Beispiel 78

### 4-(Naphthyl-1-oxy)-1-[N-(2-phenylethylsulfonyl)amino]benzol

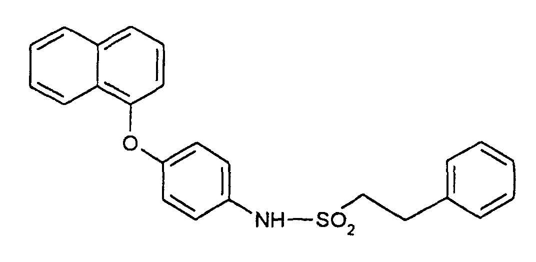

Eine Lösung von Beispiel 22 (630 mg; 1,57 mmol) in Ethanol (30 ml) und THF (20 ml) wird mit 5% Palladium auf Aktivkohle (100 mg) versetzt und 43 h unter 3 bar H₂ hydriert. Nach dem Absaugen über Kieselgur wird das Lösemittel im Vakuum abgezogen und der Rückstand an Kieselgel mit Petrolether : Diethylether (5:1) chromatographiert. Man erhält ein Gemisch der Beispiele 22 und 78 im Verhältnis 1;3:1 (R_{f} = 0,74 (II)), welches in Ethanol (20 ml) aufgenommen wird und erneut nach Zugabe von 5% Palladium auf Aktivkohle (100 mg) bei 40°C und 3 bar H₂ hydriert wird. Die Reaktionsmischung wird über Kieselgur abgesaugt, das Lösemittel wird im Vakuum abgezogen und der Rückstand wird aus Methanol umkristallisiert.
Ausbeute: 260 mg (41% d.Th.)
Smp.: 109,5°C
R_{f} = 0,74 (II)
MS (DCI, NH₃): m/z = 421 (M+NH₄)

### Beispiel 79

### 5-[4-(n-Butylsulfonyl)aminophenyl-1-oxy]-naphthalin-1-carbonsäuremethylester

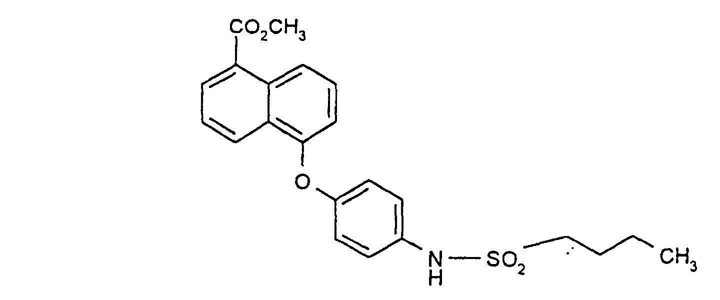

Zur Suspension der Verbindung aus Beispiel 74 (1,25 g; 3,15 mmol) in Dichlormethan (14 ml) gibt man bei -10°C nacheinander Methanol (0,64 ml; 15,8 mmol), 4-N,N-Dimethylaminopyridin (38mg; 0,32 mmol) und N'-(3-Dimethylaminopropyl)-N-ethylcarbodiimid Hydrochlorid (0,66 g; 3,46 mmol) und läßt den Ansatz über Nacht unter Rühren auf RT erwärmen. Nach Zugabe von Dichlormethan wird mit Wasser (50 ml), ges. wäßrige NaHCO₃-Lösung (2x50 ml) und Wasser (50 ml) gewaschen, getrocknet (Na₂SO₄) und im Vakuum eingeengt.

Der Rückstand wird an Kieselgel mit Toluol : Ethylacetat (10:1) chromatographiert.
Ausbeute: 0,94 g (72% d.Th.)
Smp.: 98°C
R_{f} = 0,23 (IV)
MS (DCI, NH₃): m/z = 431 (M+NH₄)

In Analogie zur Herstellung des Beispiels 79 werden die in der Tabelle 2 aufgeführten Beispiele hergestellt:

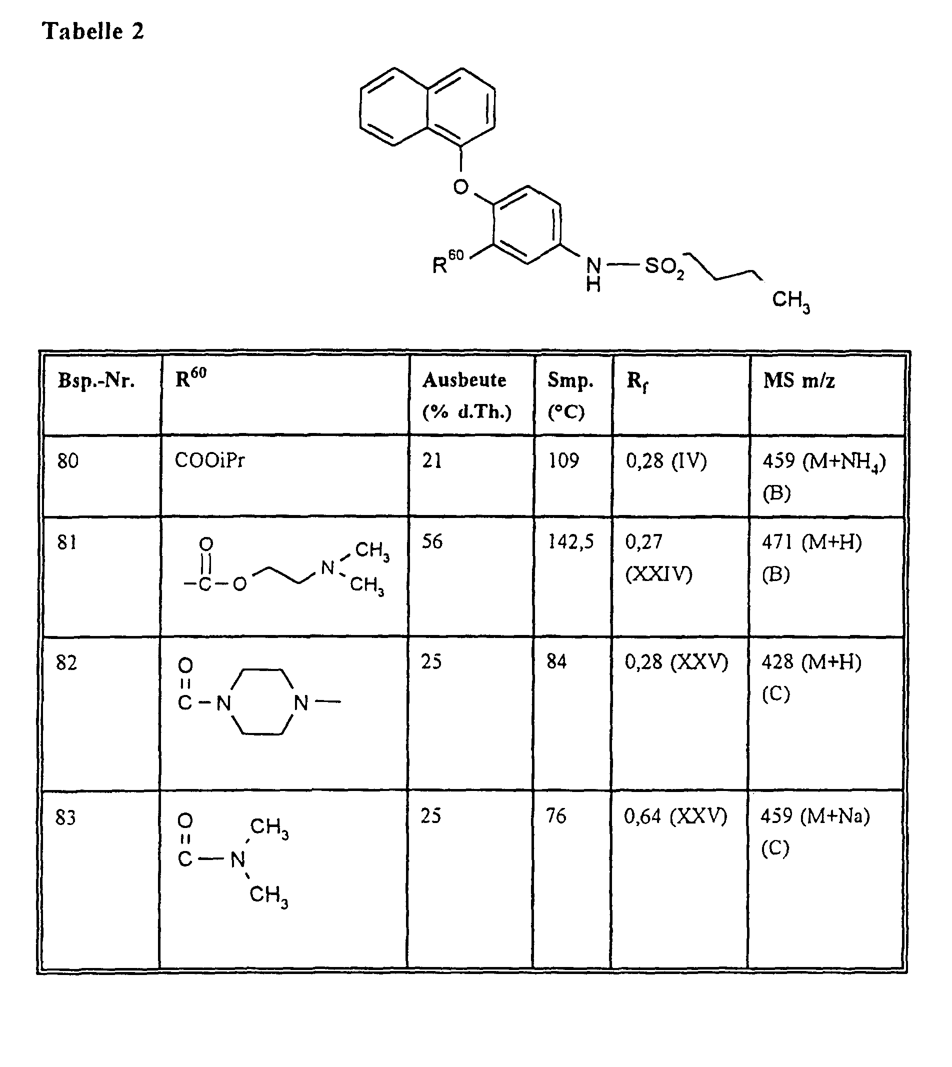

### Beispiel 84

### 5-[N-(n-Butylsulfonyl)amino]-2-(naphthyl-1-oxy)benzoesäureamid

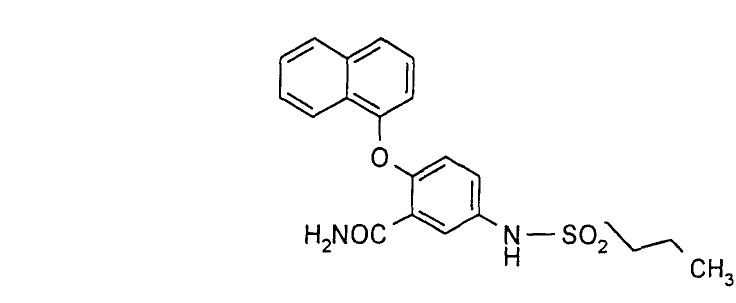

Zur Lösung des Beispiels 75 (799 mg; 2,00 mmol) und N-Methylmorpholin (0,33 ml; 3,00 mmol) in Ethylacetat (10 ml) tropft man bei -15°C unter Argon Isobutylchloroformiat (0,40 ml; 3,00 mmol) und läßt 1 h bei -15°C rühren. Anschließend wird 25%ige wäßriger Ammoniak-Lösung (0,47 ml; 6,3 mmol) zugetropft und man läßt den Ansatz auf RT erwärmen. Nach Zugabe von Ethylacetat (80 ml) und THF (20 ml) wird mit 50%ige wäßriger Na₂CO₃-Lösung (50 ml) und ges. NaCl-Lösung (50 ml) gewaschen, über Na₂SO₄ getrocknet und das Lösemittel im Vakuum abgezogen. Der Rückstand wird mit Ethylacetat /Diethylether (2:1, 6 ml) verrührt. Ausgefallenes Produkt wird abgesaugt, mit Diethylether gewaschen und im Vakuum getrocknet.
Ausbeute: 630 mg (79% d.Th.)
Smp.: 214°C
R_{f} = 0,11 (XXII)
MS (DCI, NH₃): m/z = 416 (M+NH₄)

In Analogie zur Vorschrift des Beispiels 84 werden die in Tabelle 3 aufgeführten Verbindungen hergestellt:

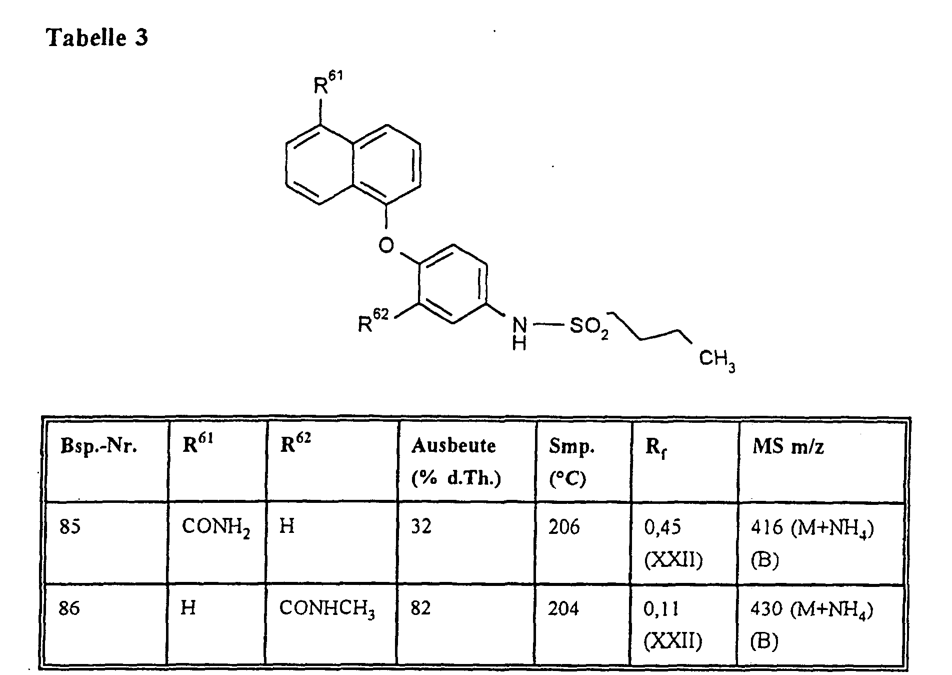

In Analogie zur Vorschrift des Beispiels 29 A erfolgt die Herstellung der in Tabelle 4 aufgeführten Verbindungen:

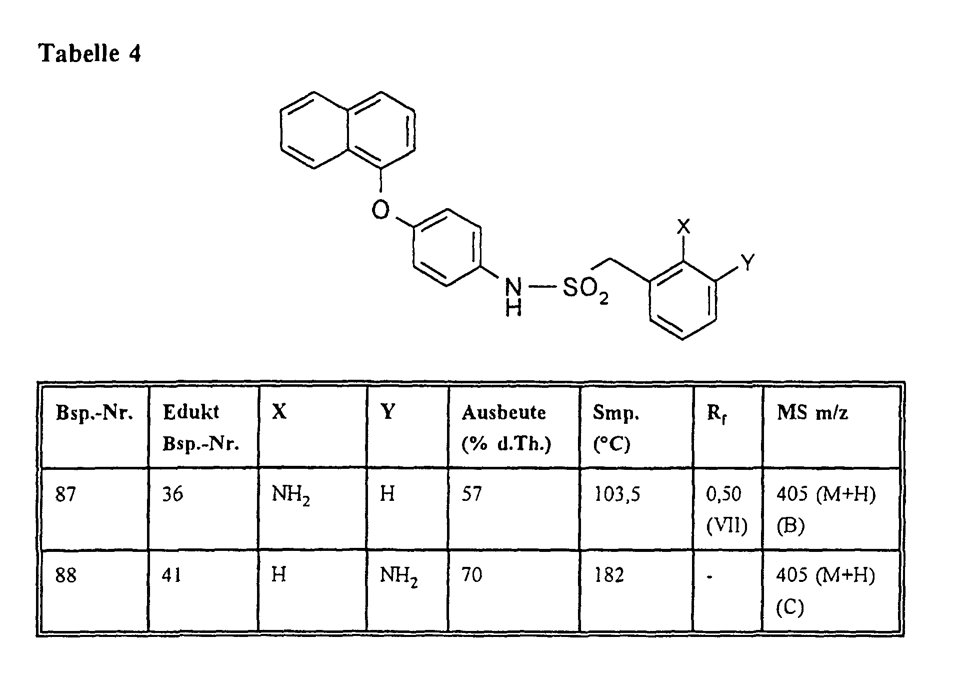

### Beispiel 89

### 1-[N-(2-Acetylaminophenylmethylsulfonyl)amino-4-(naphthyl-1-oxy)benzol

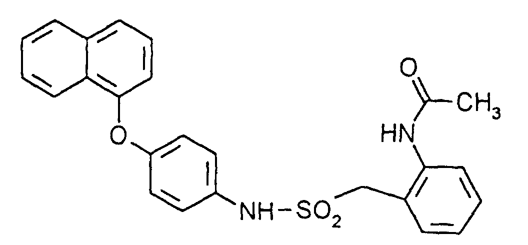

Zur Lösung der Verbindung aus Beispiel 87 (250 mg; 0,62 mmol) und Triethylamin (125 mg; 1,24 mmol) in Dichlormethan (5 ml) tropft man Acetylchlorid (49 mg; 0,62 mmol) und läßt 3 h bei RT rühren. Die Reaktionsmischung wird gewaschen mit Wasser (5 ml), 2 N Salzsäure (2 x 5 ml) und Wasser (5 ml), getrocknet (Na₂SO₄) und im Vakuum eingeengt. Der Rückstand wird in THF (8 ml) aufgenommen, bei 0°C mit einer Lösung von LiOH x H₂O (52 mg; 1,24 mmol) tropfenweise versetzt und über Nacht bei RT gerührt. Das THF wird im Vakuum abgezogen und durch Zugabe von 1 N Salzsäure wird pH 2 eingestellt. Das Produkt wird mit Ethylacetat extrahiert. Die Ethylacetat-Phasen werden getrocknet (Na₂SO₄) und im Vakuum eingeengt.
Ausbeute: 209 mg (75% d.Th.)
Smp.: 173,5°C
R_{f} = 0,38 (VII)
MS (DCI, NH₃): m/z = 464 (M+NH₄)

### Beispiel 90

### 1-[N-(3-Acetylaminophenylmethylsulfonyl)amino-4-(naphthyl-1-oxy)-benzol

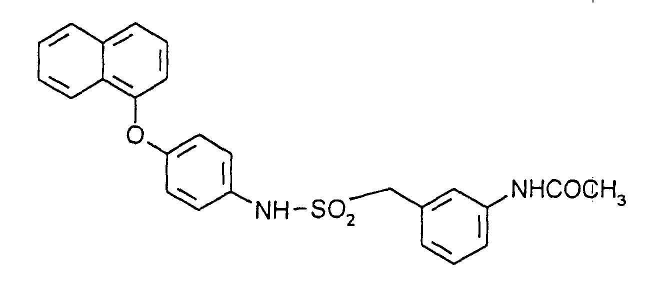

Die Herstellung erfolgt in Analogie zur Herstellung von Beispiel 89 ausgehend von Beispiel 88 (500 mg; 1,23 mmol).
Ausbeute: 232 mg (42% d.Th.)
Smp.: 169°C
MS (DCI, NH₃). m/z = 464 (M+NH₄)

### Beispiel 91

### 1-[N-(Butylsulfonyl)amino]-3-hydroxymethyl-4-(naphthyl-1-oxy)benzol

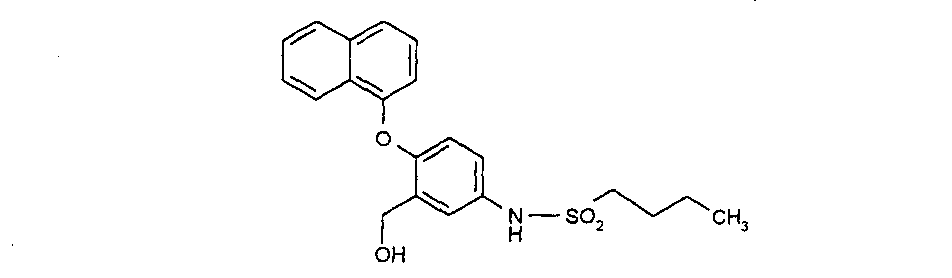

Eine Lösung des Beispiel 43 (750 mg; 1,81 mmol) in THF (6 ml) tropft man unter Argon bei RT zu einer 1 N Lösung von Lithiumaluminiumhydrid in THF (2,0 ml; 2,0 mmol) und THF (5 ml) und läßt über Nacht bei RT rühren. Nach Zugabe von ges. wäßriger NH₄Cl-Lösung (30 ml) wird mit Ethylacetat (3 x 30 ml) extrahiert. Die vereinten organischen Phasen werden getrocknet (Na₂SO₄) und im Vakuum eingeengt.
Ausbeute: 698 mg (100%)
R_{f} = 0,61 (VII)
MS (DCI, NH₃): m/z = 403 (M⁺NH₄)

In Analogie zur Vorschrift des Beispiels 91 werden die in der Tabelle 5 aufgeführten Verbindungen hergestellt:

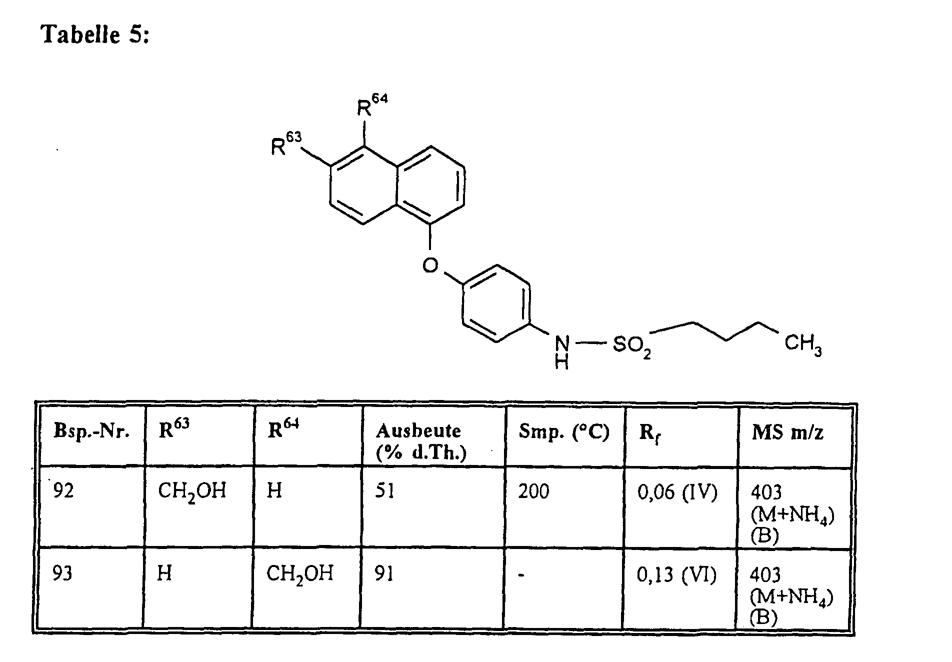

### Beispiel 94

### 1-Naphthyl-4-[N-(n-butylsulfonyl)amino]phenyl-sulfoxid

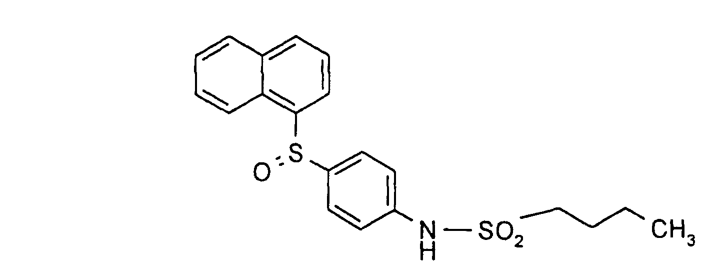

Eine Lösung des Beispiels 44 (500 mg; 1,34 mmol) in Dichlormethan (15 ml) wird mit m-Chlorperbenzoesäure, 80%ig (290 mg; 1,34 mmol) versetzt und über Nacht bei RT gerührt. Die Reaktionsmischung wird mit Wasser (2 x 20 ml) gewaschen, getrocknet (Na₂SO₄) und im Vakuum eingeengt. Der Rückstand wird aus Diethylether umkristallisiert.
Ausbeute: 402 mg (78% d.Th.)
Smp.: 161°C
R_{f} = 0,40 (VII)
MS (ESI): m/z = 426 (M+K)

### Beispiel 95

### 1-Naphthyl-4-[N-(n-butylsulfonyl)amino]phenyl-sulfon

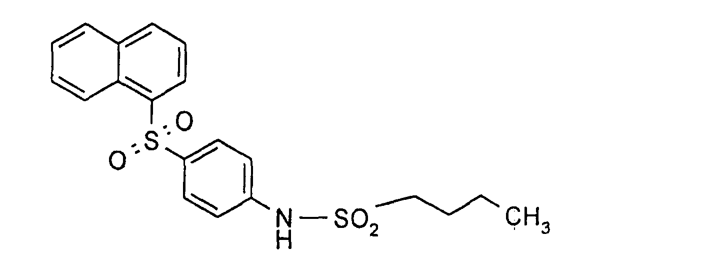

Eine Lösung des Beispiels 44 (500 mg; 1,34 mmol) in Dichlormethan (15 ml) wird mit m-Chlorperbenzoesäure, 80%ig (580 mg; 2,68 mmol) versetzt und über Nacht bei RT gerührt. Nach Filtration wird das Filtrat mit Wasser (2 x 15 ml) gewaschen, getrocknet (Na₂SO₄) und im Vakuum eingedampft. Der Rückstand wird in Diethylether verrührt und anschließend an Kieselgel mit Toluol :
Ethylacetat (8:1) chromatographiert.
Ausbeute: 218 mg (40% d.Th.)
Smp.: 180°C
R_{f} = 0,67 (VII)
MS (ESI): m/z = 442 (M+K)

### Beispiel 96

### 1-[N-(n-Butylsulfinylamino)-4-(naphthyl-1-oxy)benzol

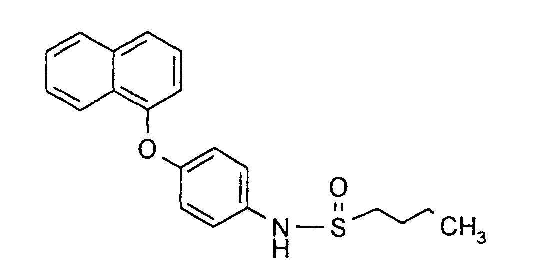

Zur Lösung des Beispiels 51 A (3,50g; 15,0 mmol) und Pyridin (2,40 g; 30,0 mmol) in Dichlormethan gibt man n-Butansulfinylchlorid (2,20 g; 15,8 mmol; Herstellung nach JOC, 1968, 33, 2104) und läßt über Nacht bei RT rühren. Das Reaktionsgemisch wird in Dichlormethan (70 ml) und Wasser (30 ml) eingetragen und gerührt. Ausgefallenes Produkt wird abgesaugt, mit Wasser gewaschen und getrocknet.
Ausbeute: 440 mg (9% d.Th.)
Smp.: 138-139°C
R_{f} = 0,06 (VI)
MS (ESI): m/z = 362 (M+Na)

### Beispiel 97

### 1-(n-Butylsulfonyloxy)-4-(naphthyl-1-oxy)benzol

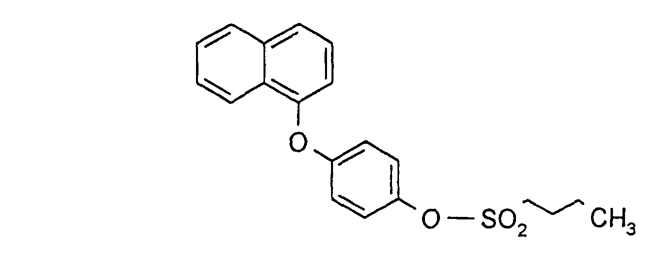

Zur Lösung von Beispiel 56 A (300 mg; 1,27 mmol) in Dichlormethan (10 ml) gibt man bei RT Triethylamin (0,35 ml; 2,54 mmol) und 1-Butansulfonsäurechlorid (0,18 ml; 1,33 mmol) und läßt über Nacht bei RT rühren. Nach Zugabe von Dichlormethan (50ml) wird mit Wasser (50 ml), 1 N Salzsäure (2 x 50 ml) und Wasser (50 ml) gewaschen, über Na₂SO₄ getrocknet und das Solvens im Vakuum abgezogen. Der Rückstand wird an Kieselgel mit Toluol chromatographiert.
Ausbeute: 384 mg (85% d.Th.)
R_{f} = 0,44 (Toluol)
MS (DCI, NH₃) : m/z = 374 (M+NH₄)

In Analogie zur Vorschrift des Beispiels 97 werden die in der Tabelle 6 aufgeführten Verbindungen hergestellt:

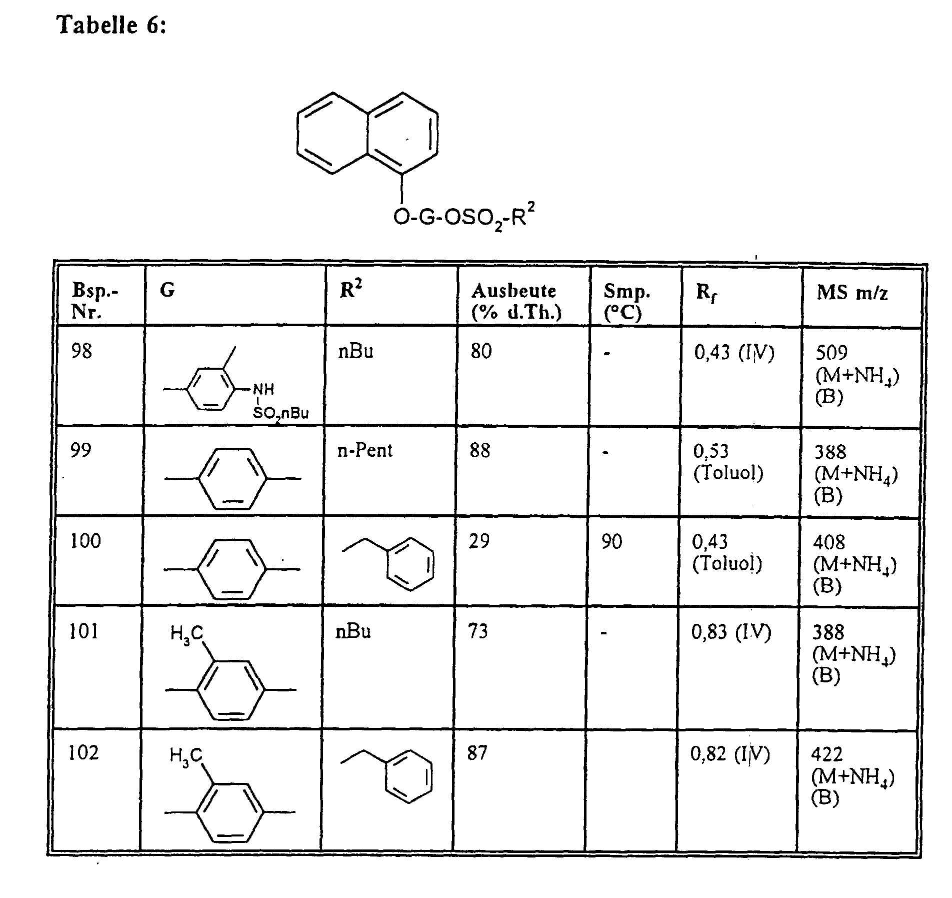

### Beispiel 103

### 1-[N-(1-Propyloxysulfonyl)amino]-4-(naphthyl-1-oxy)benzol

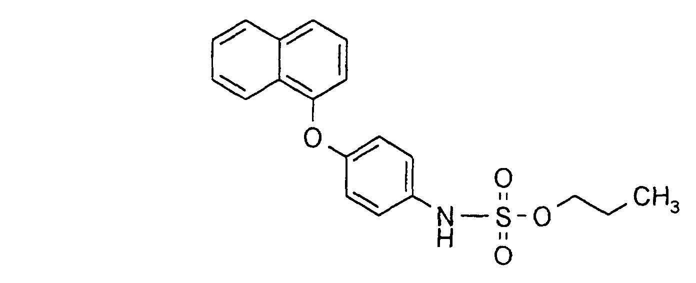

Die Verbindung aus Beispiel 58 A (3,20 g; 10,0 mmol) wird in Toluol (80 ml) vorgelegt. Nach Zugabe von Phosphorpentachlorid (2,08 g; 10,0 mmol) wird die Reaktionsmischung innerhalb von 1 h langsam auf Rückflußtemperatur erwärmt und noch 1,5 h unter Rückfluß gerührt. Anschließend wird abgekühlt auf RT, die Lösung von schwerlöslichen, viskosen Bestandteilen abdekantiert und im Vakuum eingeengt. Von den entstandenen Aminsulfonylchlorid (ca. 3,4 g ) werden 1,73 g (ca. 5 mmol) in Dichlormethan (40 ml) aufgenommen und nacheinander versetzt mit Na₂CO₃ (3,0 g), Benzyltriethylammoniumchlorid (228 mg, 1,0 mmol) und 1-Propanol (301 mg; 5,0 mmol). Der Ansatz wird über Nacht zum Rückfluß erhitzt, filtriert und im Vakuum eingeengt. Der Rückstand wird an Kieselgel mit Toluol :
Ethylacetat (12:1) chromatographiert.
Ausbeute: 700 mg (39% d.Th.)
Smp.: 95°C
R_{f} = 0,40 (IV)
MS (DCI; NH₃): m/z = 375 (M+NH₄)

### Beispiel 104

### 1-[N-(1-Propylaminosulfonyl)amino]-4-(naphthyl-1-oxy)benzol

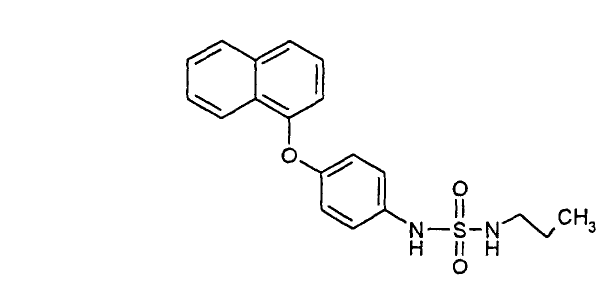

Die Herstellung erfolgt in Analogie zur Synthese des Beispiels 103 unter Verwendung von n-Propylamin statt n-Propylalkohol.
Ausbeute: 280 mg (16% d.Th.)
Smp.: 113-15°C
R_{f} = 0,38 (IV)
MS (DCI, NH₃): m/z = 374 (M+NH₄)

### Beispiel 105

### 1-(N-1-Butylsulfonyl-N-methyl)amino-4-(naphthyl-1-oxy)benzol

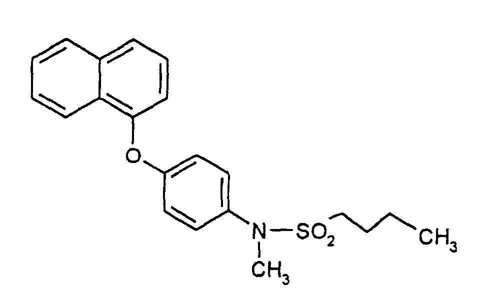

Methyljodid (0,18 ml; 2,8 mmol) wurde zu einer Mischung von 51 A (500 mg; 1,41 mmol) und Kaliumcarbonat (389 mg; 2,81 mmol) in DMF (10 ml) zugefügt. Nach 30 min Rühren bei RT wurde die Reaktionslösung auf Wasser gegeben und zweimal mit Essigester extrahiert. Die vereinigten organischen Phasen wurden mit Wasser gewaschen, über Natriumsulfat getrocknet und eingeengt, um 190 mg eines Harzes zu ergeben, das sich allmählich verfestigte.
Ausbeute: 190 mg (37% d.Th.)
R_{f} = 0,67 (XVI)
MS (DCI; NH₃): m/z = 387 (M+NH₄)

### Beispiel 106 und Beispiel 107

### 1-N-(4-Azido-1-propyl-sulfonyl)amino-4-(naphthyl-1-oxy)benzol (Beispiel 106)

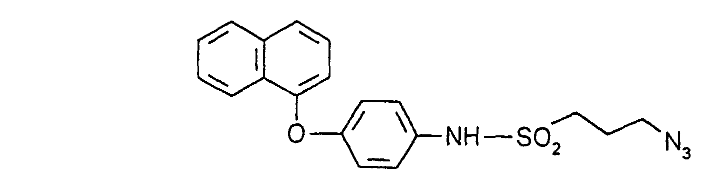

### N-(4-Naphthyl-1-oxy)phenyl-1,3-propansultam (Beispiel 107)

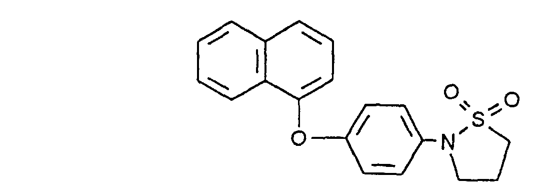

Eine Lösung des Beispiels 65 (15,51 g; 41,3 mmol) in DMSO (100 ml) wird mit Natriumazid (2,95 g; 45,4 mmol) versetzt und 15 h auf 80°C erhitzt. Nach Zugabe von Wasser (300 ml) mit mit Diethylether (3 x 200 ml) extrahiert. Die vereinigten organischen Phasen werden mit gesättigter NaCl-Lösung (200 ml) gewaschen, getrocknet (Na₂SO₄) und im Vakuum eingeengt. Der Rückstand wird an Kieselgel mit Toluol : Diethylether (10:1) chromatographiert.
Ausbeute (Beispiel 106): 9,80 g (62% d.Th.)
Smp.: 77,5°C
R_{f} = 0,29 (IV)
MS (DCI, NH₃): m/z = 400 [M+NH₄]
Ausbeute (Beispiel 107): 1,61 g (12% d.Th.)
Smp.: 150°C
R_{f} = 0,21 (IV)
MS (DCI, NH₃): m/z = 357 [M+NH₄]

### Beispiel 108

### 1-N-(4-Amino-1-propylsulfonyl)amino-4-(naphthyl-1-oxy)benzol

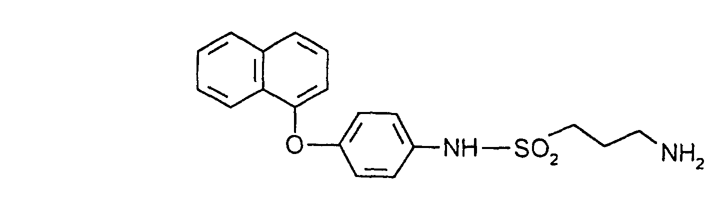

Eine Lösung des Beispiels 106 (4,76 g; 12,4 mmol) in Methanol (100 ml) wird mit 10 % Palladium auf Aktivkohle (0,5 g) versetzt und 3,5 h bei 3 bar und Raumtemperatur hydriert. Die Reaktionsmischung wird über Kieselgur filtriert und im Vakuum eingeengt.
Ausbeute: 3,67 (83% d.Th.)
Smp.: 159°C
R_{f} = 0,08 (XXIII)
MS (DCI, NH₃): m/z = 357 (M+H)

Die Herstellung der in Tabelle 7 aufgelisteten Beispiele erfolgt in Analogie zur Herstellung der Beispiele 1 bis 71 (Methoden A und B):

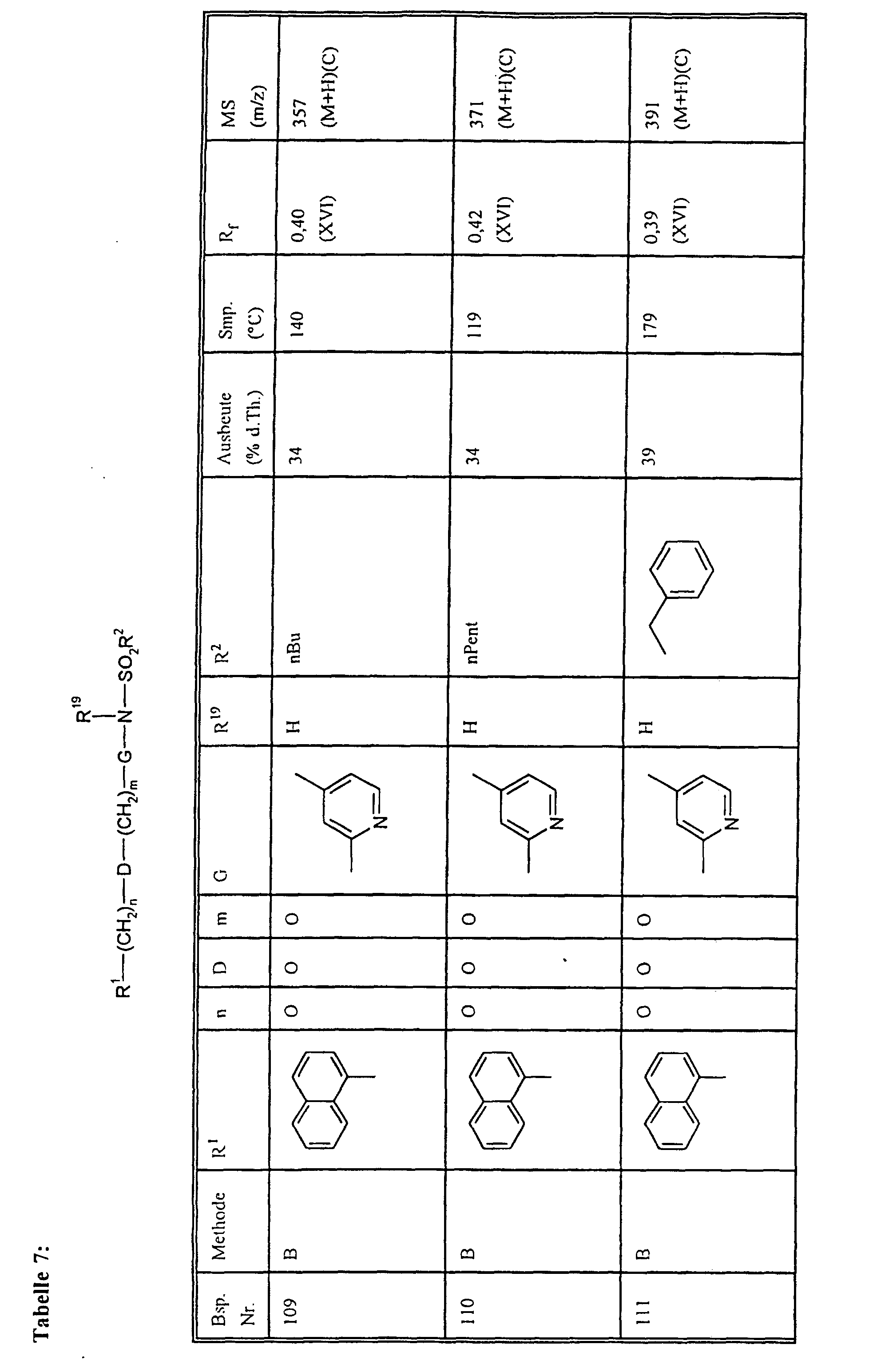

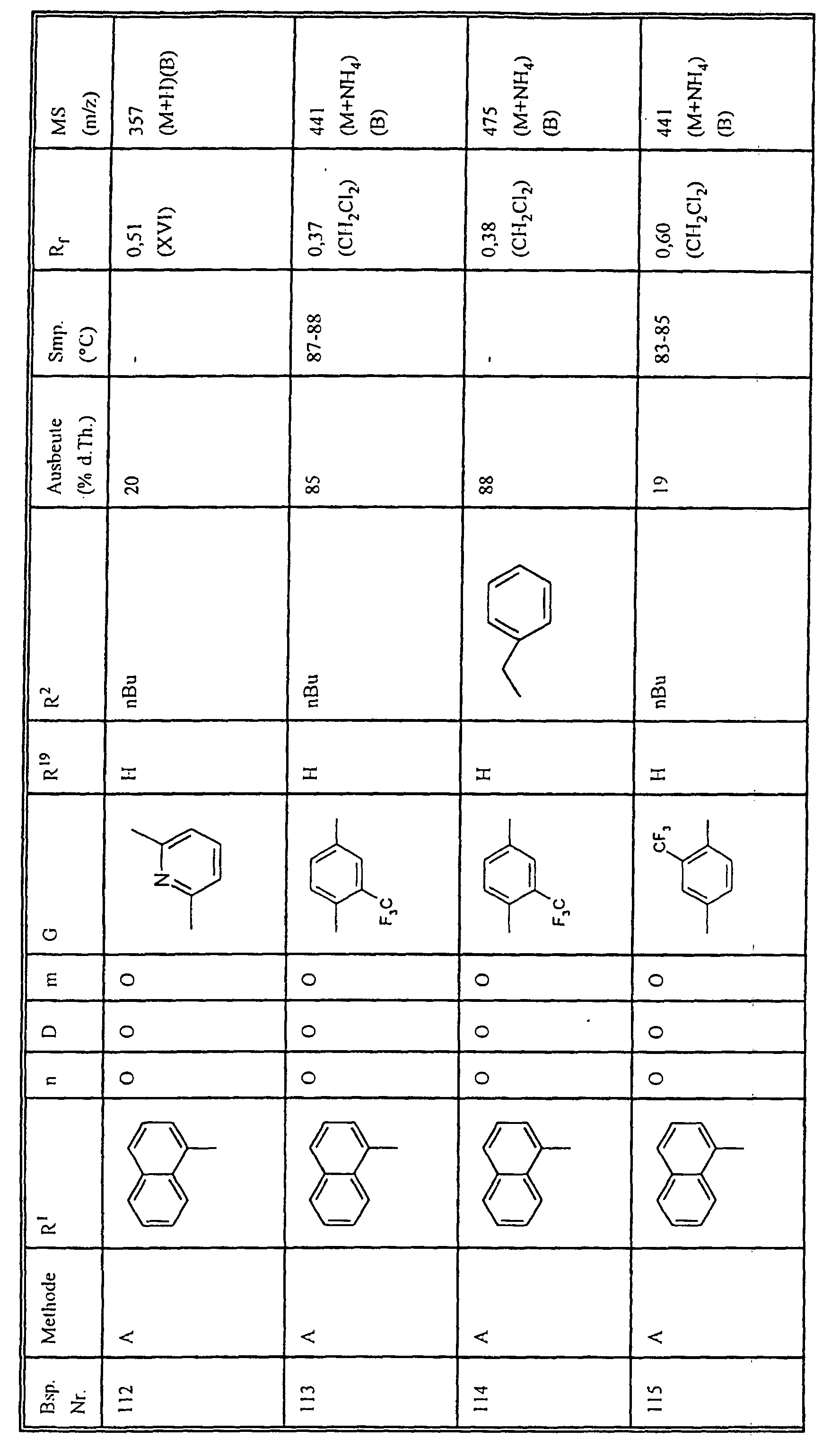

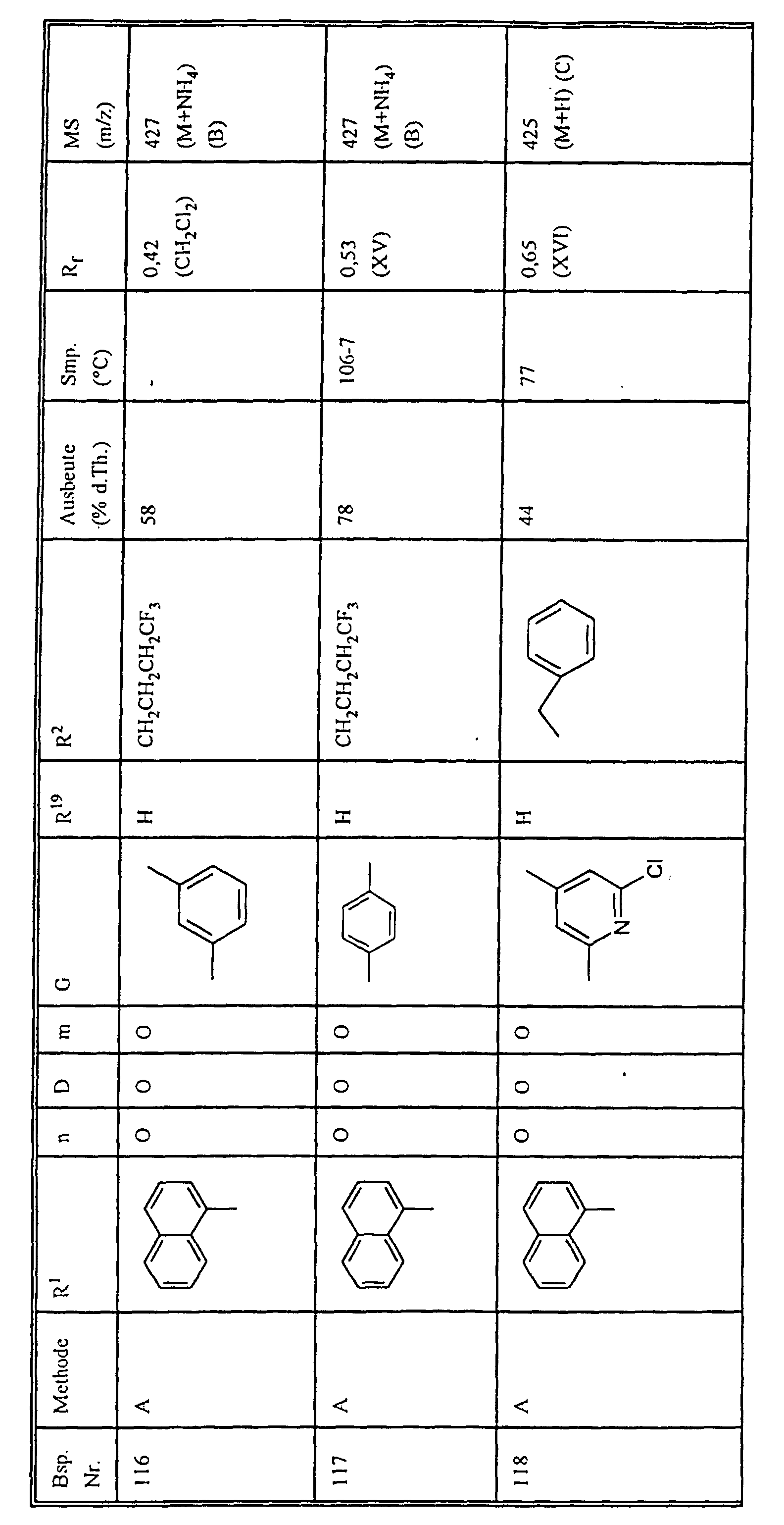

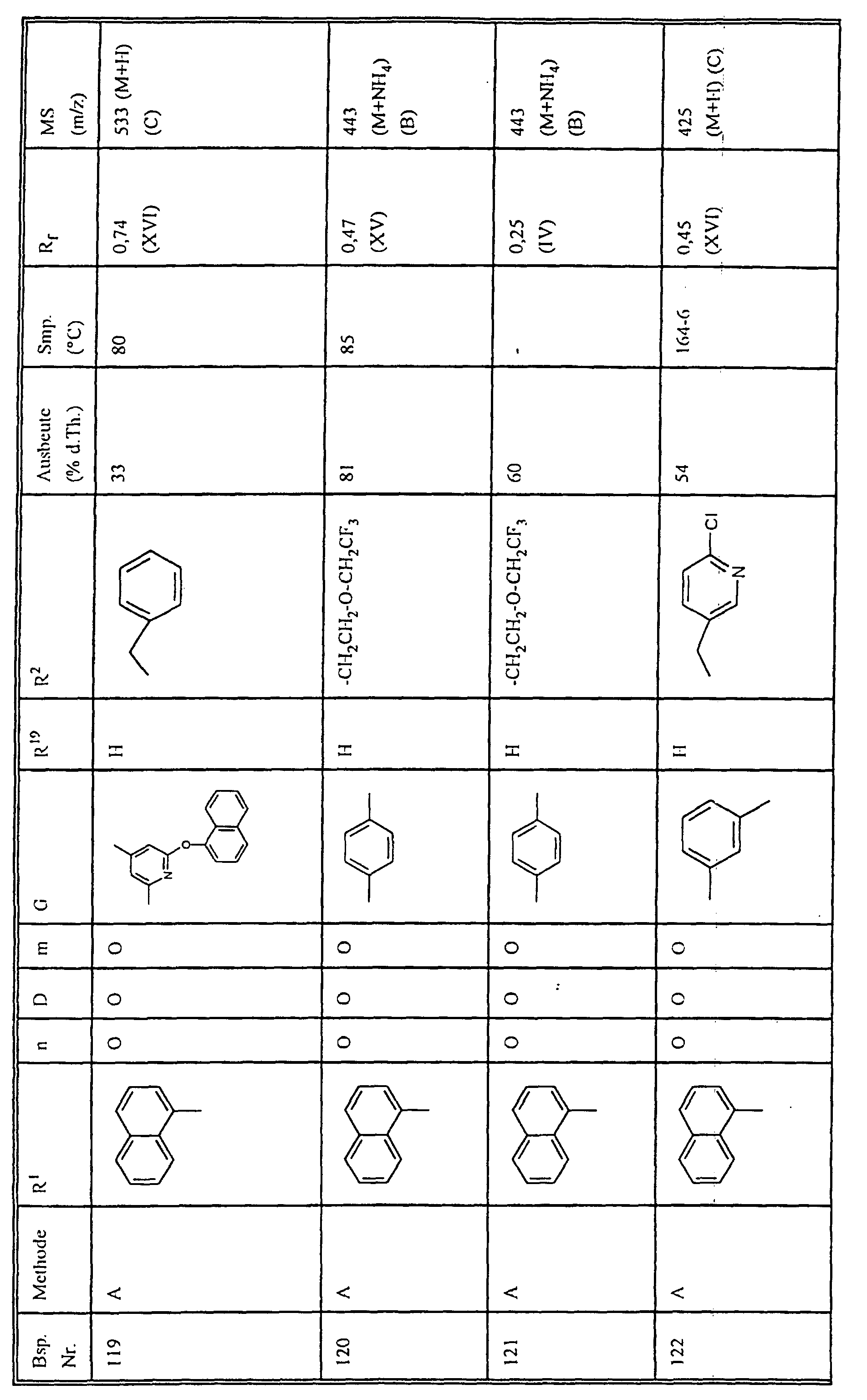

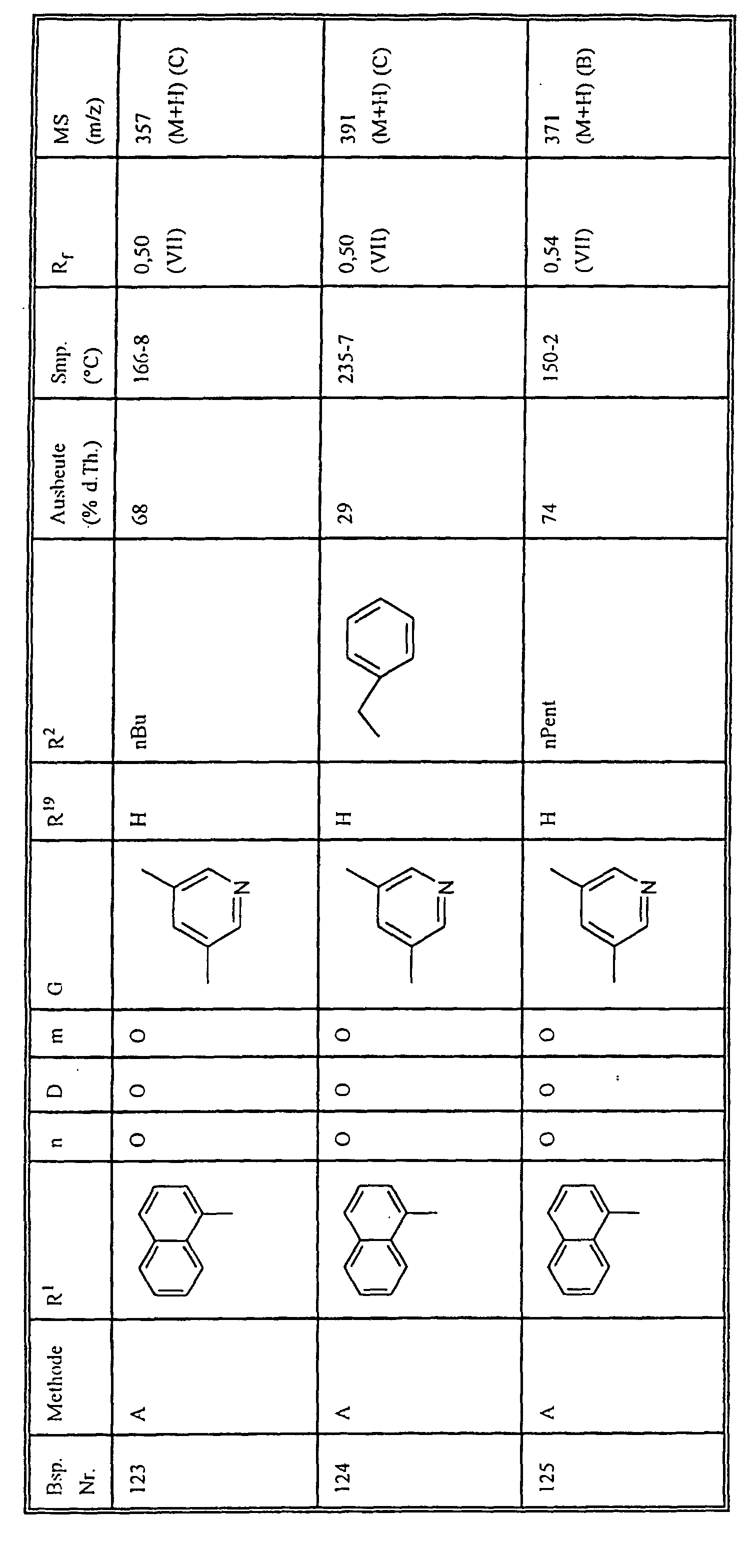

### Beispiel 126

### 1-(Benzylsulfonyloxy)-3-(naphthyl-1-oxy)benzol

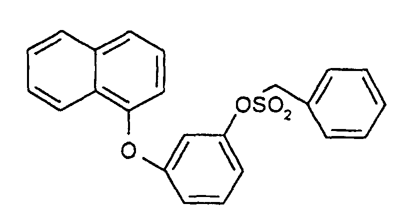

Die Herstellung erfolgte in Analogie zur Herstellung des Beispiels 97 ausgehend von Beispiel 63 A (0,709 g; 3.00 mmol).
Ausbeute: 0,680 g (58 % d.Th.)
R_{f} = 0,50 (Toluol)
MS (DCI, NH₃): m/z = 408 (M+NH₄)

### Beispiel 127

### 3-(Naphthyl-1-oxy)-1-(pentylsulfonyloxy)benzol

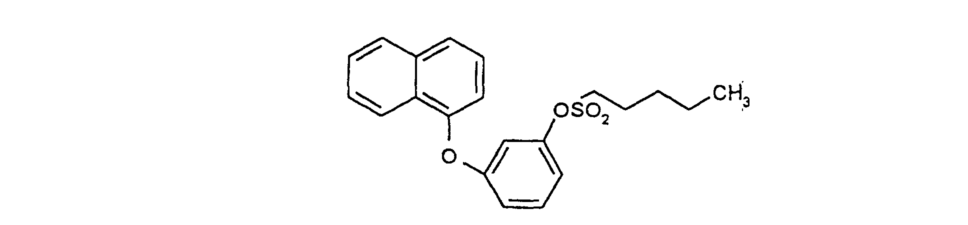

Die Herstellung erfolgte in Analogie zur Herstellung des Beispiels 97 ausgehend von Beispiel 63 A (0,709 g; 3.00 mmol)
Ausbeute: 0,800 g (72 % d.Th.)
R_{f} = 0,52 (Toluol)
MS (DCI, NH₃): m/z = 388 (M⁺NH₄)

### Beispiel 128

### 2-(Naphthyl-1-oxy)-4-(pentylsulfonylamino)pyridin Natriumsalz

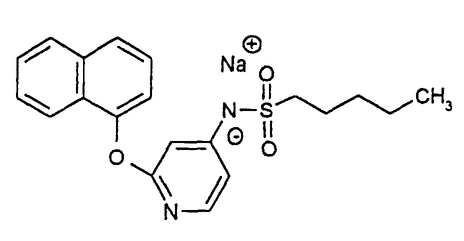

Eine Lösung des Beispiels 110 (0,227 g; 0,61 mmol) in Tetrahydrofuran (2 ml) wird bei Raumtemperatur unter Argon mit einer Lösung von Natriummethylat (0,033 g; 0,61 mmol) in MeOH (1,56 ml) versetzt. Die Reaktionsmischung wird noch 15 min gerührt und anschließend werden die Lösungsmittel im Vakuum abgezogen.

Der Rückstand wird in Diethylether verrührt, abgesaugt und im Vakuum getrocknet.
Ausbeute: 0,240 g (99 % d.Th.)
R_{f} = 168°C (Zers.)

In Analogie zur Vorschrift des Beispiels 128 erfolgt die Herstellung der in Tabelle 8 aufgelisteten Verbindungen.

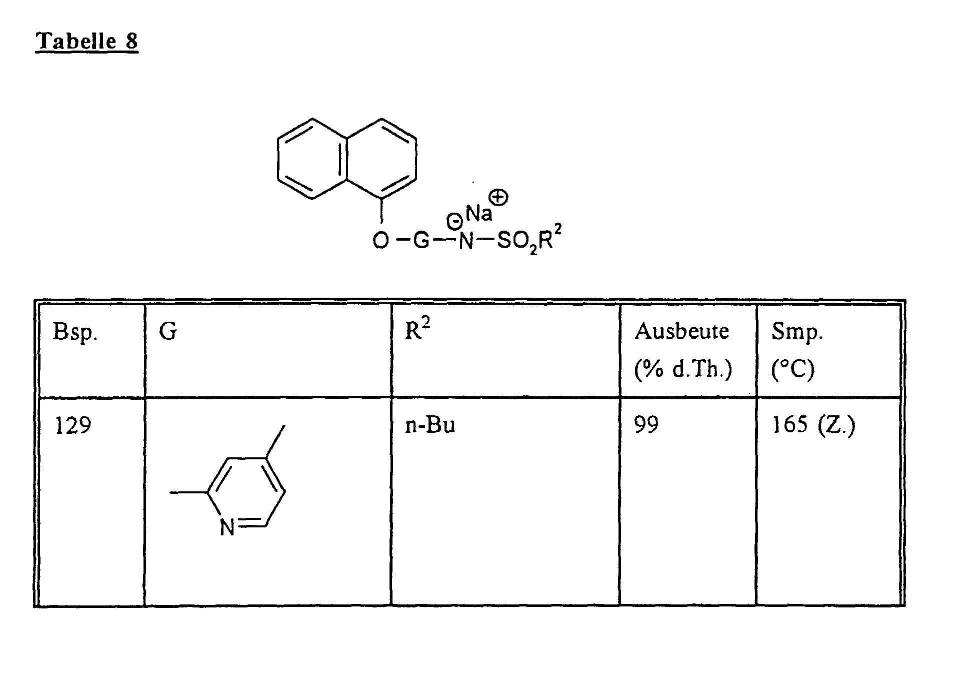

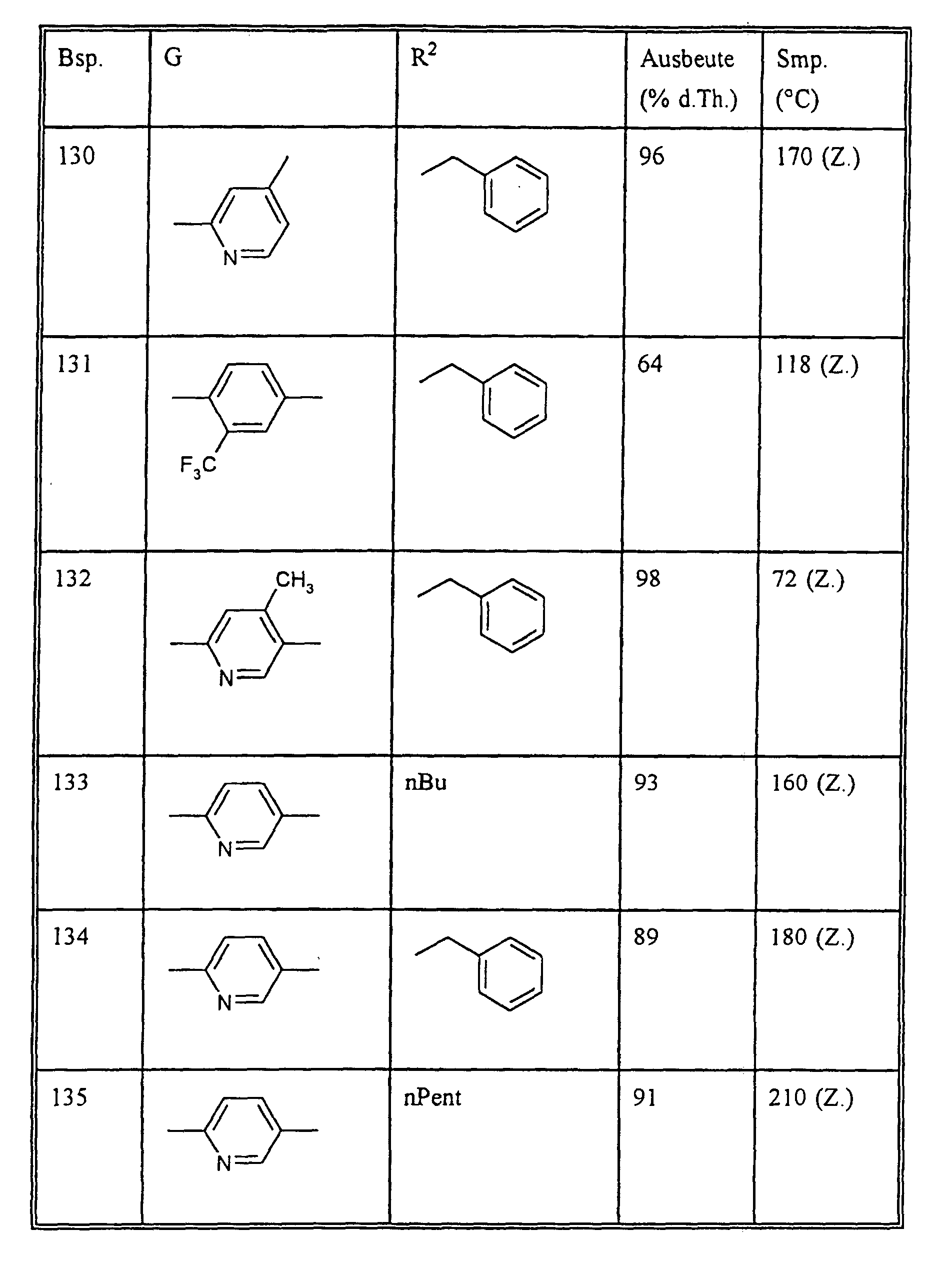

### Beispiel 136

### 1-(Naphthyl-1-oxy)-4-(3-pyridylmethylsulfonylamino)benzol

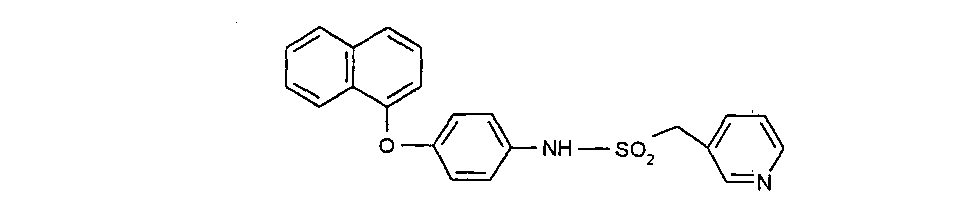

Eine Lösung des Beispiels 48 (2,1 g; 5,0 mmol) in THF (40 ml) und Methanol (100 ml) wird mit Palladium, 10 % auf Aktivkohle (0,5 g) versetzt und bei 3 bar 15 h hydriert. Das Reaktionsgemisch wird über Kieselgur filtriert und das Filtrat im Vakuum eingeengt.
Der Rückstand wird an Kieselgel mit Toluol : Ethylacetat (2:1) chromatographiert. Ausbeute: 0,668 g (34 % d.Th.)
Smp.: 174-76°C
R_{f} = 0,13 (XXVII)
MS (ESI): m/z = 391 (M+H)

### Beispiel 137

### 1-(Naphthyl-1-oxy)-3-(3-pyridylmethylsulfonylamino)benzol

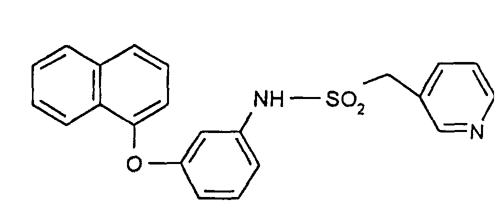

Die Herstellung erfolgte in Analogie zur Herstellung des Beispiels 136 ausgehend von Beispiel 118 (1,83 g; 4,2 mmol).
Ausbeute: 1,43 g (85 % d.Th.)
R_{f} = 0,09 (XVI)
MS (ESI): m/z = 391 (M+H)

### Beispiel 138

### 4-(n-Butylsulfonylamino)-2-(N,N-dimethylamino)methyl-1-(naphthyl-1-oxy)benzol

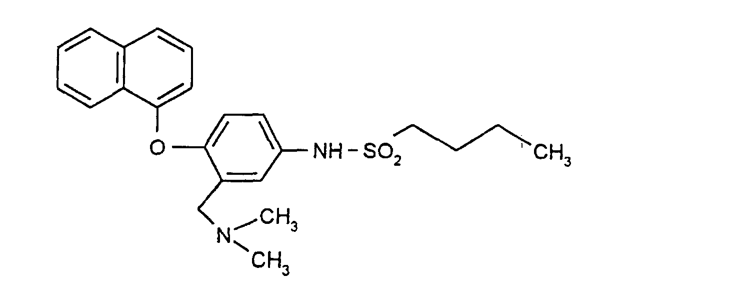

Eine Lösung des Beispiels 83 (0,200 g; 0,469 mmol) in THF (5 ml) wird bei Raumtemperatur unter Argon mit einer IN-Lösung von LiAlH₄ in THF (0,94 ml; 0,94 mmol) versetzt und 18 h zum Rückfluß erhitzt.
Nach Zugabe von Wasser (20 ml) wird die Reaktionsmischung mit Ethylacetat (3 x 20 ml) extrahiert. Die vereinten organischen Phasen werden getrocknet (Na₂SO₄) und im Vakuum eingeengt.
Ausbeute: 0,190 g (98 % d.Th.)
R_{f} = 0,77 (XXVI)
MS (DCI, NH₃): m/z = 413 (M+H)

In Analogie zur Herstellung des Beispiels 138 werden die in Tabelle 9 aufgelisteten Beispiele hergestellt.

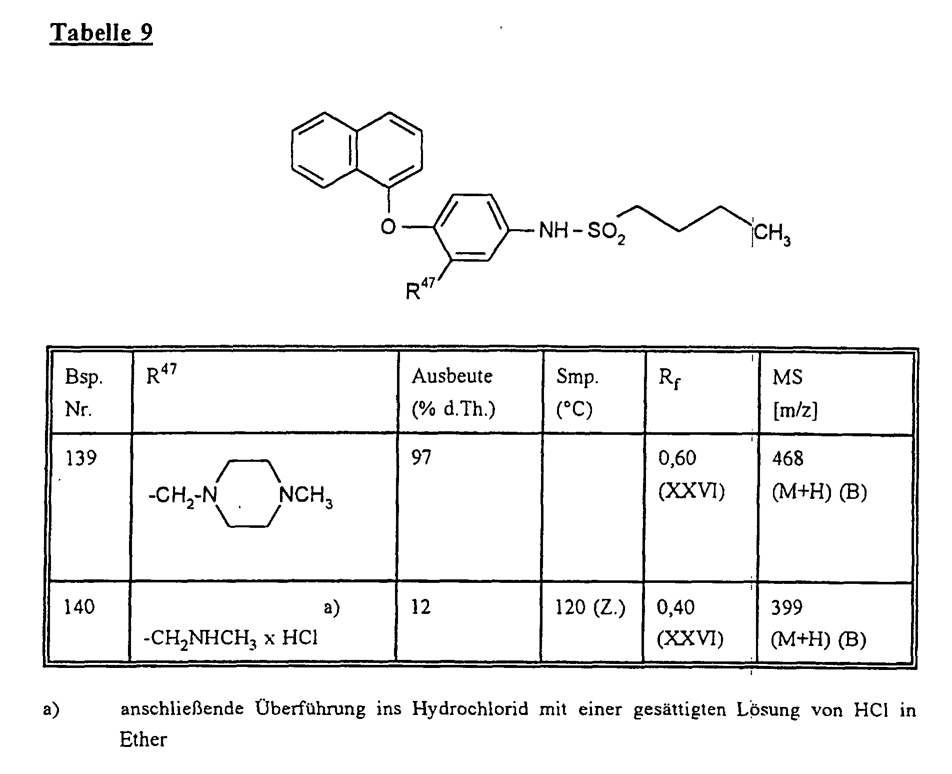

### Beispiel 141

### 1-[3-(N,N-Dimethylamino]propylsulfonyl)amino-4-(naphthyl-1-oxy)benzol

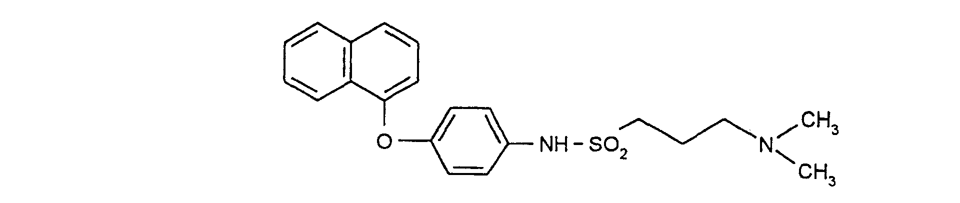

Eine Lösung des Beispiels 108 (0,505 g; 1,40 mmol), Zink-(II)-chlorid (0,772 g; 5,70 mmol) und para-Formaldehyd (0,170 g; 5,70 mmol) in Dichlormethan (25 ml) wird 1 h unter Argon bei Raumtemperatur gerührt, anschließend mit Natriumborhydrid (0,214 g; 5,70 mmol) versetzt und über Nacht bei Raumtemperatur gerührt.

Nach Zugabe einer wäßrigen 2,6N Ammoniak-Lösung (8,6 ml) wird mit Wasser (50 ml) verdünnt und zweimal mit CH₂Cl₂ (50 ml) extrahiert. Die vereinten organischen Phasen werden getrocknet (Na₂SO₄) und im Vakuum eingeengt. Der Rückstand wird an Kieselgel mit Dichlormethan : Ethanol (5:1) chromatographiert. Ausbeute: 0,107 g (20 % d.Th.)
R_{f} = 0,60 (XXVI)
MS (DCI, NH₃): m/z = 385 (M+H)

### Beispiel 142

### 3-[(4-(Naphthyl-1-oxy)-phenyl)aminosulfonyl]propyl-N,N,N-trimethylammoniumiodid

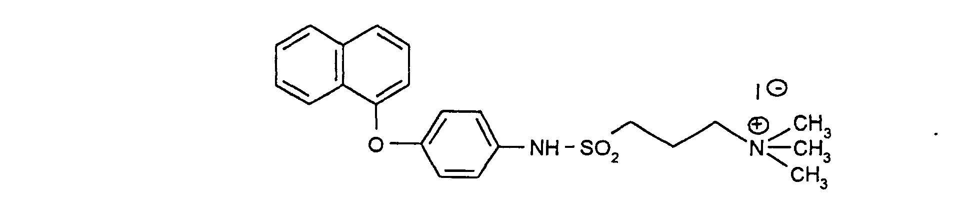

Eine Lösung des Beispiels 108 (1,07 g; 3,00 mmol) in THF (50 ml) wird bei Raumtemperatur mit Iodmethan (0,43 g; 3,00 mmol) versetzt und 72 h bei Raumtemperatur gerührt. Ausgefallenes Produkt wird abgesaugt und im Vakuum getrocknet.
Ausbeute: 0,341 g (22 % d.Th.)
Smp.: >210°C (Z.)
MS (DCI, NH₃): m/z = 399 (M+H)

In Analogie zur Herstellung der Beispiele 1 (Methode A) und 2 (Methode B) werden die in Tabelle 10 aufgeführten Beispiele hergestellt.

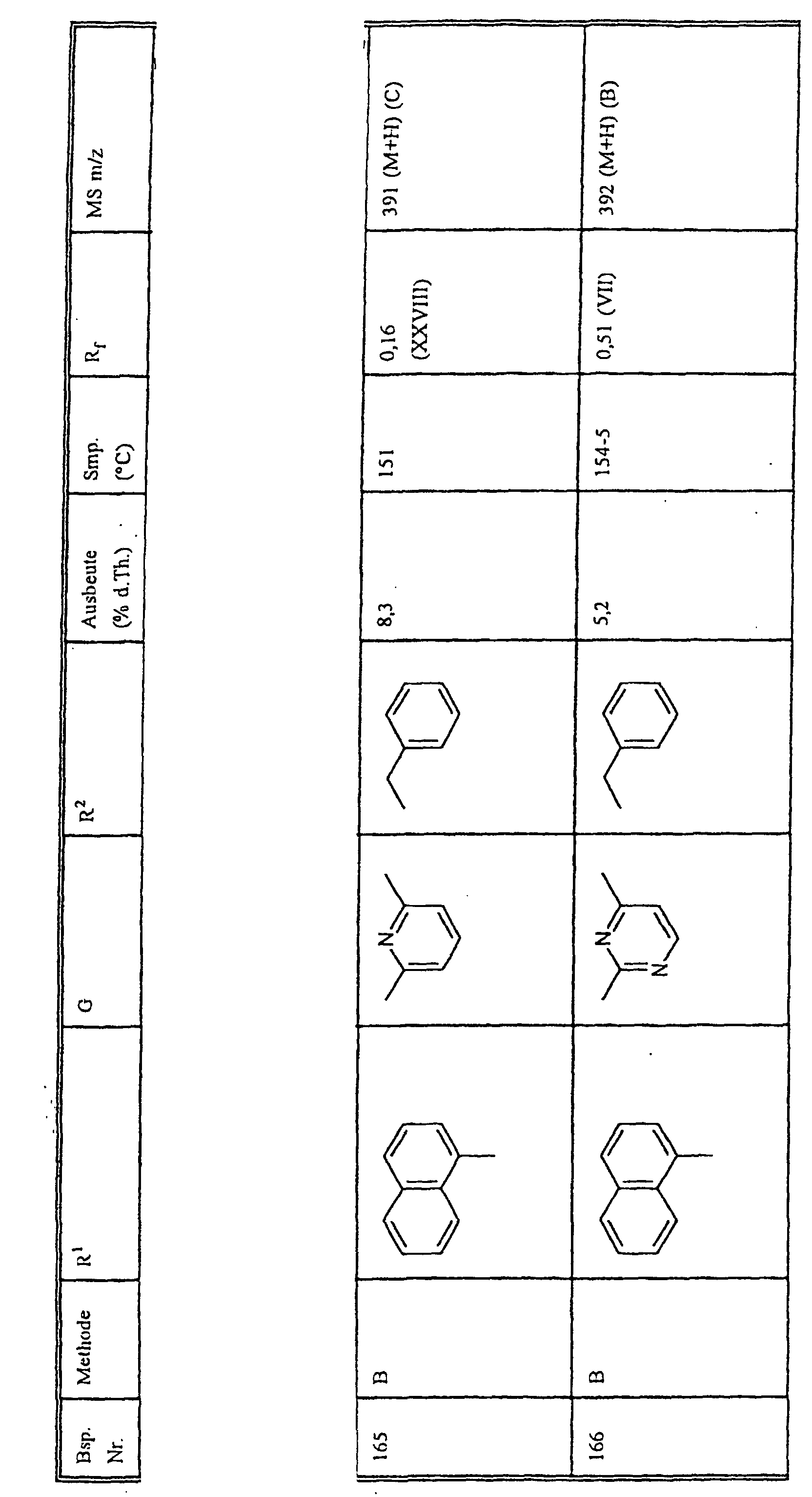

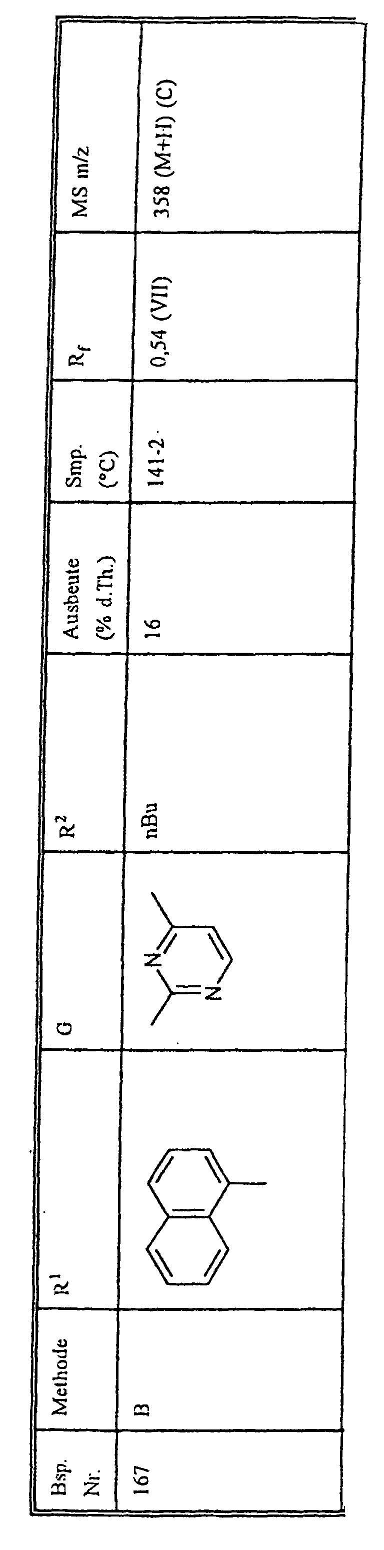

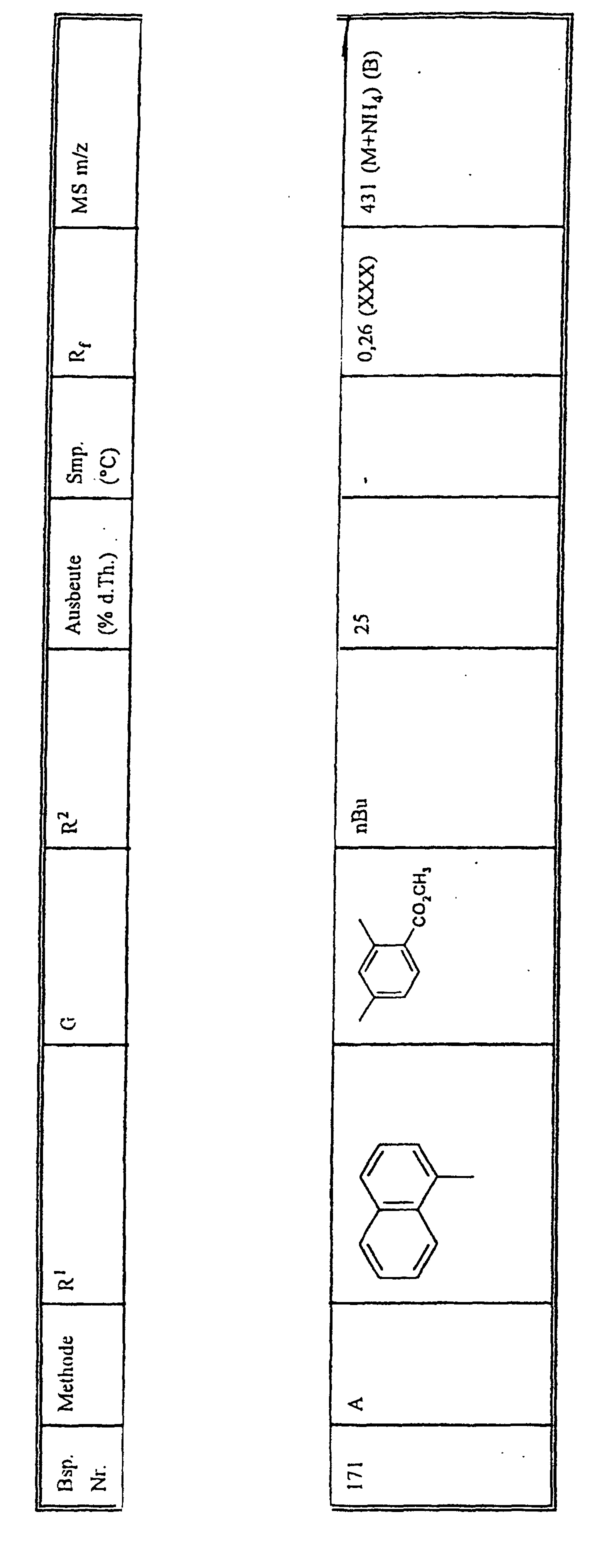

### Beispiele 172 und 173

### 2-N-(n-Butylsulfonyl)amino-4-(naphthyl-1-oxy)benzoesäure (Beispiel 172)

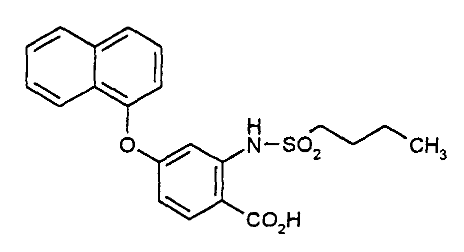

### 2-N-(n-Butylsulfonyl)amino-4-(naphthyl-1-oxy)benzoesäure-n-propylester (Beispiel 173)

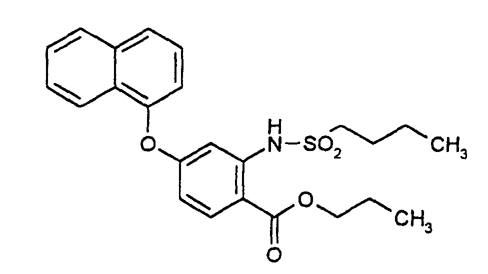

Eine Lösung von Beispiel 172 (0,500 g, 1,21 mmol) in 6 ml n-Propanol wurde mit 1N-Natronlauge (2,50 ml) versetzt und über Nacht bei 85°C gerührt. Die Reaktionsmischung wurde auf Wasser gegossen, dreimal mit Essigester extrahiert, sauer gestellt und nochmals mit Essigester extrahiert. Alle Essigesterphasen wurden vereinigt, am Vakuum eingeengt und über Kieselgel chromatographiert.

Bsp.-Nr. 172:
Ausbeute: 0,213 g (42 % d.Th.)
Smp.: 145-146°C
R_{f}: 0,35 (XXV)
MS (ESI): m/z = 400 (N+H)

Bsp.-Nr. 173:
Ausbeute: 0,195 g (36,5 % d.Th.)
gelbes Öl
R_{f}: 0,63 (IV)
MS (ESI): m/z = 364 (M+Na)

### Beispiel 174

### Natriumsalz von 4-N-(n-Butylsulfonyl)amino-2-(naphthyl-1-oxy)pyrimidin

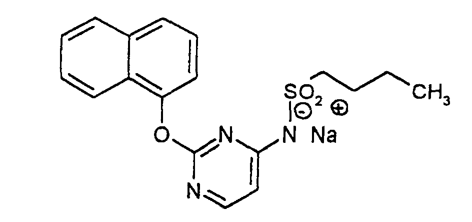

Beispiel 167 (0,310 g; 0,84 mmol) wurde in THF (2 ml) gelöst und mit 1N-Natronlauge (0,84 ml) versetzt. Das THF wurde im Vakuum abgezogen, die resultierende Lösung lyophilisiert.
Ausbeute: 0,317 g weißes Pulver (100 % d.Th.)
R_{f} = 0,47 (VII)

### Beispiel 175

### Natriumsalz von 2-N-(Benzylsulfonyl)amino-4-(naphthyl-1-oxy)benzoesäuremethylester

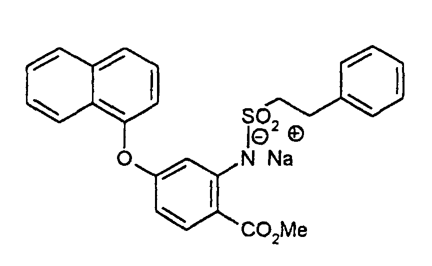

In Analogie zur Herstellung von Beispiel 1 wurde aus Beispiel 82 A (0,590 g; 2,01 mmol) 2-n-(Benzylsulfonyl)amino-4-(naphthyl-1-oxy)benzoesäuremethylester hergestellt. Das chromatographierte Produkt (0,274 g) wurde in THF (3 ml) gelöst und mit Natriummethylat (0,033 g, 0,61 mmol) versetzt. Die Suspension wurde durch Zugabe von Methanol (5 ml) vollständig gelöst, die Lösung wurde eingeengt, der feste Rückstand wurde mit wenig Methanol digeriert und abgesaugt. Ausbeute: 0,186 g weißer Feststoff (20 % d.Th.)
R_{f}: 0,67 (IV)
MS (korrespondierende Säure, DCI/NH₃): m/z = 465 (M+Na)

### Beispiel 176

### 1-(Naphthyl-1-oxy)-4-N-(n-pentanoyl)aminobenzol

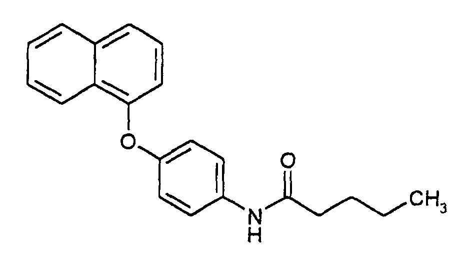

Zu einer Lösung von Beispiel 51 A (2,0 g; 8,5 mmol) und Valeriansäurechlorid (1,0 ml; 8,5 mmol) in Methylenchlorid (20 ml) wurde Pyridin (1,0 ml, 3 mmol) gegeben und über Nacht bei Raumtemperatur gerührt. Die Reaktionslösung wurde auf Wasser gegossen und mit Methylenchlorid extrahiert (2 x). Die organischen Phasen wurden mit Wasser gewaschen (2 x), über Na₂SO₄ getrocknet und eingeengt. Der resultierende Feststoff wurde mit Ether verrührt, abgesaugt und getrocknet.
Ausbeute: 2,37 g (87 % d.Th.)
Smp.: 80°C
R_{f} = 0,57 (XVI)
MS (DCI/NH₃): m/z = 320 (M+H)

In Analogie zu Beispiel 176 wurden die in Tabelle 11 aufgeführten Beispiele hergestellt.

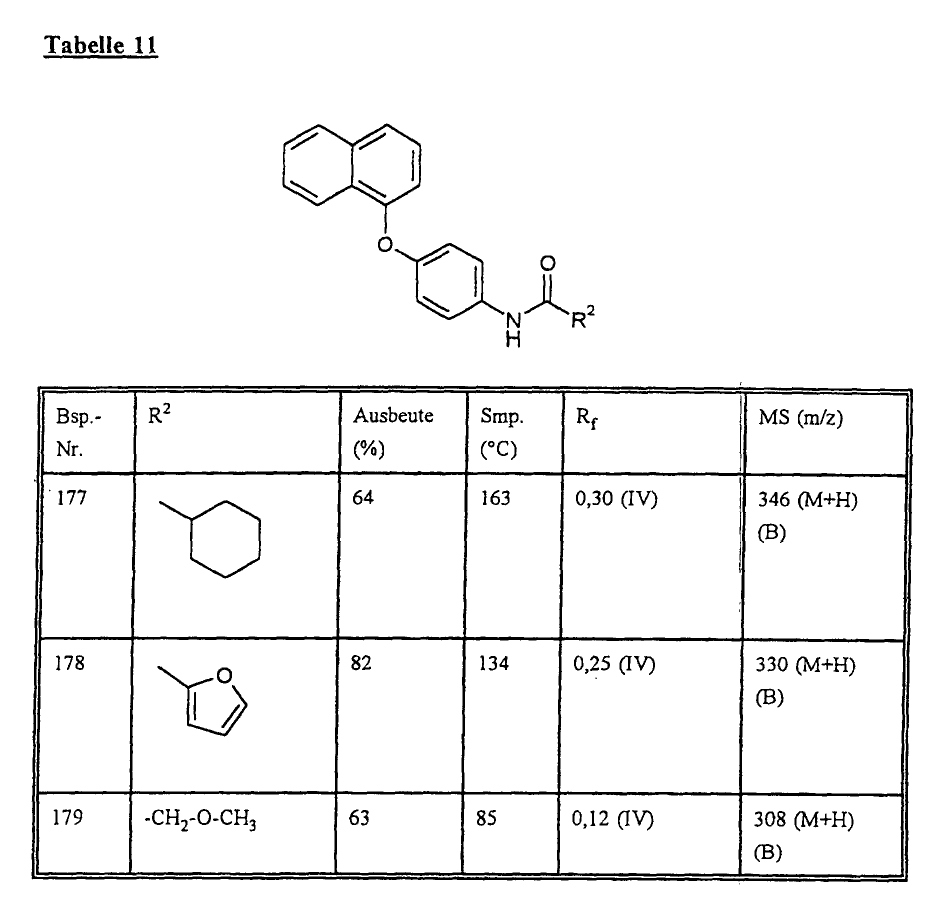

### Beispiel 180

### 1-(Naphthyl-1-oxy)-4-N-(phenylsulfonyl)aminobenzol

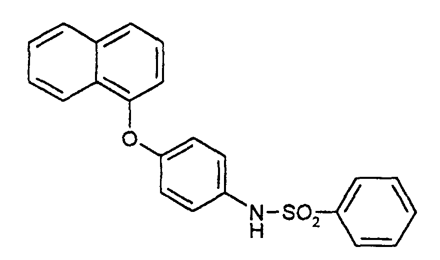

In Analogie zur Herstellung von Beispiel 1 wurde aus Beispiel 51 A (2,0 g; 8,5 mmol) Beispiel 180 hergestellt.

Ausbeute: 2,35 g (74 % d.Th.)
Smp.: 143-4°C
R_{f} = 0,25 (IV)
MS (DCI/NH₃): m/z = 393 (M+NH₄)

### Beispiel 181

### 1-N-(1-Butylsulfonyl)amino-4-(naphthyl-1-oxy)benzol Natriumsalz

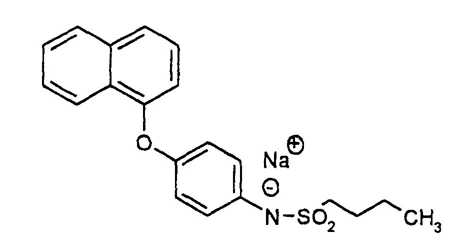

Die Herstellung erfolgte in Analogie zur Herstellung des Beispiels 128 ausgehend von Beispiel 1 (0,500 g; 1,41 mmol).
Ausbeute: 0,479 mg (91 % d.Th.)
Smp.: >210°C
R_{f} = 0,32 (IV, korrespondierende Säure)
MS (korrespondierende Säure DCI, NH₃): m/z = 373 (M+NH₄)

### Beispiel 182

### 5-(N-Butylsulfonyl)amino-2-(1-naphthyl-1-oxy)pyridin-hydrochlorid

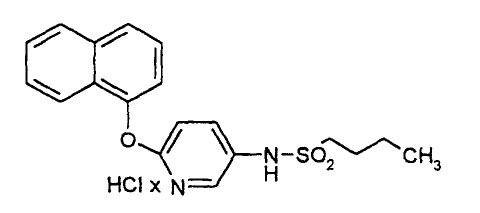

Eine Lösung des Beispiels 32 (0,500 g; 1,40 mmol) in THF (10 ml) wird mit einer 2,6-N-Lösung von HCl in Diethylether (0,77 ml; 2,0 mmol) versetzt, 10 Minuten gerührt und im Vakuum eingeengt bis das Produkt auszufallen beginnt. Nach Zugabe von Diethylether wird das Produkt abgesaugt und im Vakuum getrocknet.
Ausbeute: 0,550 g (100 % d.Th.)
Smp.: 136-38°C

### Beispiel 183

### n-Butanphosphonsäuremethylester-N-(4-(naphthyl-1-oxy)phenyl)amid

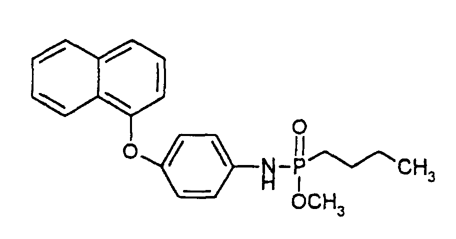

Zur Lösung von n-Butanphosphonsäuredichlorid (2,00 g; 11,9 mmol) und Triethylamin (2,30 g; 22,8 mmol) in Toluol (40 ml) tropft man unter Argon bei 0 bis 5°C eine Lösung von Methanol (0,365 g; 11,4 mmol) in Toluol (10 ml) und läßt 2h bei dieser Temperatur nachrühren. Das Reaktionsgemisch wird unter Argon filtriert und das Filtrat wird nacheinander bei Raumtemperatur mit Triethylamin (2,30 g; 22,8 mmol) und einer Lösung der Verbindung aus Beispiel 51 A (2,35 g; 10,0 mmol) in Toluol (10 ml) versetzt. Man läßt über Nacht bei Raumtemperatur rühren und trägt das Reaktionsgemisch in Ethylacetat (100 ml) ein und extrahiert mit Wasser (3 x 50 ml). Die organische Phase wird getrocknet (MgSO₄) und im Vakuum eingeengt. Der Rückstand wird an Kieselgel chromatographiert mit Toluol : Ethylacetat (1:1). Das so erhaltene Produkt wird in Ether verrührt, abgesaugt und im Vakuum getrocknet.
Ausbeute: 2,60 g (70 % d.Th.)
Smp.: 119-20°C
R_{f} = 0,14 (VII)
MS (DCI, NH₃): m/z = 387 (M+NH₄)

### Beispiel 184

### 4-(Naphthyl-1-oxy)-benzol-sulfonsäure-N-benzylamid

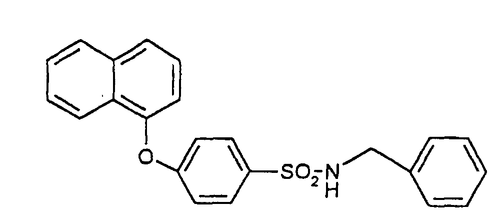

1-Naphthol (10,7 g; 74 mmol) und Kaliumcarbonat (20,5 g; 148 mmol) werden in DMF (200 ml) vorgelegt und 1,5 h bei Raumtemperatur gerührt. Nach Zugabe von 4-Fluor-benzolsulfonsäure-N-benzylamid (19,6 g; 74 mmol; Bull. Soc. Chim. Fr. 1961, 488) wird die Reaktionsmischung über Nacht bei 80°C und 5 h bei 120°C gerührt. Das DMF wird anschließend im Vakuum abkondensiert, der Rückstand wird mit Wasser versetzt und viermal mit Ethylacetat extrahiert. Die vereinten organischen Phasen werden zweimal mit Wasser gewaschen, getrocknet (MgSO₄) und im Vakuum eingeengt. Der Rückstand wird an Kieselgel mit Toluol : Ethylacetat (10:1) chromatographiert. Das so erhaltene Produkt wird mit Diethylether verrührt, abgesaugt und im Vakuum getrocknet.
Ausbeute: 3,45 g (12 % d.Th.)
Smp.: 144-46°C
R_{f} = 0,39 (IV)
MS (ESI): m/z = 390 (M+H)

### Beispiel 185

### 1-N-(n-Pentylsulfonyl)amino-4-(2-ethoxycarbonylindan-4-oxy)benzol

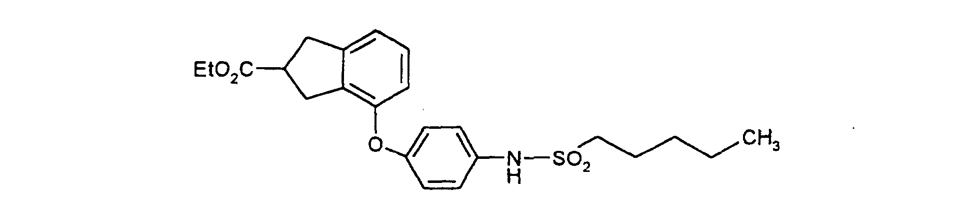

Die Herstellung erfolgte in Analogie zur Herstellung des Beispiels 1 ausgehend von Beispiel 98 A (0,546 g; 1,84 mmol) und 1-Pentansulfonsäurechlorid (0,313 g; 1,84 mmol).
Ausbeute: 0,432 g (70 % d.Th.)
R_{f} = 0,45 (VII)
MS (ESI): m/z = 432 (M+H)

### Beispiel 186

### 1-N-(n-Pentylsulfonyl)amino-4-(2-hydroxymethylindan-4-oxy)-benzol

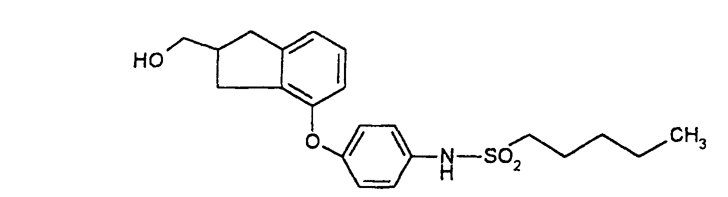

Die Herstellung erfolgt in Analogie zur Herstellung des Beispiels 91 ausgehend von Beispiel 185 (0,260 g; 0,60 mmol).
Ausbeute: 0,209 g (87 % d.Th.)
R_{f} = 0,56 (VII)
MS (ESI): m/z = 412 (M+Na)

### Beispiel 187

### N-(3-Fluor-(5-naphthyl-1-oxy)-phenyl)-N-hydroxy-1-pentan-sulfonsäurechlorid

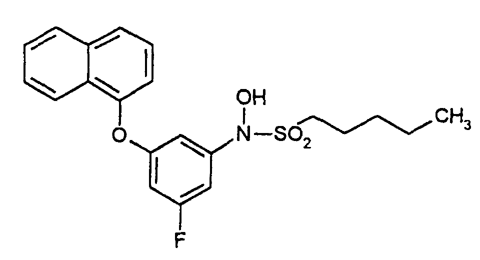

Die Herstellung erfolgte in Analogie zur Herstellung des Beispiels 1 ausgehend von Beispiel 101 A (1,29 g; 5,10 mmol) und 1-Pentansulfochlorid (0,91 g; 5,36 mmol).
Ausbeute: 0,24 g (12 % d.Th.)
R_{f} = 0,27 (X)
MS (FAB): m/z = 404 (M+H)

### Beispiel 188

### 1-[(4,4,4-Trifluor-1-butyl)sulfonyloxy]-3-(naphthyl-1-oxy)benzol

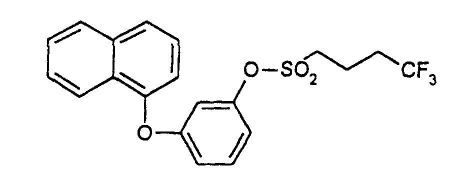

Die Herstellung erfolgte in Analogie zur Herstellung des Beispiels 97 ausgehend von Beispiel 63 A (0,709 g; 3,00 mmol).
Ausbeute: 1,10 g (89% d.Th.)
R_{f} = 0,50 (XXX)
MS (DCI, NH₃): m/z = 428 (M+NH₄)

### Beispiel 189

### 5-[(4,4,4-Trifluor-1-butyl)sulfonylamino]-2-(naphthyl-1-oxy)-pyridin

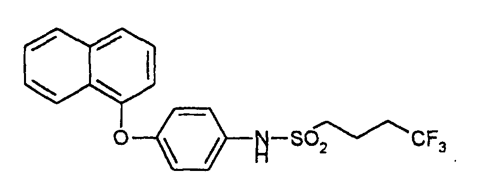

Die Herstellung erfolgte in Analogie zur Hestellung des Beispiels 1 ausgehend von Beispiel 43 A (0,945 g; 4,00 mmol).
Ausbeute: 1,20 g (75% d.Th.)
Smp.: 136-137°C
R_{f} = 0,69 (VII)
MS (DCI /NH₃): m/z = 411 (M+H)

In Analogie zur Herstellung von Beispiel 1 werden die in Tabelle 12 aufgeführten Beispiele hergestellt:

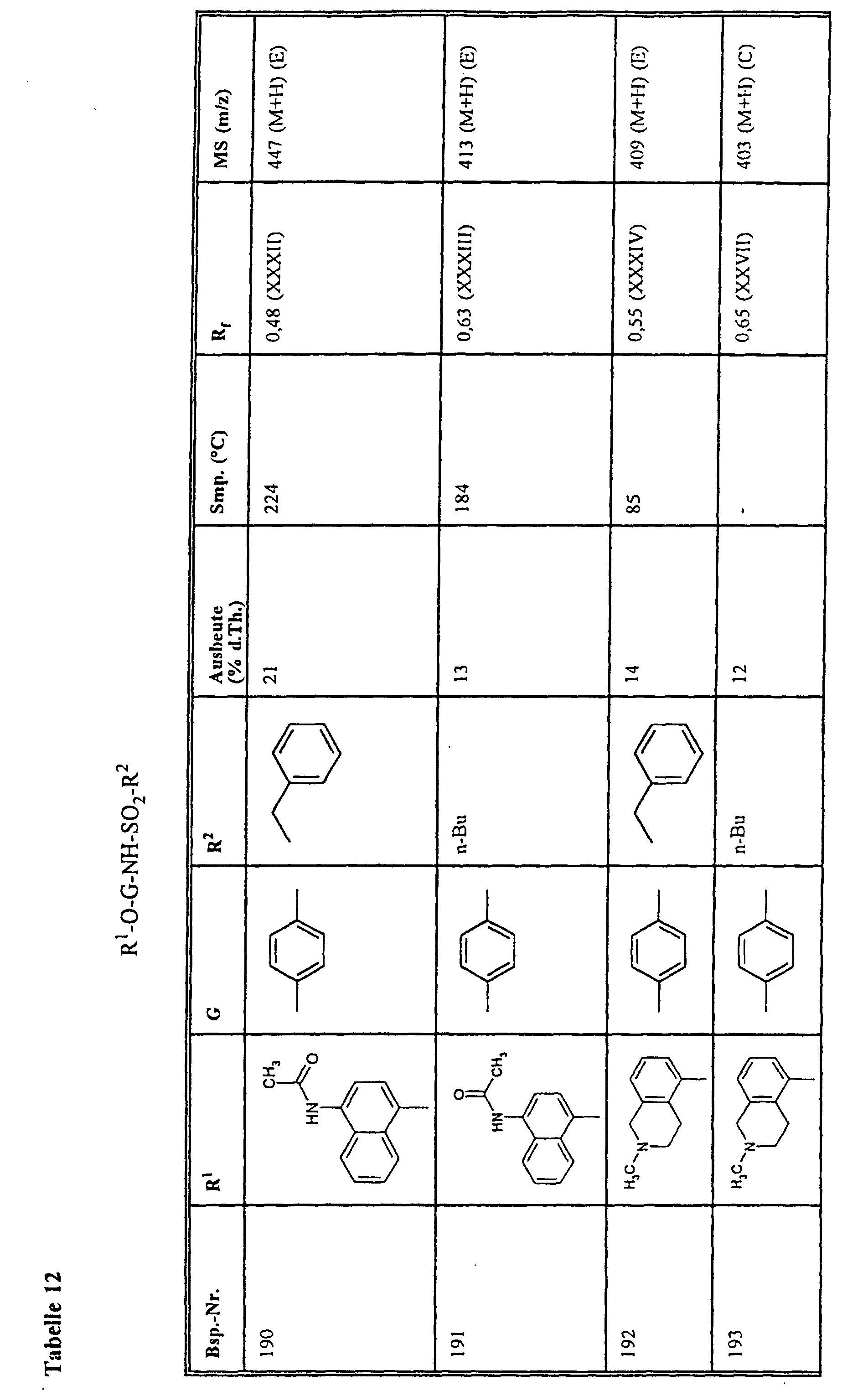

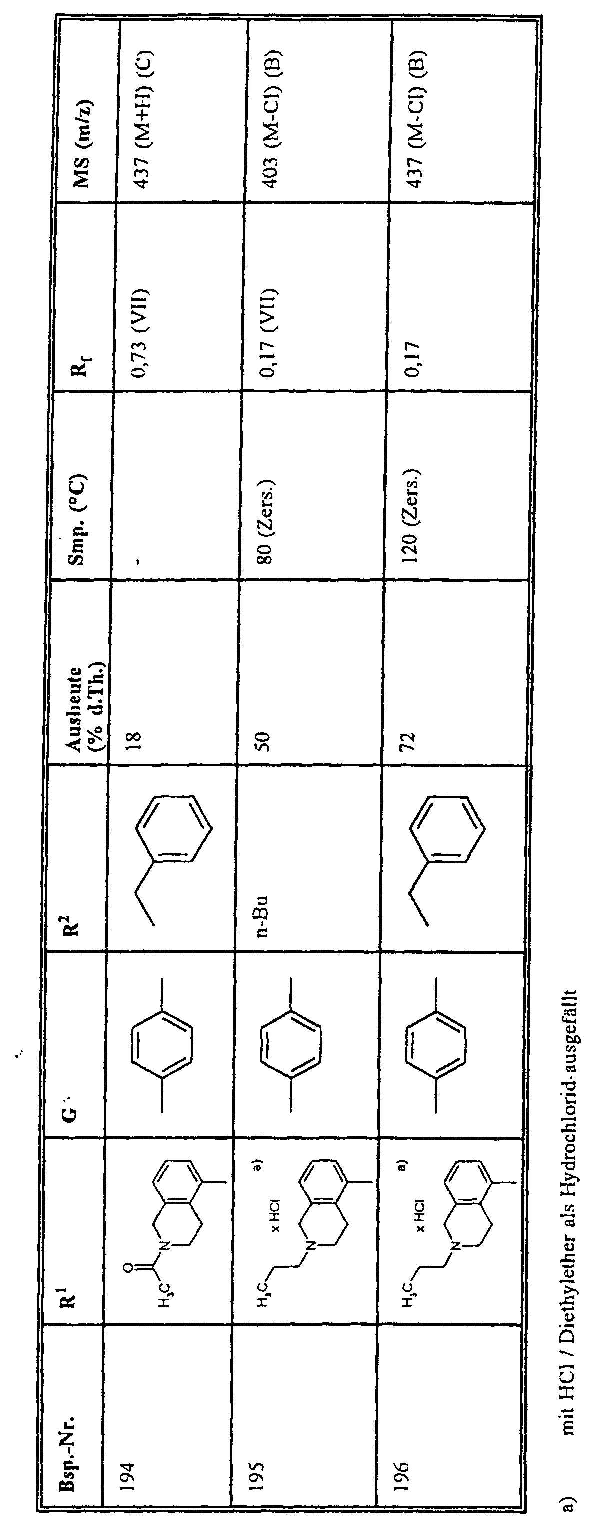

In Analogie zur Vorschrift des Beispiels 2 erfolgt die Herstellung der in Tabelle 13 aufgelisteten Verbindungen.

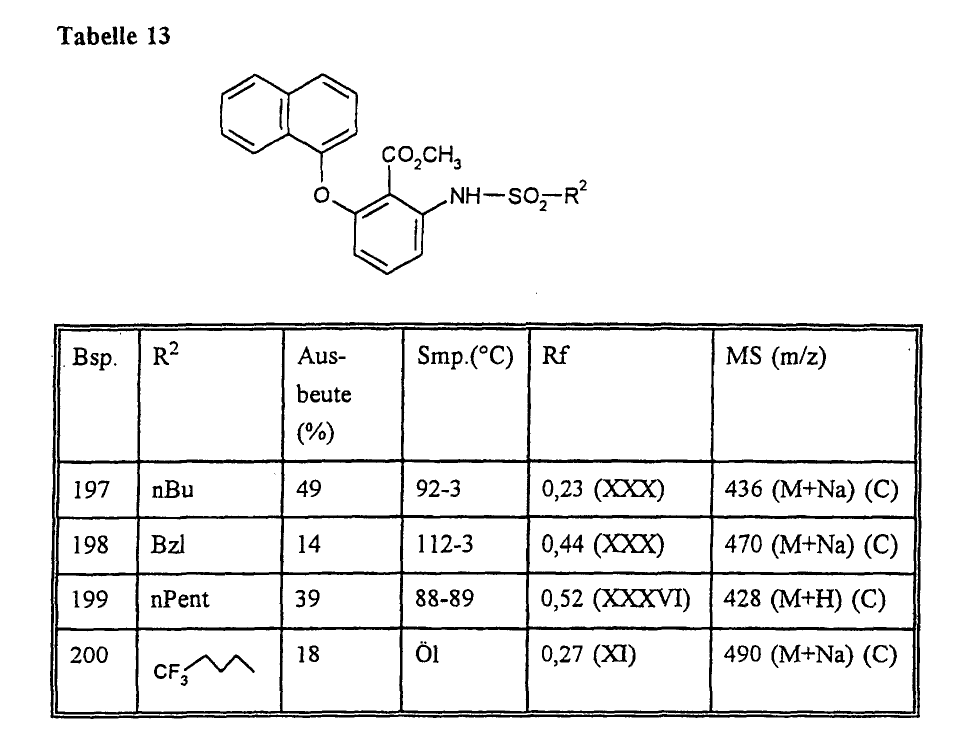

### Beispiel 201

### 2-(n-Butylsulfonylamino)-4-(1-Naphthyloxy)-benzoesäuremorpholinamid

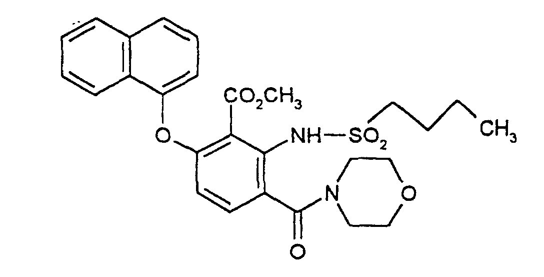

Zu einer Lösung von Beispiel 172 (0,420 g, 1,05 mmol) und Morpholin (90 µl; 11mmol) in DMF (5 ml) wurden Triethylamin (1,8 ml; 13 mmol) und 20 % Propanphosphonsäureanhydrid/ Ethylacetat (1,04 ml; 1,58 mmol) gegeben und über Nacht bei Raumtemperatur gerührt. Nach Zugabe der gleichen Mengen Morpholin, Triethylamin und Propanphosphorsäureanhydridlösung und Rühren über Nacht wurde das Reaktionsgemisch auf Wasser gegossen und mit Ethylacetat extrahiert. Die organischen Phasen wurden über Na₂SO₄ getrocknet, filtriert, eingeengt, der Rückstand wurde über Kieselgel chromatographiert (Methylenchlorid:Methanol = 30:1). Umkristallisation aus Methanol ergab weiße Kristalle.
- Ausbeute:: 33 mg (6,29 % d. Th.)
- Fp.:: 105-108°C
- R_{f}:: 0,55 (XXV)
- MS:: 469 (M+H) (B)

In Analogie zur Vorschrift des Beispiels 201 erfolgt die Herstellung der in Tabelle 14 aufgelisteten Verbindungen.

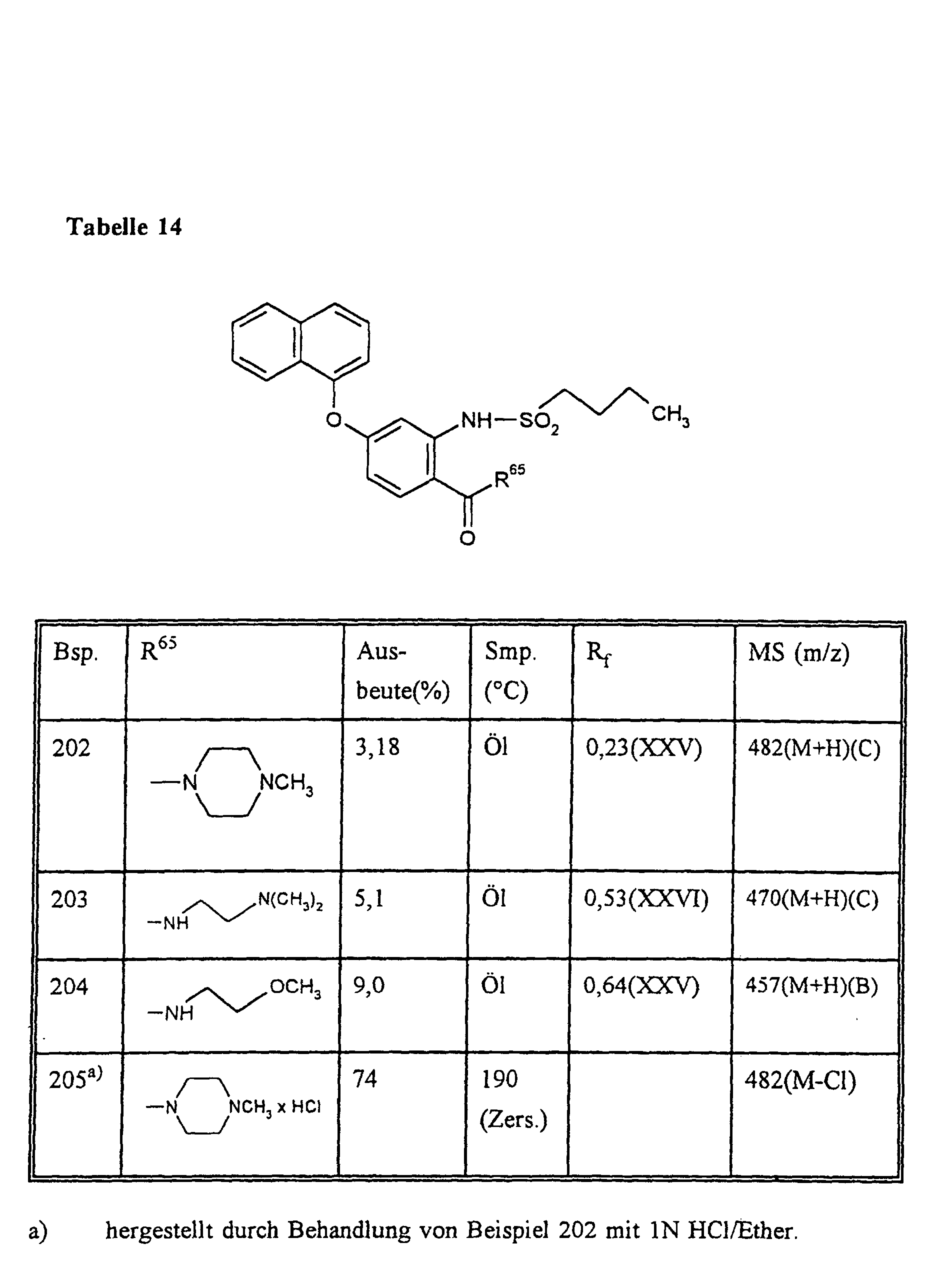

In Analogie zur Vorschrift des Beispiels 91 erfolgt die Herstellung der in Tabelle 15 aufgelisteten Verbindungen.

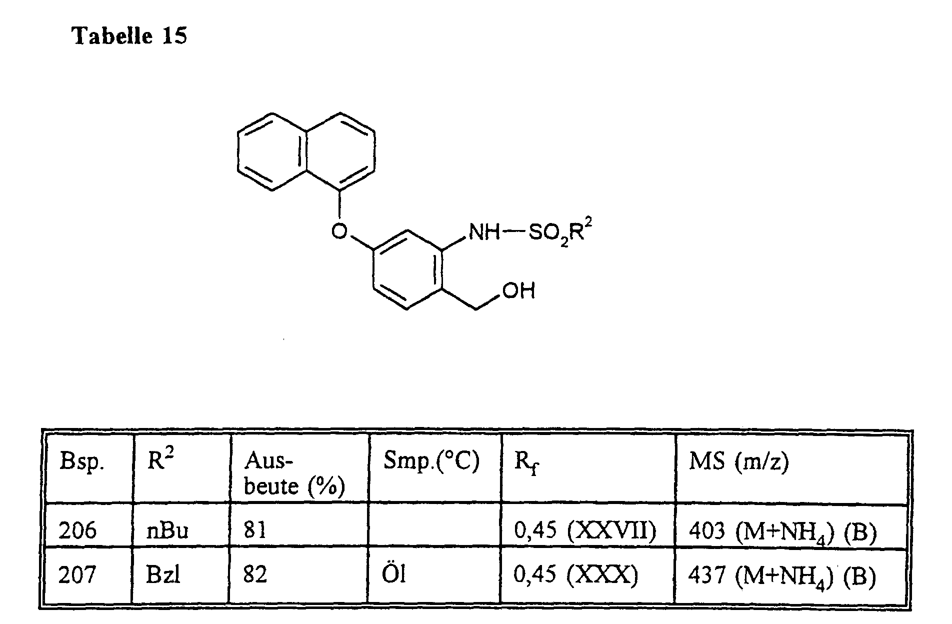

In Analogie zur Vorschrift des Beispiels 1 erfolgt die Herstellung der in Tabelle 16 aufgelisteten Verbindungen.

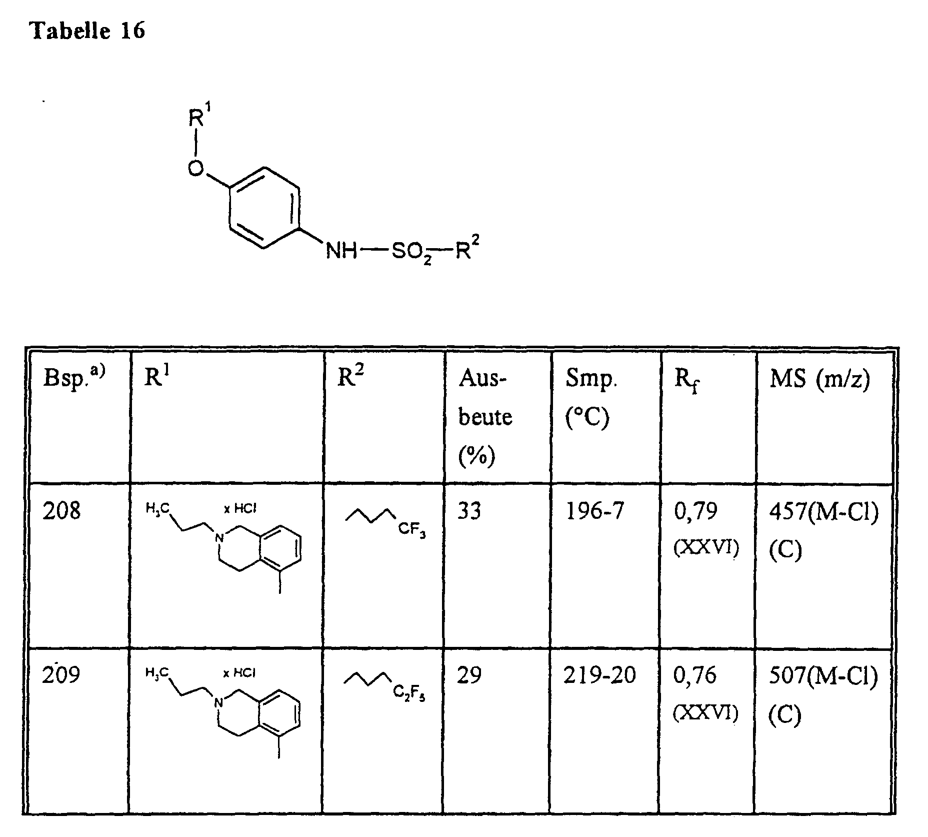

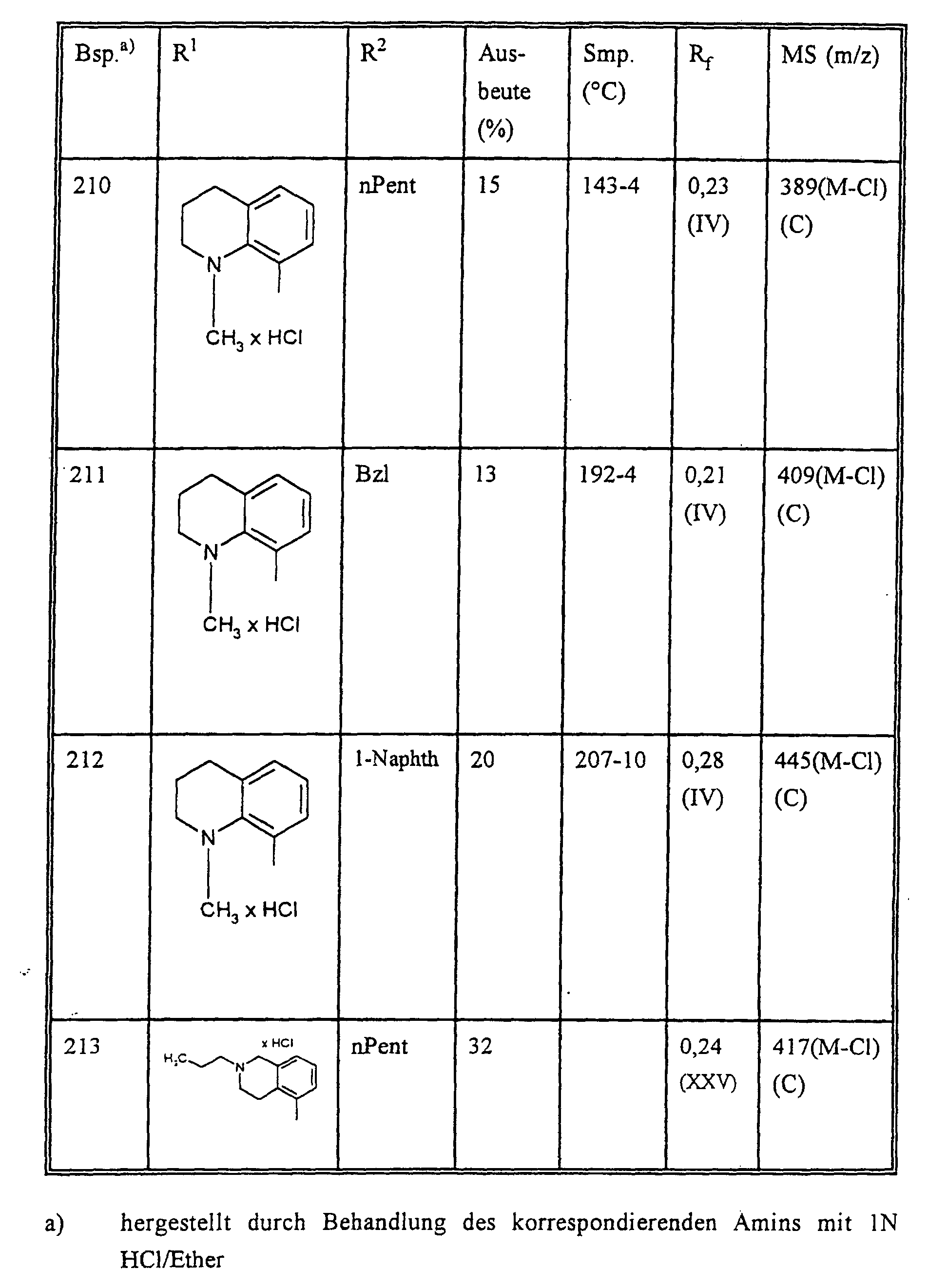

In Analogie zur Vorschrift des Beispiels 97 erfolgt die Herstellung der in Tabelle 17 aufgelisteten Verbindungen.

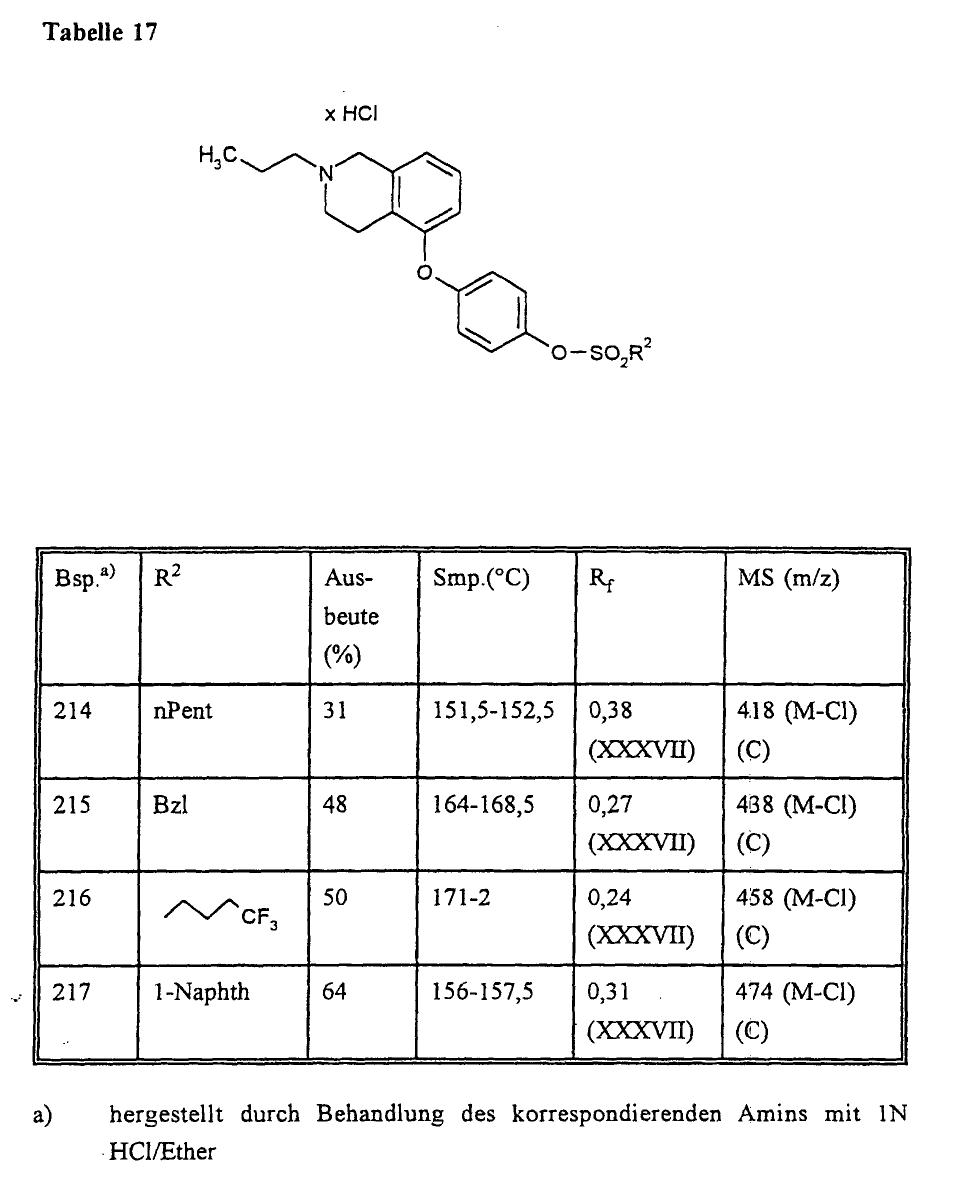

In Analogie zur Herstellung von Herstellungsbeispiel 1 wurden die in Tabelle 18 aufgeführten Verbindungen hergestellt.

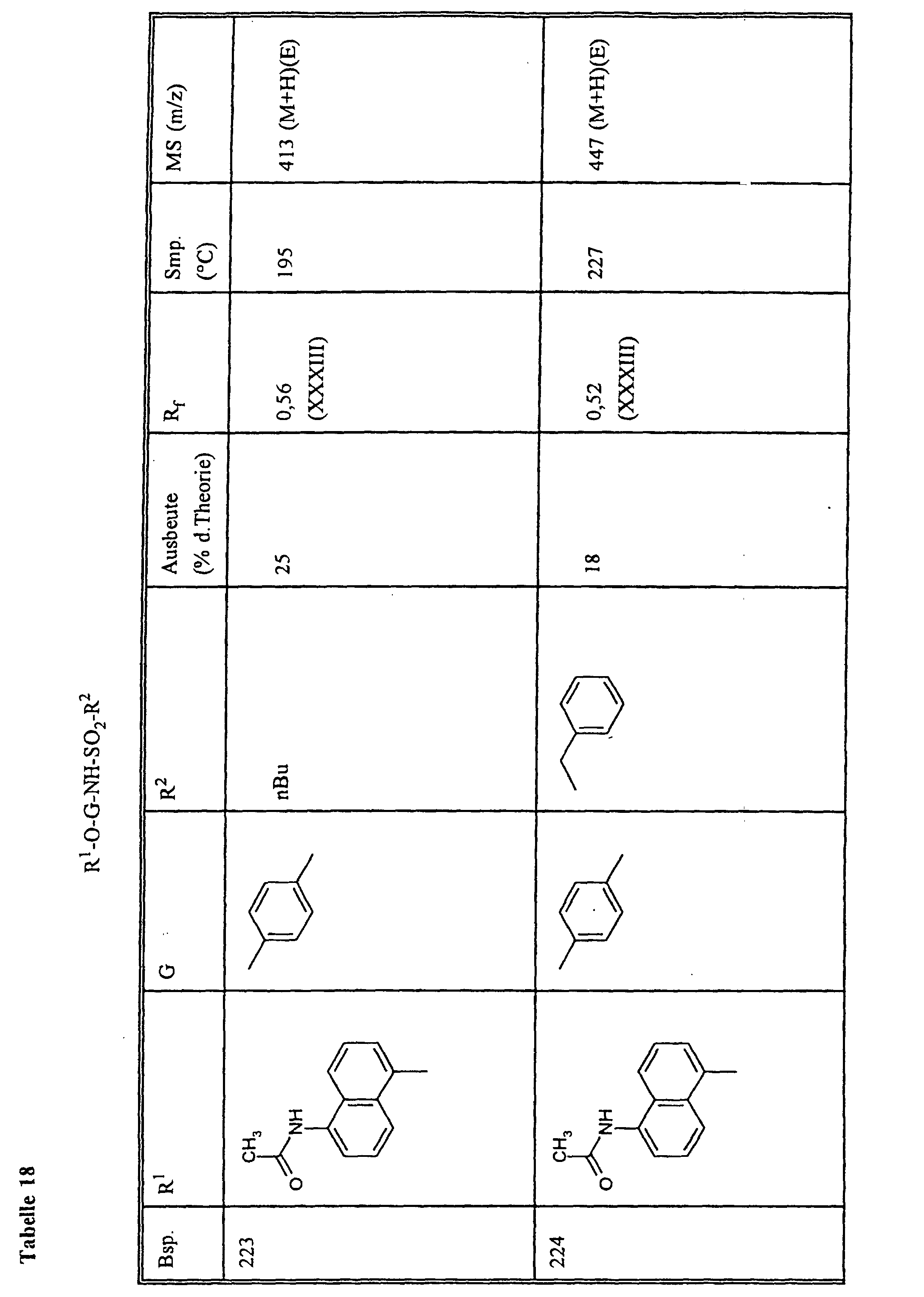

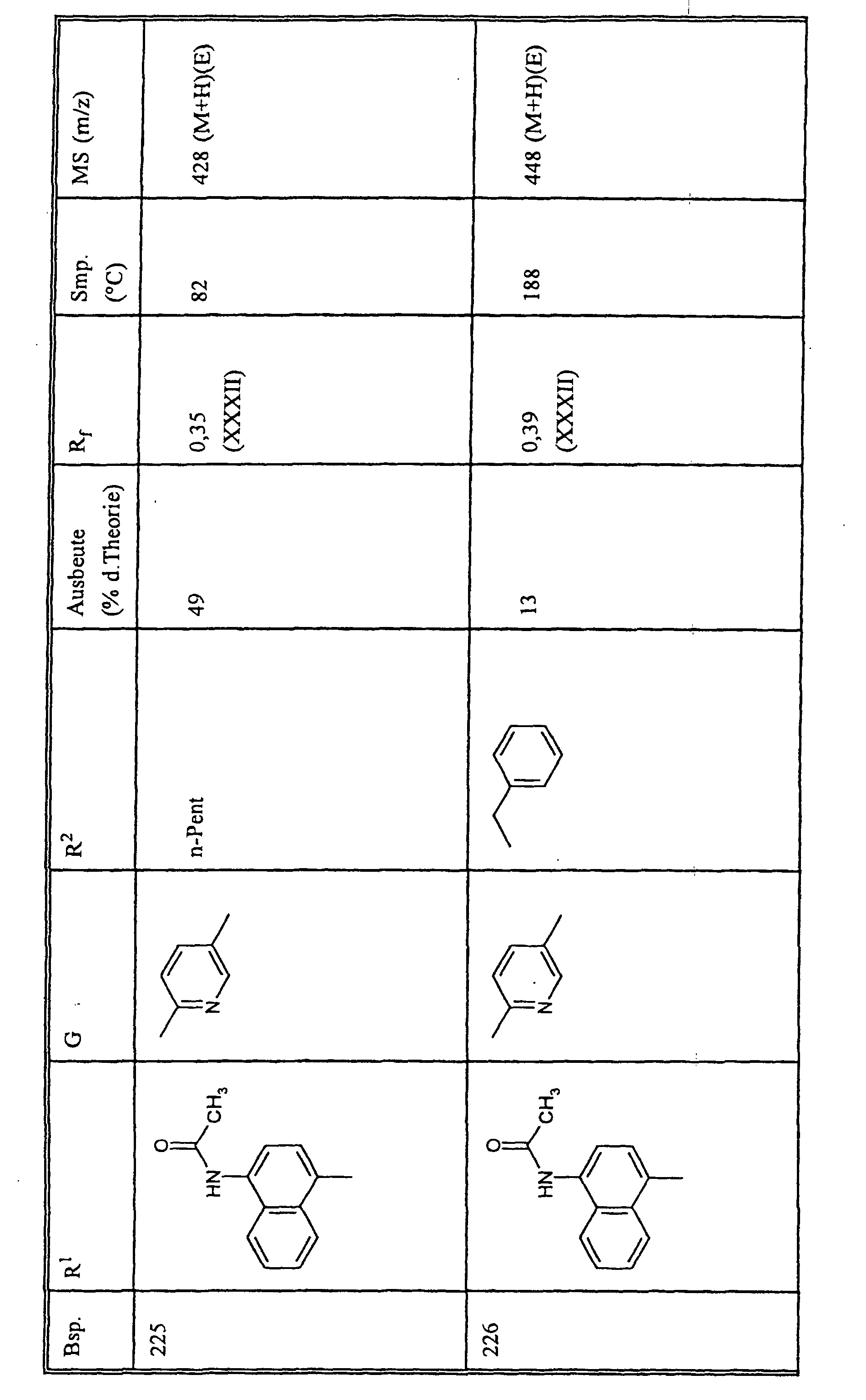

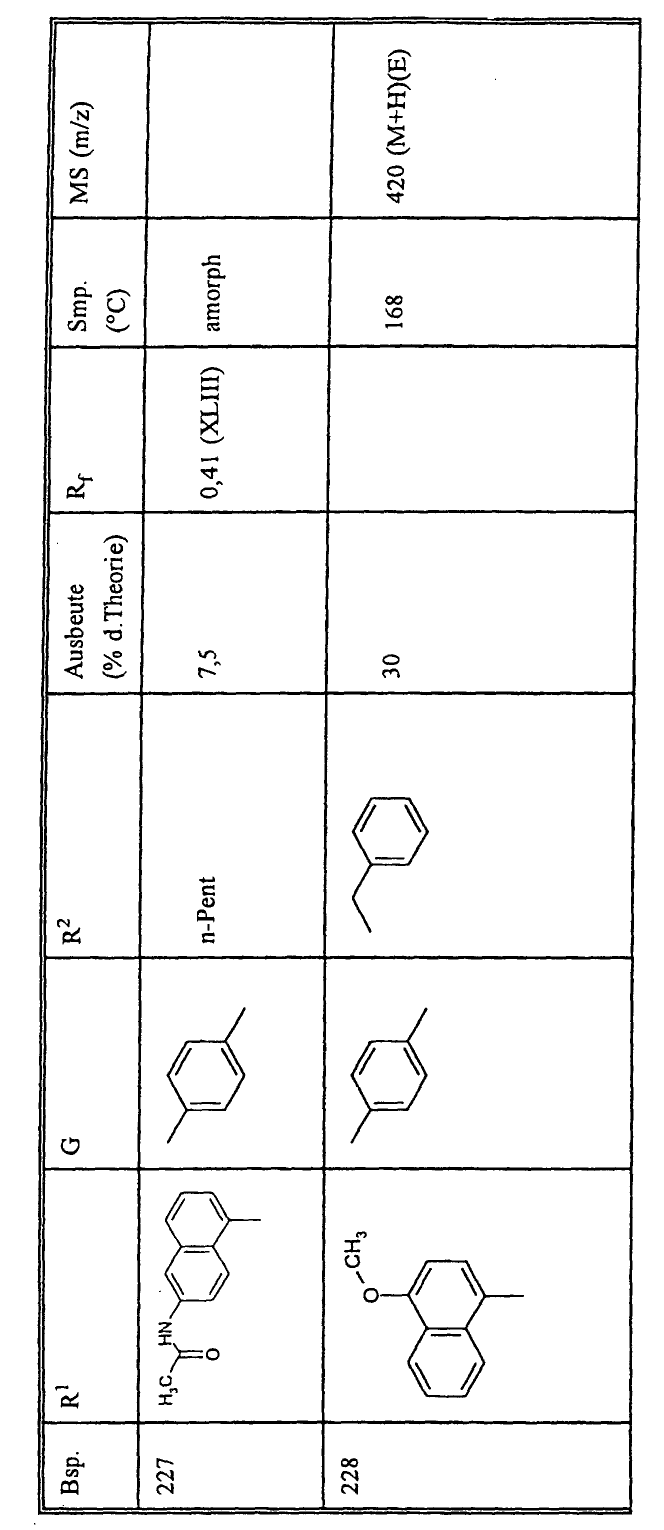

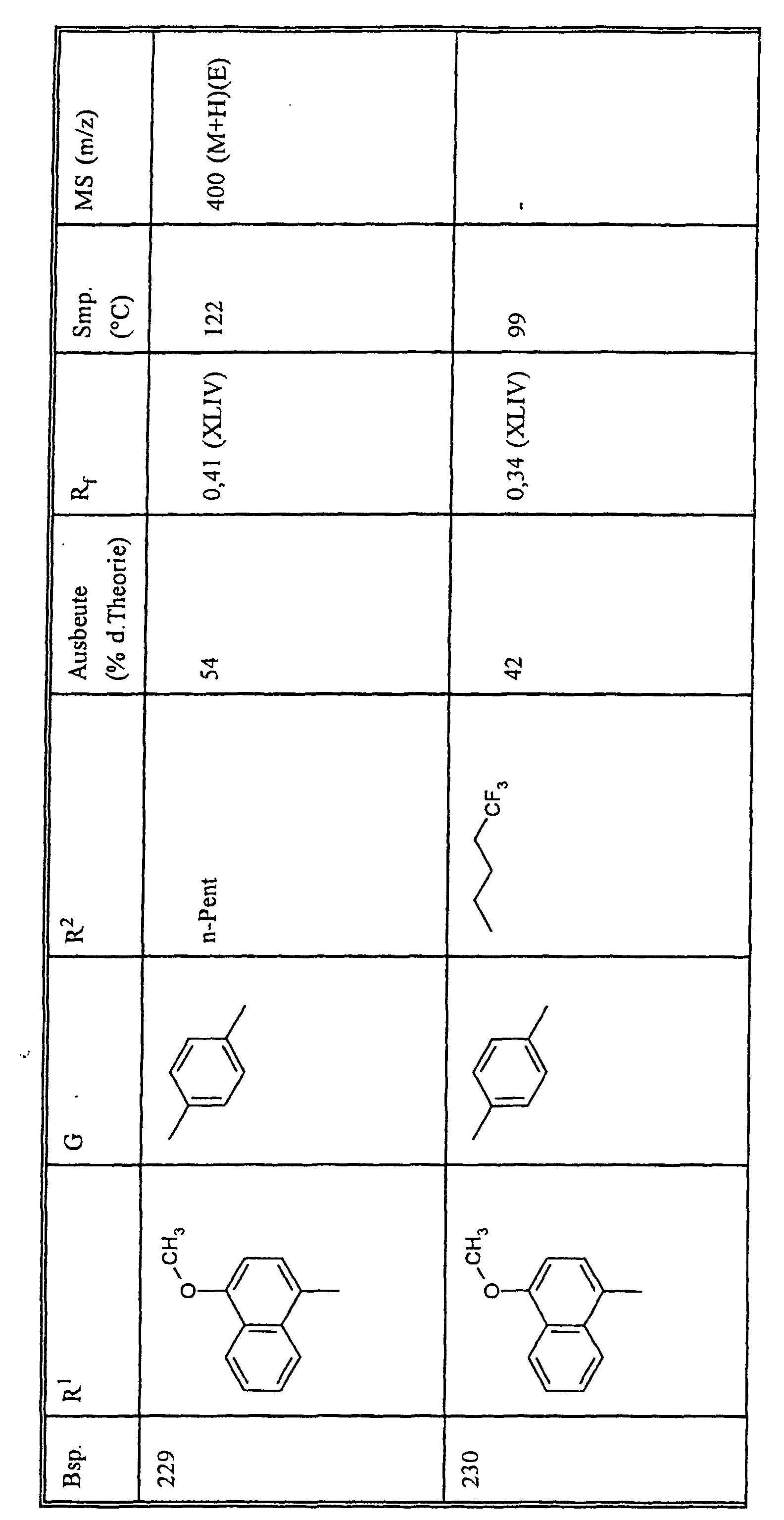

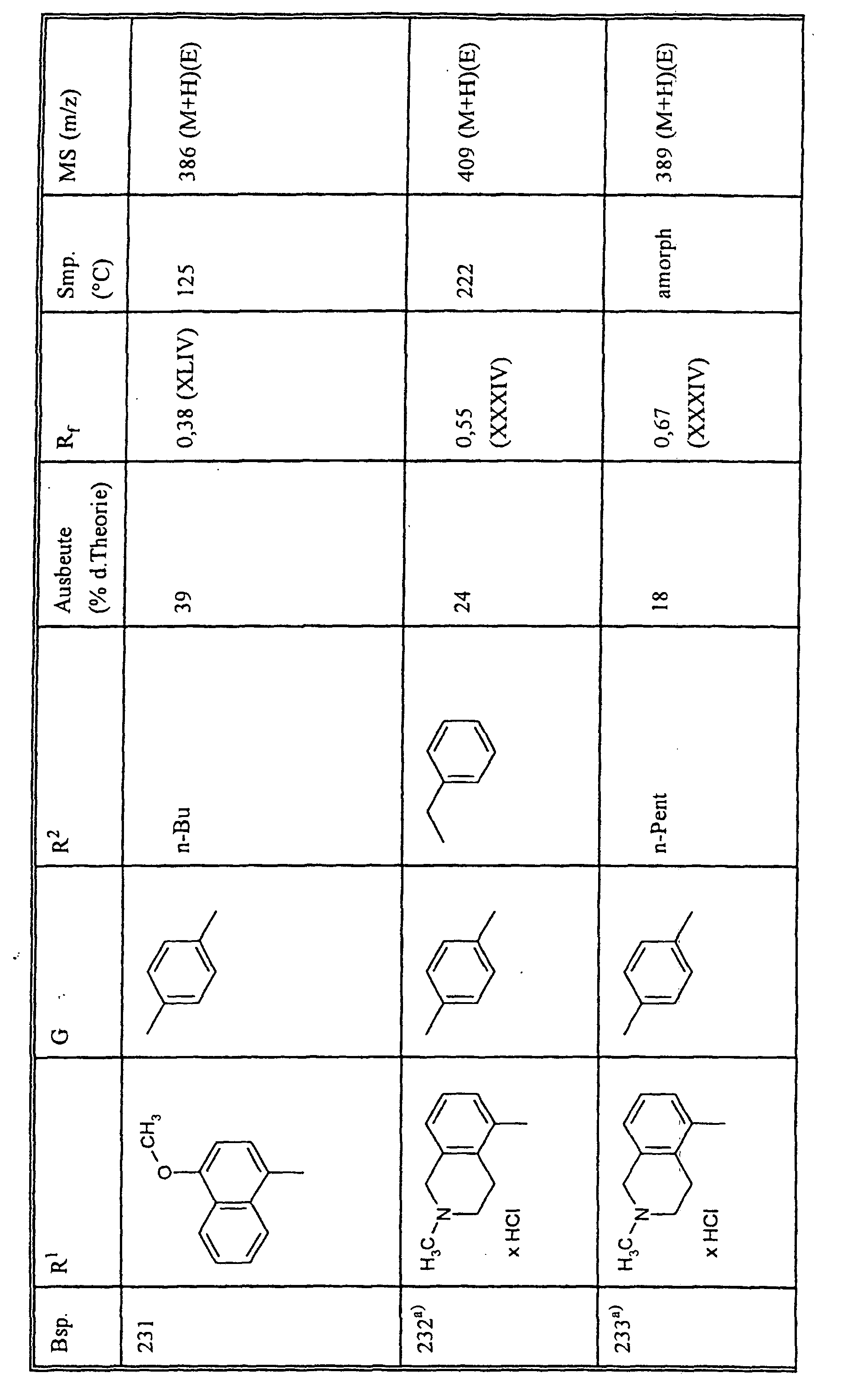

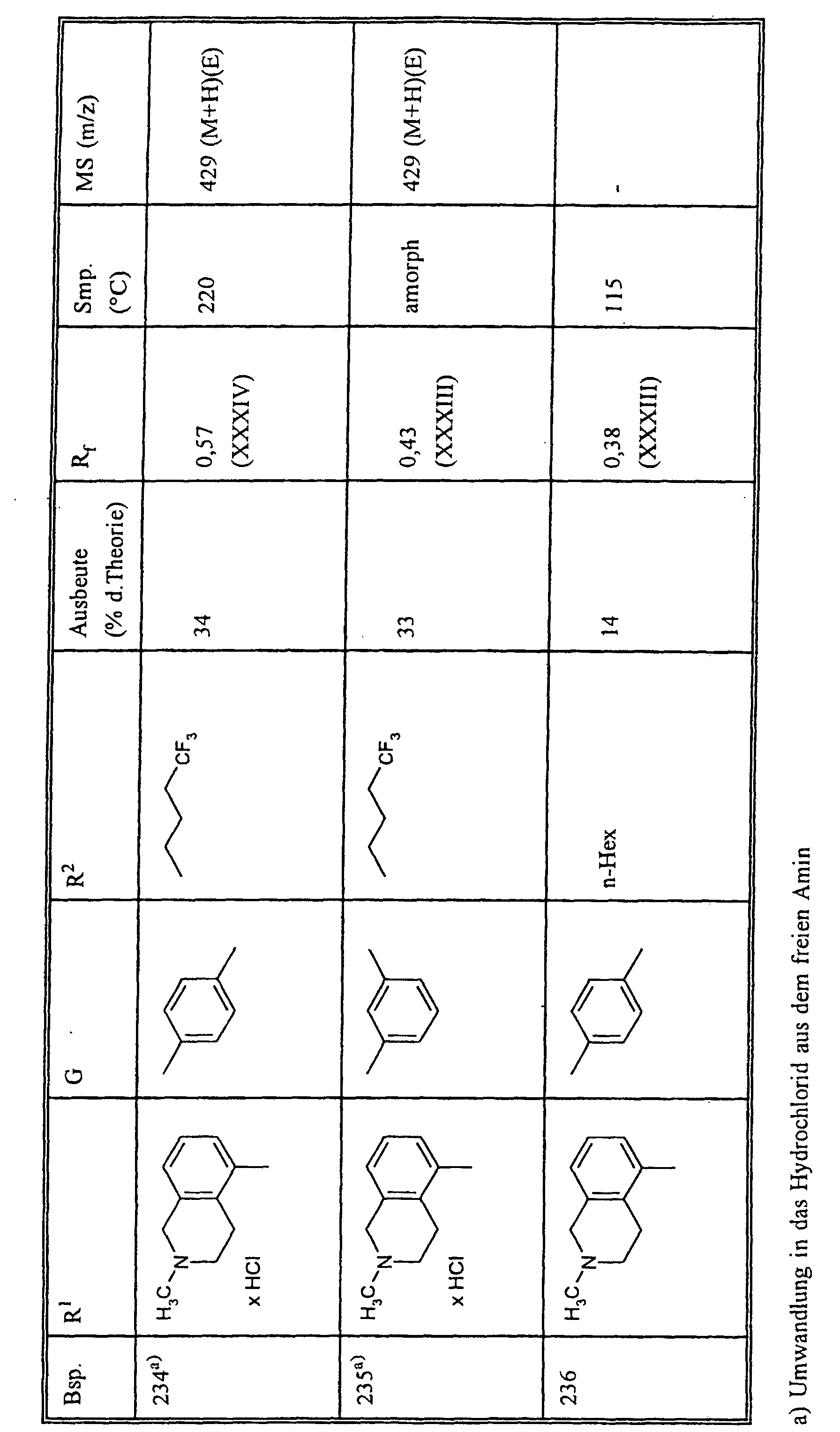

### Beispiel 237

### 1-Bis-N-(1-pentylsulfonyl)amino-4-(2-methyl-1,2,3,4-tetrahydroisochinolin-5-yloxy)-benzol

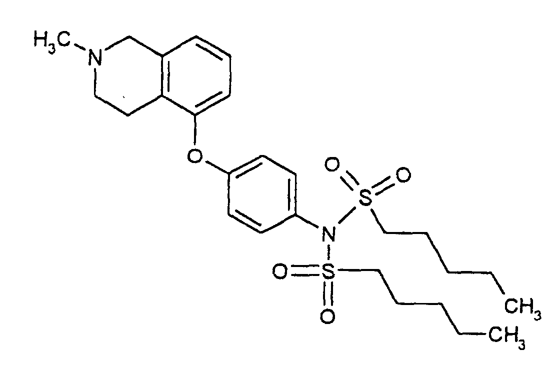

Die Verbindung aus Beispiel 108 A (3,5 g, 13,8 mmol) wird analog zur Vorschrift der Verbindung aus Beispiel 3 mit 1-Pentansulfonylchlorid (5,17 g, 30,3 mmol) und Triethylamin (9,6 ml, 70 mmol) in Dichlormethan (30 ml) bei 35 bis 40°C umgesetzt. Nach vollständiger Umsetzung wird der Ansatz mit Wasser, Natriumhydrogencarbonat-Lösung und Wasser extrahiert. Die organische Phase wird über Natriumsulfat getrocknet und im Vakuum eingeengt. Das Rohprodukt wird säulenchromatographisch über Kieselgel gereinigt (Dichlormethan/Methanol, 98:2).

Ausbeute: 1,7 g, (24 % d. Th.)
R_{f} = 0,58 (XLV)
MS (DCI, Isobutan): m/z = 523 (M+H)

### Beispiel 238

### 1-Bis-N-(1-pentylsulfonyl)amino-4-(1,2,3,4-tetrahydroisochinolin-5-yl-oxy)-benzol-Hydrochlorid

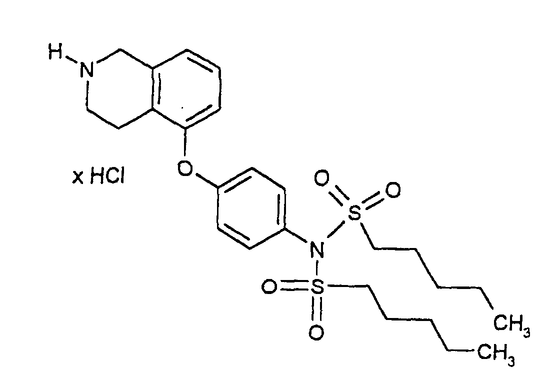

Zu einer Lösung aus der Verbindung aus Beispiel 237 (1 g, 1,92 mmol) in absolutem 1,2-Dichlorethan wird bei 0°C α-Chlorethylchlorformiat (1,1 g, 7,7 mmol) gegeben. Anschließend wird 16 Stunden unter Rückfluß erhitzt. Der Reaktionsansatz wird im Vakuum eingeengt, mit Methanol (20 ml) versetzt und 1 h unter Rückfluß erhitzt. Nach Beendigung der Reaktion wird im Vakuum eingeengt und der Rückstand aus absolutem Ethanol (13 ml) umkristallisiert.

Ausbeute: 625 mg (64,0 % d. Th.)
R_{f} = 0,22 (XXXIII)
Smp.: 162°C
MS (DCI, Isobutan): m/z = 509 (M+H)

### Beispiel 239

### 1-Bis-N-(1-pentylsulfonyl)amino-4-(2-isopropyl-1,2,3,4-tetrahydroisochinolin-5-yloxy)-benzol

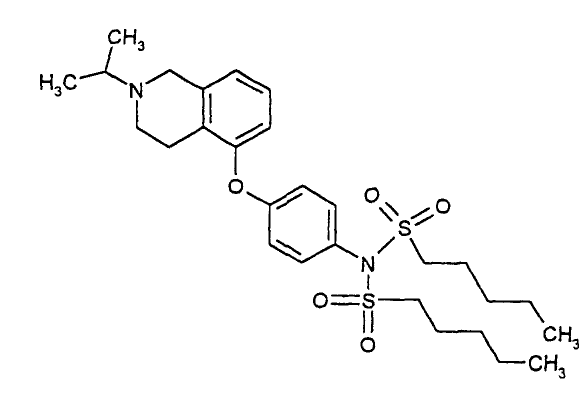

Zu einer Lösung aus der Verbindung aus Beispiel 238 (300 mg, 0,55 mmol) in absolutem Methanol (15 ml) wird bei Raumtemperatur Aceton (1,0 g, 17,2 mmol), Molekularsieb (20 Perlen, 3 Å) und Natriumcyanoborhydrid (240 mg, 2,81 mmol) gegeben. Der pH des Reaktionsansatzes wird mit wenigen Tropfen Essigsäure zwischen 5 und 6 eingestellt. Es wird 20 Stunden bei Raumtemperatur gerührt. Anschließend wird der Ansatz mit Natronlauge alkalisch gestellt, mit Dichlormethan extrahiert, die organische Phase über Natriumsulfat getrocknet und im Vakuum eingeengt.

Ausbeute: 300 mg Rohprodukt, welcher direkt weiter zu Beispiel 240 umgesetzt wird.
R_{f} = 0,37 (XXXIII)
MS (DCI, Isobutan): m/z = 551 (M+H)

### Beispiel 240

### N-(1-Pentylsulfonyl)amino-4-(2-isopropyl-1,2,3,4-tetrahydroisochinolin-5-yl-oxy)-benzol-Hydrochlorid

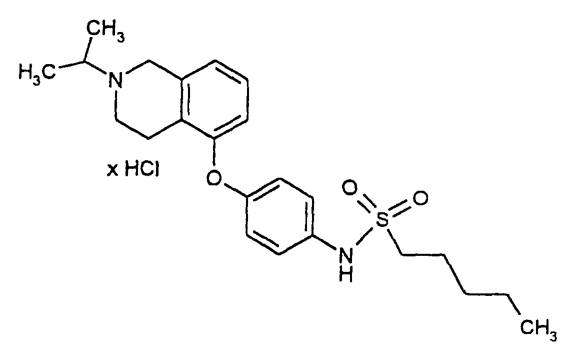

Eine Lösung aus der Verbindung aus Beispiel 239 (370 mg, 0,672 mmol) in Tetrahydrofuran (10 ml) und 1 N Natronlauge (1,35 ml, 1,35 mmol) wird 8 Stunden bei Raumtemperatur gerührt. Anschließend wird der Ansatz mit 1 N Salzsäure auf pH 1 angesäuert und mit Dichlormethan extrahiert. Die organische Phase wird über Natriumsulfat getrocknet und im Vakuum eingeengt. Das Produkt wird säulenchromatographisch über Kieselgel gereinigt (Eluationsmittel: Dichlormethan/Methanol, 98:2). Das Produkt wird nach Lösen in Ethanol, Versetzen mit 1 N Salzsäure und anschließendem Einengen im Vakuum in das Hydrochlorid überführt.

Ausbeute: 239 mg (79 % d. Th.)
R_{f} = 0,39 (XXXIII)
Smp.: amorph
MS (DCI, Isobutan): m/z = 417 (M+H)

### Beispiel 241

### 1-Bis-N-(1-pentylsulfonyl)amino-4-(2-butyl-1,2,3,4-tetrahydroisochinolin-5-yl-oxy)-benzol

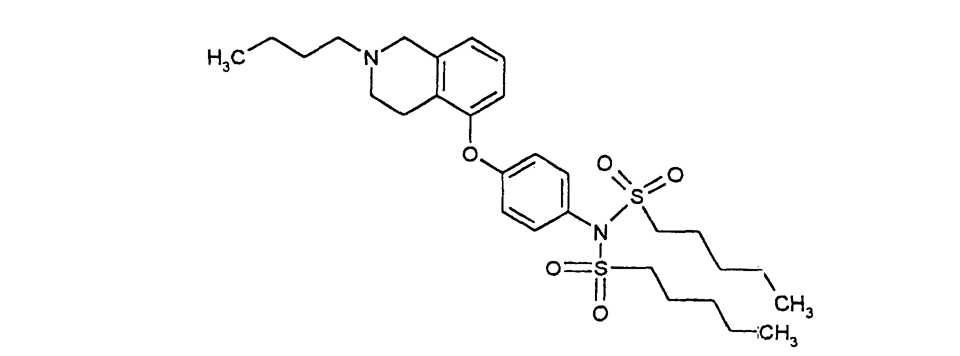

Das Produkt wird analog zum Beispiel 239 aus der Verbindung aus Beispiel 238 (215 mg, 0,394 mmol) und Butyraldehyd (889 mg, 12,3 mmol) hergestellt.

Ausbeute: 260 mg Rohprodukt, das direkt zu Beispiel 242 umgesetzt wird.
R_{f} = 0,7 (XXXIII)
MS (DCI, Isobutan): m/z = 565 (M+H)

### Beispiel 242

### N-(1-Pentansulfonyl)amino-4-(2-butyl-1,2,3,4-tetrahydroisochinolin-5-yl-oxy)-benzol

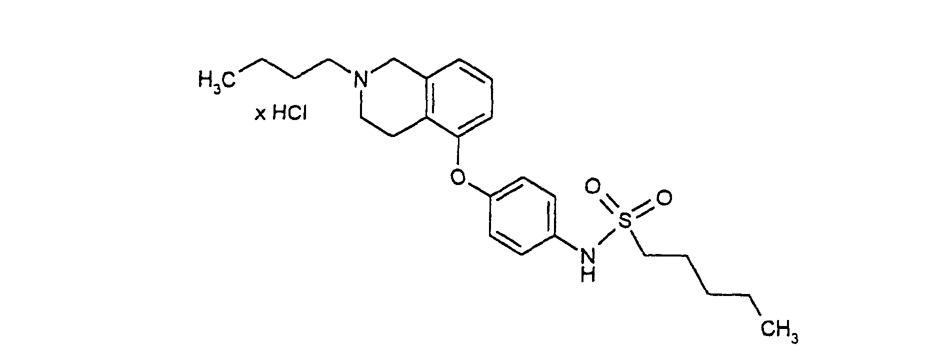

Das Produkt wird analog zum Beispiel 240 aus der Verbindung aus Beispiel 241 (255 mg, 0,451 mmol) hergestellt.

Ausbeute: 236 mg (64 % d. Th.)
R_{f} = 0,25 (XXXIII)
Smp.: 187 °C
MS (DCI, Isobutan): m/z = 431 (M+H)

In Analogie zur Herstellung des Beispiel 97 wurden die in der Tabelle 19 aufgeführten Beispiele hergestellt:

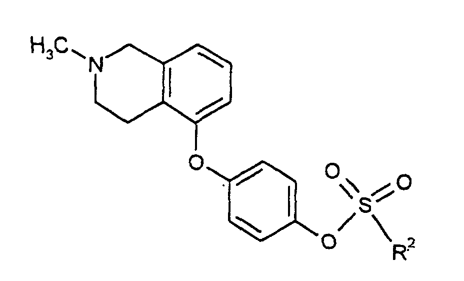

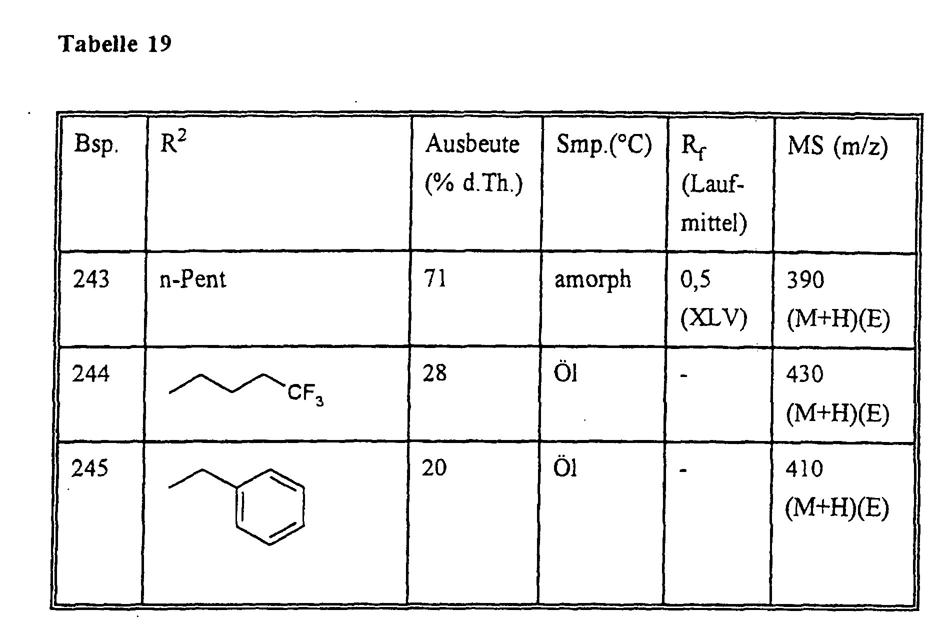

Die Verbindungen aus Tabelle 19 werden durch Lösen in Methanol oder Ethanol, Versetzen mit 1 N Salzsäure und anschließendem Einengen im Vakuum in die entsprechenden in der Tabelle 20 aufgeführten Hydrochloride überführt.

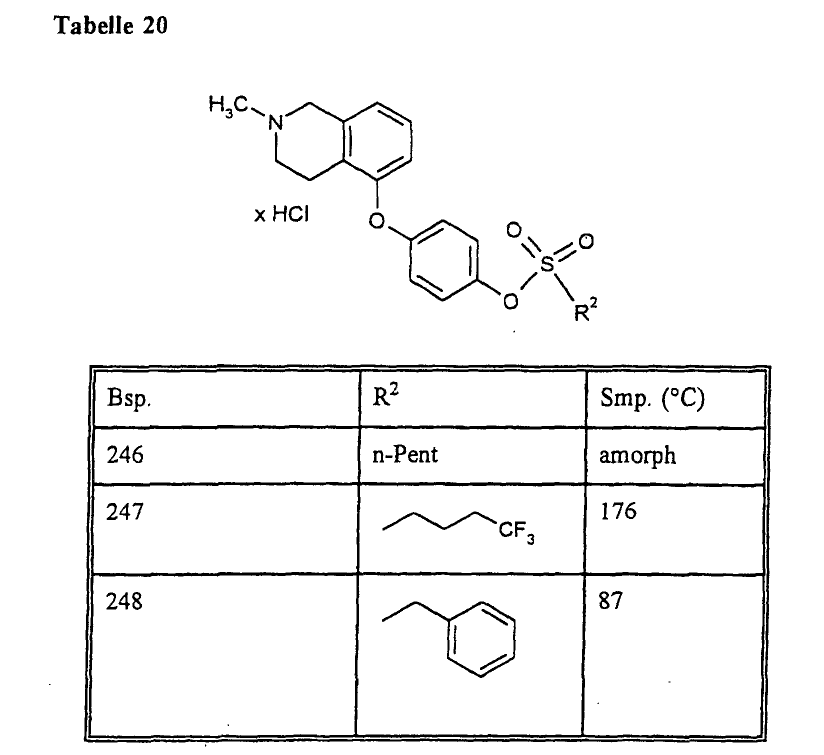

### Beispiel 249

### 4-(1,2,3,4-Tetrahydroisochinolin-5-yl-oxy)-1-(1-pentansulfonyl)oxy-benzol

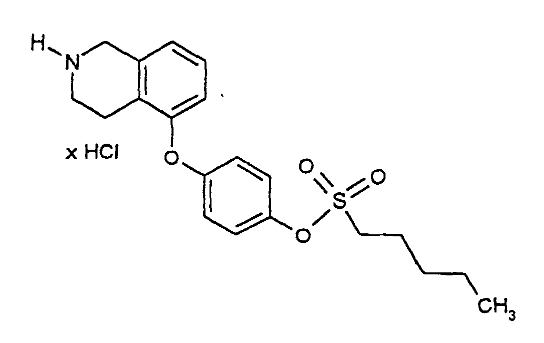

Das Produkt wird analog zum Beispiel 238 aus der Verbindung aus Beispiel 243 (2 g, 5,14 mmol) hergestellt.

Ausbeute: 1,60 g (75 % d. Th.)
R_{f} = 0,23 (XXXIII)
Smp.: 143°C
MS (DCI, Isobutan): m/z = 376 (M+H)

In Analogie zur Herstellung des Beispiels 97 wurden die in der Tabelle 2 aufgeführten Beispiele hergestellt. Die Amine werden durch Lösen in Methanol oder Ethanol, Versetzen mit 1 N Salzsäure und anschließendem Einengen im Vakuum in die Hydrochloride überführt.

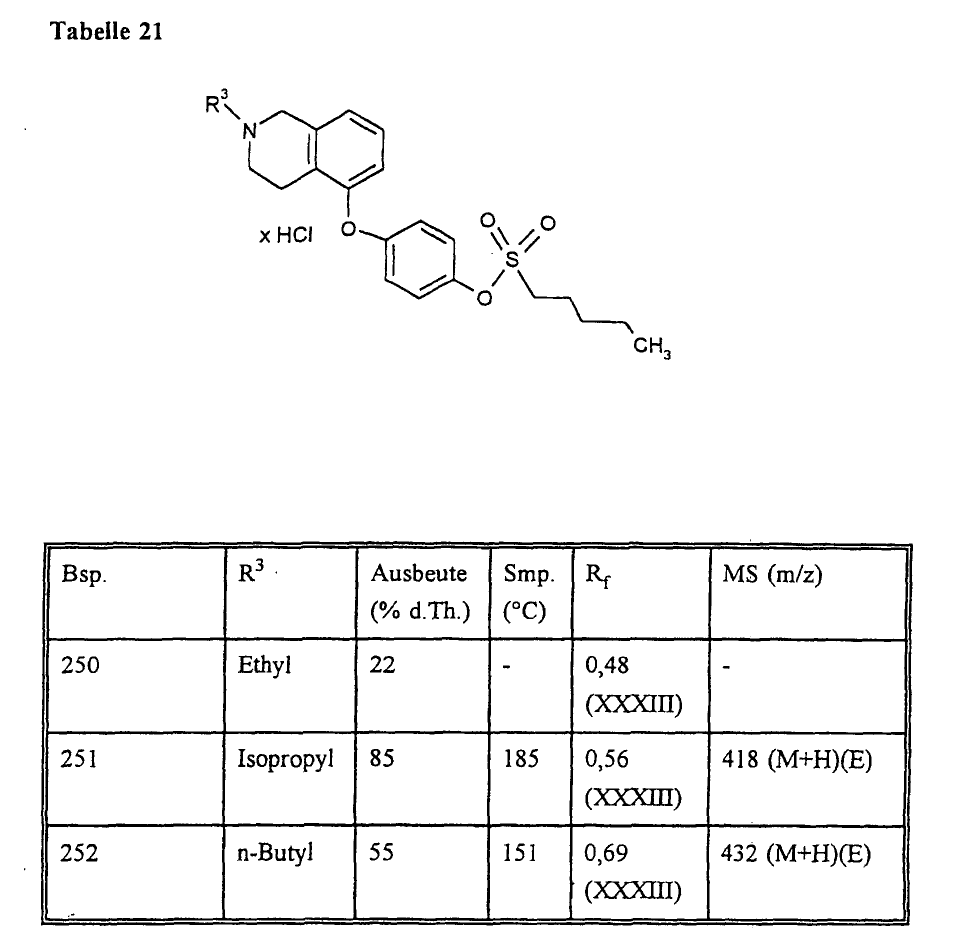

### Beispiel 253

### 1-(4-Aminonaphth-1-yl-oxy)-4-(benzylsulfonylamino)-benzol-Hydrochlorid

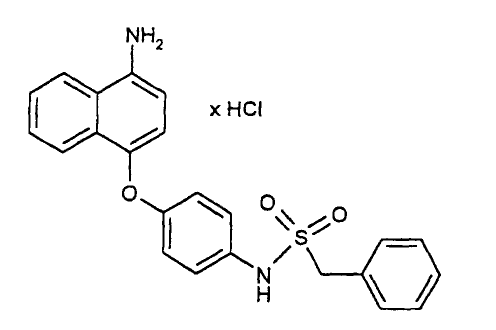

Die Verbindung aus Beispiel 190 (374 mg, 0,839 mmol) wird in warmem Ethanol (200 ml) gelöst. Nach Zugabe von halbkonzentrierter Salzsäure (200 ml) wird eineinhalb Stunden unter Rückfluß erhitzt und anschließend im Vakuum eingeengt.

Ausbeute: 370 mg (100 % d. Th.)
R_{f} = 0,46 (XLI)
Smp.: 252°C
MS (FAB): m/z = 405 (M+H)

### Beispiel 254

### 4-(Benzylsulfonylamino)-1-(4-ethylcarbonylamino-naphth-1-yl-oxy)benzol

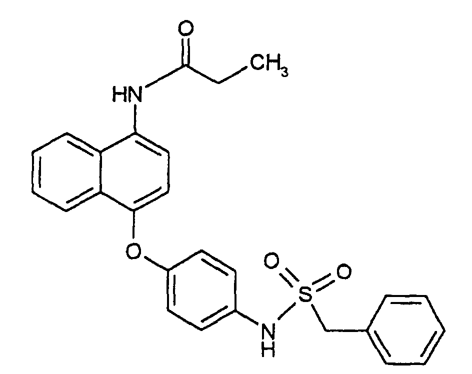

Eine Mischung bestehend aus der Verbindung aus Beispiel 253 (52 mg, 0,12 mmol) in absolutem Dichlormethan (40 ml) und absolutem Tetrahydrofuran (30 ml), Triethylamin (24 mg, 0,24 mmol) und Propionsäurechlorid (18 mg, 0,18 mmol) wird 16 Stunden bei Raumtemperatur gerührt. Der Reaktionsansatz wird im Vakuum eingeengt, und das Rohprodukt aus Ethanol umkristallisiert.

Ausbeute: 42 mg ( % d. Th.)
R_{f} = 0,35 (XLI)
Smp.: 180°C
MS (DCI, Isobutan): m/z = 461 (M+H)

In Analogie zur Herstellung des Beispiels 254 wurden die in der Tabelle 22 aufgeführten Beispiele hergestellt:

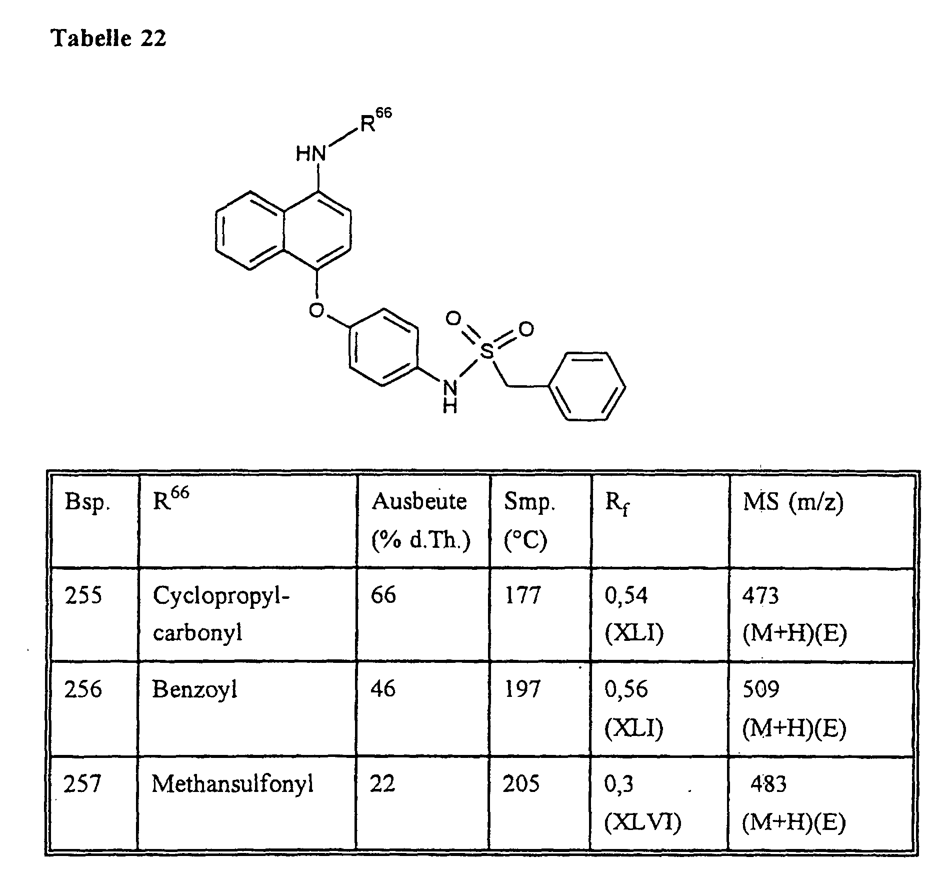

### Beispiel 258

### 2-(6-Hydroxymethyl-naphthyl-1-oxy)-5-(N-1-pentylsulfonyl)amino-pyridin

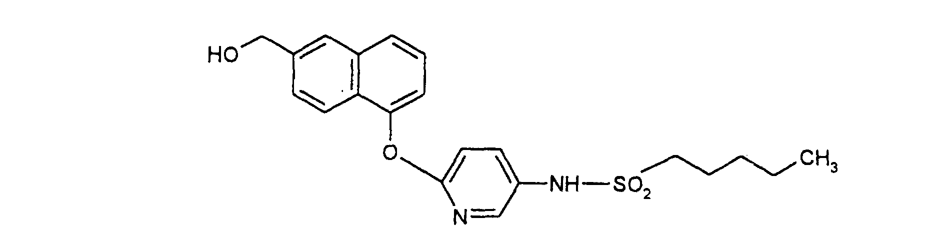

Die Herstellung erfolgte in Analogie zur Herstellung des Beispiels 1 ausgehend von Beispiel 143 A (7,30 g; 27,4 mmol).

Ausbeute: 2,98 g (27 % d. Th.)
R_{f} = 0,42 (VII)
MS (ESI): m/z = 401 (M+H)

### Beispiel 259

### 2-(6-Hydroxymethyl-naphthyl-1-oxy)-5-(4,4,4-trifluor-1-butylsulfonyl)aminopyridin

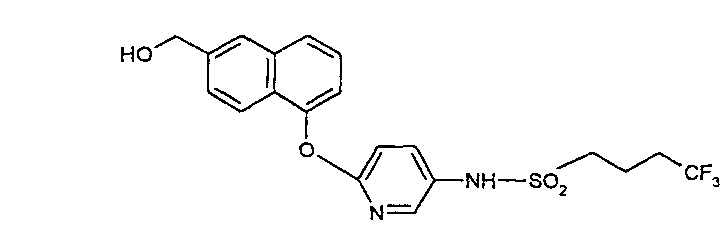

Die Herstellung erfolgte in Analogie zur Herstellung des Beispiels 1 ausgehend von Beispiel 143 A (1,01 g; 3,78 mmol).

Ausbeute: 0,62 g (36 % d. Th.)
Smp.: 60°C
R_{f} = 0,36 (VII)
MS (DCI/NH₃): m/z = 441 (M+H)

### Beispiel 260

### 3-(6-Methyl-naphthyl-1-oxy)-1-(4,4,4-trifluor-1-butylsulfonyl)amino-benzol

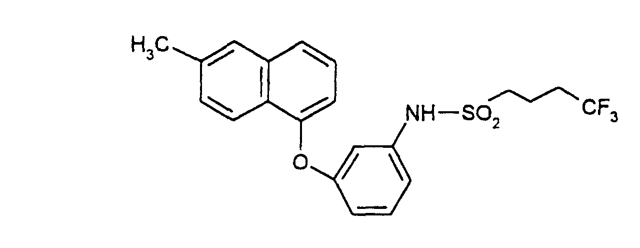

Die Herstellung erfolgte in Analogie zur Herstellung des Beispiels 1 ausgehend von Beispiel 141 A (0,90 g; 3,61 mmol).

Ausbeute: 1,09 g (71 % d. Th.)
Smp.: 75-77°C
R_{f} = 0,38 (Dichlormethan)
MS (ESI): m/z = 424 (M+H)

### Beispiel 261

### 5-(1-Butylsulfonyl)amino-2-(naphthyl-1-oxy)-benzoesäure-N-morpholinamid

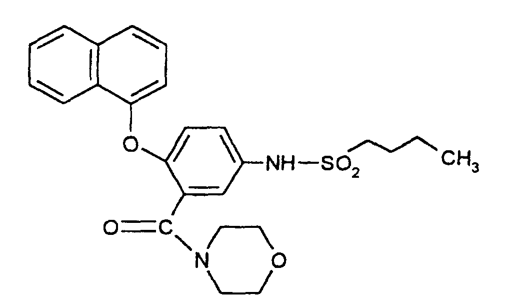

Die Herstellung erfolgte in Analogie zur Herstellung des Beispiels 79 ausgehend von Beispiel 75 (0,509 g; 1,27 mmol).

Ausbeute: 0,425 g (71 % d. Th.)
R_{f} = 0,29 (Dichlormethan:MeOH = 40:1)
MS (DCI, NH₃): m/z = 486 (M+H)

### Beispiel 262

### 4-(Naphth-1-yl-oxy)-2-(1-N-pentylsulfonyl)aminopyridin

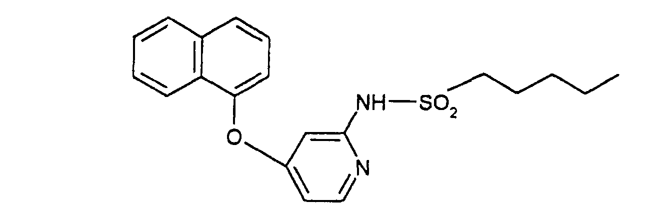

Die Herstellung erfolgte in Analogie zur Herstellung des Beispiels 2 ausgehend von Beispiel 139 A (0,300 g; 1,27 mmol).

Ausbeute: 0,164 g (35 % d. Th.)
R_{f} = 0,66 (VII)
MS (ESI): m/z = 371 (M+H)

### Beispiel 263

### 2-(N-Benzylsulfonyl)amino-4-(naphthyl-1-oxy)-pyridin

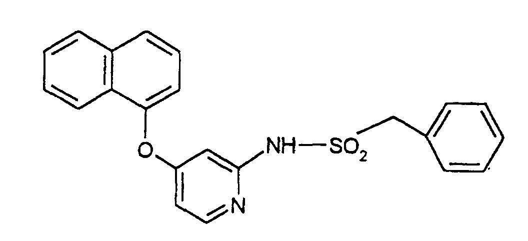

Die Herstellung erfolgte in Analogie zur Herstellung des Beispiels 2 ausgehend von Beispiel 139 A (0,300 g; 1,27 mmol).

Ausbeute: 0,289 g (58% d. Th.)
R_{f} = 0,55 (VII)
MS (ESI): m/z = 391 (M+H)

### Beispiel 264

### 3-Fluor-5-(naphthyl-1-oxy)-1-(N-1-pentylsulfonyl)amino-benzol

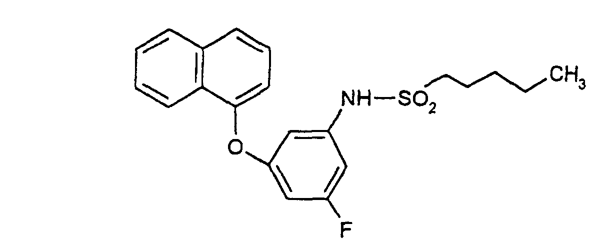

Die Herstellung erfolgte in Analogie zur Herstellung des Beispiels 1 ausgehend von Beispiel 100 A (1,00 g; 3,95 mmol).

Ausbeute: 1,49 g (96 % d. Th.)
Smp.: 72°C
R_{f} = 0,50(IV)
MS (ESI): m/z = 410 (M+Na)

### Beispiel 265

### 1-(N-Benzylsulfonyl)amino-3-fluor-5-(naphthyl-1-oxy)-benzol

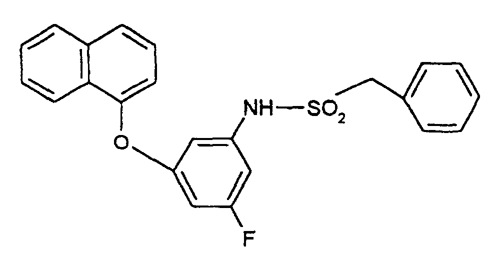

Die Herstellung erfolgte in Analogie zur Herstellung des Beispiels 1 ausgehend von Beispiel 100 A (1,00 g; 3,95 mmol).

Ausbeute: 1,29 g (77 % d. Th.)
Smp.: 122°C
R_{f} = 0,54 (IV)
MS (DCI, NH₃): m/z = 425 (M+NH₄)

### Beispiel 266

### 3-Fluor-5-(naphthyl-1-oxy)-1-(4,4,4-trifluor-1-butylsulfonyl)amino-benzol

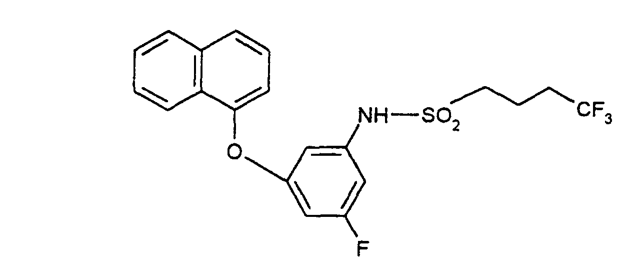

Die Herstellung erfolgte in Analogie zur Herstellung des Beispiels 1 ausgehend von Beispiel 100 A (1,00 g; 3,95 mmol).

Ausbeute: 1,18 g (69 % d. Th.)
R_{f} = 0,49 (IV)
MS (DCI, NH₃): m/z = 445 (M+NH₄)

### Beispiele 267 und 268

### (R)- und (S)-1-N-(n-Pentylsulfonyl)amino-4-(2-hydroxymethylindanyl-4-oxy)-benzol

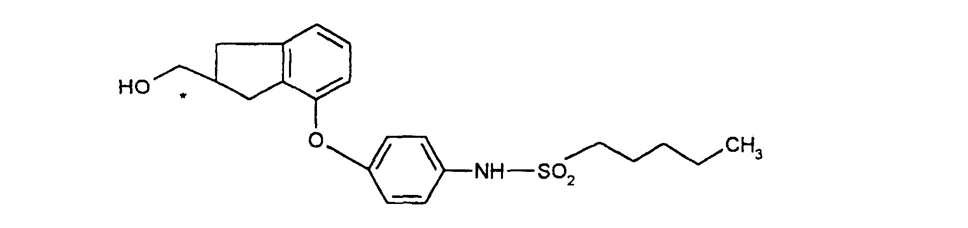

Enantiomer A (Beispiel 267) und Enantiomer B (Beispiel 268)

Die Verbindung aus Beispiel 186 (0,100 g; 0,257 mmol) wird mittels präparativer HPLC (Chiralpak AD, 250 mm x 20 mm, Laufmittel 82 % Petroleumbenzin/18 % iPrOH, T = 50°C, Fluß = 0,2 ml/min) in die beiden Enantiomere A (Beispiel 267) und B (Beispiel 268) getrennt.
Beispiel 267:
   Ausbeute: 34,3 mg (68 % d. Th.)
   Retentionszeit: 10,6 min
Beispiel 268:
   Ausbeute: 13,3 mg (26 % d. Th.)
   Retentionszeit: 11,4 min

### Beispiel 269

### 3-(Naphthyl-1-oxy)-1-[2-(bis-trifluormethyl-methoxy)ethylsulfonyl]amino-benzol

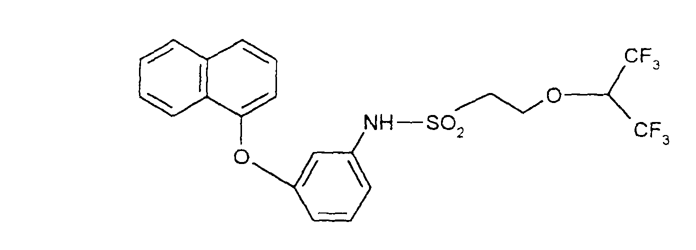

Die Herstellung erfolgte in Analogie zur Herstellung des Beispiels 1 ausgehend von Beispiel 45 A (0,518 g; 2,20 mmol).

Ausbeute: 0,315 g (28% d. Th.)
R_{f} = 0,56 (Dichlormethan)
MS (DCI, NH₃): m/z = 511 (M+NH₄)

### Beispiel 270

### 3-(Naphthyl-1-oxy)-1-(4,4,5,5,5-pentafluor-1-pentylsulfonyl)amino-benzol

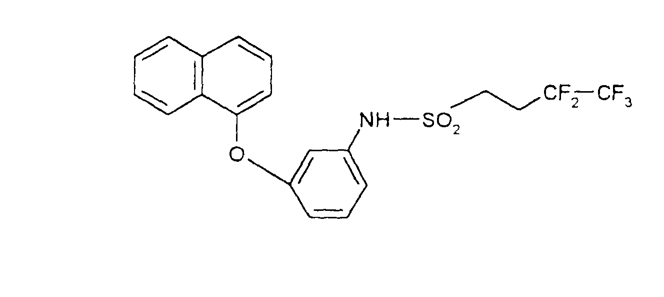

Die Herstellung erfolgte in Analogie zur Herstellung des Beispiels 1 ausgehend von Beispiel 45 A (0,518 g, 2,20 mmol).

Ausbeute: 0,665 g (63 % d. Th.)
R_{f} = 0,54 (Dichlormethan)
MS (DCI, NH₃): m/z = 477 (M+NH₄)

### Beispiel 271

### 3-(Naphthyl-1-oxy)-1-(4,4,5,5,5-pentafluor-1-pentylsulfonyl)oxy-benzol

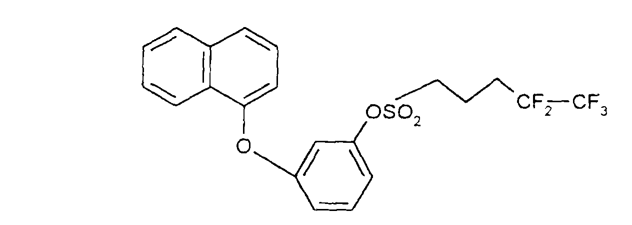

Die Herstellung erfolgte in Analogie zur Herstellung des Beispiels 97 ausgehend von Beispiel 63 A (0,210 g; 0,89 mmol).

Ausbeute: 0,346 g (85 % d. Th.)
R_{f} = 0,38 (Dichlormethan)
MS (ESI): m/z = 461 (M+H)

### Beispiel 272

### 3-(6-Methoxymethyl-naphthyl-1-oxy)-1-(N-1-pentylsulfonyl)amino-benzol

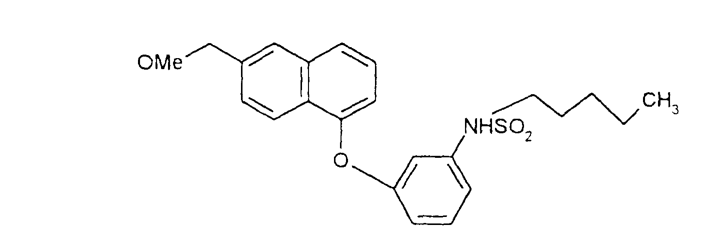

Die Herstellung erfolgte in Analogie zur Herstellung des Beispiels 1 ausgehend von Beispiel 144 A (59,0 mg; 0,21 mmol).

Ausbeute: 64 g (74 % d. Th.)
R_{f} = 0,77 (Dichlormethan:EE = 10:1)
MS (OCI, NH₃): m/z = 431 (M+NH₄)

### Beispiel 273

### (R,S)-1-N-(4,4,4-Trifluor-1-butylsulfonyl)amino-3-(2-hydroxymethyl-indanyl-4-oxy)-benzol

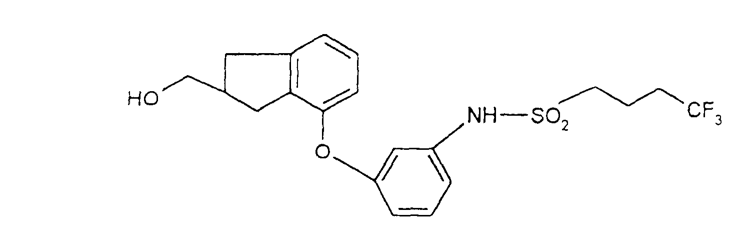

Die Herstellung erfolgte in Analogie zur Herstellung des Beispiels 1 ausgehend von Beispiel 146 A (0,800 g; 3,13 mmol).

Ausbeute: 0,832 g (64 % d. Th.)
R_{f} = 0,50 (VII)
MS (DCl, NH₃): m/z = 447 (M+NH₄)

### Beispiele 274 und 275

### (R) und (S)-1-N-(4,4,4-Trifluor-1-butylsulfonyl)amino-3-(2-hydroxymethyl-indanyl-4-oxy)-benzol

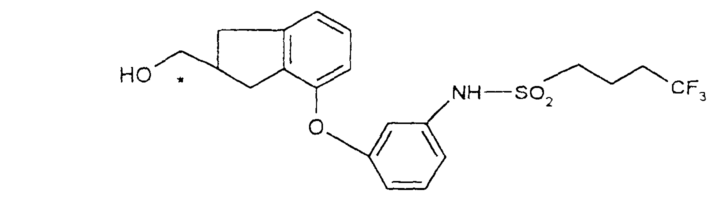

Enantiomer A (Beispiel 274) und Enantiomer B (Beispiel 275)

Die Verbindung aus Beispiel 273 (0,560 g; 1,30 mmol) wird mittels präparativer HPLC (Chiralpak AD 10 um, 250 x 20 mm, Laufmittel 88 % Petroleumbenzin 40°C-70°C/12 % EtOH, T = 15°C) in die Enantiomeren A (Beispiel 274) und B (Beispiel 275) getrennt.
Beispiel 274:
   Ausbeute: 85 mg (15 % d. Th.)
   Retentionszeit: 13,3 min.
Beispiel 275:
   Ausbeute: 80 mg (14 % d. Th.)
   Retentionszeit: 15,6 min.

### Beispiel 276

### (R,S)-1-(4,4,4-Trifluor-1-butylsulfonyl)oxy-3-(2-hydroxymethylindanyl-4-oxy)-benzol

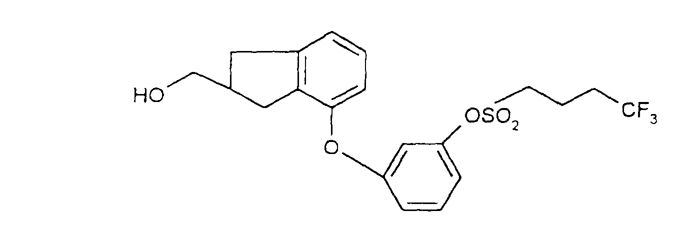

Eine Lösung des Beispiels 147 A (1,228 g; 4,79 mmol) in THF (10 ml) wird bei Raumtemperatur unter Argon mit Kalium-tert-butanolat (0,538 g, 4,79 mmol) versetzt und 30 min bei Raumtemperatur gerührt. Anschließend wird 4,4,4-Trifluorbutan-1-sulfonsäurechlorid (1,009 g; 4,79 mmol) zugetropft und die Reaktionsmischung wird 16 h nachgerührt. Nach Zugabe von Ethylacetat (50 ml) wird mit Wasser (50 ml) und ges. wäßriger NaCl-Lösung (50 ml) gewaschen, getrocknet (Na₂SO₄) und das Lösungsmittel im Vakuum abgezogen. Der Rückstand wird an Kieselgel mit Toluol:EE (3:1) chromatographiert.

Ausbeute: 0,894 g (41 % d. Th.)
R_{f} = 0,39 (Tol:EE = 3:1)
MS (DCI, NH₃): m/z = 448 (M+NH₄)

### Beispiele 277 und 278

### (R)- und (S)-1-(4,4,4-Trifluor-1-butylsulfonyl)oxy-3-(2-hydroxymethylindanyl-4-oxy)-benzol

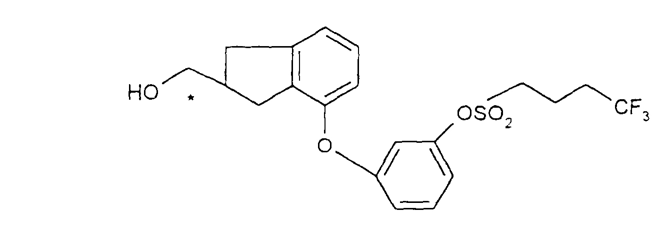

(+)-Enantiomer A (Beispiel 277) und (-)-Enantiomer B (Beispiel 278)

Die Verbindung aus Beispiel 276 (490 mg, 1,14 mmol) wird mittels präparativer HPLC (Chiracel OD, 10 µm, 250 x 20 mm, Fluß 10 ml/min, Laufmittel 80 % Petroleumbenzin 40-70°C / 20 % Isopropanol, T = 10°C) in die Enantiomeren A (Beispiel 277) und B (Beispiel 278) getrennt.
Beispiel 277:
   Ausbeute: 111 mg (23 % d. Th.)
   Smp.: 60-61°C
   Retentionszeit: 12,5 min
   [α]_{D}²⁰ (c = 1, MeOH)= + 10,70
Beispiel 278:
   Ausbeute 105 mg (21 % d. Th.)
   Smp.: 60-61°C
   Retentionszeit: 15,4 min
   [α]_{D}²⁰ (c= 1, MeOH) = - 10,35

### Beispiel 279

### 5-[(4,4,4-Trifluor-1-butyl)sulfonylamino]-2-(naphthyl-1-oxy)-pyridin Natriumsalz

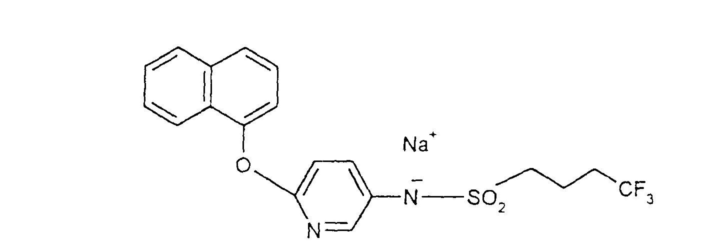

Die Herstellung erfolgte in Analogie zur Herstellung des Beispiels 128 ausgehend von Beispiel 189 (452 mg; 1,10 mmol).

Ausbeute: 315 mg (66 % d. Th.)
Smp.: 170°C (Z)

In Analogie zu Beispiel 279 erfolgt die Herstellung der in Tabelle 23 aufgelisteten Beispiele.

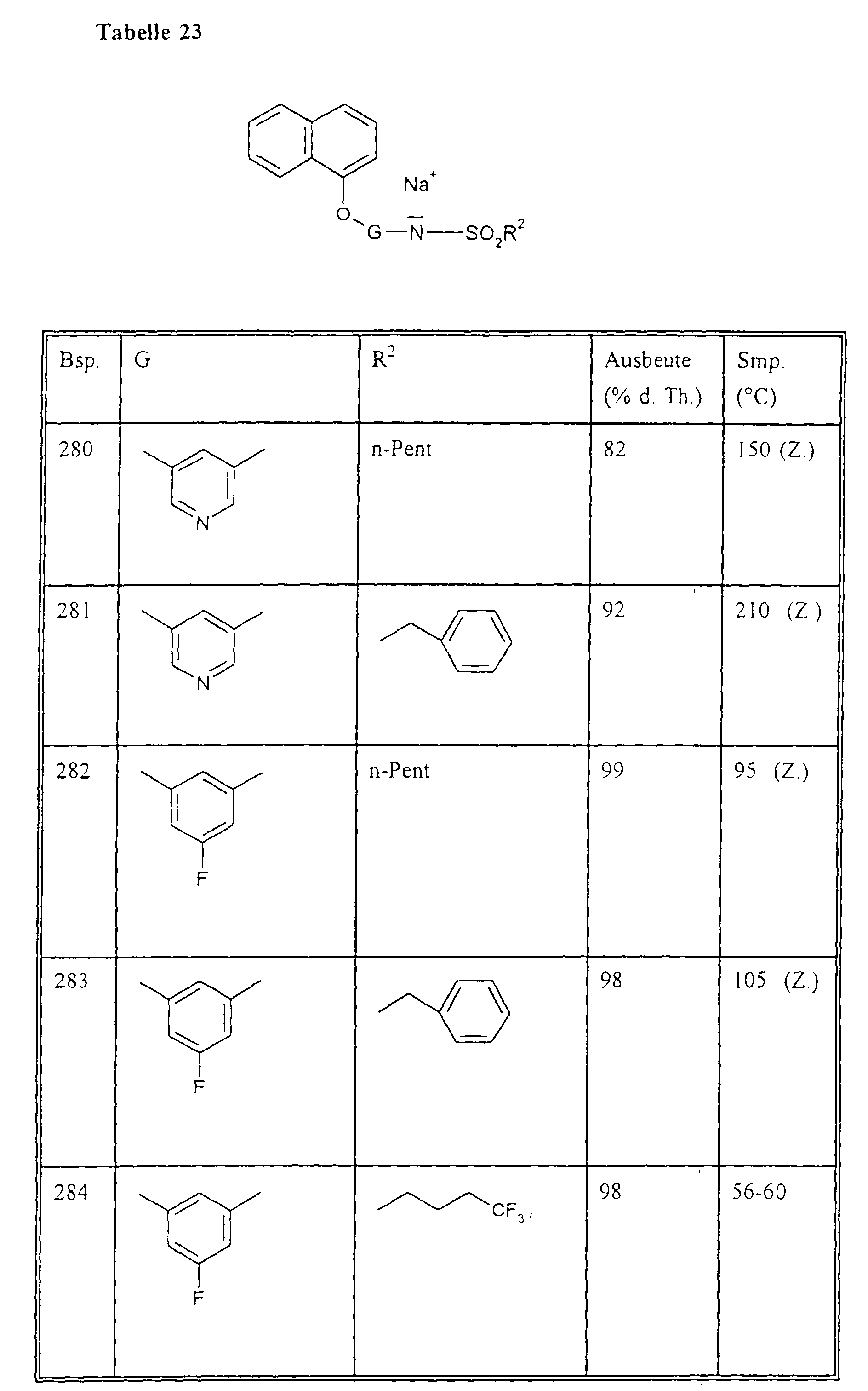

### Beispiel 285

### 5-Fluor-1-[(4,4,4-trifluor-1-butyl)sulfonyl]amino-3-(naphthyl-1-oxy)-benzol Kaliumsalz

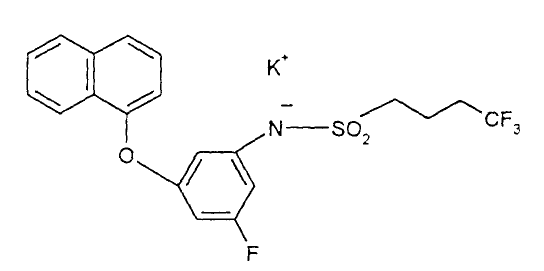

Die Herstellung erfolgte in Analogie zur Herstellung des Beispiels 128 ausgehend von Beispiel 266 (400 mg; 0,94 mmol) mit Kalium-tert-butanolat (105 mg; 0,94 mmol) statt Natriummethylat.

Ausbeute: 433 mg (99 % d. Th.)
Smp.: 46-50°C

### Beispiel 286

### (R,S)-1-[(4,4,4-Trifluor-1-butyl)sulfonyl]amino-3-(2-methansulfonyloxymethylindanyl-4-oxy)-benzol

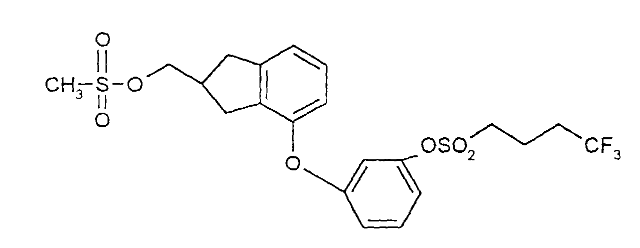

Zur Lösung des Beispiels 276 (665 mg; 1,55 mmol) und Triethylamin (235 mg; 2,32 mmol) in Dichlormethan (10 ml) tropft man unter Argon bei -10°C Methansulfonsäurechlorid (195 mg; 1,70 mmol), läßt noch 30 min bei -10°C rühren und läßt den Ansatz auf Raumtemperatur erwärmen. Die Reaktionsmischung wird mit Dichlormethan (10 ml) verdünnt und mit Wasser (20 ml), 1 N Salzsäure (10 ml), ges. wäßriger NaHCO₃-Lösung (20 ml) und Wasser (20 ml) gewaschen, getrocknet (Na₂SO₄) und im Vakuum eingeengt.

Ausbeute: 706 mg (88 % d. Th.)
R_{f} = 0,74 (VII)
MS (ESI): m/z = 509 (M+H)

### Beispiel 287

### (R,S)-3-(2-Azidomethyl-indanyl-4-oxy)-1-[(4,4,4-trifluor-1-butyl)sulfonyl]amino-benzol

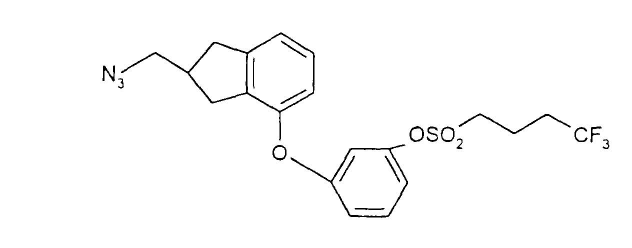

Eine Lösung des Beispiels 286 (637 mg; 1,25 mmol) in DMSO (5 ml) wird mit Natriumazid (407 mg; 6,26 mmol) versetzt und 1 h bei 80°C unter Argon gerührt. Nach Zugabe von Wasser (50 ml) wird mit Diethylether (2 x 50 ml) extrahiert. Die vereinigten organischen Phasen werden mit Wasser (30 ml) gewaschen, getrocknet (Na₂SO₄) und im Vakuum eingeengt.

Ausbeute: 507 mg (87% d. Th.)
R_{f} = 0,78 (IV)
MS (EI): m/z = 427 (M-N₂)

### Beispiel 288

### (R,S)-3-(2-Aminomethyl-indanyl-4-oxy)-1-[(4,4,4-trifluor-1-butyl)sulfonyl]amino-benzol Hydrochlorid

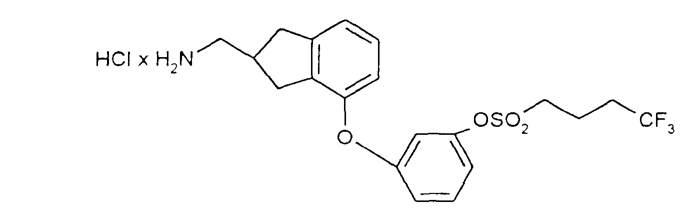

Beispiel 287 (457 mg; 1,00 mmol) wird in MeOH (10 ml) gelöst, mit Palladium auf Aktivkohle, 10 %ig (50 mg) versetzt und 1,5 h bei 1 bar Wasserstoff hydriert. Der Ansatz wird über Kieselgel filtriert und im Vakuum eingeengt. Der Rückstand wird in Diethylether (5 ml) und MeOH (4 ml) aufgenommen und mit einer gesättigten Lösung von HCI in Diethylether (2 ml) versetzt. Anschließend wird das Solvens im Vakuum abgezogen und der Rückstand in Diethylether verrührt, abgesaugt und im Vakuum getrocknet.

Ausbeute: 321 mg (69 % d. Th.)
Smp.: 192°C
R_{f} = 0,10 (Dichlormethan:MeOH = 20:1)
MS (DCI, NH₃): m/z = 430 (M+H)

### Beispiel 289

### (R,S)-3-(2-Dimethylaminomethyl-indanyl-4-oxy)-1-[(4,4,4-trifluoro-1-butyl)sulfonyl]amino-benzol Hydrochlorid

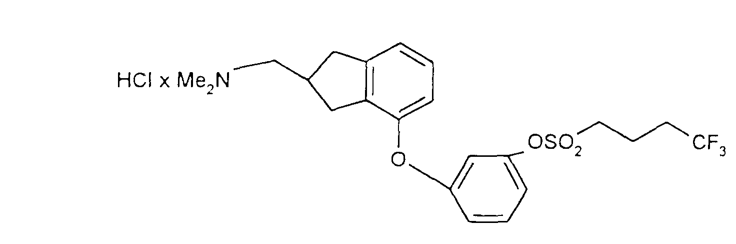

Beispiel 288 (140 mg; 0,30 mmol) wird in Dichlormethan gelöst und mit wäßriger NH₃-Lösung gewaschen. Die wäßrige Phase wird mit Dichlormethan gewaschen (2 x 20 ml). Die vereinten org. Phasen werden getrocknet (Na₂SO₄) und i. Vak. eingeengt. Der Rückstand wird in Acetonitril (5,0 ml) gelöst und bei Raumtemperatur mit einer 37 %igen wäßrigen Formaldehydlösung (246 mg; 3,0 mmol) und Natriumcyanoborhydrid (191 mg; 3,0 mmol) versetzt. Man läßt 30 min bei Raumtemperatur rühren, stellt mit Essigsäure pH 3 ein, läßt 5 min rühren und gibt 20 ml 1 N NaOH zu. Die Reaktionsmitschung wird mit Dichlormethan (2 x 20 ml) gewaschen. Die vereinten org. Phasen werden getrocknet (Na₂SO₄) und im Vakuum einrotiert Der Rückstand wird in MeOH (5 ml) gelöst und mit einer gesättigten Lösung von HCl in Diethylether (0,1 ml) versetzt. Anschließend wird die Lösung im Vakuum eingeengt.

Ausbeute: 134 mg (90 % d. Th.)
R_{f} = 0,33 (XXV)
MS (DCI, NH₃): m/z = 458 (M+H)

### Beispiel 290

### 1-[(4,4,4-Trifluor-1-butyl)-sulfonyl]amino-3-(6-hydroxy-methyl-naphthyl-1-oxy)benzol

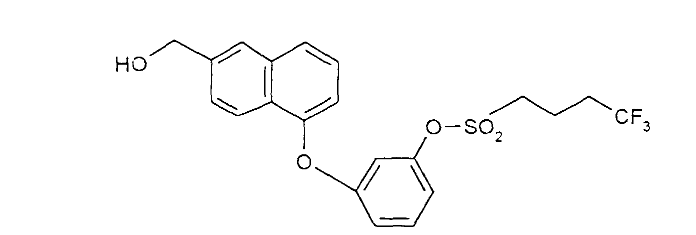

Die Herstellung erfolgte in Analogie zur Herstellung des Beispiels 276 ausgehend von Beispiel 148 A (1,01 g; 3,80 mmol).

Ausbeute: 0,72 g (43 % d. Th.)
R_{f} = 0,60 (Tol:EE = 5:4)
MS (DCI, NH₃): m/z = 458(M+NH₄)

### Beispiel 291

### 3-(6-Hydroxymethyl-naphthyl-1-oxy)-1-(1-pentylsulfonyl)oxy-benzol

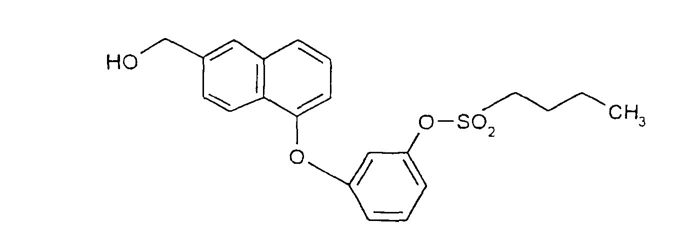

Die Herstellung erfolgte in Analogie zur Herstellung des Beispiels 276 ausgehend von Beispiel 148 (5,33 g; 20,0 mmol).

Ausbeute: 4,00 g (49 % d. Th.)
R_{f} = 0,67 (VI)
MS (DCI, NH₃): m/z = 418 (M+NH₄)

### Beispiel 292

### 3-(6-Methansulfonyloxymethyl-naphthyl-1-oxy)-1-(1-pentylsulfonyl)oxy-benzol

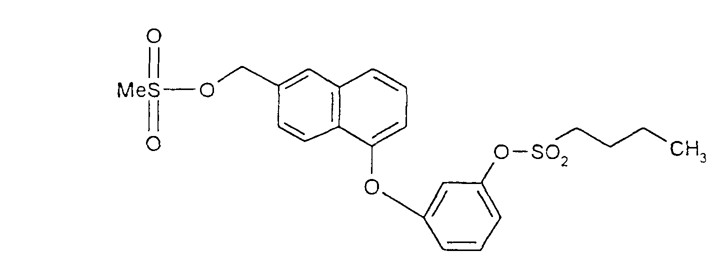

Die Herstellung erfolgte in Analogie zur Herstellung des Beispiels 286 ausgehend von Beispiel 291 (3,73 g, 9,00 mmol).

Ausbeute: 3,19 g (74 % d. Th.)
R_{f} = 0,64 (Tol:EE = 5:2)
MS (DCI, NH₃): m/z = 496 (M+NH₄)

### Beispiel 293

### 3-(6-Azidomethyl-naphthyl-1-oxy)-1-(1-pentylsulfonyl)-oxy-benzol

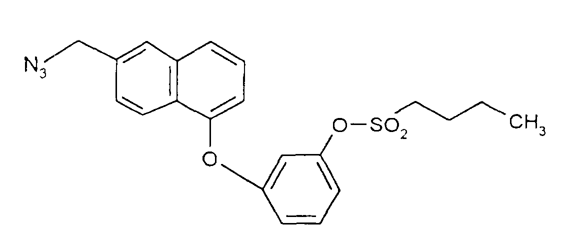

Die Herstellung erfolgte in Analogie zur Herstellung des Beispiels 287 ausgehend von Beispiel 292 (3,60 g; 7,52 mmol).

Ausbeute 2,68 g (84 % d. Th.)
R_{f} = 0,88 (Tol:EE = 5:2)
MS (DCI, NH₃): m/z = 443 (M+NH₄)

### Beispiel 294

### 3-(6-Aminomethyl-naphthyl-1-oxy)-1-(1-pentylsulfonyl)-oxy-benzol Hydrochlorid

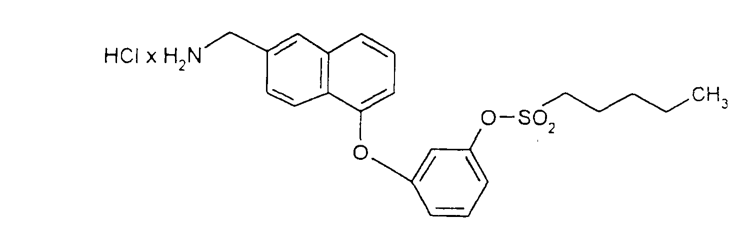

Die Herstellung erfolgte in Analogie zur Herstellung des Beispiels 288 ausgehend von Beispiel 293 (2,40 g; 5,64 mmol).

Ausbeute: 2,23 g (90 % d. Th.)
Smp.: >150°C (Z.)
R_{f} = 0,41 (XXV)
MS (DCI, NH₃): m/z = 400 (M+H)

### Beispiel 295

### 3-(6-N,N-Dimethylaminomethyl-naphthyl-1-oxy)-1-(1-pentylsulfonyl)oxy-benzol Hydrochlorid

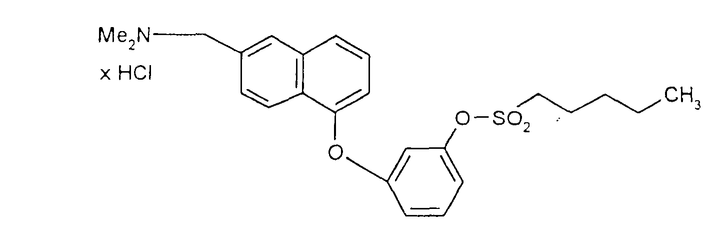

Die Herstellung erfolgte in Analogie zur Herstellung des Beispiels 289 ausgehend von Beispiel 294 (1,09 g, 2,50 mmol).

Ausbeute: 0,220 g (19 % d. Th.)
R_{f} = 0,49 (XXV)
MS (DCI, NH₃): m/z = 428 (M+H)

### Beispiel 296

### 1-(1-Pentylsulfonyl)amino-4-(2,3-dimethyl-phenyl-1-oxy)-benzol

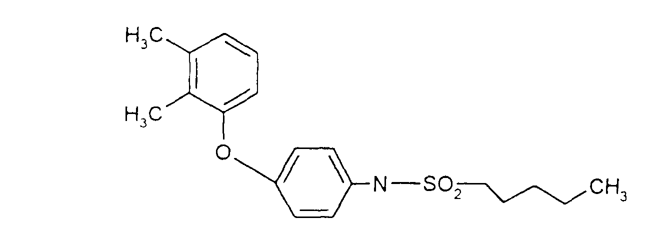

Die Herstellung erfolgte in Analogie zur Herstellung des Beispiels 1 ausgehend von Beispiel 29 A (7,25 g; 34,0 mmol).

Ausbeute: 10,9 g (93 % d. Th.)
R_{f} = 0,43 (IV)
MS (ESI): m/z = 348 (M+H)

### Beispiel 297

### 1-[N,N-Bis-(1-pentylsulfonyl)amino]-4-(2,3-dimethyl-phenyl-1-oxy)benzol

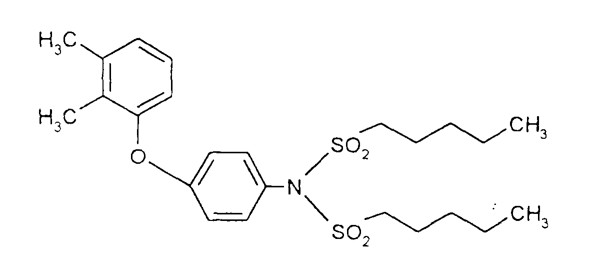

Zur Lösung von Beispiel 296 (3,48 g; 10,0 mmol) in THF (40 ml) gibt man unter Eiskühlung Kalium-tert.-butylat (1,18 g; 10,5 mmol), läßt 20 min rühren und tropft dann 1-Pentansulfonylchlorid (2,04 g; 12,0 mmol) bei 0°C zu. Man läßt über Nacht bei Raumtemperatur rühren und extrahiert nach Zugabe von Wasser dreimal mit Ethylacetat. Die vereinten org. Phasen werden zweimal mit Wasser gewaschen, getrocknet (MgSO₄) und im Vakuum eingeengt. Der Rückstand wird an Kieselgel mit Toluol chromatographiert.

Ausbeute. 3,71 g (77 % d. Th.)
Smp.: 91°C
R_{f} = 0,64 (PE:Diethylether = 10:3)
MS (ESI): m/z = 482 (M+H)

### Beispiel 298

### 1-[N,N-Bis-(1-pentylsulfonyl)amino]-4-[2,3-(bis-brommethyl)-phenyl-1-oxy]benzol

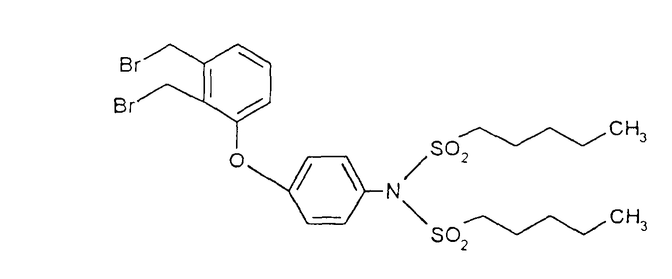

Zur Lösung des Beispiels 297 (13,0 g; 27,0 mmol) in CCl₄ (250 ml) gibt man N-Bromsuccinimid (10,2 g; 57,4 mmol) und erhitzt den Ansatz bei gleichzeitiger Bestrahlung mit einer 300 W-Lampe 4 h zum Rückfluß. Nach dem Abkühlen wird der Ansatz filtriert und das Filtrat im Vakuum eingeengt. Der Rückstand wird an Kieselgel mit Cyclohexan/Diethylether (10:1) chromatographiert. Das so erhaltene Produkt wird aus Cyclohexan kristallisiert.

Ausbeute: 13,4 g (78 % d. Th.)
Smp.: 68-75°C
R_{f} = 0,90 (PE:Diethylether = 10:3)
MS (ESI): m/z = 662 (M+Na)

### Beispiel 299

### 4-(1-N-Butyl-isoindolinyl-3-oxy)-1-(1-pentylsulfonyl)-amino-benzol Hydrochlorid

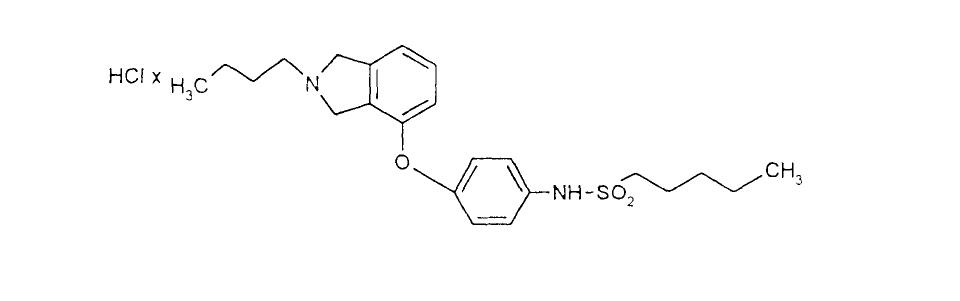

Eine Lösung von Beispiel 298 (0,750 g, 1,17 mmol) und n-Butylamin (0,858 g, 11,7 mmol) in THF (150 ml) wird über Nacht bei Raumtemperatur gerührt. Anschließend versetzt man mit 1 N NaOH (5,0 ml) und rührt den Ansatz 24 h bei 50°C. Das Lösungsmittel wird im Vakuum abgezogen, der Rückstand wird in Ethylacetat (50 ml) aufgenommen und mit Wasser (50 ml) gewaschen. Die wäßrige Phase wird mit Ethylacetat (25 ml) extrahiert und die vereinten organischen Phasen werden getrocknet (Na₂SO₄) und im Vakuum eingeengt. Der Rückstand wird an Kieselgel mit Toluol:Ethylacetat (1:1) chromatographiert. Das so erhaltene Amin wird in Diethylether (5 ml) gelöst und mit einer gesättigten Lösung von HCI in Diethylether (1 ml) versetzt. Das Lösungsmittel wird im Vakuum abgezogen und das Produkt im Vakuum getrocknet.

Ausbeute: 0,255 g (47 % d. Th.)
Smp.: 70-73°C (Z.)
R_{f} = 0,37 (VII)
MS (DCI, NH₃): m/z = 417 (M+H)

In Analogie zu Beispiel 299 werden die in Tabelle 24 aufgelisteten Beispiele hergestellt.

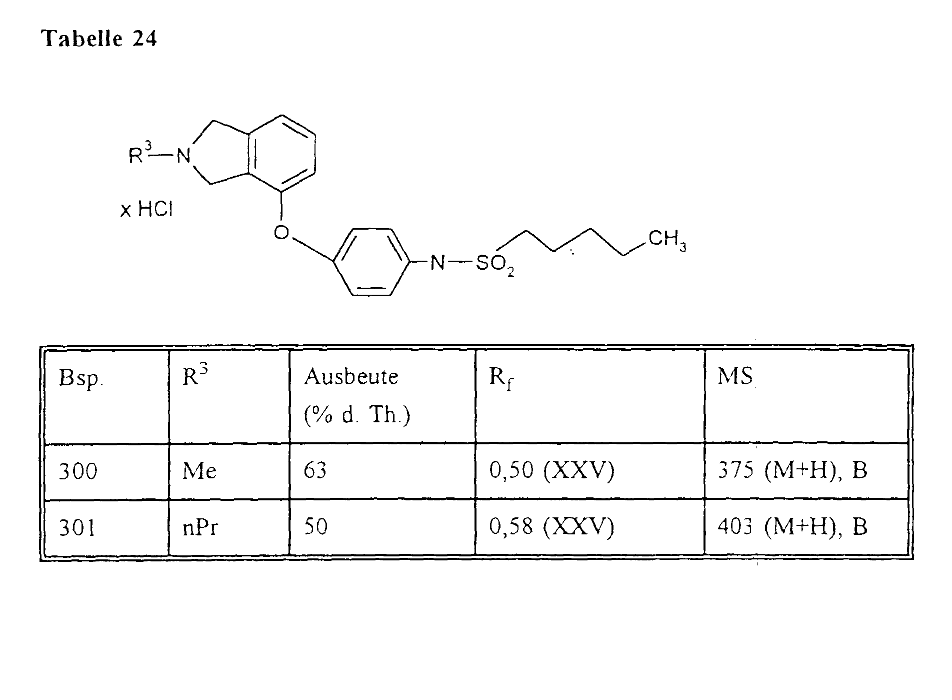

### Beispiel 302

### 4-[2,2-Bis-(ethoxycarbonyl)-indanyl-4-oxy]-1-[N,N-bis(1-pentylsulfonyl)amino]-benzol

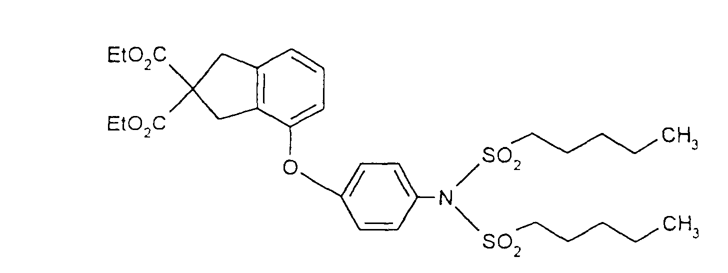

Eine Lösung von Beispiel 298 (2,00 g; 3,13 mmol) und Malonsäurediethylester (0,50 g; 3,13 mmol) in 2-Butanon (30 ml) wird mit Kaliumcarbonat (1,88 g; 13,6 mmol) versetzt und 18 h unter Rückfluß gerührt. Man läßt auf Raumtemperatur abkühlen, filtriert und engt das Filtrat im Vakuum ein. Der Rückstand wird an Kieselgel mit Tol:EE (30:1) chromatographiert.

Ausbeute: 0,480 g (24 % d. Th.)
R_{f} = 0,53 (X)
MS (ESI): m/z = 638 (M+H)

### Beispiel 303

### 4-[2,2-Bis(hydroxymethyl)-indanyl-4-oxy)-1-[N-1-pentylsulfonyl]amino-benzol

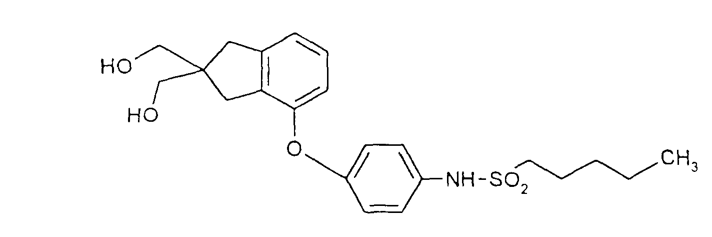

Zur Lösung von Beispiel 302 (452 mg; 0,71 mmol) in THF (5,0 ml) tropft man unter Argon bei Raumtemperatur Lithiumaluminiumhydrid, 1 N Lösung in THF (1,42 ml; 1,42 mmol) und läßt 18 h bei Raumtemperatur rühren Nach Zugabe von ges. wäßriger NH₄Cl-Lösung (20 ml) wird mit Ethylacetat (1 x 50 ml, 2 x 25 ml) extrahiert. Die vereinten org. Phasen werden mit ges. wäßriger NaCl-Lösung (25 ml) gewaschen, getrocknet (Na₂SO₄) und im Vakuum eingeengt. Der Rückstand wird an Kieselgel mit Tol:EE = 1:1 chromatographiert.

Ausbeute: 149mg (49 % d. Th.)
Smp.: 135-137°C
R_{f} = 0,25 (VII)
MS (ESI): m/z = 442 (M+Na)

### Beispiel 304

### 3-(2,3-Dimethyl-phenyl- 1-oxy)-1-(4,4,4-trifluor-1-butyl-sulfonyl)oxy-benzol

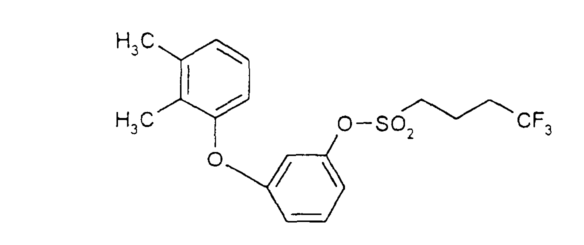

Die Herstellung erfolgte in Analogie zur Herstellung des Beispiels 97 ausgehend von Beispiel 150A (4,54 g; 21,2 mmol).

Ausbeute: 7,80 g (95 % d. Th.)
R_{f} = 0,51 (Toluol)
MS (DCI, NH₃): m/z = 406 (M+NH₄)

### Beispiel 305

### 3-(2,3-Bis-brommethyl-phenyl-1-oxy)-1-(4,4,4-trifluor-1-butylsulfonyl)oxy-benzol

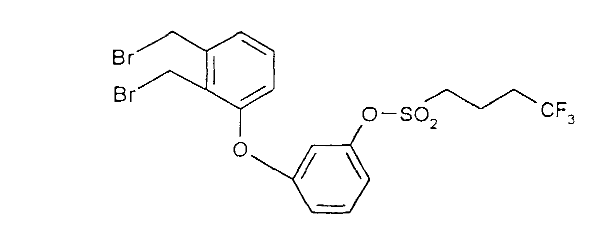

Die Herstellung erfolgte in Analogie zur Herstellung des Beispiels 298 ausgehend von Beispiel 304 (6,76 g; 17,4 mmol).

Ausbeute: 7,98 g (84 % d. Th.)
R_{f} = 0,71 (IV)
MS (DCI, NH₃): m/z = 564 (M+NH₄)

### Beispiel 306

### 1-(4,4,4-Trifluor-1-butylsulfonyl)oxy-3-[2,2-bis-(methoxycarbonyl)-indanyl-4-oxy-benzol

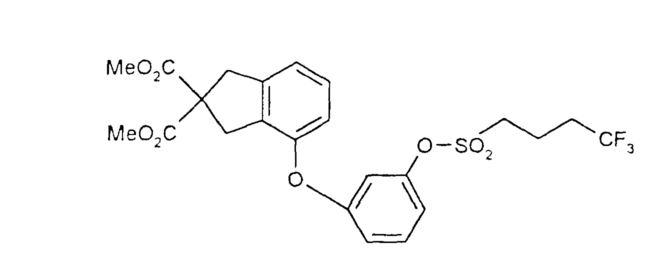

Die Herstellung erfolgte in Analogie zur Herstellung des Beispiels 302 ausgehend von Beispiel 305 (6,00 g; 10,2 mmol).

Ausbeute: 1,95 g (37 % d. Th.)
R_{f} = 0,45 (X)
MS (DCI, NH₃): m/z = 534 (M+NH₄)

### Beispiel 307

### 1-(4,4,4-Trifluor-1-butylsulfonyl)oxy-3-(1-N-propylisoindolinyl-3-oxy)benzol

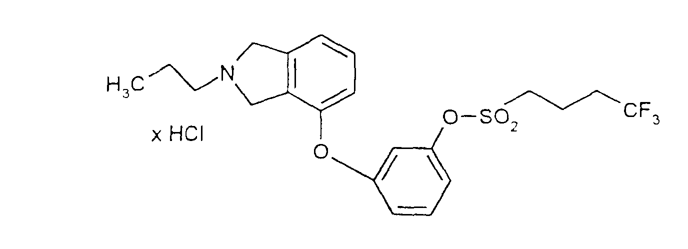

Eine Lösung von Beispiel 305 (2,00 g; 3,66 mmol) und n-Propylamin (2,16 g; 36,6 mmol) in THF (200 ml) wird 5 h bei Raumtemperatur gerührt. Das THF wird im Vakuum abgezogen, der Rückstand wird in Wasser aufgenommen und mit Ethylacetat extrahiert. Die org. Phase wird mit 5 %iger wäßriger K₂CO₃-Lösung und zweimal mit Wasser extrahiert, getrocknet (MgSO₄) und im Vakuum eingeengt. Der Rückstand wird an Kieselgel mit Dichlormethan:MeOH = 20:1 chromatographiert. Das so erhaltene Amin wird in Diethylether (5 ml) gelöst und mit einer ges. Lösung von HCl in Diethylether (1,5 ml) versetzt. Das Lösungsmittel wird im Vakuum abgezogen und der Rückstand wird mit Diethylether verrieben, abgesaugt und im Vakuum getrocknet.

Ausbeute: 0,775 g (44 % d. Th.)
R_{f} = 0,29 (XXXII)
MS (ESI): m/z = 444 (M+H)

### Beispiel 308

### 3-(1-Hexyl)oxy-3-(naphthyl-1-oxy)benzol

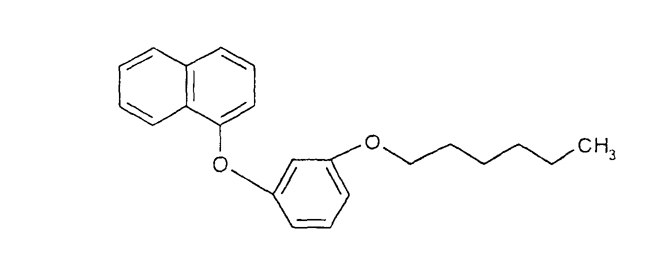

Eine Lösung von Beispiel 63 A (300 mg; 1,27 mmol) in Aceton (5,0 ml) wird mit Kaliumcarbonat (193 mg; 1,40 mmol) und 1-Jodhexan (296 mg; 1,40 mmol) versetzt und 18 h unter Rückfluß gerührt. Anschließend wird das Aceton im Vakuum abgezogen, der Rückstand wird in Wasser (30 ml) aufgenommen und mit Diethylether (3 x 30 ml) extrahiert. Die vereinten org. Phasen werden getrocknet (Na₂SO₄) und im Vakuum eingeengt. Der Rückstand wird an Kieselgel mit Cyclohexan:Dichlormethan (4:1) chromatographiert.

Ausbeute: 285 mg (69% d. Th.)
R_{f} = 0,50 (PE:Dichlormethan = 4:1)
MS (DCI, NH₃): m/z = 321 (M+H)

### Beispiel 309

### N-1-Hexyl-3-(naphthyl-1-oxy)anilin

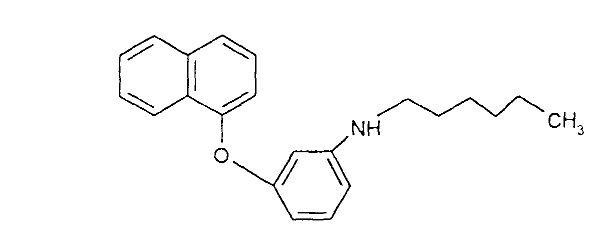

Eine Lösung von Beispiel 45 A (1,176 g; 5,00 mmol) und 1-Jodhexan (0,509 g; 2,40 mmol) in Petrolether (10 ml) wird über Nacht zum Rückfluß erhitzt. Nach Zugabe von 1-Jodhexan (0,170 g, 0,80 mmol) und THF (4 ml) wird weitere 3 h unter Rückfluß gerührt. Nach Zugabe von Diethylether (50 ml) wird mit verd. Ammoniak-Lösung (50 ml) und Wasser (2 x 50 ml) gewaschen, getrocknet (Na₂SO₄) und im Vakuum das Lösungsmittel abgezogen. Der Rückstand wird an Kieselgel chromatographiert mit Cyclohexan:Dichlormethan (3:1).

Ausbeute: 0,211 g (28 % d. Th.)
R_{f} = 0,86 (IV)
MS (DCI, NH₃): m/z = 320 (M+H)

## Patentansprüche

1. Verbindungen der allgemeinen Formel (I)
R¹-A-D-E-G-L-R² (I)
in welcher
R¹ für Naphthyl oder für einen Rest der Formel 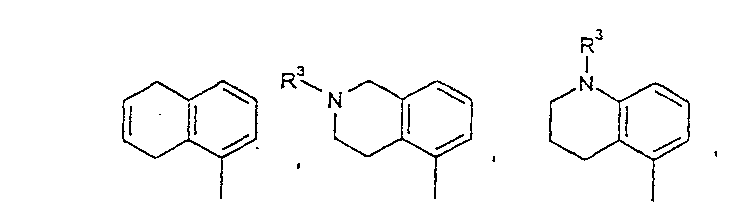 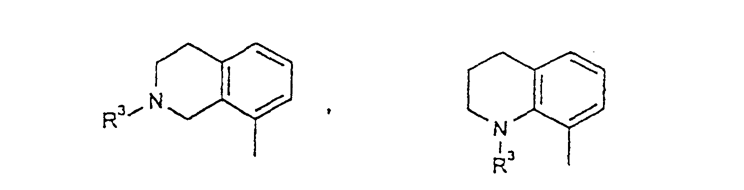 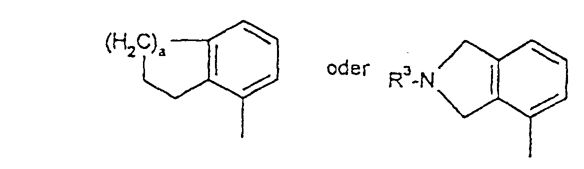 steht,
worin
a eine Zahl 1 oder 2 bedeutet,
R³ Wasserstoff, (C₂-C₆)-Alkenyl, (C₁-C₆)-Alkyl oder (C₁-C₆)-Acyl bedeutet,
und wobei alle oben aufgefuhrten Ringsysteme und Reste gegebenenfalls mit einem oder mehreren, gleichen oder verschiedenen Substituenten gegebenenfalls geminal substituiert sind, die aus der Gruppe ausgewählt sind, die besteht aus:
Halogen, Carboxyl, Hydroxy, Phenyl, (C₁-C₆)-Alkoxy, (C₁-C₆)-Alkoxycarbonyl, (C₁-C₈)-Alkyl, das seinerseits durch Halogen, (C₁-C₆)-Alkylsulfonyloxy, Azid, Amino, Mono(C₁-C₆)-Alkylamino, Di(C₁-C₆)-Alkylamino oder Hydroxy substituiert sein kann,
einer Gruppe der Formel -(CO)_{b}-NR⁴R⁵,
worin
b eine Zahl 0 oder 1 bedeutet,
R⁴ und R⁵ gleich oder verschieden sind und unabhängig voneinander Wasserstoff, Phenyl, (C₁-C₆)-Acyl, cyclo(C₄-C₇)-Acyl, Benzoyl oder (C₁-C₆)-Alkyl, das gegebenenfalls durch Amino, Mono(C₁-C₆)-Alkylamino, Di(C₁-C₆)-Alkylamino substituiert ist, bedeuten,
oder
R⁴ und R⁵ gemeinsam mit dem Stickstoffatom einen 5- oder 6-gliedrigen gesättigten Heterocyclus bilden, der gegebenenfalls ein oder mehrere weitere(s) Heteroatom(e) aus der Reihe S, O und/oder einen oder mehrere Rest(e) der Formel -NR⁸ enthalten kann,
worin
R⁸ Wasserstoff, (C₁-C₆)-Alkyl oder (C₁-C₆)-Acyl bedeutet,
und
einer Gruppe der Formel -NR⁶-SO₂-R⁷
worin
R⁶ Wasserstoff, Phenyl, (C₁-C₆)-Alkyl oder (C₁-C₆)-Acyl bedeutet,
R⁷ Phenyl oder (C₁-C₆)-Alkyl bedeutet,
A und E gleich oder verschieden sind und für eine Bindung oder für (C₁-C₄)-Alkylen stehen,
D für ein Sauerstoffatom oder für einen Rest der Formel -S(O)_{c}- oder -N(R⁹)- steht,
worin
c eine Zahl 0, 1 oder 2 bedeutet,
R⁹ Wasserstoff, (C₁-C₆)-Alkyl oder (C₁-C₆)-Acyl bedeutet,
G für zweifach gebundenes (C₆-C₁₀)-Aryl oder für einen zweifach gebundenen 5- bis 7-gliedrigen aromatischen Heterocyclus mit bis zu 3 Heteroatomen aus der Reihe S, N und/oder O steht, die gegebenenfalls mit einem oder mehreren, gleichen oder verschiedenen Substituenten substituiert sind, die aus der Gruppe ausgewählt sind, die besteht aus:
Hydroxy, Trifluormethyl, Carboxyl, Halogen, (C₁-C₆)-Alkyl, Hydroxy(C₁-C₆)alkyl, (C₁-C₆)-Alkoxy, (C₁-C₆)-Alkoxycarbonyl,
sowie Gruppen der Formeln
-CO-O-(CH₂)_{d}-NR¹⁰R¹¹, -NR¹²-SO₂R¹³,
-(CH₂)ₑ-(CO)_{f}-NR¹⁴R¹⁵ und -OR¹⁶,
worin
d eine Zahl 1, 2, 3 oder 4 bedeutet,
e und f gleich oder verschieden sind und eine Zahl 0 oder 1 bedeuten,
R¹⁰ und R¹¹ die oben angegebene Bedeutung von R⁴ und R⁵ haben und mit dieser gleich oder verschieden sind,
R¹² die oben angegebene Bedeutung von R⁶ hat und mit dieser gleich oder verschieden ist,
R¹³ die oben angegebene Bedeutung von R⁷ hat und mit dieser gleich oder verschieden ist,
R¹⁴ und R¹⁵ die oben angegebene Bedeutung von R⁴ und R⁵ haben und mit dieser gleich oder verschieden sind,
oder unabhängig voneinander einen Rest der Formel -(CH₂)_{g}-NR¹⁷R¹⁸ darstellen,
worin
g eine Zahl 1, 2, 3 oder 4 bedeutet,
und
R¹⁷ und R¹⁸ die oben angegebene Bedeutung von R⁴ und R⁵ haben und mit dieser gleich oder verschieden sind,
R¹⁶ (C₆-C₁₀)-Aryl bedeutet,
L für einen Rest der Formel
-O-, -NH-, 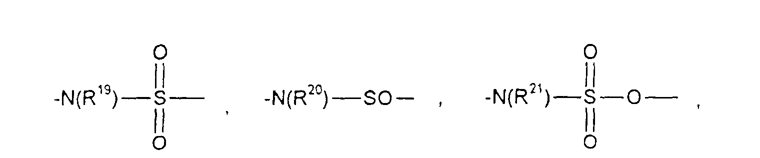 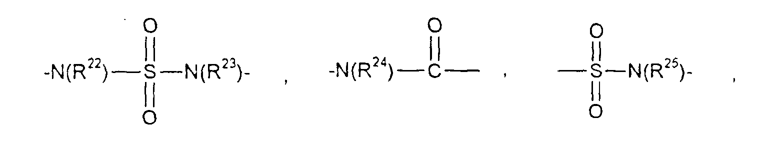 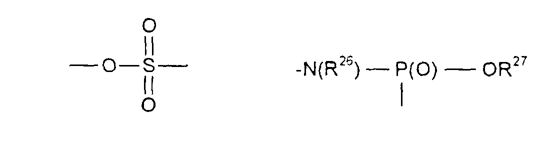
oder 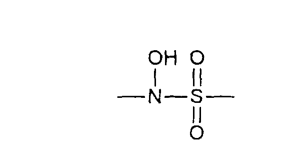 steht,
wobei die Anbindung der Reste an G linksbündig erfolgt,
und worin R¹⁹, R²⁰, R²¹, R²², R²³, R²⁴, R²⁵, R²⁶ und R²⁷ gleich oder verschieden sind und Wasserstoff oder (C₁-C₄)-Alkyl bedeuten, oder
R¹⁹ einen Rest der Formel -SO₂R² bedeutet,
R² für (C₆-C₁₀)-Aryl oder für einen 5- bis 7-gliedrigen gesättigten oder aromatischen Heterocyclus mit bis zu 3 Heteroatomen aus der Reihe S, N und/oder O steht, die gegebenenfalls mit einem oder mehreren, gleichen oder verschiedenen Substituenten substituiert sind, die aus der Gruppe ausgewählt sind, die besteht aus:
Halogen, Trifluormethyl, Nitro, Amino und (C₁-C₆)-Alkyl,
oder
für den Rest der Formel 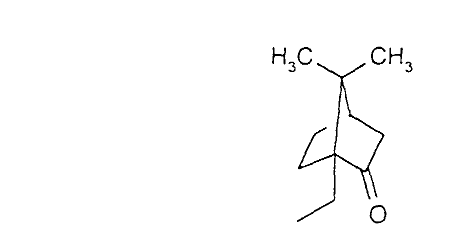 oder Morpholin steht, oder
für C₃-C₈-Cycloalkyl steht, oder
für (C₁-C₁₂)-Alkyl, (C₂-C₁₂)-Alkenyl oder (C₂-C₁₂)-Alkinyl steht, die gegebenenfalls mit einem oder mehreren, gleichen oder verschiedenen Substituenten substituiert sind, die aus der Gruppe ausgewählt sind, die besteht aus:
Halogen, Trifluormethyl, Hydroxy, Cyano, Azido, (C₁-C₆)-Alkoxy, (C₁-C₆)-Perfluoralkoxy, partiell fluoriertem (C₁-C₆)-Alkoxy, einem Rest der Formel 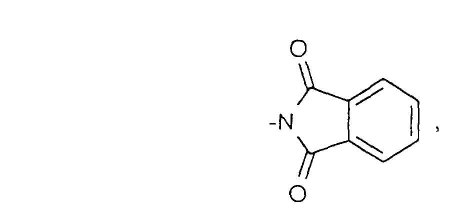
-NR²⁸R²⁹,
worin R²⁸ und R²⁹ die oben angegebene Bedeutung von R⁴ und R⁵ haben und mit dieser gleich oder verschieden sind,
Phenyl, gegebenenfalls substituiert mit einem oder mehreren, gleichen oder verschiedenen Substituenten, die aus der Gruppe ausgewählt sind, die besteht aus:
Halogen, Nitro, Hydroxy, (C₁-C₆)-Alkyl, (C₁-C₆)-Alkoxy und einer Gruppe der Formel -NR³⁰R³¹,
worin R³⁰ und R³¹ gleich oder verschieden sind und Wasserstoff oder (C₁-C₆)-Alkyl oder (C₁-C₆)-Acyl bedeuten,
und einem 5- bis 6-gliedrigen aromatischen Heterocyclus mit bis zu drei Heteroatomen aus der Reihe S, N und/oder O, gegebenenfalls substituiert mit einem oder mehreren, gleichen oder verschiedenen Substituenten, die aus der Gruppe ausgewählt sind, die besteht aus:
Halogen, Nitro, Hydroxy, (C₁-C₆)-Alkyl, (C₁-C₆)-Alkoxy und einer Gruppe der Formel -NR³⁰R³¹,
worin R³⁰ und R³¹ wie oben definiert sind,
oder
L und R² gemeinsam für einen Rest der Formel 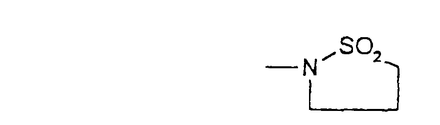 stehen,
und deren Salze,
zur Verwendung als Medikament in der Behandlung von Menschen und Tieren.

2. Verbindungen der Formel (1) nach Anspruch 1, worin
R¹ für Naphthyl oder für einen Rest der Formel 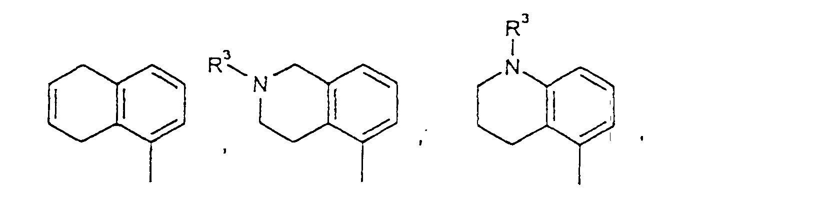 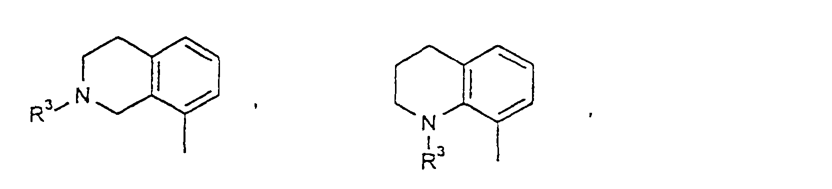 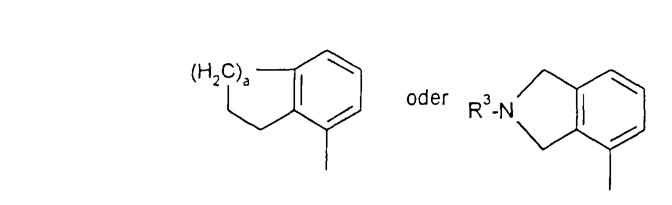 steht,
worin
a eine Zahl 1 oder 2 bedeutet,
R³ Wasserstoff, (C₂-C₄)-Alkenyl, (C₁-C₄)-Alkyl oder (C₁-C₄)-Acyl bedeutet,
und wobei alle oben aufgeführten Ringsysteme und Reste gegebenenfalls mit einem oder mehreren, gleichen oder verschiedenen Substituenten gegebenenfalls geminal substituiert sind, die aus der Gruppe ausgewählt sind, die besteht aus:
Halogen, Carboxyl, Hydroxyl, Phenyl, (C₁-C₄)-Alkoxy, (C₁-C₅)-Alkoxycarbonyl, (C₁-C₆)-Alkyl, das seinerseits durch Halogen, (C₁-C₄)-Alkylsulfonyloxy, Azid, Amino, Mono-(C₁-C₄)-Alkylamino, Di(C₁-C₄)-Alkylamino oder Hydroxy substituiert sein kann,
einer Gruppe der Formel -(CO)_{b}-NR⁴R⁵
worin
b eine Zahl 0 oder 1 bedeutet,
R⁴ und R⁵ gleich oder verschieden sind und unabhängig voneinander Wasserstoff, Phenyl, (C₁-C₄)-Acyl, cyclo(C₄-C₇)-Acyl, Benzoyl oder (C₁-C₄)-Alkyl, das gegebenenfalls durch Amino, Mono(C₁-C₄)-Alkylamino, Di(C₁-C₄)-Alkyl substituiert ist, bedeuten,
oder
R⁴ und R⁵ gemeinsam mit dem Stickstoffatom einen Morpholin-, Piperidin- oder N-Methylpiperazinring bilden,
und
einer Gruppe
der Formel -NR⁶-SO₂-R⁷ worin
R⁶ Wasserstoff, Phenyl, (C₁-C₄)-Alkyl oder (C₁-C₄)-Acyl bedeutet
und
R⁷ Phenyl oder (C₁-C₅)-Alkyl bedeutet,
A und E gleich oder verschieden sind und für eine Bindung oder für (C₁-C₄)-Alkylen stehen,
D für ein Sauerstoffatom oder für einen Rest der Formel -S(O)_{c}- oder -NR⁹ -steht,
worin
c eine Zahl 0, 1 oder 2 bedeutet,
R⁹ Wasserstoff oder (C₁-C₄)-Alkyl oder (C₁-C₄)-Acyl bedeutet,
G für zweifach gebundenes Phenyl, Naphthyl, Pyrimidyl, Pyridazinyl oder Pyridyl steht, die gegebenenfalls mit einem oder mehreren, gleichen oder verschiedenen Substituenten substituiert sind, die aus der Gruppe ausgewählt sind, die besteht aus:
Hydroxy, Trifluormethyl, Carboxyl, Halogen, (C₁-C₄)-Alkyl, Hydroxy(C₁-C₄)alkyl, (C₁-C₄)-Alkoxy, (C₁-C₄)-Alkoxycarbonyl, sowie Gruppen der Formeln
-CO-O-(CH₂)_{d}-NR¹⁰R¹¹, -NR¹²-SO₂R¹³,
-(CH₂)ₑ-(CO)_{f}-NR¹⁴R¹⁵ und -OR¹⁶,
worin
d eine Zahl 1, 2, 3 oder 4 bedeutet,
e und f gleich oder verschieden sind und eine Zahl 0 oder 1 bedeuten,
R¹⁰ und R¹¹ die oben angegebene Bedeutung von R⁴ und R⁵ haben und mit dieser gleich oder verschieden sind,
R¹² die oben angegebene Bedeutung von R⁶ hat und mit dieser gleich oder verschieden ist,
R¹³ die oben angegebene Bedeutung von R⁷ hat und mit dieser gleich oder verschieden ist,
R¹⁴ und R¹⁵ die oben angegebene Bedeutung von R⁴ und R⁵ haben und mit dieser gleich oder verschieden sind, oder unabhängig voneinander einen Rest der Formel
-(CH₂)_{g}-NR¹⁷R¹⁸
darstellen,
worin
g eine Zahl 1, 2 oder 3 bedeutet,
und
R¹⁷ und R¹⁸ die oben angegebene Bedeutung von R¹⁰ und R¹¹ haben und mit dieser gleich oder verschieden sind,
R¹⁶ Phenyl oder Naphthyl bedeutet,
L für einen Rest der Formel
-O-, -NH-, 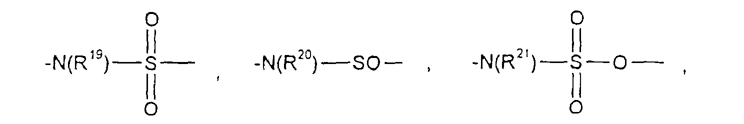 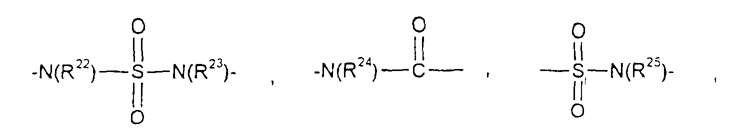 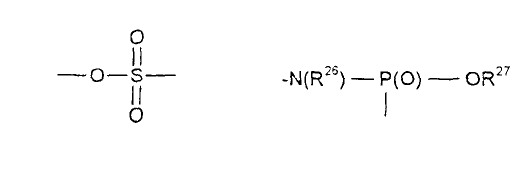
oder 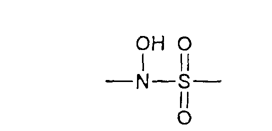 steht,
wobei die Anbindung der Reste an G linksbündig erfolgt,
und
worin
R¹⁹, R²⁰, R²¹, R²², R²³, R²⁴, R²⁵, R²⁶ und R²⁷ gleich oder verschieden sind und Wasserstoff oder (C₁-C₃)-Alkyl bedeuten,
oder
R¹⁹ einen Rest der Formel -SO₂R² bedeutet,
R² für Phenyl, Naphthyl, Pyridyl, Furyl, Thienyl oder Pyrimidyl steht, die gegebenenfalls mit einem oder mehreren, gleichen oder verschiedenen Substituenten substituiert sind, die aus der Gruppe ausgewählt sind, die besteht aus:
Halogen, Amino, Trifluormethyl, Nitro und (C₁-C₄)-Alkyl,
oder
für den Rest der Formel 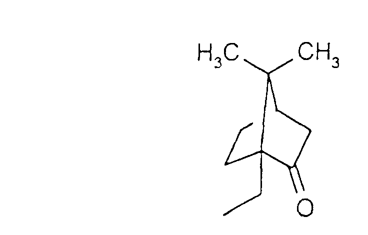 oder für Morpholin steht,
oder
für Cyclopropyl, Cyclohexyl oder Cyclopentyl steht, oder
für (C₁-C₁₀)-Alkyl, (C₂-C₁₀)-Alkenyl oder (C₂-C₁₀)-Alkinyl steht, die gegebenenfalls mit einem oder mehreren, gleichen oder verschiedenen Substitutenen substituiert sind, die aus der Gruppe ausgewählt sind die besteht aus:
Halogen, Trifluormethyl, Hydroxy, Azido, (C₁-C₄)-Alkoxy, (C₁-C₅)-Perfluoralkoxy, partiell fluoriertem (C₁-C₄)-Alkoxy, einem Rest der Formel 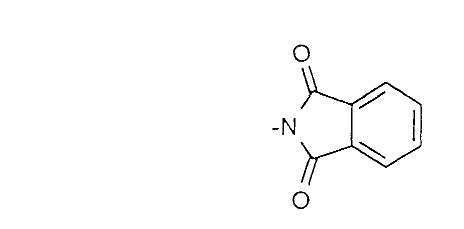 und -NR²⁸R²⁹,
worin
R²⁸ und R²⁹ die oben angegebene Bedeutung von R⁴ und R⁵ haben und mit dieser gleich oder verschieden sind,
Phenyl, gegebenenfalls substituiert mit einem oder mehreren, gleichen oder verschiedenen Substituenten, die aus der Gruppe ausgewählt sind, die besteht aus:
Halogen, Nitro, Hydroxy, (C₁-C₄)-Alkyl, (C₁-C₄)-Alkoxy und einer Gruppe der Formel -NR³⁰R³¹,
worin R³⁰ und R³¹ gleich oder verschieden sind und Wasserstoff oder (C₁-C₄)-Alkyl oder (C₁-C₄)-Acyl bedeuten,
Pyridyl und Pyrimidyl, gegebenenfalls substituiert mit einem oder mehreren, gleichen oder verschiedenen Substituenten, die aus der Gruppe ausgewählt sind, die besteht aus:
Halogen, Nitro, Hydroxy, (C₁-C₄)-Alkyl, (C₁-C₄)-Alkoxy und einer Gruppe der Formel -NR³⁰R³¹,
worin R³⁰ und R³¹ wie oben definiert sind,
oder
L und R² gemeinsam für einen Rest der Formel 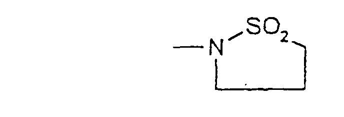 stehen, und deren Salze,
zur Verwendung als Medikament in der Behandlung von Menschen und Tieren.

3. Verbindungen der Formel (I) nach Anspruch 1, worin
R¹ für Naphthyl, oder für einen Rest der Formel 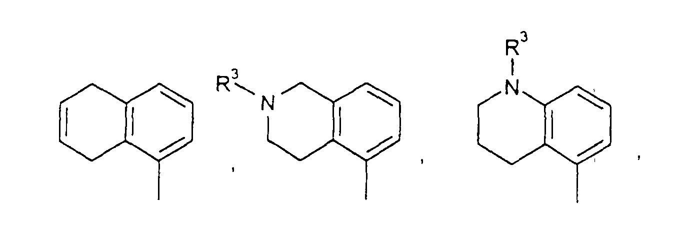 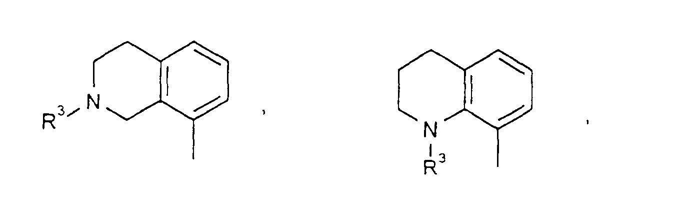 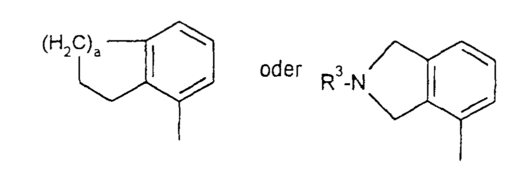 steht,
worin
a eine Zahl 1 oder 2 bedeutet,
R³ Wasserstoff, (C₂-C₃)-Alkenyl, (C₁-C₃)-Alkyl oder (C₁-C₃)-Acyl bedeutet,
und wobei alle oben aufgeführten Ringsysteme gegebenenfalls mit einem oder mehreren, gleichen oder verschiedenen Substituenten gegebenenfalls geminal substituiert sind, die aus der Gruppe ausgewählt sind, die besteht aus:
Chlor, Fluor, Carboxyl, Hydroxyl, Phenyl, (C₁-C₃)-Alkoxy, (C₁-C₄)-Alkoxycarbonyl, (C₁-C₄)-Alkyl, das seinerseits durch Chlor oder Hydroxy substituiert sein kann,
einer Gruppe der Formel -(CO)_{b}-NR⁴R⁵
worin
b eine Zahl 0 oder 1 bedeutet,
R⁴ und R⁵ gleich oder verschieden sind und unabhängig voneinander Wasserstoff, (C₁-C₃)-Acyl, cyclo(C₄-C₆)-Acyl, Benzoyl oder (C₁-C₃)-Alkyl, das gegebenenfalls durch Amino, Mono(C₁-C₃)-Alkylamino, Di(C₁-C₃)-Alkylamino substituiert ist, bedeuten,
oder
R⁴ und R⁵ gemeinsam mit dem Stickstoffatom einen Morpholin-, Piperidin- oder N-Methylpiperazinring bilden,
und
einer Gruppe der Formel -NR⁶-SO₂-R⁷
worin
R⁶ Wasserstoff, (C₁-C₃)-Alkyl oder (C₁-C₃)-Acyl bedeutet,
und
R⁷ Phenyl oder (C₁-C₄)-Alkyl bedeutet,
A und E gleich oder verschieden sind und für eine Bindung oder für (C₁-C₃)- Alkyl stehen,
D für ein Sauerstoffatom oder für einen Rest der Formel -S(O)_{c}- oder -NR⁹- steht,
worin
c eine Zahl 0, 1 oder 2 bedeutet,
R⁹ Wasserstoff oder (C₁-C₃)-Alkyl oder (C₁-C₃)-Acyl bedeutet,
G für zweifach gebundenes Phenyl, Naphthyl, Pyrimidyl, Pyridazinyl oder Pyridyl steht, die gegebenenfalls mit einem oder mehreren, gleichen oder verschiedenen Substituenten substituiert sind, die aus der Gruppe ausgewählt sind, die besteht aus:
Hydroxy, Trifluormethyl, Carboxyl, Fluor, Chlor, Brom, (C₁-C₃)-Alkyl, Hydroxy(C₁-C₃)alkyl, (C₁-C₃)-Alkoxy, (C₁-C₃)-Alkoxycarbonyl, sowie Gruppen der Formeln
-CO-O-(CH₂)_{d}-NR¹⁰R¹¹, -NR¹²-SO₂R¹³,
-(CH₂)ₑ-(CO)_{f}-NR¹⁴R¹⁵, -CH₂OH und -OR¹⁶,
worin
d eine Zahl 1, 2 oder 3 bedeutet,
e und f gleich oder verschieden sind und eine Zahl 0 oder 1 bedeuten,
R¹⁰ und R¹¹ Wasserstoff oder Methyl bedeuten,
R¹² Wasserstoff bedeutet,
R¹³ (C₁-C₄)-Alkyl bedeutet,
R¹⁴ und R¹⁵ die oben angegebene Bedeutung von R⁴ und R⁵ haben und mit dieser gleich oder verschieden sind oder einen Rest der Formel -(CH₂)_{g}-NR¹⁷R¹⁸ bedeuten,
worin
g eine Zahl 1, 2 oder 3 bedeutet,
und
R¹⁷ und R¹⁸ Wasserstoff oder Methyl bedeuten,
oder
R¹⁴ und R¹⁵ gemeinsam mit dem Stickstoffatom einen Rest der Formel 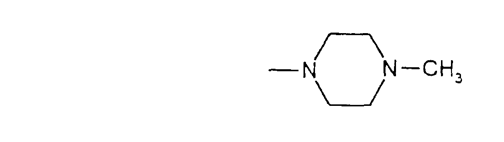 bilden,
R¹⁶ Phenyl oder Naphthyl bedeutet,
L für einen Rest der Formel
O-, -NH-, 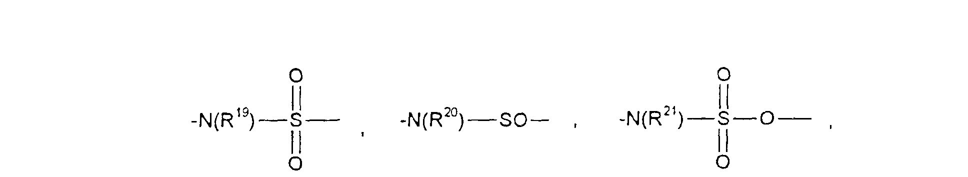 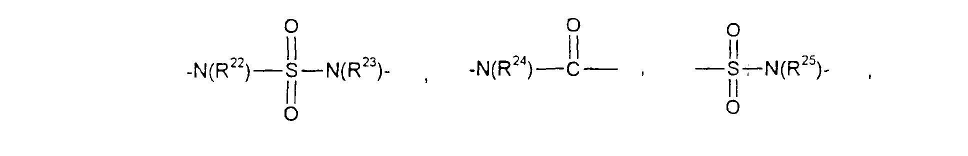 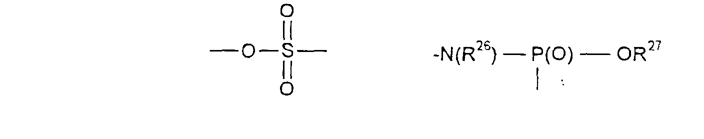
oder 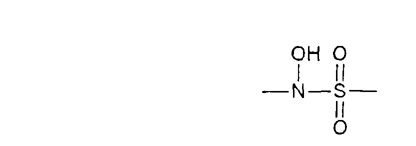 steht,
wobei die Anbindung der Reste an G linksbündig erfolgt,
und
worin
R¹⁹, R²⁰, R²¹, R²², R²³, R²⁴, R²⁵, R²⁶ und R²⁷ gleich oder verschieden sind und Wasserstoff, Methyl oder Ethyl bedeuten,
oder
R¹⁹ einen Rest der Formel -SO₂R² bedeutet,
R² für Phenyl, Furyl oder Pyridyl steht, die gegebenenfalls mit einem oder mehreren, gleichen oder verschiedenen Substituenten substituiert sind, die aus der Gruppe ausgewählt sind, die besteht aus: Fluor, Chlor, Brom oder Trifluormethyl,
oder
für den Rest der Formel 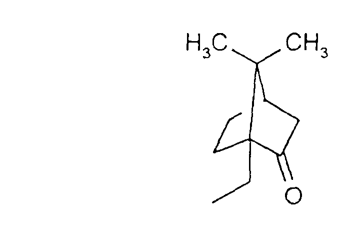 oder Morpholin steht,
oder
für Cyclopentyl oder Cyclohexyl steht, oder
für (C₁-C₈)-Alkyl, (C₂-C₈)-Alkenyl oder (C₂-C₈)-Alkinyl steht, die gegebenenfalls mit einem oder mehreren, gleichen oder verschiedenen Substitutenen substituiert sind, die aus der Gruppe ausgewählt sind, die besteht aus:
Fluor, Chlor, Brom, Trifluormethyl, Hydroxy, Azido, (C₁-C₃)-Alkoxy, (C₁-C₄)-Perfluoralkoxy, Trifluormethyl-substituiertem (C₁-C₄)-Alkoxy, einem Rest der Formel 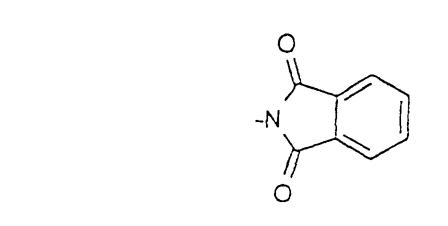 und -NR²⁸R²⁹,
worin
R²⁸ und R²⁹ Wasserstoff oder Methyl bedeuten,
Phenyl, Pyridyl und Pyrimidyl, gegebenenfalls substituiert mit einem oder mehreren, gleichen oder verschiedenen Substituenten, die aus der Gruppe ausgewählt sind, die besteht aus:
Fluor, Chlor, Brom, Nitro, Hydroxy, (C₁-C₃)-Alkyl, (C₁-C₃)-Alkoxy und einer Gruppe der Formel -NR³⁰R³¹,
worin R³⁰ und R³¹ gleich oder verschieden sind und Wasserstoff, Methyl oder Methylcarbonyl bedeuten,
oder
L und R² gemeinsam für einen Rest der Formel 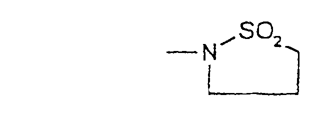 stehen,
und deren Salze
zur Verwendung als Medikament in der Behandlung von Menschen und Tieren.

4. Verbindungen der allgemeinen Formel (I),
R¹-A-D-E-G-L-R² (I)
in welcher R¹, A, D, E, G, L und R² die in einem der Ansprüche 1 bis 3 angegebene Bedeutung haben,
mit der Ausnahme von Verbindungen der allgemeinen Formel (I),
in welchen
R¹ für Naphth-1-yl steht, wobei die 3-Position des Naphth-1-yl-Rests gegebenenfalls mit Chlor oder (C₁-C₄)-Alkyl substituiert ist und die 4-Position des Naphth-1-yl-Rests gegebenenfalls mit Chlor oder Phenyl substituiert ist,
A und E für eine Bindung stehen,
D für ein Sauerstoffatom steht,
G für 1,4-Phenylen, das gegebenenfalls durch (C₁-C₄)-Alkyl substituiert ist, steht,
L für ein Sauerstoffatom steht, und
R² für Methyl steht,
und mit der Ausnahme von m-bis-(1-Naphthyloxy)benzol.

5. Verbindungen der Formel (I) nach Anspruch 1,
worin
R¹ für Maphthyl oder für einen Rest der Formel 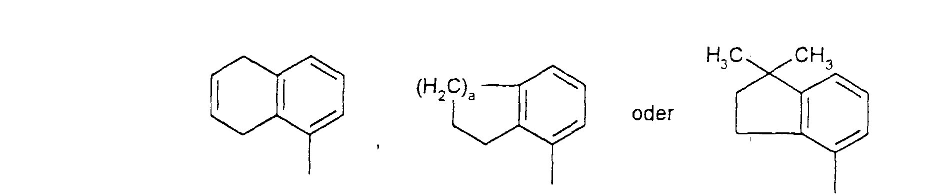 steht,
worin
a eine Zahl 1 oder 2 bedeutet,
und wobei alle oben aufgeführten Ringsysteme und Reste gegebenenfalls mit 1 bis 3, gleichen oder verschiedenen Substituenten, gegebenenfalls geminal, substituiert sind, die aus der Gruppe ausgewählt sind, die besteht aus:
Halogen, Carboxyl, Hydroxy, (C₁-C₆)-Alkoxy, (C₁-C₆)-Alkoxycarbonyl, (C₁-C₈)-Alkyl, das seinerseits durch Halogen oder Hydroxy substituiert sein kann,
einer Gruppe der Formel -(CO)_{b}-NR⁴R⁵,
worin
b eine Zahl 0 oder 1 bedeutet,
R⁴ und R⁵ Phenyl gleich oder verschieden sind und Wasserstoff, oder (C₁-C₆)-Alkyl bedeuten,
und
einer Gruppe der Formel -NR⁶-SO₂-R⁷
worin
R⁶ Wasserstoff, Phenyl oder (C₁-C₆)-Alkyl bedeutet,
R⁷ Phenyl oder (C₁-C₆)-Alkyl bedeutet,
A und E gleich oder verschieden sind und für eine Bindung oder für (C₁-C4)-Alkylen stehen,
D für ein Sauerstoffatom oder für einen Rest der Formel -S(O)_{c}- oder -NH- steht,
worin
c eine Zahl 0, 1 oder 2 bedeutet,
G für zweifach gebundenes (C₆-C₁₀)-Aryl oder für einen zweifach gebundenen 5- bis 7-gliedrigen aromatischen Heterocyclus mit bis zu 3 Heteroatomen aus der Reihe S, N und/oder O steht, die gegebenenfalls mit einem bis drei, gleichen oder verschiedenen Substituenten substituiert sind, die aus der Gruppe ausgewählt sind, die besteht aus:
Hydroxy, Carboxyl, Halogen, (C₁-C₆)-Alkyl, Hydroxy-(C₁-C₆)alkyl, (C₁-C₆)-Alkoxy, (C₁-C₆)-Alkoxycarbonyl, sowie Gruppen der Formeln
-CO-O-(CH₂)_{d}-NR¹⁰R¹¹, -NR¹²-SO₂R¹³ und -CO-NR¹⁴R¹⁵
worin
d eine Zahl 1, 2, 3 oder 4 bedeutet,
R¹⁰ und R¹¹ die oben angegebene Bedeutung von R⁴ und R⁵ haben und mit dieser gleich oder verschieden sind,
R¹² die oben angegebene Bedeutung von R⁶ hat und mit dieser gleich oder verschieden ist,
R¹³ die oben angegebene Bedeutung von R⁷ hat und mit dieser gleich oder verschieden ist,
R¹⁴ und R¹⁵ die oben angegebene Bedeutung von R⁴ und R⁵ haben und mit dieser gleich oder verschieden sind, oder gemeinsam mit dem Stickstoffatom einen 5- bis 6-gliedrigen gesättigten Heterocyclus bilden, der gegebenenfalls noch ein weiteres Heteroatom aus der Reihe S und O oder eine Gruppe der Formel -NH- enthalten kann,
L für einen Rest der Formel 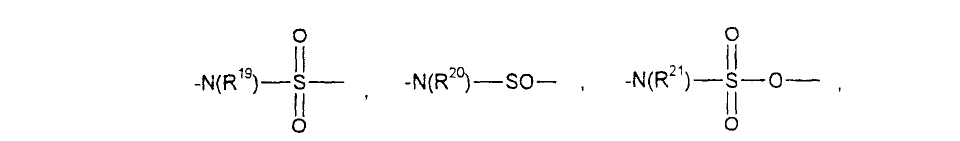 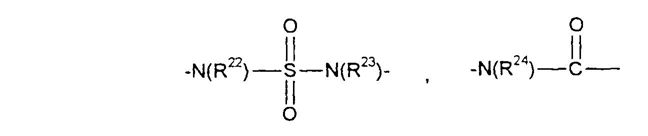 oder 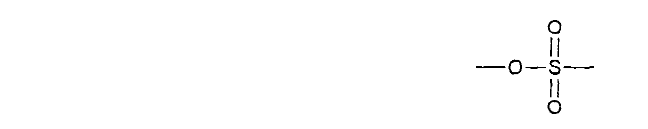 steht,
wobei die Anbindung der Reste an G linksbündig erfolgt,
und worin R¹⁹, R²⁰, R²¹, R²², R²³ und R²⁴ gleich oder verschieden sind und Wasserstoff oder (C₁-C₄)-Alkyl bedeuten,
R² für Phenyl steht, das gegebenenfalls mit Halogen, Trifluormethyl, Nitro, Amino, oder (C₁-C₆)-Alkyl substituiert ist,
R² für den Rest der Formel 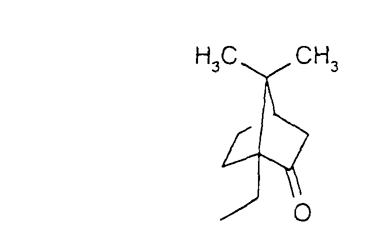 oder Morpholin steht,
oder
für Perfluoralkyl mit bis zu 12 Fluoratomen steht, oder
für (C₁-C₁₂)-Alkyl oder (C₂-C₁₂)-Alkinyl steht, die gegebenenfalls mit Halogen, Trifluormethyl, Hydroxy, Azido oder durch einen Rest der Formel 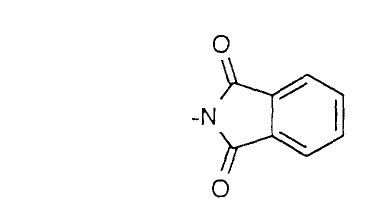 oder -NR²⁸R²⁹ substituiert sind,
worin R²⁸ und R²⁹ die oben angegebene Bedeutung von R⁴ und R⁵ haben und mit dieser gleich oder verschieden sind,
und/oder gegebenenfalls durch Phenyl oder durch einen 5- bis 6-gliedrigen aromatischen Heterocyclus mit bis zu 3 Heteroatomen aus der Reihe S, N und/oder O substituiert sind, die ihrerseits bis zu 2-fach gleich oder verschieden durch Halogen, Nitro, Hydroxy, (C₁-C₆)-Alkyl, (C₁-C₆)-Alkoxy oder durch eine Gruppe der Formel -NR³⁰R³¹ substituiert sein können,
worin R³⁰ und R³¹ gleich oder verschieden sind und Wasserstoff, (C₁-C₆)-Alkyl oder (C₁-C₆)-Acyl bedeuten,
L und R² gemeinsam für einen Rest der Formel 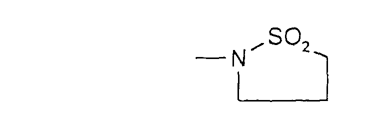 stehen,
und deren Salze.

6. Verbindungen der Formel (I) nach Anspruch 1,
worin
R¹ für Naphth-1-yl, gegebenenfalls substituiert durch (C₁-C₆)-Alkyl substituiert mit Hydroxy, (C₁-C₆)-Acylamino, Amino oder (C₁-C₆)-Alkoxy, Indan-4-yl, substituiert durch Hydroxy(C₁-C₆)-Alkyl,
für einen Rest der Formel 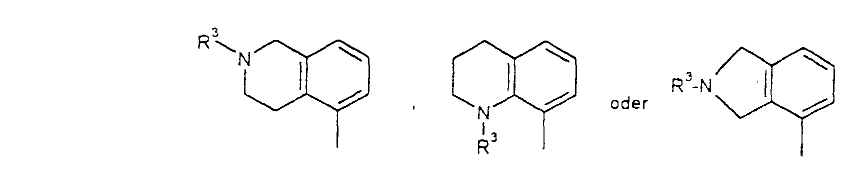 steht,
worin
R³ (C₁-C₆)-Alkyl ist,
E und A für eine Bindung stehen,
D für ein Sauerstoffatom stehen,
G für 1,3-Phenylen, 1,4-Phenylen oder 2,5-Pyrildylen steht, die gegebenenfalls durch Halogen substituiert sind,
L für einen Rest der Formel -NH-SO₂- oder -O-SO₂- steht und
R² für (C₁-C₆)-Alkyl steht, das gegebenenfalls durch Chlor, Trifluormethyl, durch einen Rest der Formel -O-CH₂-CF₃ oder durch Phenyl oder durch Pyridyl substituiert ist, die ihrerseits durch Brom oder Chlor substituiert sein können,
und deren Salze.

7. Verbindungen der folgenden Formeln:

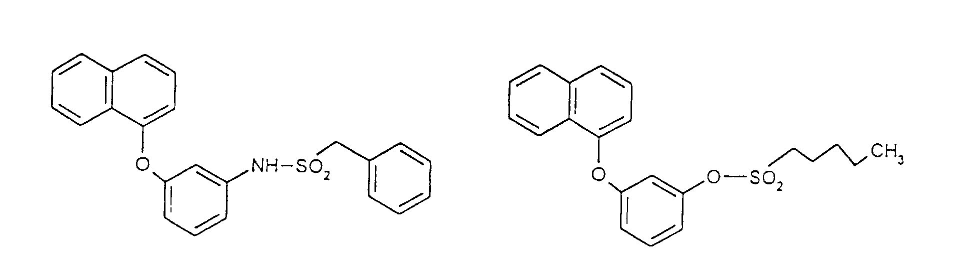

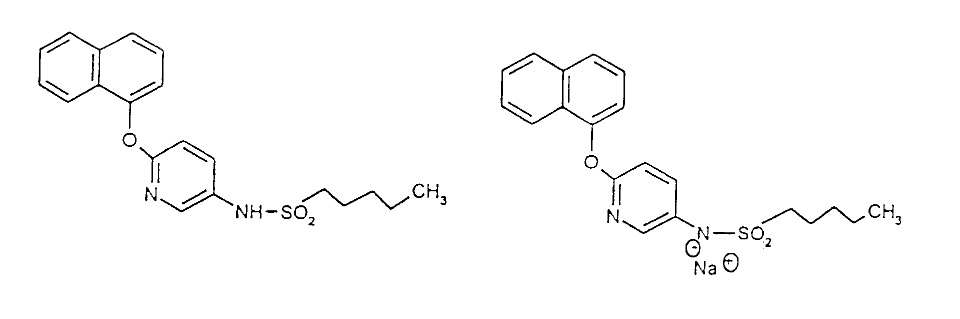

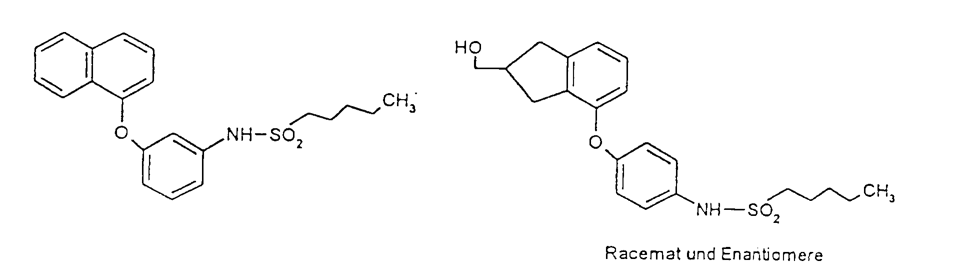

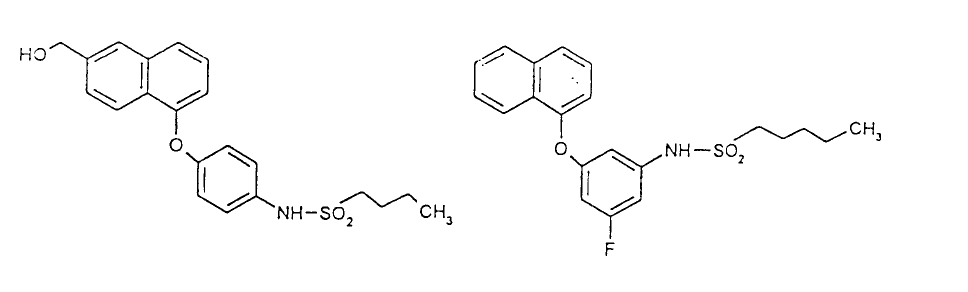

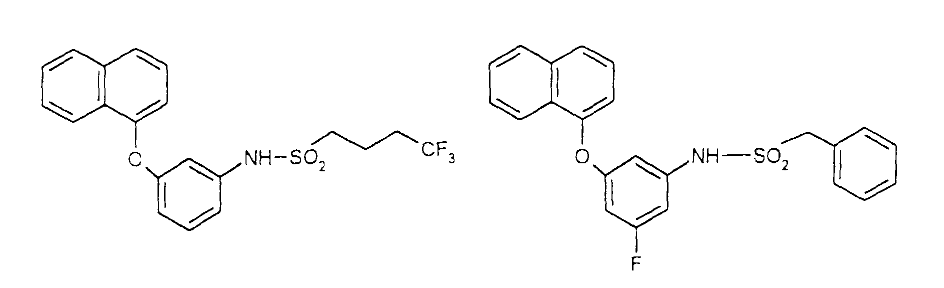

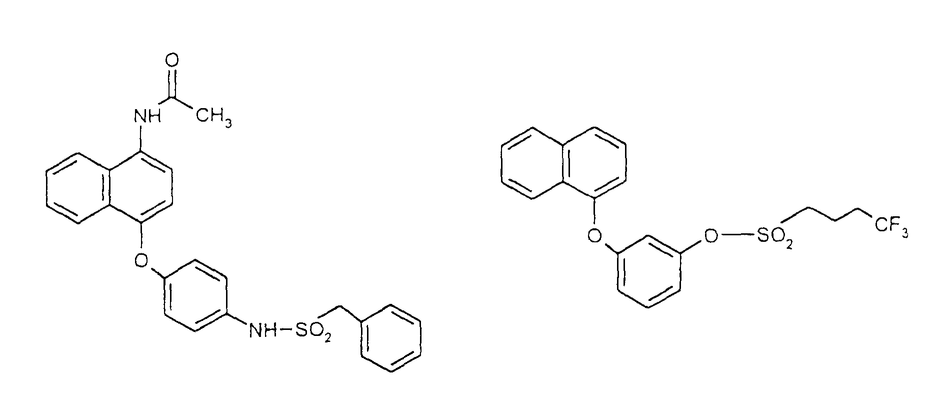

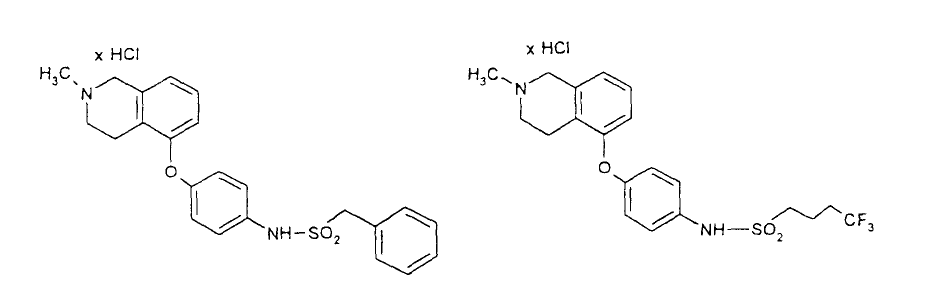

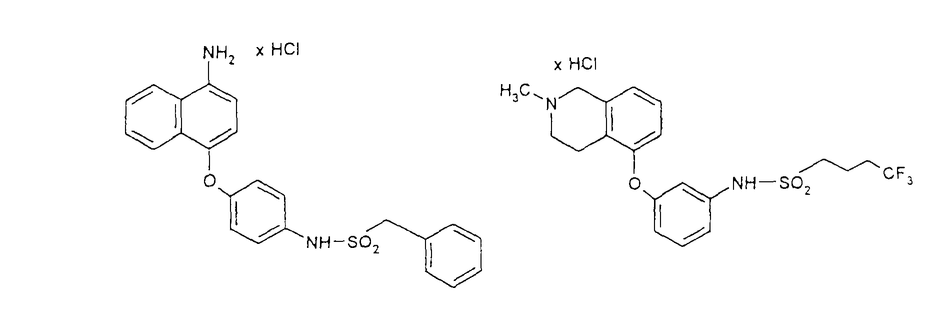

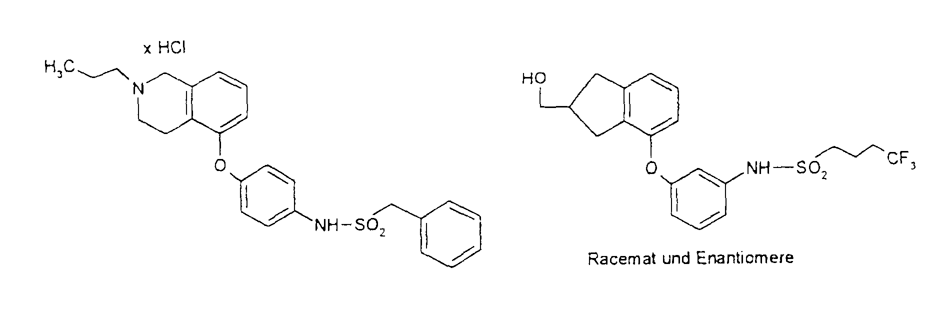

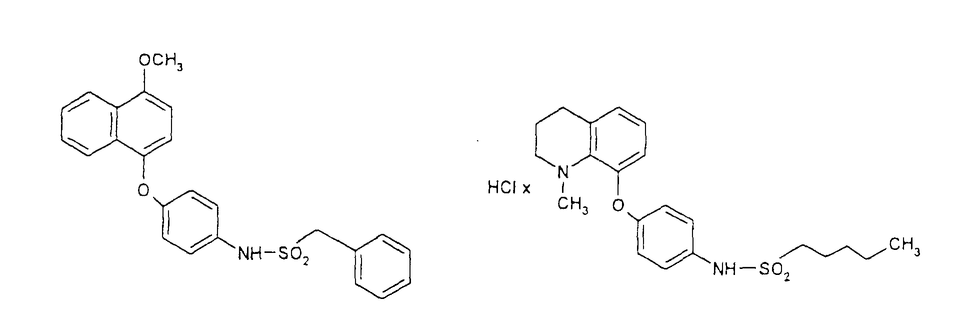

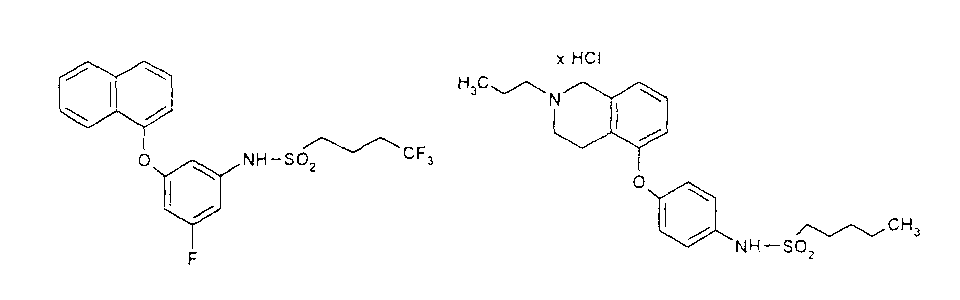

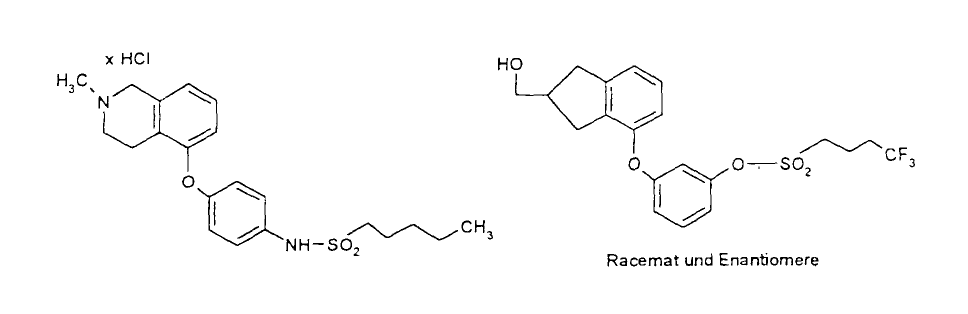

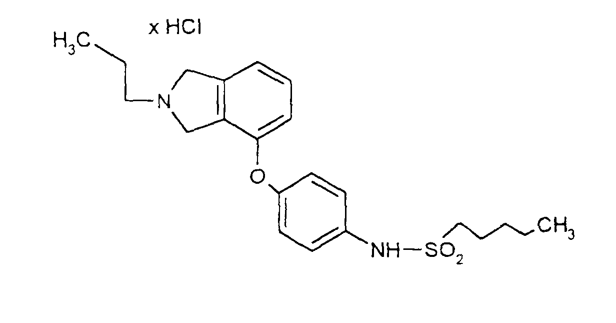

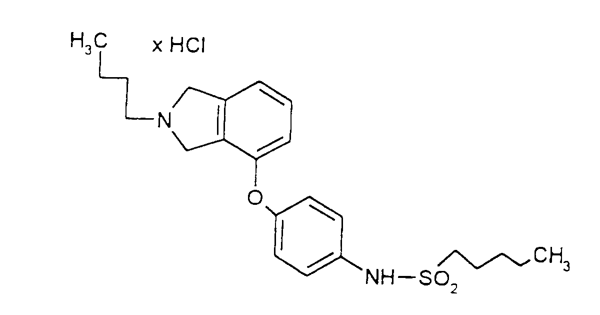

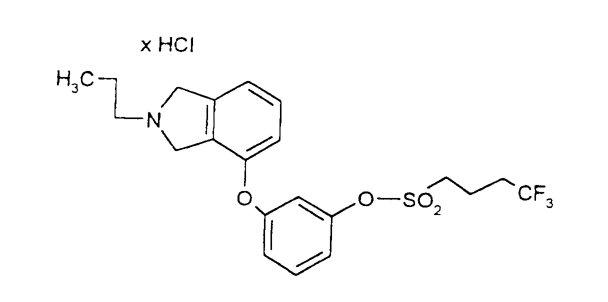

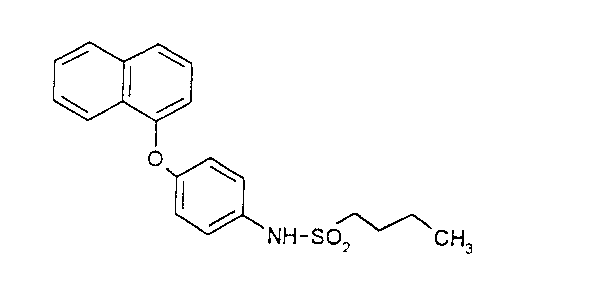

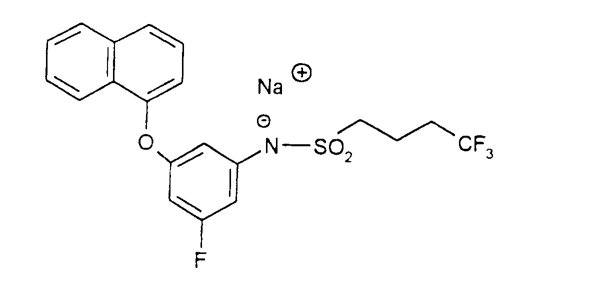

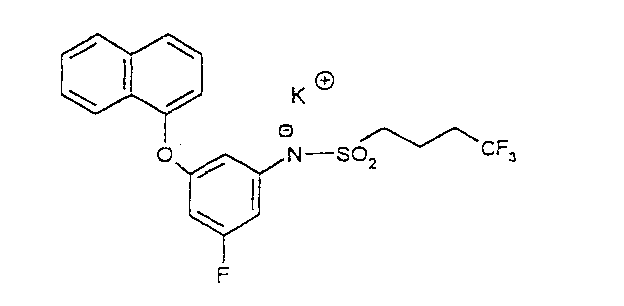

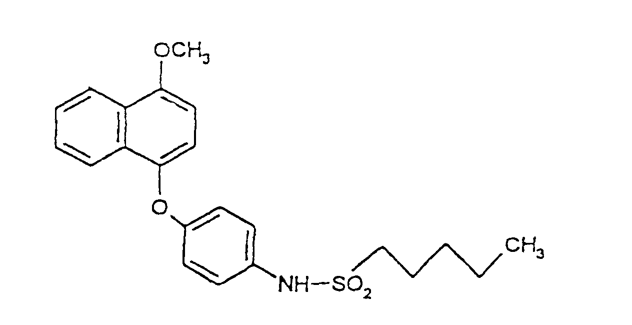

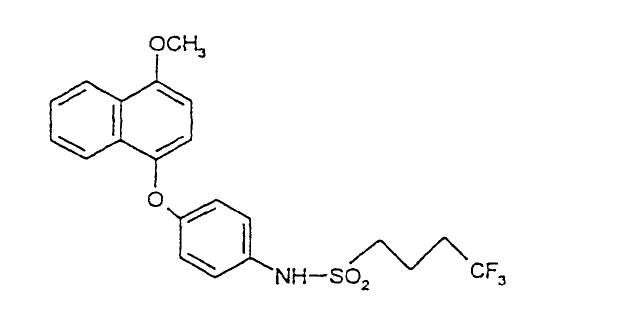

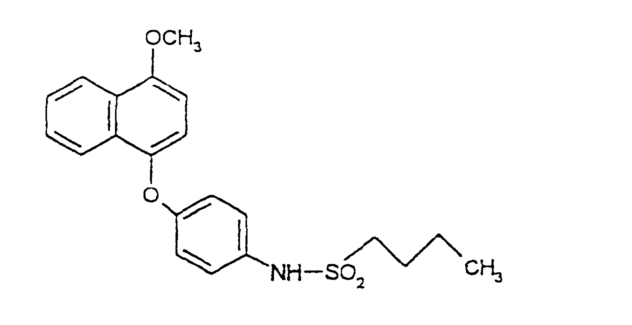

8. Verfahren zur Herstellung der Verbindungen nach Anspruch 4,
worin
[A] Verbindungen der allgemeinen Formel (II)
R¹-A-D-E-G-M-H (II)
in welcher
R¹, A, D, E und G die im Anspruch 1 angegebene Bedeutung haben und
M für Sauerstoff oder -N(R³²)- steht und
R³² Wasserstoff oder (C₁-C₄)-Alkyl ist,
mit Verbindungen der allgemeinen Formel (III)
R³³-Q-R² (III)
in welcher
R² die im Anspruch 1 angegebene Bedeutung hat,
R³³ für Halogen, vorzugsweise Chlor oder Iod steht,
Q für einen Rest der Formel -SO₂-, -SO-, -CO- , -P(O)(OR²⁷)- oder eine Einfachbindung steht,
worin
R²⁷ die im Anspruch 1 angegebene Bedeutung hat,
zu Verbindungen der allgemeinen Formel (Ia)
R¹-A-D-E-G-M-Q-R² (Ia)
in welcher
R¹, A, D, E, G, M, Q und R² die oben angegebene Bedeutung haben,
in inerten Lösemitteln, gegebenenfalls in Anwesenheit einer Base, umgesetzt werden
oder
[B] Verbindungen der allgemeinen Formel (II)
zunächst mit Chlorsulfonsäuretrialkylsilylester, vorzugsweise Chlorsulfonsäuretrimethylsilylester, umgesetzt werden, mit einer Säure versetzt werden und dann mit einem Chlorierungsmittel, vorzugsweise Phosphorpentachlorid, zu einer Verbindung der allgemeinen Formel (IV)
R¹-A-D-E-G-M-SO₂-Cl (IV)
in welcher
R¹, A, D, E, G und M die oben angegebene Bedeutung haben,
umgesetzt werden und anschließend mit Verbindungen der allgemeinen Formel (V)
H-T-R² (V)
in welcher
R² die im Anspruch 1 angegebene Bedeutung hat und
T für Sauerstoff oder Stickstoff steht,
zu Verbindungen der allgemeinen Formel (Ib)
R¹-A-D-E-G-M-SO₂-T-R² (Ib)
in welcher
R¹, A, D, E, G, M, T und R² die oben angegebene Bedeutung haben,
in inerten Lösemitteln, gegebenenfalls in Anwesenheit von Bzl-NEt₃+Cl⁻ und einer Base, umgesetzt werden
oder
[C] Verbindungen der allgemeinen Formel (VI)
R¹-A-D'-H (VI)
in welcher
R¹ und A die oben angegebene Bedeutung haben und
D' für Sauerstoff, Schwefel oder -N(R⁹)- steht und
R⁹ die in Anspruch 1 angegebene Bedeutung hat,
mit Verbindungen der allgemeinen Formel (VII)
R³⁴-E-G-SO₂-NH-R² (VII)
in welcher
E, G und R² die oben angegebene Bedeutung haben und
R³⁴ für eine Abgangsgruppe, bevorzugt Halogen, besonders bevorzugt Fluor, Chlor oder Brom steht,
zu Verbindungen der allgemeinen Formel (Ic)
R¹-A-D'-E-G-SO₂-NH-R² (Ic)
in welcher
R¹, A, D', E, G und R² die oben angegebene Bedeutung haben,
umgesetzt werden,
oder
[D] Verbindungen der allgemeinen Formel (Id)
R³⁷-A-D-E-G-L-R² (Id)
in welcher
A, D, E, G, L und R² die oben angegebene Bedeutung haben und
R³⁷ für einen Rest der Formel 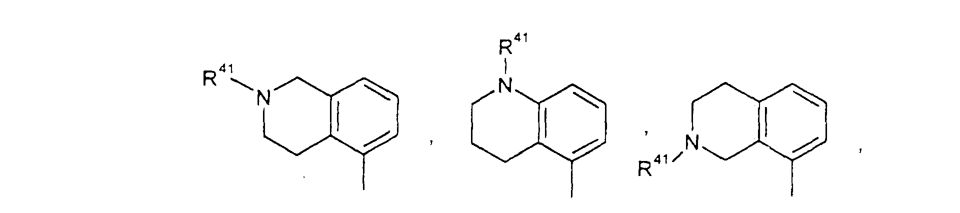 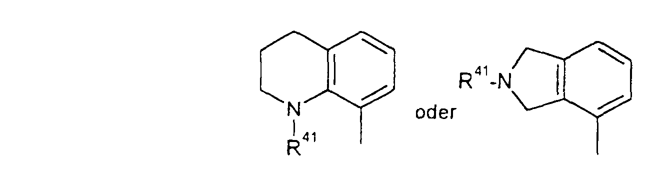 steht,
worin
R⁴¹ für (C₁-C₆)-Alkyl steht,
mit Chlorameisensäureester, vorzugsweise Chlorameisensäure-1-(1-chlor)-ethylester oder Chlorameisensäuremethylester, und anschließend mit Alkoholen, bevorzugt Methanol, gegebenenfalls in Anwesenheit einer Base, zu Verbindungen der allgemeinen Formel (Ie)
R³⁸-A-D-E-G-L-R² (Ie)
in welcher
A, D, E, G, L und R² die oben angegebene Bedeutung haben und
R³⁸ für einen Rest der Formel 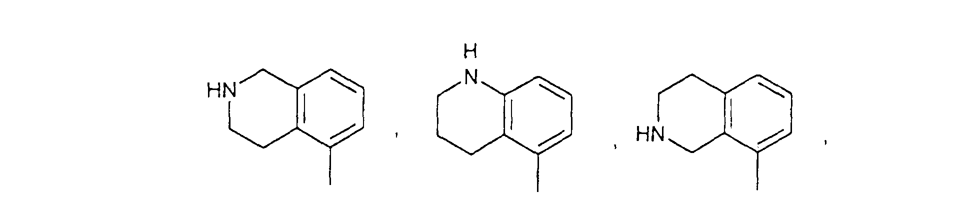 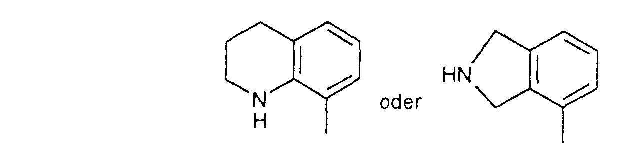 steht,
umgesetzt werden
oder
[E] Verbindungen der allgemeinen Formel (Ie)
mit (C₁-C₆)-Ketonen oder (C₁-C₆)-Aldehyden in Gegenwart eines Reduktionsmittels, vorzugsweise Natriumcyanoborhydrid, gegebenenfalls in der Gegenwart einer Säure zu Verbindungen der allgemeinen Formel (If)
R³⁹-A-D-E-G-L-R² (If)
in welcher
A, D, E, G, L und R² die oben angegebene Bedeutung haben und
R³⁹ für (C₃-C₆)-Alkenyl oder (C₁-C₆)-Alkyl steht,
umgesetzt werden
oder
[F] Verbindungen der allgemeinen Formel (Ie) mit Verbindungen der allgemeinen Formel (VIII)
R³⁵-R³ (VIII)
in welcher
R³ die in Anspruch 1 genannte Bedeutung hat,
R³⁵ für eine Abgangsgruppe, bevorzugt Halogen steht,
in inerten Lösemitteln, gegebenenfalls in Anwesenheit einer Base, zu Verbindungen der allgemeinen Formel (Ig)
R⁴⁰-A-D-E-G-L-R² (Ig)
in welcher
A, D, E, G, L und R² die oben angegebene Bedeutung haben und
R⁴⁰ für einen Rest der Formel 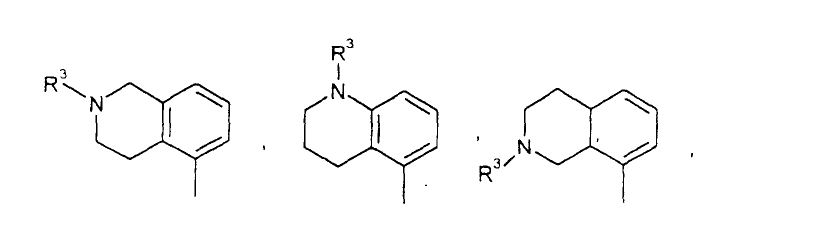 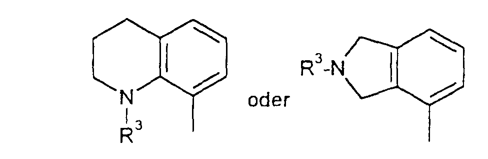 steht,
worin
R³ die oben angegebene Bedeutung hat,
umgesetzt werden,
oder
[G] Verbindungen der allgemeinen Formel (Ih) 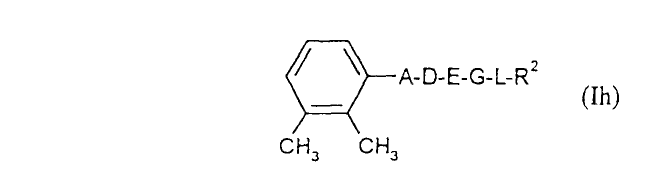 in welcher
A, D, E, G, L und R² die oben angegebene Bedeutung haben,
durch radikalische Bromierung, beispielsweise mit N-Bromsuccinimid, in einem inerten Lösungsmittel in Verbindungen der allgemeinen Formel (Ii) 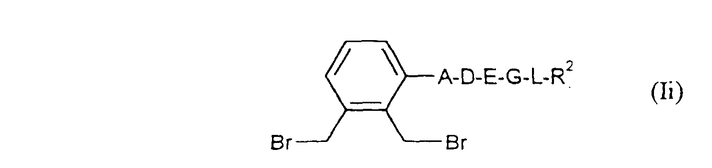 in welcher
A, D, E, G, L und R² die oben angegebene Bedeutung haben,
übergeführt werden,
und anschließend mit Verbindungen der allgemeinen Formel (IX) oder (X)
CH₂(CO₂R⁴²)₂ (IX)
H₂N-R³ (X)
in welchen
R⁴² für (C₁-C₆)-Alkyl steht und
R³ die oben angegebene Bedeutung hat,
in inerten Lösemitteln, gegebenenfalls in Anwesenheit einer Base, zu Verbindungen der allgemeinen Formel (Ij)
R⁴³-A-D-E-G-L-R² (Ij)
in welcher
A, D, E, G, L und R² die oben genannte Bedeutung haben und
R⁴³ für 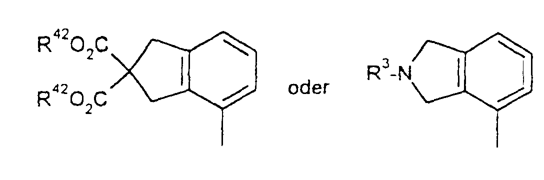 steht,
worin
R⁴² und R³ die oben genannte Bedeutung haben,
umgesetzt werden,
und gegebenenfalls die oben aufgeführten Substituenten nach üblichen Methoden eingeführt und derivatisiert werden,
und im Fall D ist = -SO- oder -SO₂- ausgehend von den entsprechenden Thioethern (D = S) eine Oxidation nach üblichen Methoden durchgeführt wird,
und im Fall der Ammoniumverbindungen ausgehend von den entsprechenden Aminen eine Alkylierung durchgeführt wird.

9. Verbindungen der allgemeinen Formel (II)
R¹-A-D-E-G-M-H (II)
in welcher
R¹ für einen Rest der Formel 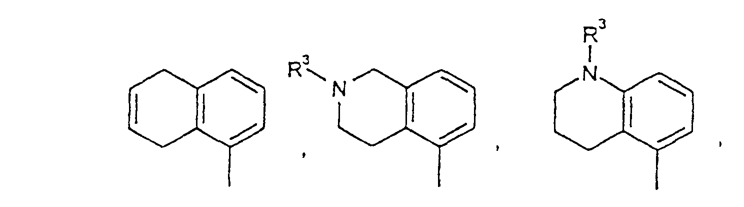 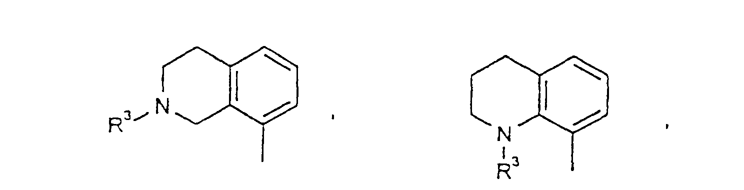 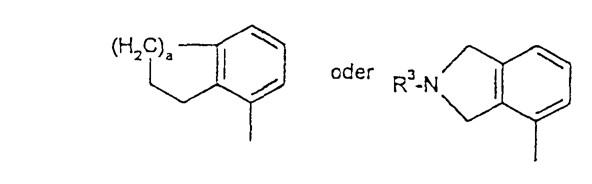 steht,
worin
a eine Zahl 1 bedeutet,
R³ Wasserstoff, (C₂-C₆)-Alkenyl, (C₁-C₆)-Alkyl oder (C₁-C₆)-Acyl bedeutet,
und wobei alle oben aufgeführten Ringsysteme und Reste gegebenenfalls wie in Anspruch 1 angegeben substituiert sind, und
A, D, E, G und M die in den Ansprüchen 1 und 8 angegebenen Bedeutungen haben.

10. Verbindungen der allgemeinen Formel (II) gemäß Anspruch 9, in welcher
R¹ für Indan-4-yl, substituiert durch Hydroxy(C₁-C₆)-Alkyl, oder für einen Rest der Formel 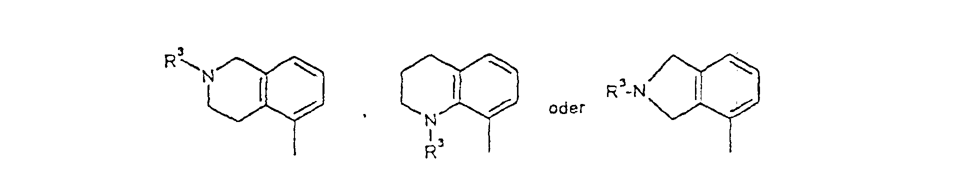 steht,
worin
R³ (C₁-C₆)-Alkyl ist,
A, D, E und G die in Anspruchs genannten Bedeutungen haben und
M die in Anspruch 8 angegebene Bedeutung hat.

11. Verfahren zur Herstellung der Verbindungen nach Anspruch 9, wobei
[A] Verbindungen der allgemeinen Formel (VI)
R¹-A-D'-H (VI)
in welcher
R¹, A und D' die in Anspruch 8 angegebene Bedeutung haben,
mit Verbindungen der allgemeinen Formel (XI)
R⁴⁴-E-G-NO₂ (XI)
in welcher
E und G die in Anspruch 1 angegebene Bedeutung haben und
R⁴⁴ eine Abgangsgruppe, bevorzugt Halogen, ist,
in inerten Lösemitteln, gegebenenfalls in Anwesenheit einer Base, umgesetzt werden und anschließend mit üblichen Reduktionsmitteln, vorzugsweise H₂/Pd/C in einem inerten Lösemittel oder mit Hydrazinhydrat, Pd/C, gegebenenfalls unter gleichzeitiger Hydrogenierung von (C-C)-Mehrfachbindungen, zu Verbindungen der allgemeinen Formel (IIa)
R¹-A-D'-E-G-NH₂ (IIa)
in welcher
R¹, A, D', E und G die oben angegebene Bedeutung haben, umgesetzt werden,
oder
[B] Verbindungen der allgemeinen Formel (IIb)
R¹-A-D-E-G-NH₂ (IIb)
in welcher
R¹, A, D, E und G die oben angegebene Bedeutung haben, mit einem Nitrosierungsmittel, bevorzugt einer wäßrigen Lösung von Schwefelsäure und Natriumnitrit und anschließender Erwärmung, bevorzugt auf 60 bis 100°C, zu Verbindungen der allgemeinen Formel (IIc)
R¹-A-D-E-G-OH (IIc)
in welcher
R¹, A, D, E und G die oben genannte Bedeutung haben,
umgesetzt werden,
oder
[C] Verbindungen der allgemeinen Formel (XII)
R¹-R³⁶ (XII)
in welcher
R¹ die oben angegebene Bedeutung hat und
R³⁶ für eine Abgangsgruppe, bevorzugt Halogen, besonders bevorzugt Brom, steht,
mit Verbindungen der allgemeinen Formel (XIII)
HO-G-O-R⁴⁵ (XIII)
in welcher
G die oben angegebene Bedeutung hat und
R⁴⁵ für (C₁-C₆)-Alkyl, bevorzugt Methyl, steht,
in einem inerten Lösungsmittel, bevorzugt Dimethylformamid oder Pyridin, gegebenenfalls in Anwesenheit einer Base, bevorzugt Kaliumcarbonat, und gegebenenfalls in Anwesenheit von Kupfer (I/II)-Salzen, bevorzugt Kupfer (II)-Oxid oder Kupfer (I)-Iodid, in einem Temperaturbereich von 0°C bis 200°C, bevorzugt 80 bis 150°C und Normaldruck zu Verbindungen der allgemeinen Formel (Ik)
R¹-O-G-O-R⁴⁵ (Ik)
in welcher
R¹, G und R⁴⁵ die oben genannte Bedeutung haben,
umgesetzt werden und anschließend in Gegenwart einer Säure, bevorzugt Bromwasserstoffsäure, zu Verbindungen der allgemeinen Formel (IId)
R¹-O-G-OH (IId)
reagiert werden
oder
[D] Verbindungen der allgemeinen Formel (VI)
R¹-A-D'-H (VI)
in welcher
R¹, A und D' die in Anspruch 8 angegebene Bedeutung haben,
mit Verbindungen der allgemeinen Formel (XIV)
R⁴⁶-E-G'-R⁴⁷ (XIV)
in welcher
R⁴⁶ die für R³⁶ angegebene Bedeutung hat und mit dieser gleich oder verschieden ist,
E die oben genannte Bedeutung hat,
G' für einen zweifach gebundenen 5- bis 7-gliedrigen aromatischen Heterocyclus mit bis zu 3 Heteroatomen aus der Reihe Schwefel, Stickstoff und/oder Sauerstoff steht, der gegebenenfalls mit einem oder mehreren, gleichen, oder verschiedenen Substituenten wie im Anspruch 1 für G definiert substituiert sein kann und
R⁴⁷ für Halogen, bevorzugt für Chlor oder Brom, steht,
zu einer Verbindung der allgemeinen Formel (XV)
R¹-A-D'-E-G'-R⁴⁷ (XV)
in welcher
R¹, A, D', E, G' und R⁴⁷ die oben genannte Bedeutung haben,
in inerten Lösungsmitteln, gegebenenfalls in Anwesenheit einer Base, umgesetzt werden und anschließend mit Kaliumamid in flüssigem Ammoniak in die entsprechenden freien Amine der allgemeinen Formel (IIe)
R¹-A-D'-E-G'-NH₂ (IIe)
in welcher
R¹, A, D', E und G' die oben genannte Bedeutung haben,
transformiert wird.

12. Verbindungen der Formeln
Cl -SO₂-CH₂-CH₂-CH₂-CF₃
oder
Cl -SO₂-CH₂-CH₂-CH₂-CF₂-CF₃,

13. Pharmazeutische Zusammensetzung, die als aktiven Bestandteil mindestens eine Verbindung gemäß irgendeinem der Ansprüche 1 bis 7 in Zusammenmischung mit mindestens einem pharmazeutisch verträglichen im wesentlichen nichtgiftigen Träger oder Exzipienten umfaßt.

14. Verbindungen nach irgendeinem der Ansprüche 5 bis 7 zur Verwendung als Medikament in der Behandlung von Menschen und Tieren.

15. Verwendung der Verbindungen gemäß irgendeinem der Ansprüche 1 bis 7 für die Herstellung eines Medikamentes zur Prävention und/oder Behandlung neurodegenerativer Erkrankungen.

16. Verwendung der Verbindungen gemäß irgendeinem der Ansprüche 1 bis 7 für die Herstellung eines Medikamentes zur Prävention und/oder Behandlung von cerebralen Ischämien und Schädel/Hirn-Trauma.

17. Verwendung der Verbindungen gemäß irgendeinem der Ansprüche 1 bis 7 für die Herstellung eines Medikaments zur Behandlung von Schmerzzuständen, Emesis, Übelkeit, Glaukom, Asthma, Anorexie, Konvulsionen, Rheuma, Sedation und Bewegungsstörungen.

## Claims

1. Compounds of the general formula (I)
R¹ - A - D - E - G - L - R² (I)
in which
R¹ represents naphthyl or a radical of the formula 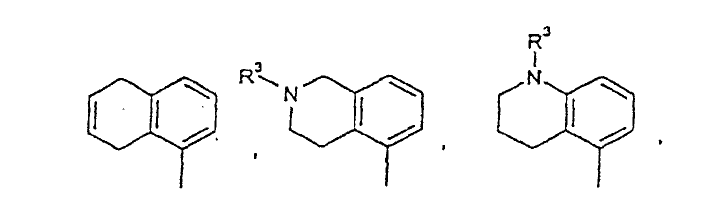 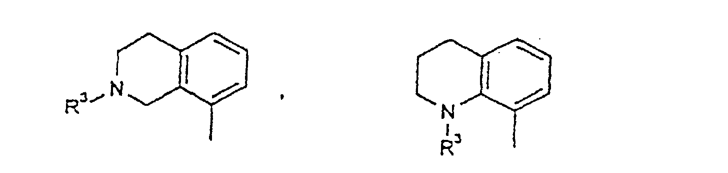 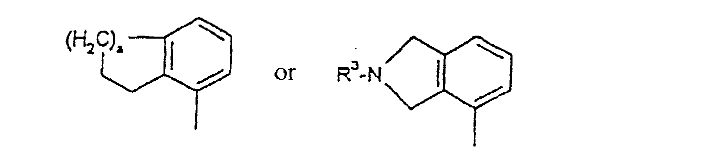
in which
a denotes a number 1 or 2,
R³ denotes hydrogen, (C₂-C₆)-alkenyl, (C₁-C₆)-alkyl or (C₁-C₆)-acyl,
and where all the abovementioned ring systems and radicals are optionally substituted, if appropriate geminally, by one or more, identical or different substituents which are selected from the group which consists of:
halogen, carboxyl, hydroxyl, phenyl, (C₁-C₆)-alkoxy, (C₁-C₆)-alkoxycarbonyl, C₁-C₈-alkyl, which, for its part, can be substituted by halogen, (C₁-C₆)-alkylsulphonyloxy, azide, amino, mono(C₁-C₆)-alkylamino, di(C₁-C₆)-alkylamino or hydroxyl,
a group of the formula -(CO)_{b}-NR⁴R⁵,
in which
b denotes a number 0 or 1,
R⁴ and R⁵ are identical or different and independently of one another denote hydrogen, phenyl, (C₁-C₆)-acyl, cyclo(C₄-C₇)-acyl, benzoyl or (C₁-C₆)-alkyl which is optionally substituted by amino, mono(C₁-C₆)-alkylamino, di(C₁-C₆)-alkylamino,
or
R⁴ and R⁵, together with the nitrogen atom, form a 5- or 6-membered saturated heterocycle which can optionally contain one or more further heteroatoms from the group consisting of S and O and/or one or more radicals of the formula -NR⁸,
in which
R⁸ denotes hydrogen, (C₁-C₆)-alkyl or (C₁-C₆)-acyl,
and
a group of the formula -NR⁶-SO₂-R⁷
in which
R⁶ denotes hydrogen, phenyl, (C₁-C₆)-alkyl or (C₁-C₆)-acyl,
R⁷ denotes phenyl or (C₁-C₆)-alkyl,
A and E are identical or different and represent a bond or (C₁-C₄)-alkylene,
D represents an oxygen atom or a radical of the formula -S(O)_{c}- or -N(R⁹)-,
in which
c denotes a number 0, 1 or 2,
R⁹ denotes hydrogen, (C₁-C₆)-alkyl or (C₁-C₆)-acyl,
G represents doubly bonded (C₆-C₁₀)-aryl or a doubly bonded 5- to 7-membered aromatic heterocycle having up to 3 heteroatoms from the group consisting of S, N and/or O, each of which is optionally substituted by one or more, identical or different substituents which are selected from the group which consists of:
hydroxyl, trifluoromethyl, carboxyl, halogen, (C₁-C₆)-alkyl, hydroxy(C₁-C₆)-alkyl, (C₁-C₆)-alkoxy, (C₁-C₆)-alkoxycarbonyl,
and also groups of the formulae
-CO-O-(CH₂)_{d}-NR¹⁰R¹¹, -NR¹²-SO₂R¹³,
-(CH₂)ₑ-(CO)_{f}-NR¹⁴R¹⁵ and -OR¹⁶,
in which
d denotes a number 1, 2, 3 or 4,
e and f are identical or different and denote a number 0 or 1,
R¹⁰ and R¹¹ have the meaning of R⁴ and R⁵ indicated above and are identical to or different from this,
R¹² has the meaning of R⁶ indicated above and is identical to or different from this,
R¹³ has the meaning of R⁷ indicated above and is identical to or different from this,
R¹⁴ and R¹⁵ have the meaning of R⁴ and R⁵ indicated above and are identical to or different from this,
or independently of one another represent a radical of the formula -(CH₂)_{g}-NR¹⁷R¹⁸,
in which
g denotes a number 1, 2, 3 or 4,
and
R¹⁷ and R¹⁸ have the meaning of R⁴ and R⁵ indicated above and are identical to or different from this,
R¹⁶ denotes (C₆-C₁₀)-aryl,
L represents a radical of the formula
-O-, -NH-, 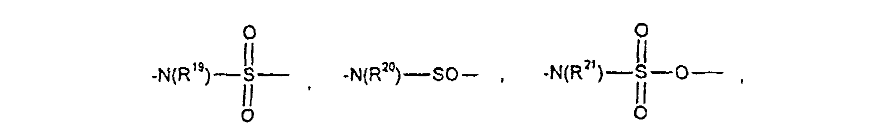 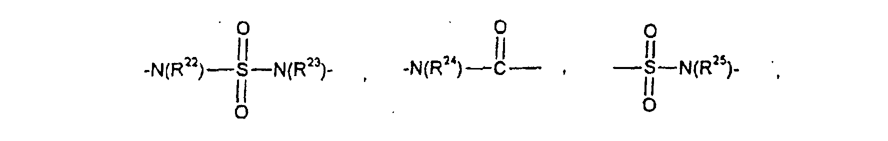 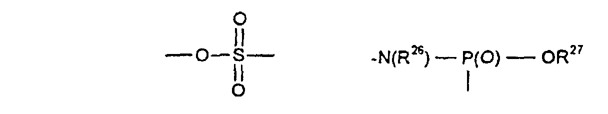
or 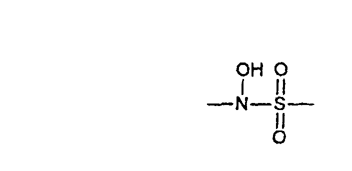 where the bonding of the radicals to G takes place at the left bond,
and in which R¹⁹, R²⁰, R²¹, R²², R²³, R²⁴, R²⁵, R²⁶ and R²⁷ are identical or different and denote hydrogen or (C₁-C₄)-alkyl, or
R¹⁹ denotes a radical of the formula -SO₂R²,
R² represents (C₆-C₁₀)-aryl or a 5- to 7-membered saturated or aromatic heterocycle having up to 3 heteroatoms from the group consisting of S, N and/or O, each of which is optionally substituted by one or more, identical or different substituents which are selected from the group which consists of:
halogen, trifluoromethyl, nitro, amino and (C₁-C₆)-alkyl,
or
represents the radical of the formula 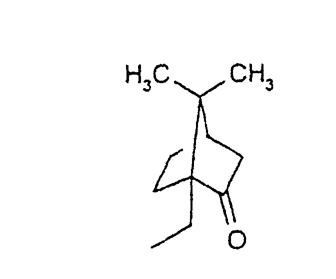 or morpholine, or
represents C₃-C₈-cycloalkyl, or
represents (C₁-C₁₂)-alkyl, (C₂-C₁₂)-alkenyl or (C₂-C₁₂)-alkinyl, each of which is optionally substituted by one or more, identical or different substituents which are selected from the group which consists of:
halogen, trifluoromethyl, hydroxyl, cyano, azido, (C₁-C₆)-alkoxy, (C₁-C₆)-perfluoroalkoxy, partially fluorinated (C₁-C₆)-alkoxy, a radical of the formula 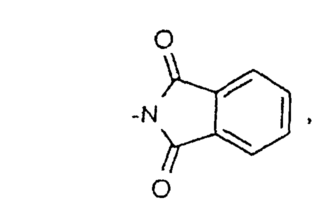
-NR²⁸R²⁹,
in which R²⁸ and R²⁹ have the meaning of R⁴ and R⁵ indicated above and are identical to or different from this,
phenyl, optionally substituted by one or more, identical or different substituents which are selected from the group which consists of:
halogen, nitro, hydroxyl, (C₁-C₆)-alkyl, (C₁-C₆)-alkoxy and a group of the formula -NR³⁰R³¹,
in which R³⁰ and R³¹ are identical or different and denote hydrogen or (C₁-C₆)-alkyl or (C₁-C₆)-acyl,
and a 5- to 6-membered aromatic heterocycle having up to [lacuna] heteroatoms from the group consisting of S, N and/or O, optionally substituted by one or more, identical or different substituents which are selected from the group which consists of:
halogen, nitro, hydroxyl, (C₁-C₆)-alkyl, (C₁-C₆)-alkoxy and a group of the formula -NR³⁰R³¹,
in which R³⁰ and R³¹ are as defined above,
or
L and R² together represent a radical of the formula 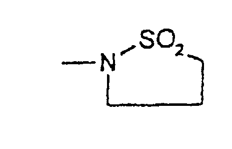
and their salts,
for use as a medicament in the treatment of humans and animals.

2. Compounds of the formula (I) according to Claim 1, in which
R¹ represents naphthyl or a radical of the formula 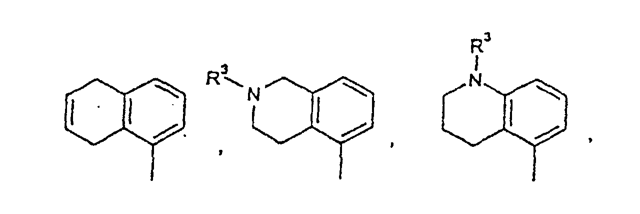 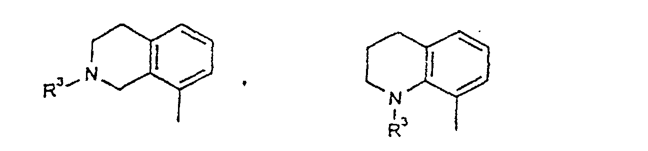 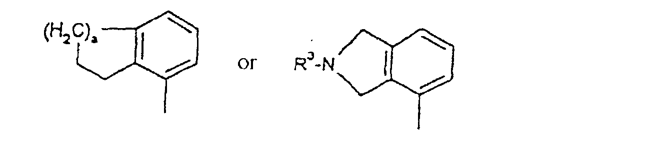 in which
a denotes a number 1 or 2,
R³ denotes hydrogen, (C₂-C₄)-alkenyl, (C₁-C₄)-alkyl or (C₁-C₄)-acyl,
and where all the abovementioned ring systems and radicals are optionally substituted, if appropriate geminally, by one or more, identical or different substituents which are selected from the group which consists of:
halogen, carboxyl, hydroxyl, phenyl, (C₁-C₄)-alkoxy, (C₁-C₅)-alkoxycarbonyl, (C₁-C₆)-alkyl which, for its part, can be substituted by halogen, (C₁-C₄)-alkylsulphonyloxy, azide, amino, mono(C₁-C₄)-alkylamino, di(C₁-C₄)-alkylamino or hydroxyl,
a group of the formula -(CO)_{b}-NR⁴R⁵
in which
b denotes a number 0 or 1,
R⁴ and R⁵ are identical or different and independently of one another denote hydrogen, phenyl, (C₁-C₄)-acyl, cyclo(C₄-C₇)-acyl, benzoyl or (C₁-C₄)-alkyl which is optionally substituted by amino, mono(C₁-C₄)-alkylamino, di(C₁-C₄)-alkyl,
or
R⁴ and R⁵, together with the nitrogen atom, form a morpholine, piperidine or N-methylpiperazine ring,
and
a group of the formula -NR⁶-SO₂-R⁷
in which
R⁶ denotes hydrogen, phenyl, (C₁-C₄)-alkyl or (C₁-C₄)-acyl
and
R⁷ denotes phenyl or (C₁-C₅)-alkyl,
A and E are identical or different and represent a bond or (C₁-C₄)-alkylene,
D represents an oxygen atom or a radical of the formula -S(O)_{c}- or -NR⁹-,
in which
c denotes a number 0, 1 or 2,
R⁹ denotes hydrogen or (C₁-C₄)-alkyl or (C₁-C₄)-acyl,
G represents doubly bonded phenyl, naphthyl, pyrimidyl, pyridazinyl or pyridyl, each of which is optionally substituted by one or more, identical or different substituents which are selected from the group which consists of:
hydroxyl, trifluoromethyl, carboxyl, halogen, (C₁-C₄)-alkyl, hydroxy(C₁-C₄)alkyl, (C₁-C₄)-alkoxy, (C₁-C₄)-alkoxycarbonyl, and also groups of the formulae
-CO-O-(CH₂)_{d}-NR¹⁰R¹¹, -NR¹²-SO₂R¹³,
-(CH₂)ₑ-(CO)_{f}-NR¹⁴R¹⁵ and -OR¹⁶,
in which
d denotes a number 1, 2, 3 or 4,
e and f are identical or different and denote a number 0 or 1,
R¹⁰ and R¹¹ have the meaning of R⁴ and R⁵ indicated above and are identical to or different from this,
R¹² has the meaning of R⁶ indicated above and is identical to or different from this,
R¹³ has the meaning of R⁷ indicated above and is identical to or different from this,
R¹⁴ and R¹⁵ have the meaning of R⁴ and R⁵ indicated above and are identical to or different from this, or independently of one another represent a radical of the formula
-(CH₂)_{g}-NR¹⁷R¹⁸,
in which
g denotes a number 1, 2 or 3,
and
R¹⁷ and R¹⁸ have the meaning of R¹⁰ and R¹¹ indicated above and are identical to or different from this,
R¹⁶ denotes phenyl or naphthyl,
L represents a radical of the formula
-O-, -NH-, 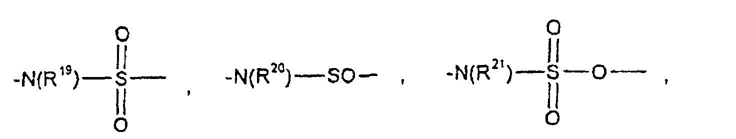 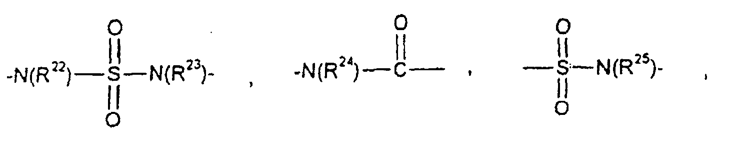 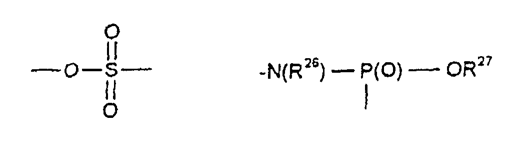
or 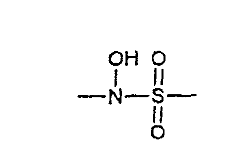 where the bonding of the radicals to G takes place at the left bond,
and
in which
R¹⁹, R²⁰, R²¹, R²², R²³, R²⁴, R²⁵, R²⁶ and R²⁷ are identical or different and denote hydrogen or (C₁-C₃)-alkyl,
or
R¹⁹ denotes a radical of the formula -SO₂R²,
R² represents phenyl, naphthyl, pyridyl, fury], thienyl or pyrimidyl, each of which is optionally substituted by one or more, identical or different substituents which are selected from the group which consists of:
halogen, amino, trifluoromethyl, nitro and (C₁-C₄)-alkyl,
or represents the radical of the formula 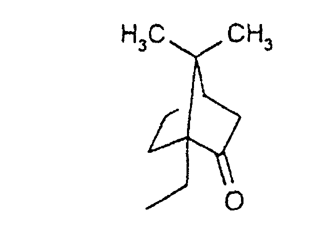 or morpholine,
or
represents cyclopropyl, cyclohexyl or cyclopentyl, or
represents (C₁-C₁₀)-alkyl, (C₂-C₁₀)-alkenyl or (C₂-C₁₀)-alkinyl, each of which is optionally substituted by one or more, identical or different substituents which are selected from the group which consists of:
halogen, trifluoromethyl, hydroxyl, azido, (C₁-C₄)-alkoxy, (C₁-C₅)-perfluoroalkoxy, partially fluorinated (C₁-C₄)-alkoxy, a radical of the formula 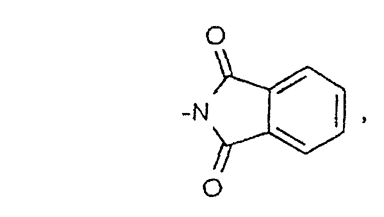 and -NR²⁸R²⁹,
in which
R²⁸ and R²⁹ have the meaning of R⁴ and R⁵ indicated above and are identical to or different from this,
phenyl, optionally substituted by one or more, identical or different substituents which are selected from the group which consists of:
halogen, nitro, hydroxyl, (C₁-C₄)-alkyl, (C₁-C₄)-alkoxy and a group of the formula -NR³⁰R³¹,
in which R³⁰ and R³¹ are identical or different and denote hydrogen or (C₁-C₄)-alkyl or (C₁-C₄)-acyl,
pyridyl and pyrimidyl, optionally substituted by one or more, identical or different substituents which are selected from the group which consists of:
halogen, nitro, hydroxyl, (C₁-C₄)-alkyl, (C₁-C₄)-alkoxy and a group of the formula -NR³⁰R³¹,
in which R³⁰ and R³¹ are as defined above,
or
L and R² together represent a radical of the formula 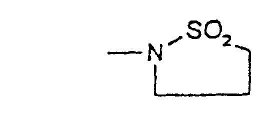
and their salts,
for use as a medicament in the treatment of humans and animals.

3. Compounds of the formula (I) according to Claim 1, in which
R¹ represents naphthyl or a radical of the formula 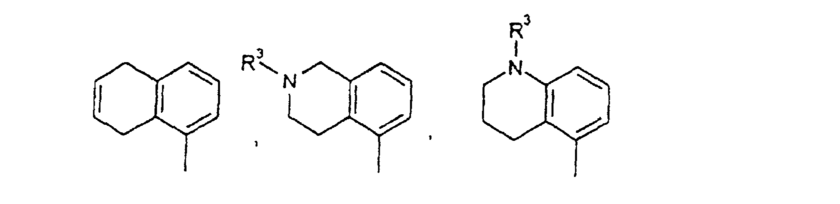 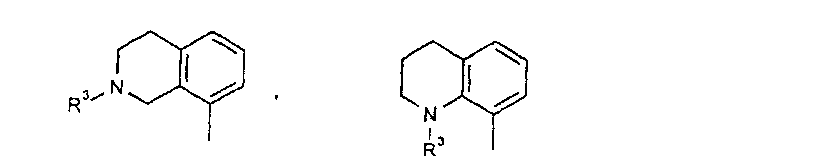 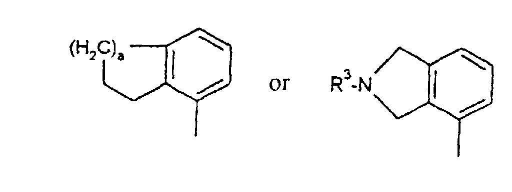 in which
a denotes a number 1 or 2,
R³ denotes hydrogen, (C₂-C₃)-alkenyl, (C₁-C₃)-alkyl or (C₁-C₃)-acyl,
and where all the abovementioned ring systems are optionally substituted, if appropriate geminally, by one or more, identical or different substituents which are selected from the group which consists of:
chlorine, fluorine, carboxyl, hydroxyl, phenyl, (C₁-C₃)-alkoxy, (C₁-C₄)-alkoxycarbonyl, (C₁-C₄)-alkyl which, for its part, can be substituted by chlorine or hydroxyl,
a group of the formula -(CO)_{b}-NR⁴R⁵
in which
b denotes a number 0 or 1,
R⁴ and R⁵ are identical or different and independently of one another denote hydrogen, (C₁-C₃)-acyl, cyclo(C₄-C₆)-acyl, benzoyl or (C₁-C₃)-alkyl which is optionally substituted by amino, mono(C₁-C₃)-alkylamino, di(C₁-C₃)-alkylamino,
or
R⁴ and R⁵, together with the nitrogen atom, form a morpholine, piperidine or N-methylpiperazine ring,
and
a group of the formula -NR⁶-SO₂-R⁷
in which
R⁶ denotes hydrogen, (C₁-C₃)-alkyl or (C₁-C₃)-acyl
and
R⁷ denotes phenyl or (C₁-C₄)-alkyl,
A and E are identical or different and represent a bond or (C₁-C₃)-alkyl,
D represents an oxygen atom or a radical of the formula -S(O)_{c}- or -NR⁹-,
in which
c denotes a number 0, 1 or 2,
R⁹ denotes hydrogen or (C₁-C₃)-alkyl or (C₁-C₃)-acyl,
G represents doubly bonded phenyl, naphthyl, pyrimidyl, pyridazinyl or pyridyl, each of which is optionally substituted by one or more, identical or different substituents which are selected from the group which consists of:
hydroxyl, trifluoromethyl, carboxyl, fluorine, chlorine, bromine, (C₁-C₃)-alkyl, hydroxy(C₁-C₃)alkyl, (C₁-C₃)-alkoxy, (C₁-C₃)-alkoxycarbonyl, and also groups of the formulae
-CO-O-(CH₂)_{d}-NR¹⁰R¹¹, -NR¹²-SO₂R¹³,
-(CH₂)ₑ-(CO)_{f}-NR¹⁴R¹⁵, -CH₂OH and -OR¹⁶,
in which
d denotes a number 1, 2 or 3,
e and f are identical or different and denote a number 0 or 1,
R¹⁰ and R¹¹ denote hydrogen or methyl,
R¹² denotes hydrogen,
R¹³ denotes (C₁-C₄)-alkyl,
R¹⁴ and R¹⁵ have the meaning of R⁴ and R⁵ indicated above and are identical to or different from this, or denote a radical of the formula -(CH₂)_{g}-NR¹⁷R¹⁸,
in which
g denotes a number 1, 2 or 3,
and
R¹⁷ and R¹⁸ denote hydrogen or methyl,
or
R¹⁴ and R¹⁵, together with the nitrogen atom, form a radical of the formula 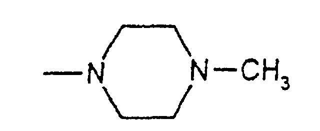
R¹⁶ denotes phenyl or naphthyl,
L represents a radical of the formula
-O-, -NH-, 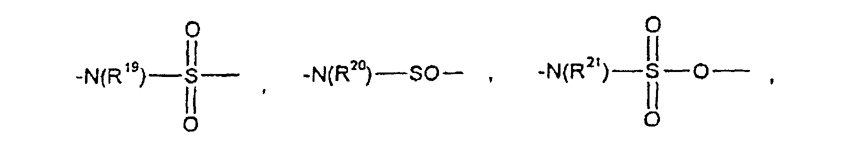 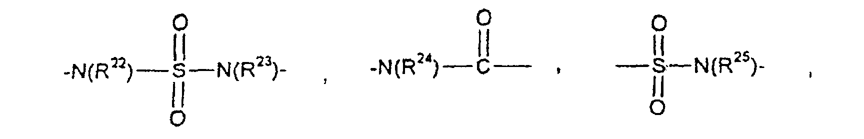 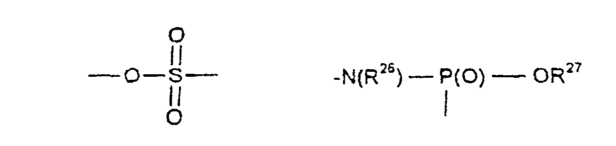
or 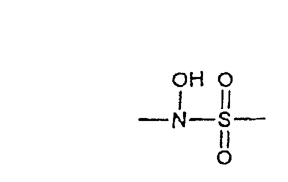 where the bonding of the radicals to G takes place at the left bond,
and
in which
R¹⁹, R²⁰, R²¹, R²², R²³, R²⁴, R²⁵, R²⁶ and R²⁷ are identical or different and denote hydrogen, methyl or ethyl,
or
R¹⁹ denotes a radical of the formula -SO₂R²,
R² represents phenyl, furyl or pyridyl, each of which is optionally substituted by one or more, identical or different substituents which are selected from the group which consists of: fluorine, chlorine, bromine or trifluoromethyl,
or
represents the radical of the formula 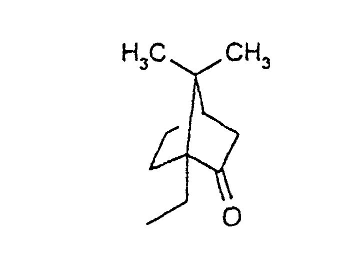 or morpholine,
or
represents cyclopentyl or cyclohexyl, or
represents (C₁-C₈)-alkyl, (C₂-C₈)-alkenyl or (C₂-C₈)-alkinyl, each of which is optionally substituted by one or more, identical or different substituents which are selected from the group which consists of:
fluorine, chlorine, bromine, trifluoromethyl, hydroxyl, azido, (C₁-C₃)-alkoxy, (C₁-C₄)-perfluoroalkoxy, trifluoromethyl-substituted (C₁-C₄)-alkoxy, a radical of the formula 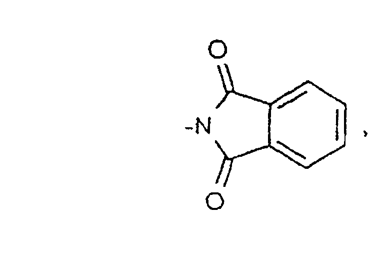 and -NR²⁸R²⁹,
in which
R²⁸ and R²⁹ denote hydrogen or methyl,
phenyl, pyridyl and pyrimidyl, optionally substituted by one or more, identical or different substituents which are selected from the group which consists of:
fluorine, chlorine, bromine, nitro, hydroxyl, (C₁-C₃)-alkyl, (C₁-C₃)-alkoxy and a group of the formula -NR³⁰R³¹,
in which R³⁰ and R³¹ are identical or different and denote hydrogen, methyl or methylcarbonyl,
or
L and R² together represent a radical of the formula 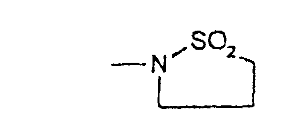 and their salts,
for use as a medicament in the treatment of humans and animals.

4. Compounds of the general formula (I)
R¹-A-D-E-G-L-R² (I)
in which
R¹, A, D, E, G, L and R² are each as defined in any of Claims 1 to 3, with the exception of compounds of the general formula (I)
in which
R¹ represents 1-naphthyl whose 3-position is optionally substituted by chlorine or (C₁-C₄)-alkyl and the 4-position is optionally substituted by chlorine or phenyl,
A and E each represent a bond,
D represents an oxygen atom,
G represents 1,4-phenylene which is optionally substituted by (C₁-C₄)-alkyl,
L represents an oxygen atom, and
R² represents methyl,
and with the exception of m-bis-(1-naphthyloxy)benzene.

5. Compounds of the formula (I) according to Claim 1,
in which
R¹ represents naphthyl or a radical of the formula 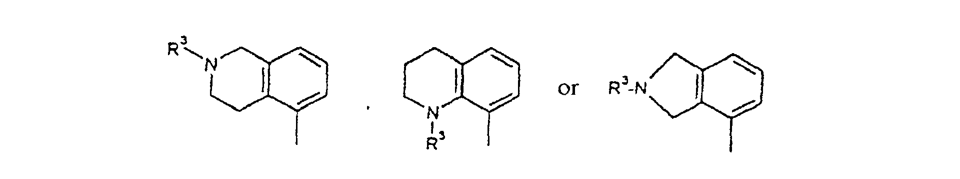 in which
a denotes a number 1 or 2,
and where all the abovementioned ring systems and radicals are optionally substituted, if appropriate geminally, by 1 to 3 identical or different substituents which are selected from the group which consists of:
halogen, carboxyl, hydroxyl, (C₁-C₆)-alkoxy, (C₁-C₆)-alkoxycarbonyl, (C₁-C₈)-alkyl which, for its part, can be substituted by halogen or hydroxyl,
a group of the formula -(CO)_{b}-NR⁴R⁵
in which
b denotes a number 0 or 1,
R⁴ and R⁵ are identical or different and denote hydrogen, phenyl or (C₁-C₆)-alkyl,
and
a group of the formula -NR⁶-SO₂-R⁷
in which
R⁶ denotes hydrogen, phenyl or (C₁-C₆)-alkyl,
R⁷ denotes phenyl or (C₁-C₆)-alkyl,
A and E are identical or different and represent a bond or (C₁-C₄)-alkylene,
D represents an oxygen atom or a radical of the formula -S(O)_{c}- or -NH-,
in which
c denotes a number 0, 1 or 2,
G represents doubly bonded (C₆-C₁₀)-aryl or a double bonded 5- to 7-membered aromatic heterocycle having up to 3 heteroatoms from the group consisting of S, N and/or O, each of which is optionally substituted by up to three identical or different substituents which are selected from the group which consists of:
hydroxyl, carboxyl, halogen, (C₁-C₆)-alkyl, hydroxy(C₁-C₆)alkyl, (C₁-C₆)-alkoxy, (C₁-C₆)-alkoxycarbonyl, and also groups of the formulae
-CO-O-(CH₂)_{d}-NR¹⁰R¹¹, -NR¹²-SO₂R¹³ and -CO-NR¹⁴R¹⁵
in which
d denotes a number 1, 2, 3 or 4,
R¹⁰ and R¹¹ have the meaning of R⁴ and R⁵ indicated above and are identical to or different from this,
R¹² has the meaning of R⁶ indicated above and is identical to or different from this,
R¹³ has the meaning of R⁷ indicated above and is identical to or different from this,
R¹⁴ and R¹⁵ have the meaning of R⁴ and R⁵ indicated above and are identical to or different from this, or together with the nitrogen atom form a 5- to 6-membered saturated heterocycle which can optionally additionally contain a further heteroatom from the group consisting of S and O or a group of the formula -NH-,
L represents a radical of the formula 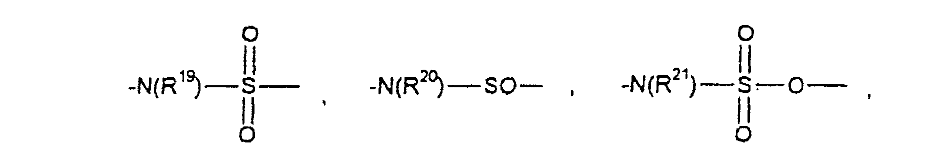 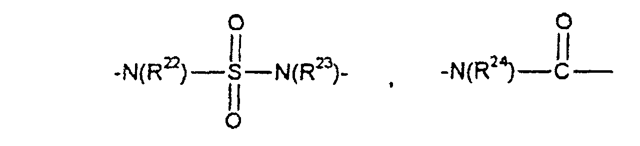 or 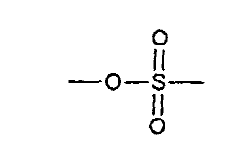 where the bonding of the radicals to G takes place at the left bond,
and in which R¹⁹, R²⁰, R²¹, R²², R²³ and R²⁴ are identical or different and denote hydrogen or (C₁-C₄)-alkyl,
R² represents phenyl which is optionally substituted by halogen, trifluoromethyl, nitro, amino or (C₁-C₆)-alkyl,
R² represents the radical of the formula 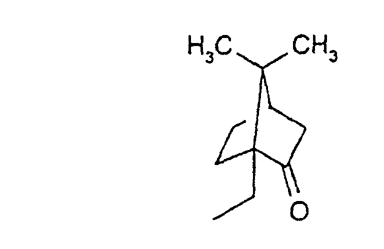 or morpholine,
or
represents perfluoroalkyl having up to 12 fluorine atoms, or
represents (C₁-C₁₂)-alkyl or (C₂-C₁₂)-alkinyl, each of which is optionally substituted by halogen, trifluoromethyl, hydroxyl, azido or by a radical of the formula 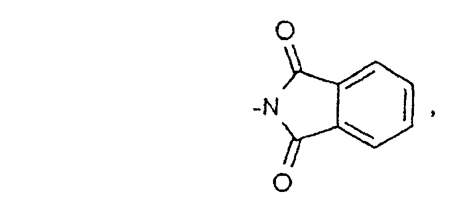 or -NR²⁸R²⁹,
in which R²⁸ and R²⁹ have the meaning of R⁴ and R⁵ indicated above and are identical to or different from this,
and/or are optionally substituted by phenyl or a 5- to 6-membered aromatic heterocycle having up to 3 heteroatoms from the group consisting of S, N and/or O, which, for their part, can be substituted up to 2 times identically or differently by halogen, nitro, hydroxyl, (C₁-C₆)-alkyl, (C₁-C₆)-alkoxy or by a group of the formula -NR³⁰R³¹,
in which R³⁰ and R³¹ are identical or different and denote hydrogen, (C₁-C₆)-alkyl or (C₁-C₆)-acyl,
L and R² together represent a radical of the formula 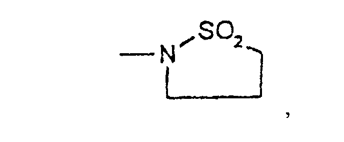 and their salts.

6. Compounds of the formula (I) according to Claim 1, in which
R¹ represents naphth-1-yl, optionally substituted by (C₁-C₆)-alkyl substituted by hydroxyl, (C₁-C₆)-acylamino, amino or (C₁-C₆)-alkoxy, indan-4-yl, substituted by hydroxy(C₁-C₆)-alkyl,
a radical of the formula 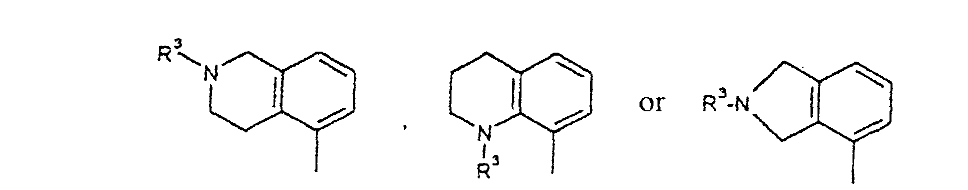 in which
R³ is (C₁-C₆)-alkyl,
E and A represent a bond,
D represents an oxygen atom,
G represents 1,3-phenylene, 1,4-phenylene or 2,5-pyridylene, each of which is optionally substituted by halogen,
L represents a radical of the formula -NH-SO₂- or -O-SO₂- and
R² represents (C₁-C₆)-alkyl which is optionally substituted by chlorine, trifluoromethyl, by a radical of the formula -O-CH₂-CF₃ or by phenyl or by pyridyl, which for their part can be substituted by bromine or chlorine,
and their salts.

7. Compounds of the following formulae:

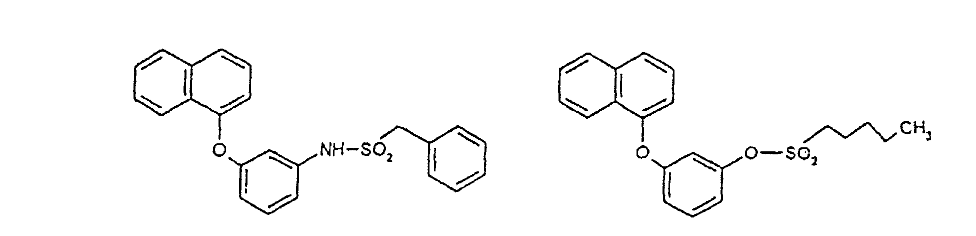

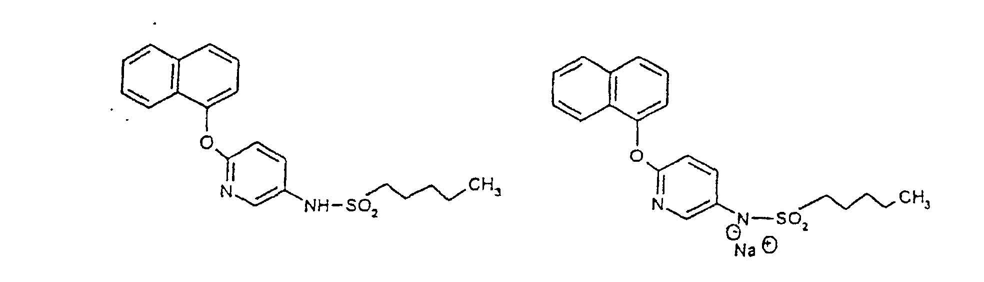

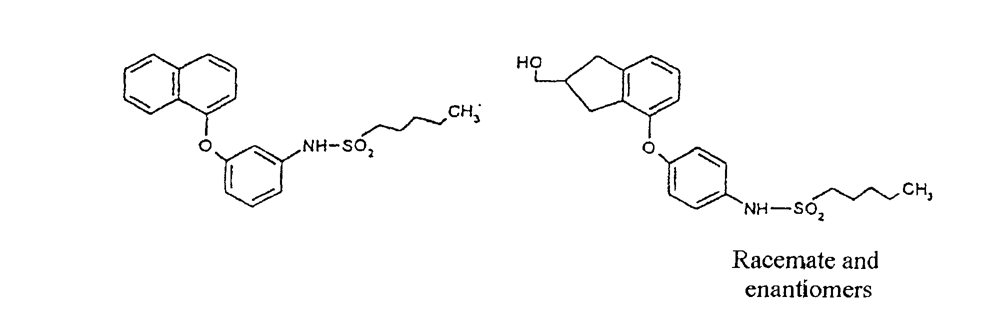

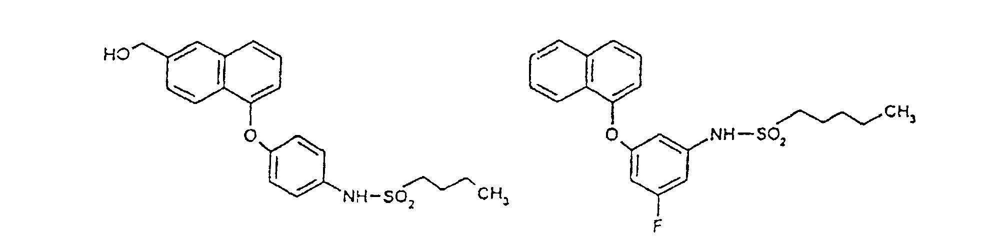

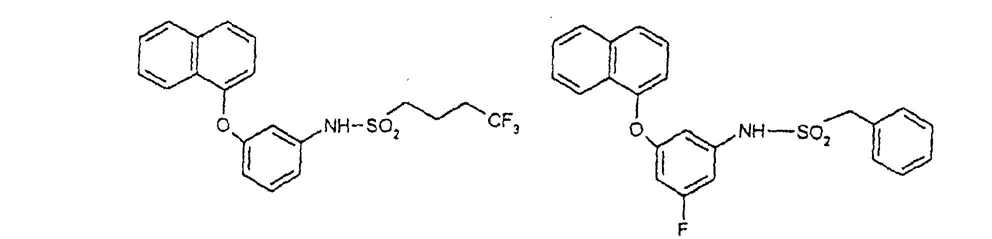

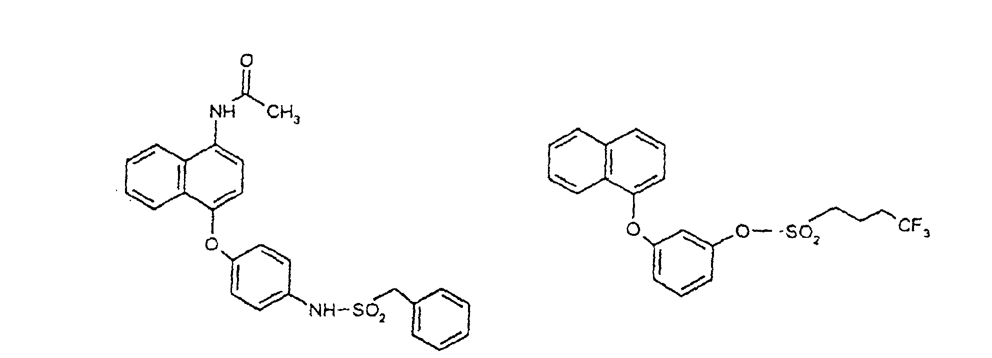

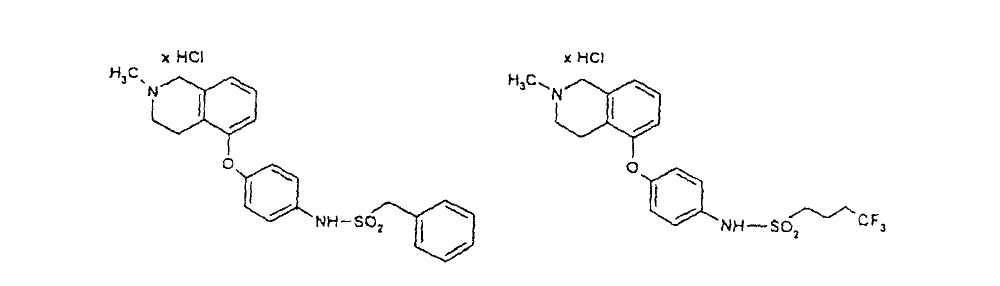

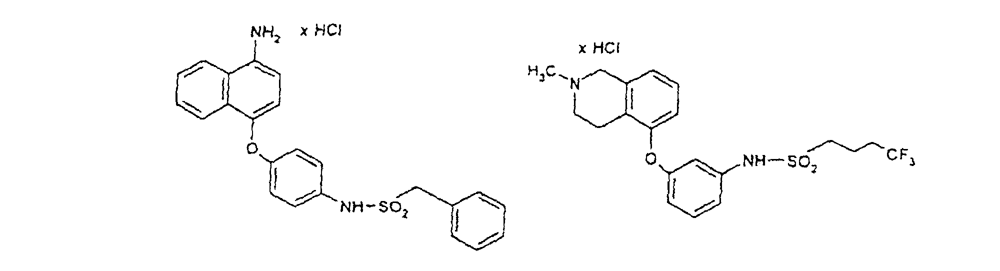

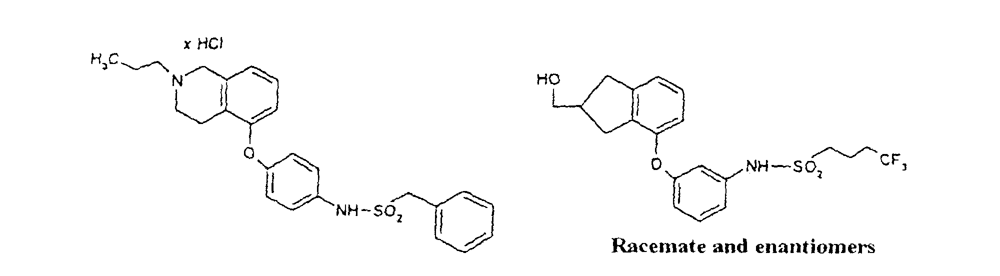

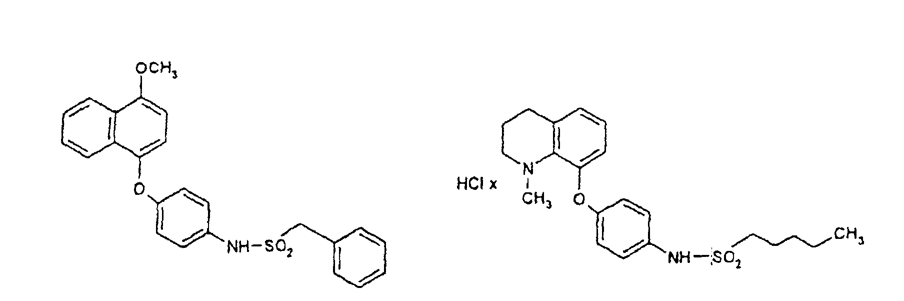

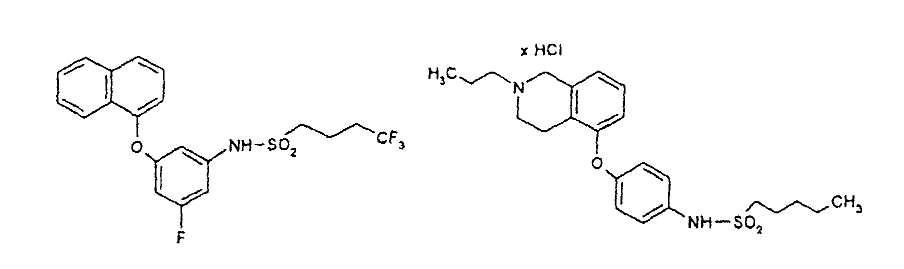

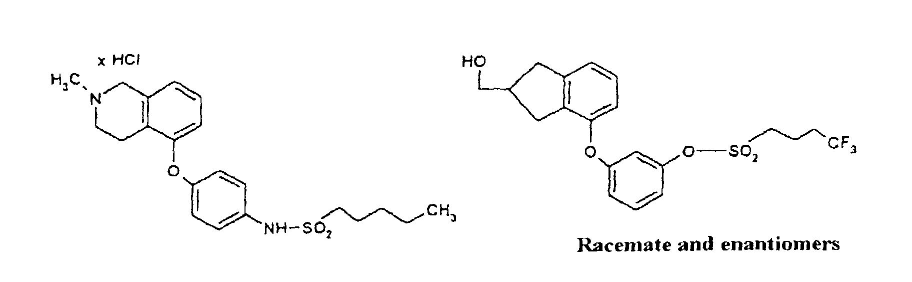

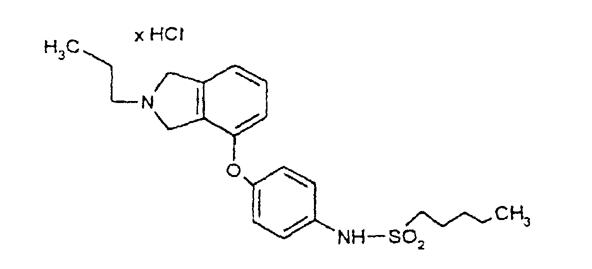

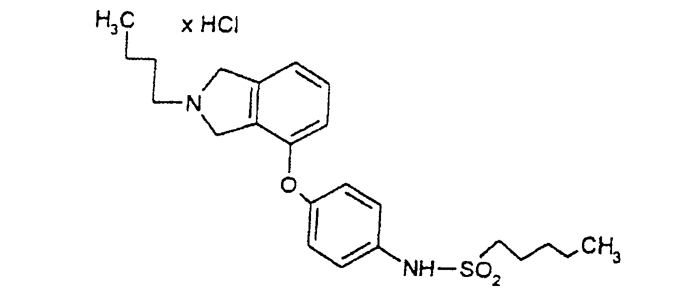

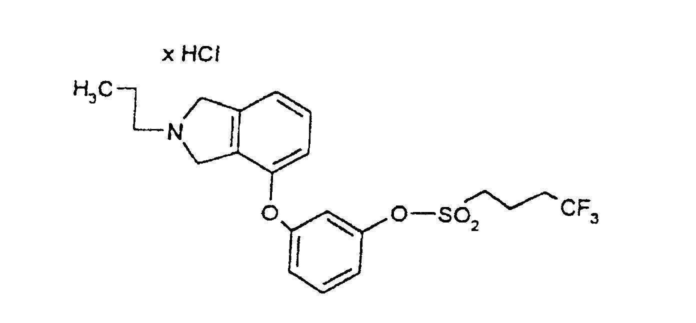

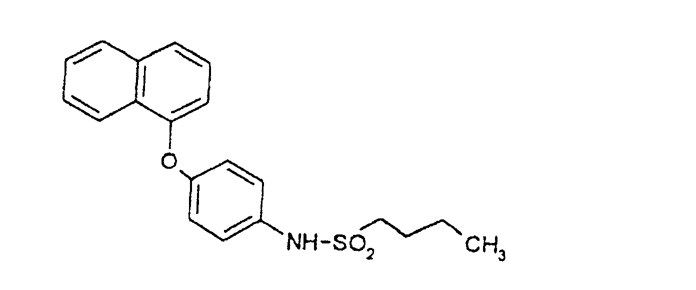

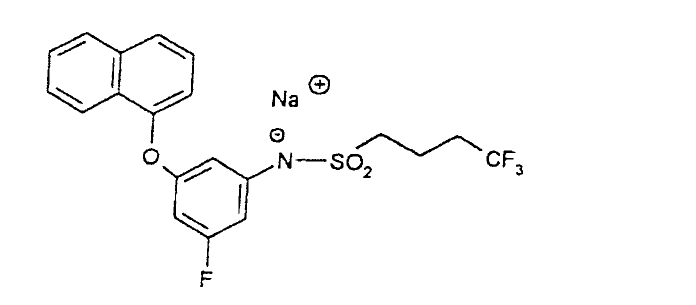

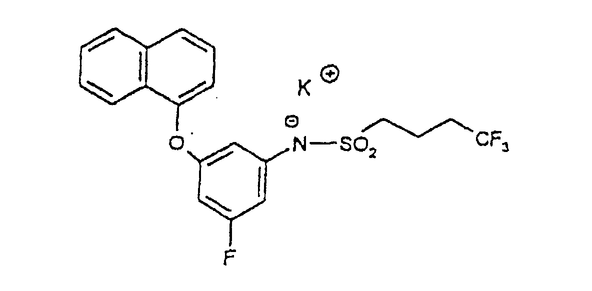

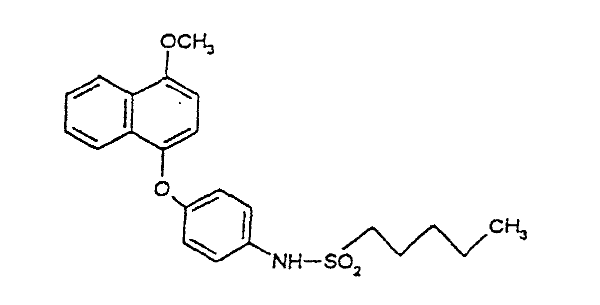

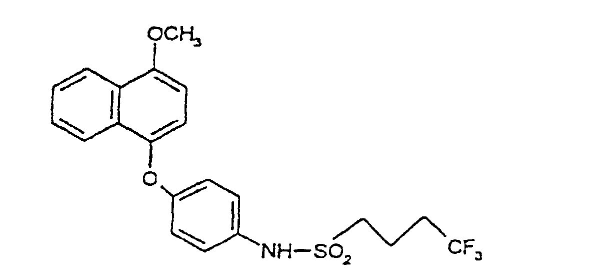

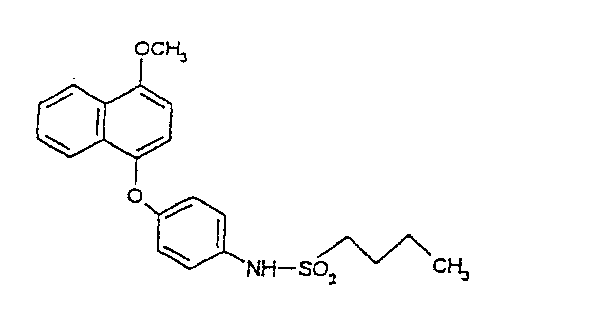

8. Process for the preparation of the compounds according to Claim 4,
in which
[A] compounds of the general formula (II)
R¹-A-D-E-G-M-H (II)
in which
R¹, A, D, E and G have the meaning indicated in Claim 1 and
M represents oxygen or -N(R³²)- and
R³² is hydrogen or (C₁-C₄)-alkyl,
are reacted with compounds of the general formula (III)
R³³-Q-R² (III)
in which
R² has the meaning indicated in Claim 1,
R³³ represents halogen, preferably chlorine or iodine,
Q represents a radical of the formula -SO₂-, -SO-, -CO-, -P(O)(OR²⁷)- or a single bond,
in which
R²⁷ has the meaning indicated in Claim 1,
to give compounds of the general formula (Ia)
R¹-A-D-E-G-M-Q-R² (Ia)
in which
R¹, A, D, E, G, M, Q and R² have the meaning indicated above,
in inert solvents, if appropriate in the presence of a base,
or
[B] compounds of the general formula (II)
are reacted first with trialkylsilyl chlorosulphonates, preferably trimethylsilyl chlorosulphonate, treated with an acid and then reacted with a chlorinating agent, preferably phosphorus pentachloride, to give a compound of the general formula (IV)
R¹-A-D-E-G-M-SO₂-Cl (IV)
in which
R¹, A, D, E, G, and M have the meaning indicated above,
and then reacted with compounds of the general formula (V)
H-T-R² (V)
in which
R² has the meaning indicated in Claim 1 and
T represents oxygen or nitrogen,
to give compounds of the general formula (Ib)
R¹-A-D-E-G-M-SO₂-T-R² (Ib)
in which
R¹, A, D, E, G, M, T and R² have the meaning indicated above,
in inert solvents, if appropriate in the presence of Bzl-NEt₃+Cl⁻ and a base,
or
[C] compounds of the general formula (VI)
R¹-A-D'-H (VI)
in which
R¹ and A have the meaning indicated above and
D' represents oxygen, sulphur or -N(R⁹)- and
R⁹ has the meaning indicated in Claim 1,
are reacted with compounds of the general formula (VII)
R³⁴-E-G-SO₂-NH-R² (VII)
in which
E, G and R² have the meaning indicated above and
R³⁴ represents a leaving group, preferably halogen, particularly preferably fluorine, chlorine or bromine,
to give compounds of the general formula (Ic)
R¹-A-D'-E-G-SO₂-NH-R² (Ic)
in which
R¹, A, D', E, G and R² have the meaning indicated above,
or
[D] compounds of the general formula (Id)
R³⁷-A-D-E-G-L-R² (Id)
in which
A, D, E, G, L and R² have the meaning indicated above and
R³⁷ represents a radical of the formula 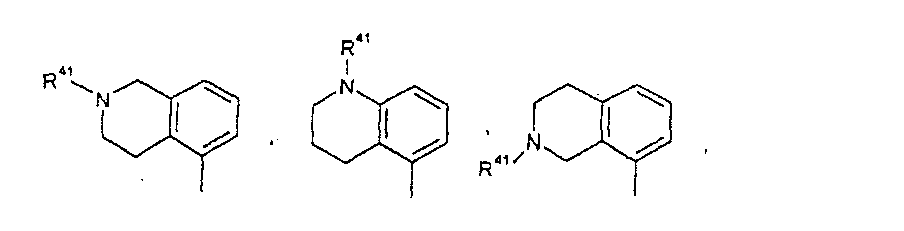 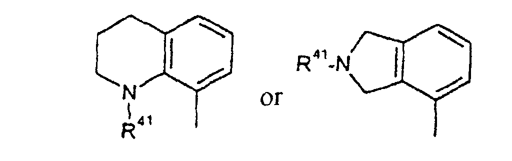
in which
R⁴¹ represents (C₁-C₆)-alkyl,
are reacted with a chloroformic acid ester, preferably 1-(1-chloro)ethyl chloroformate or methyl chloroformate, and then with alcohols, preferably methanol, if appropriate in the presence of a base, to give compounds of the general formula (Ie)
R³⁸-A-D-E-G-L-R² (Ie)
in which
A, D, E, G, L and R² have the meaning indicated above and
R³⁸ represents a radical of the formula 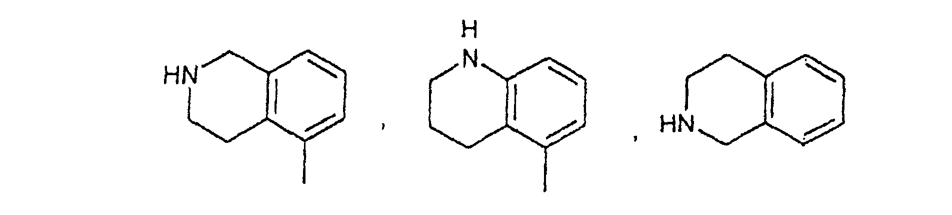 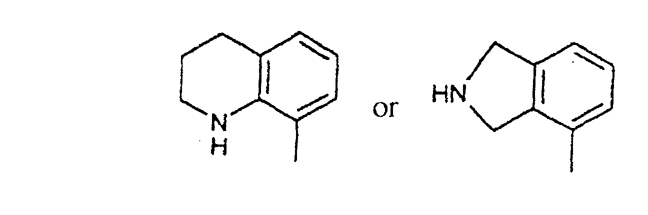
or
[E] compounds of the general formula (Ie)
are reacted with (C₁-C₆)-ketones or (C₁-C₆)-aldehydes in the presence of a reducing agent, preferably sodium cyanoborohydride, if appropriate in the presence of an acid, to give compounds of the general formula (If)
R³⁹-A-D-E-G-L-R² (If)
in which
A, D, E, G, L and R² have the meaning indicated above and
R³⁹ represents (C₃-C₆)-alkenyl or (C₁-C₆)-alkyl,
or
[F] compounds of the general formula (Ie) are reacted with compounds of the general formula (VIII)
R³⁵-R³ (VIII)
in which
R³ has the meaning mentioned in Claim 1,
R³⁵ represents a leaving group, preferably halogen,
in inert solvents, if appropriate in the presence of a base, to give compounds of the general formula (Ig)
R⁴⁰-A-D-E-G-L-R² (Ig)
in which
A, D, E, G, L and R² have the meaning indicated above and
R⁴⁰ represents a radical of the formula 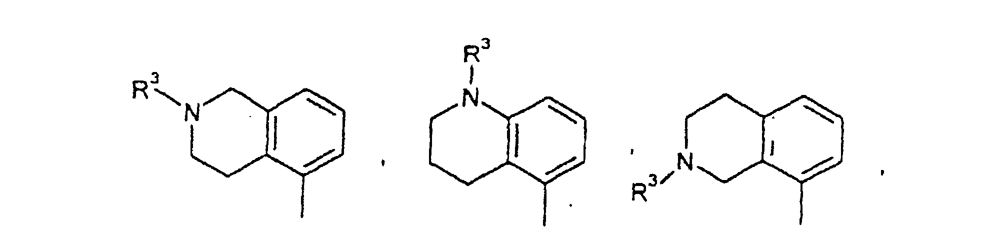 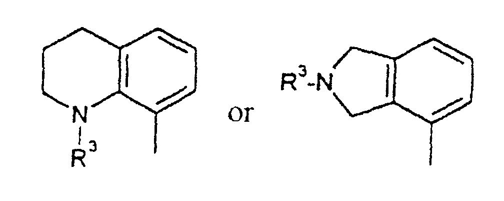
in which
R³ has the meaning indicated above,
or
[G] compounds of the general formula (Ih) 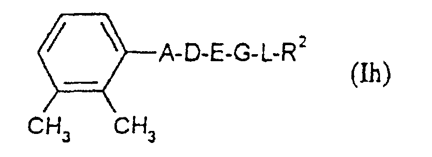 in which
A, D, E, G, L and R² have the meaning indicated above,
are converted by means of free-radical bromination, for example with N-bromosuccinimide, in an inert solvent into compounds of the general formula (Ii) 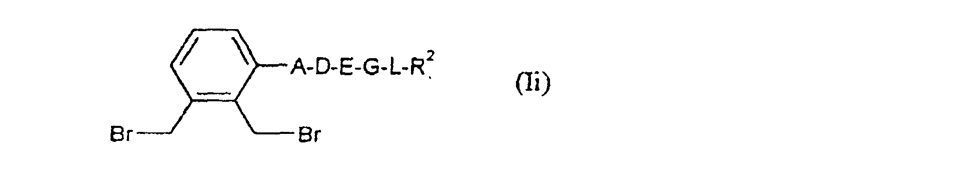 in which
A, D, E, G, L and R² have the meaning indicated above,
and then reacted with compounds of the general formula (IX) or (X)
CH₂(CO₂R⁴²)₂ (IX)
H₂N-R³ (X)
in which
R⁴² represents (C₁-C₆)-alkyl and
R³ has the meaning indicated above,
in inert solvents, if appropriate in the presence of a base, to give compounds of the general formula (Ij)
R⁴³-A-D-E-G-L-R² (Ij)
in which
A, D, E, G, L and R² have the abovementioned meaning and
R⁴³ represents 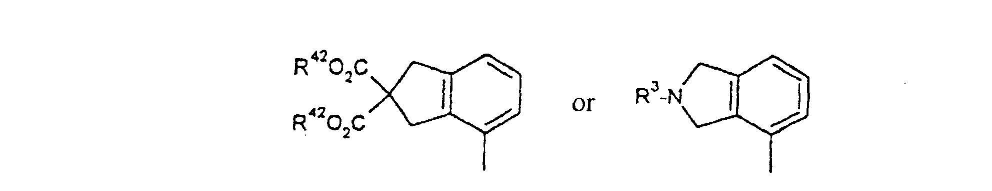
R⁴² and R³ have the abovementioned meaning,
and, if appropriate, the abovementioned substituents are introduced and derivatized according to customary methods,
and if D = -SO- or -SO₂-, starting from the corresponding thioethers (D = S), an oxidation is carried out according to customary methods,
and in the case of the ammonium compounds, starting from the corresponding amines, an alkylation is carried out.

9. Compounds of the general formula (II)
R¹-A-D-E-G-M-H (II)
in which
R¹ represents a radical of the formula 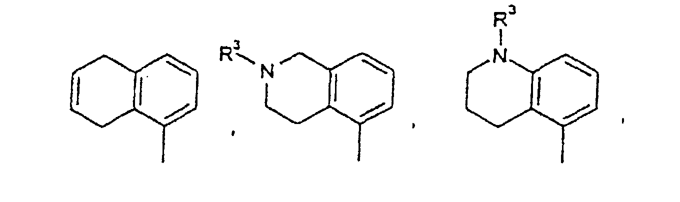 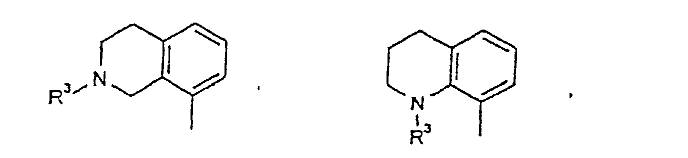 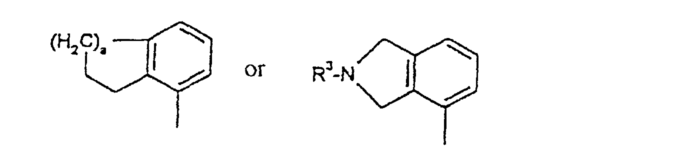
in which
a denotes a number 1,
R³ represents hydrogen, (C₂-C₆)-alkenyl, (C₁-C₆)-alkyl or (C₁-C₆)-acyl,
and where all of the abovementioned ring systems and radicals are optionally substituted as indicated in Claim 1, and
A, D, E, G and M have the meanings indicated in Claims 1 and 8.

10. Compounds of the general formula (II) according to Claim 9, in which
R¹ represents indan-4-yl substituted by hydroxy(C₁-C₆)-alkyl or represents a radical of the formula 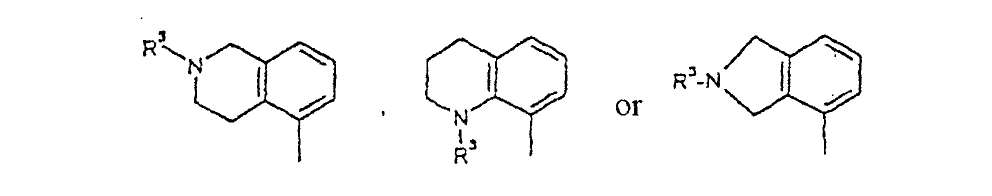 in which
R³ is (C₁-C₆)-alkyl,
A, D, E, and G have the meanings mentioned in Claim 6 and
M has the meaning indicated in Claim 8.

11. Process for the preparation of the compounds according to Claim 9, where
[A] compounds of the general formula (VI)
R¹-A-D'-H (VI)
in which
R¹, A and D' have the meaning indicated in Claim 8,
are reacted with compounds of the general formula (XI)
R⁴⁴-E-G-NO₂ (XI)
in which
E and G have the meaning indicated in Claim 1 and
R⁴⁴ is a leaving group, preferably halogen,
in inert solvents, if appropriate in the presence of a base, and then reacted with customary reducing agents, preferably H₂/Pd/C in an inert solvent or with hydrazine hydrate, Pd/C, if appropriate with simultaneous hydrogenation of (C-C) multiple bonds, to give compounds of the general formula (IIa)
R¹-A-D'-E-G-NH₂ (IIa)
in which
R¹, A, D', E and G have the meaning indicated above,
or
[B] compounds of the general formula (IIb)
R¹-A-D-E-G-NH₂ (IIb)
in which
R¹, A, D, E and G have the meaning indicated above, are reacted with a nitrosating agent, preferably an aqueous solution of sulphuric acid and sodium nitrite, and with subsequent warming, preferably to 60 to 100°C, to give compounds of the general formula (IIc)
R¹-A-D-E-G-OH (IIc)
in which
R¹, A, D, E and G have the abovementioned meaning,
or
[C] compounds of the general formula (XII)
R¹-R³⁶ (XII)
in which
R¹ has the meaning indicated above and
R³⁶ represents a leaving group, preferably halogen, particularly preferably bromine,
are reacted with compounds of the general formula (XIII)
HO-G-O-R⁴⁵ (XIII)
in which
G has the meaning indicated above and
R⁴⁵ represents (C₁-C₆)-alkyl, preferably methyl,
in an inert solvent, preferably dimethylformamide or pyridine, if appropriate in the presence of a base, preferably potassium carbonate, and if appropriate in the presence of copper(I/II) salts, preferably copper(II) oxide or copper(I) iodide, in a temperature range from 0°C to 200°C, preferably 80 to 150°C and normal pressure, to give compounds of the general formula (Ik)
R¹-O-G-O-R⁴⁵ (Ik)
in which
R¹, G and R⁴⁵ have the abovementioned meaning,
and are then reacted in the presence of an acid, preferably hydrobromic acid, to give compounds of the general formula (lid)
R¹-O-G-OH (IId)
or
[D] compounds of the general formula (VI)
R¹-A-D'-H (VI)
in which
R¹, A and D' have the meaning indicated in Claim 8,
are reacted with compounds of the general formula (XIV)
R⁴⁶-E-G'-R⁴⁷ (XIV)
in which
R⁴⁶ has the meaning indicated for R³⁶ and is identical to or different from this,
E has the abovementioned meaning,
G' represents a doubly bonded 5- to 7-membered aromatic heterocycle having up to 3 heteroatoms from the group consisting of sulphur, nitrogen and/or oxygen, which can optionally be substituted by one or more, identical or different substituents as defined for G in Claim 1 and
R⁴⁷ represents halogen, preferably chlorine or bromine,
to give a compound of the general formula (XV)
R¹-A-D'-E-G'-R⁴⁷ (XV)
in which
R¹, A, D', E, G' and R⁴⁷ have the abovementioned meaning,
in inert solvents, if appropriate in the presence of a base, and are then transformed with potassium amide in liquid ammonia into the corresponding free amines of the general formula (IIe)
R¹-A-D'-E-G'-NH₂ (IIe)
in which
R¹, A, D', E and G' have the abovementioned meaning.

12. Compounds of the formulae
-SO₂-CH₂-CH₂-CH₂-CF₃
or
-SO₂-CH₂-CH₂-CH₂-CF₂-CF₃.

13. Pharmaceutical composition which as active constituent comprises at least one compound according to any one of Claims 1 to 7 mixed together with at least one pharmaceutically tolerable essentially non-toxic vehicle or excipient.

14. Compounds according to any one of Claims 5 to 7 for use as a medicament in the treatment of humans and animals.

15. Use of the compounds according to any one of Claims 1 to 7 for the production of a medicament for the prevention and/or treatment of neurodegenerative disorders.

16. Use of the compounds according to any one of Claims 1 to 7 for the production of a medicament for the prevention and/or treatment of cerebral ischaemias and craniocerebral trauma.

17. Use of the compounds according to any one of Claims 1 to 7 for the production of a medicament for the treatment of states of pain, emesis, nausea, glaucoma, asthma, anorexia, convulsions, rheumatism, sedation and mobility disorders.

## Revendications

1. Composés de formule générale (I)
R¹-A-D-E-G-L-R² (I)
dans laquelle
R¹ est un reste naphtyle ou un reste de formule 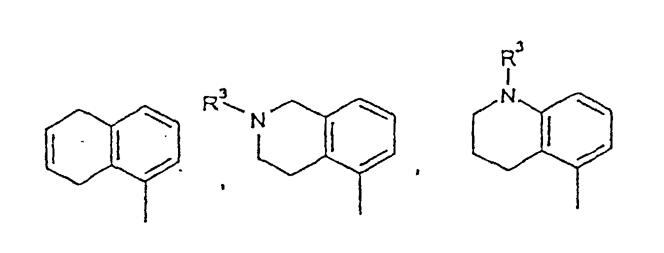 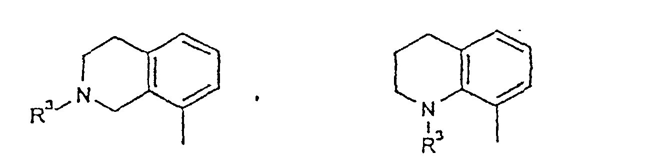 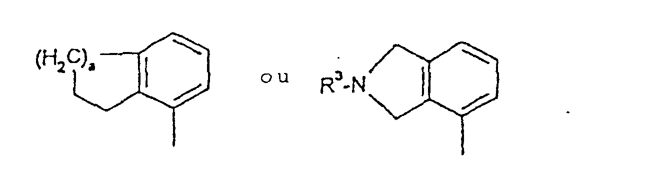
dans laquelle
a représente le nombre 1 ou 2,
R³ est l'hydrogène, un reste alcényle en C₂ à C₆, alkyle en C₁ à C₆ ou acyle en C₁ à C₆,
tous les systèmes de noyaux et les restes énumérés ci-dessus portant éventuellement un ou plusieurs restes identiques ou différents éventuellement géminés, qui sont choisis dans le groupe comprenant les substituants suivants :
halogéno, carboxyle, hydroxy, phényle, alkoxy en C₁ à C₆, (alkoxy en C₁ à C₆)-carbonyle, alkyle en C₁ à C₈ qui peut être substitué de son côté par un radical halogéno, alkylsulfonyloxy en C₁ à C₆, azide, amino, mono-(alkyle en C₁ à C₆)-amino, di-(alkyle en C₁ à C₆)-amino ou hydroxy,
un groupe de formule -(CO)_{b}-NR⁴R⁵,
dans laquelle
b est le nombre 0 ou 1,
R⁴ et R⁵ sont identiques ou différents et représentent indépendamment l'un de l'autre l'hydrogène, un radical phényle, acyle en C₁ à C₆, cycloacyle en C₄ à C₇, benzoyle ou alkyle en C₁ à C₆ qui est éventuellement substitué par un radical amino, mono-(alkyle en C₁ à C₆)-amino, di-(alkyle en C₁ à C₆)-amino,
ou bien
R⁴ et R⁵ forment conjointement avec l'atome d'azote un hétérocycle saturé pentagonal ou hexagonal, qui peut contenir le cas échéant un ou plusieurs autres hétéro-atomes de la série S, O et/ou un ou plusieurs restes de formule -NR⁸
dans laquelle
R⁸ est l'hydrogène, un radical alkyle en C₁ à C₆ ou acyle en C₁ à C₆,
et
un groupe de formule -NR⁶-SO₂-R⁷
dans laquelle
R⁶ est l'hydrogène, un radical phényle, alkyle en C₁ à C₆ ou acyle en C₁ à C₆,
R⁷ est un radical phényle ou alkyle en C₁ à C₆,
A et E sont identiques ou différents et représentent une liaison ou un groupe alkylène en C₁ à C₄,
D représente un atome d'oxygène ou un reste de formule -S(O)_{c}- ou -N(R⁹)-,
où
c représente le nombre 0, 1 ou 2,
R⁹ est l'hydrogène, un radical alkyle en C₁ à C₆ ou acyle en C₁ à C₆,
G représente un reste aryle en C₆ à C₁₀, comprenant deux liaisons ou un hétérocycle aromatique pentagonal à heptagonal comprenant deux liaisons et ayant jusqu'à 3 hétéro-atomes de la série S, N et/ou O, qui sont éventuellement substitués avec un ou plusieurs substituants identiques ou différents qui sont choisis dans le groupe comprenant les substituants suivants :
hydroxy, trifluorométhyle, carbonyle, halogéno, alkyle en C₁ à C₆, hydroxyalkyle en C₁ à C₆, alkoxy en C₁ à C₆, (alkoxy en C₁ à C₆)-carbonyle ainsi que des groupes de formules
-CO-O-(CH₂)_{d}-NR¹⁰R¹¹, -NR¹²-SO₂R¹³,
-(CH₂)_{c}-(CO)ᵢ-NR¹⁴R¹⁵ et -OR¹⁶,
dans lesquelles
d représente un nombre 1, 2, 3 ou 4,
e et f sont identiques ou différents et désignent le nombre 0 ou 1,
R¹ et R¹¹ ont la définition indiquée ci-dessus pour R⁴ et R⁵ et y sont identiques ou en sont différents,
R¹² a la définition indiquée ci-dessus pour R⁶ et il est identique ou en est différent,
R¹³ a la définition indiquée ci-dessous pour R⁷ et il est identique ou en est différent,
R¹⁴ et R¹⁵ ont la définition indiquée ci-dessus pour R⁴ et R⁵ et y sont identiques ou en sont différents, ou forment indépendamment l'un de l'autre un reste de formule -(CH₂)_{g}-NR¹⁷R¹⁸,
dans laquelle
g est le nombre 1, 2, 3 ou 4
et
R¹⁷ et R¹⁸ ont la définition indiquée ci-dessus pour R⁴ et R⁵ et y sont identiques ou en sont différents,
R¹⁶ est un groupe aryle en C₆ à C₁₀,
L représente un reste de formule
-O-, -NH-, 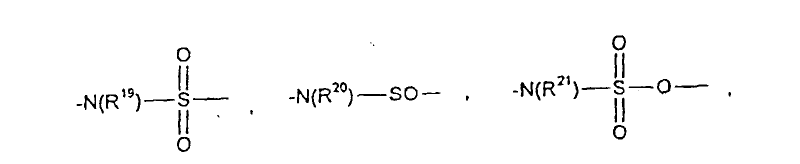 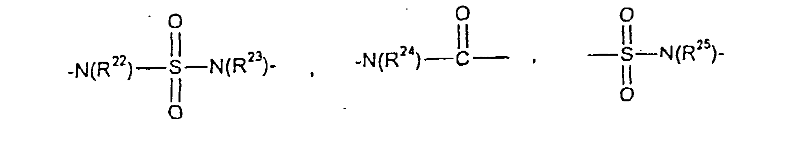 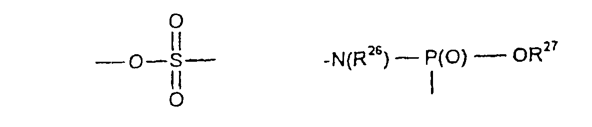
ou 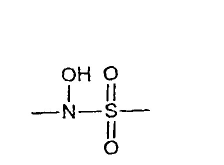 où le rattachement des restes à G s'effectue de façon cadrée à gauche,
et où
R¹⁹' R²⁰' R²¹' R²²' R²³' R²⁴' R²⁵' R²⁶ et R²⁷ sont identiques ou différents et représentent l'hydrogène ou un groupe alkyle en C₁ à C₄,
ou bien
R¹⁹ est un reste de formule -SO₂R²
R² est un reste aryle en C₆ à C₁₀, ou un hétérocycle saturé ou aromatique, pentagonal ou heptagonal ayant jusqu'à 3 hétéro-atomes de la série S, N et/ou O, qui sont éventuellement substitués avec un ou plusieurs substituants identiques ou différents qui sont choisis dans le groupe des substituants suivants :
halogène, trifluorométhyle, nitro, amino et alkyle en C₁ à C₆,
ou bien
le reste de formule 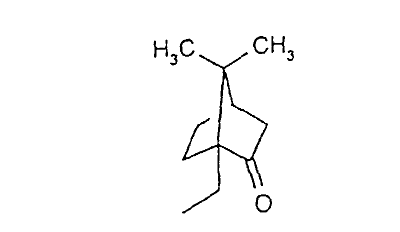 ou le reste morpholine, ou bien
un reste cycloalkyle en C₃ à C₈, ou bien
un reste alkyle en C₁ à C₁₂, alcényle en C₂ à C₁₂ ou alcynyle en C₂ à C₁₂, ces restes pouvant porter un ou plusieurs substituants, identiques ou différents, du groupe constitué des substituants :
halogéno, trifuorométhyle, hydroxy, cyano, azido, alkoxy en C₁ à C₆, perfluoralkoxy en C₁ à C₆, alkoxy en C₁ à C₆ partiellement fluoré, un reste de formule 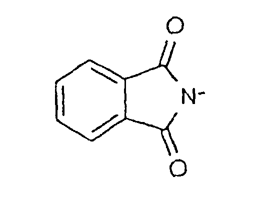 où R²⁸ et R²⁹ ont la définition indiquée ci-dessus pour R⁴ et R⁵ et y sont identiques ou en sont différents,
phényle, portant éventuellement ou plusieurs substituants identiques ou différents qui sont choisis dans le groupe des substituants :
halogéno, nitro, hydroxy, alkyle en C₁ à C₆, alkoxy en C₁ à C₆ et un groupe de formule -NR³⁰R³¹,
dans laquelle R³⁰ et R³¹ sont identiques ou différents et représentent l'hydrogène ou un radical alkyle en C₁ à C₆ ou acyle en C₁ à C₆,
et un hétérocycle aromatique pentagonal ou hexagonal ayant jusqu'à 3 hétéro-atomes de la série S, N et/ou O, portant éventuellement un ou plusieurs substituants identiques ou différents qui sont choisis dans le groupe des substituants suivants :
halogéno, nitro, hydroxy, alkyle en C₁ à C₆, alkoxy en C₁ à C₆ et un groupe de formule -NR³⁰R³¹
dans laquelle R³⁰ et R³¹ sont tels que définis ci-dessus,
ou bien
L et R² forment ensemble un reste de formule 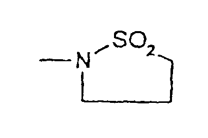
et des sels de ces composés, destinés à être utilisés comme médicament en médecine humaine et en médecine vétérinaire.

2. Composés de formule (I) suivant la revendication 1, dans lesquels
R¹ est un reste naphtyle ou un reste de formule 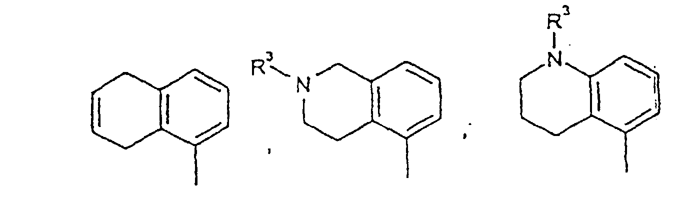 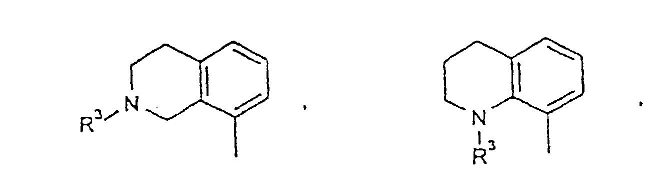 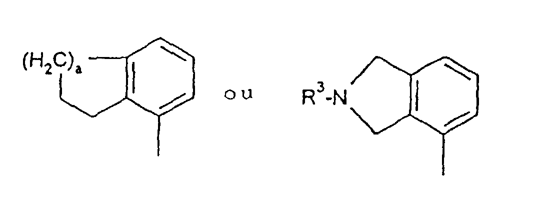 où
a représente le nombre 1 ou 2,
R³ est l'hydrogène, un radical alcényle en C₂ à C₄, alkyle en C₁ à C₄ ou acyle en C₁ à C₄,
et où tous les systèmes de noyaux et les restes énumérés ci-dessus portent éventuellement un ou plusieurs substituants identiques ou différents éventuellement géminés, qui sont choisis dans le groupe des substituants suivants :
halogéno, carboxyle, hydroxyle, phényle, alkoxy en C₁ à C₄, (alkoxy en C₁ à C₅)-carbonyle, alkyle en C₁ à C₆ qui peut éventuellement être lui-même substitué par un radical halogéno, alkylsulfonyloxy en C₁ à C₄, azido, amino, mono-(alkyle en C₁ à C₄)-amino, di-(alkyle en C₁ à C₄)-amino ou hydroxy,
un groupe de formule -(CO)_{b}-NR⁴R⁵,
dans laquelle
b a la valeur 0 ou 1,
R⁴ et R⁵ sont identiques ou différents et représentent indépendamment l'un de l'autre l'hydrogène, un reste phényle, acyle en C₁ à C₄, cycloacyle en C₄ à C₇, benzoyle ou un reste alkyle en C₁ à C₄ qui est éventuellement substitué par un radical amino, mono-(alkyle en C₁ à C₄), di-(alkyle en C₁ à C₄),
ou bien
R⁴ et R⁵ forment conjointement avec l'atome d'azote un noyau morpholine, pipéridine ou N-méthylpipérazine,
et
un groupe de formule -NR⁶-SO₂-R⁷
dans laquelle
R⁶ est l'hydrogène, un radical phényle, alkyle en C₁ à C₄ ou acyle en C₁ à C₄,
et
R⁷ est un radical phényle ou alkyle en C₁ à C₅,
A et E sont identiques ou différents et représentent une liaison ou un groupe alkylène en C₁ à C₄,
D est un atome d'oxygène ou un reste de formule -S(O)_{c}- ou -N(R⁹)-,
où
c représente le nombre 0, 1 ou 2,
R⁹ est l'hydrogène ou un reste alkyle en C₁ à C₄ ou acyle en C₁ à C₄,
G représente un reste phényle, naphtyle, pyrimidyle, pyridazinyle ou pyridyle à deux liaisons, ces restes portant éventuellement un ou plusieurs substituants identiques ou différents qui sont choisis dans le groupe des substituants suivants :
hydroxy, trifluorométhyle, carboxyle, halogéno, alkyle en C₁ à C₄, hydroxyalkyle en C₁ à C₄, alkoxy en C₁ à C₄, (alkoxy en C₁ à C₄)-carbonyle, ainsi que des groupes de formules
-CO-O-(CH₂)_{d}-NR¹⁰R¹¹, -NR¹²-SO₂R¹³,
-(CH₂)_{c}-(CO)ᵢ-NR¹⁴R¹⁵ et -OR¹⁶,
dans lesquelles
d représente le nombre 1, 2, 3 ou 4,
e et f sont identiques ou différents et représentent le nombre 0 ou 1,
R¹⁰ et R¹¹ ont la définition indiquée ci-dessus pour R⁴ et R⁵ et y sont identiques ou en sont différents,
R¹² a la définition indiquée ci-dessus pour R⁶ et il est identique ou en est différent,
R¹³ a la définition indiquée ci-dessous pour R⁷ et il est identique ou en est différent,
R¹⁴ et R¹⁵ ont la définition indiquée ci-dessus pour R⁴ et R⁵ ou y sont identiques et en sont différents, ou représentent indépendamment l'un de l'autre un reste de formule - (CH₂)_{g}-NR¹⁷R¹⁸,
dans laquelle
g représente le nombre 1, 2 ou 3
et
R¹⁷ et R¹⁸ ont la définition indiquée ci-dessus pour R¹⁰ et R¹¹ et y sont identiques ou en sont différents,
R¹⁶ est un groupe phényle ou naphtyle,
L représente un reste de formule
-O-, -NH-, 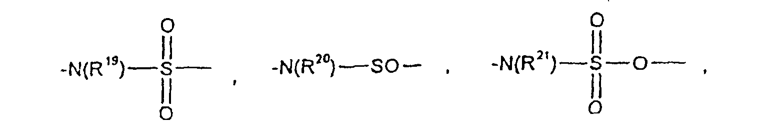 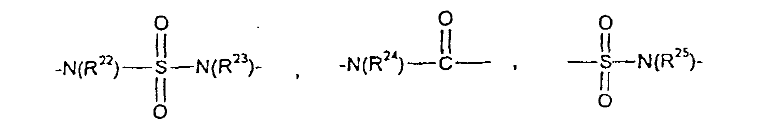 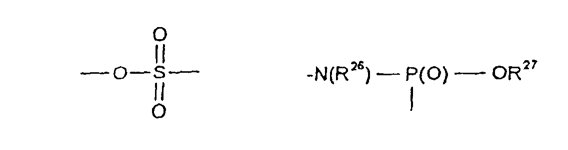
ou 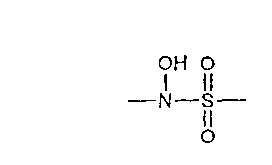 où le rattachement des restes à G est cadré à gauche,
et
où
R^{19,} R^{20,} R^{21,} R^{22,} R^{23,} R^{24,} R^{25,} R²⁶ et R²⁷ sont identiques ou différents et représentent l'hydrogène ou des groupes alkyle en C₁ à C₃,
ou bien
R¹⁹ représente un reste de formule -SO₂R²
R² est un reste phényle, naphtyle, pyridyle, furyle, thiényle ou pyrimidine, ces restes portant éventuellement un ou plusieurs substituants identiques ou différents choisis dans le groupe des substituants suivants :
halogéno, amino, trifluorométhyle, nitro, et alkyle en C₁ à C₄,
ou bien
le reste de formule 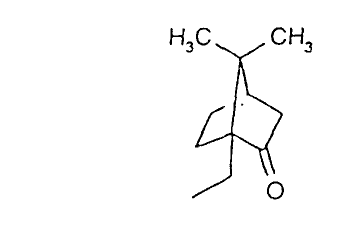 ou un reste morpholine,
ou bien
un reste cyclopropyle, cyclohexyle ou cyclopentyle, ou bien
un reste alkyle en C₁ à C₁₀, alcényle en C₂ à C₁₀ ou alcynyle en C₂ à C₁₀, ces restes portant éventuellement un ou plusieurs substituants, identiques ou différents, du groupe des substituants suivants :
halogéno, trifuorométhyle, hydroxy, azido, alkoxy en C₁ à C₄, perfluoralkyle en C₁ à C₅, alkoxy en C₁ à C₄ partiellement fluoré, un reste de formule 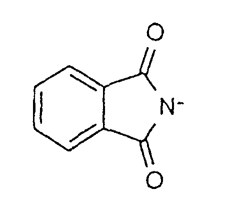 dans laquelle
R²⁸ et R²⁹ ont la définition indiquée ci-dessus pour R⁴ et R⁵ et y sont identiques ou en sont différents,
un reste phényle, portant éventuellement un ou plusieurs substituants identiques ou différents, du groupe des substituants :
halogéno, nitro, hydroxy, alkyle en C₁ à C₄, alkoxy en C₁ à C₄ et un groupe de formule -NR³⁰R³¹,
où R³⁰ et R³¹ sont identiques ou différents et représentent l'hydrogène ou un radical alkyle en C₁ à C₄ ou acyle en C₁ à C₄,
pyridyle et pyrimidine portant chacun le cas échéant un ou plusieurs substituants identiques ou différents choisis dans le groupe des substituants suivants :
halogéno, nitro, hydroxy, alkyle en C₁ à C₄, alkoxy en C₁ à C₄ et un groupe de formule -NR³⁰R³¹
dans laquelle R³⁰ et R³¹ sont tels que définis ci-dessus, ou bien
L et R² forment ensemble un reste de formule 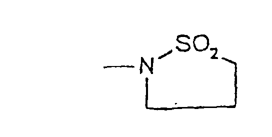 et des sels de ces composés, destinés à être utilisés comme médicament en médecine humaine et en médecine vétérinaire.

3. Composés de formule (I) suivant la revendication 1, formule dans laquelle
R¹ est un reste naphtyle ou un reste de formule 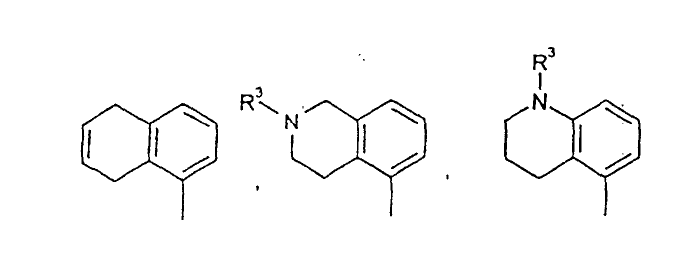 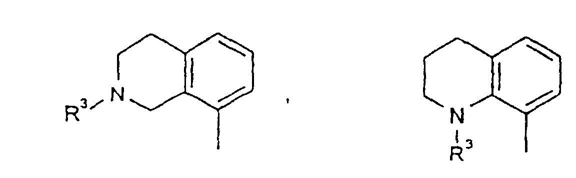 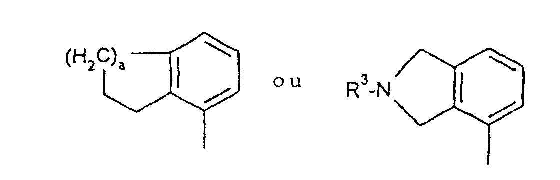 dans laquelle
a représente le nombre 1 ou 2,
R³ est l'hydrogène, un groupe alcényle en C₂ ou C₃, alkyle en C₁ à C₃ ou acyle en C₁ à C₃,
tous les systèmes de noyaux indiqués ci-dessus portant éventuellement un ou plusieurs substituants identiques ou différents éventuellement géminés, qui sont choisis dans le groupe des substituants suivants :
chloro, fluoro, carboxyle, hydroxyle, phényle, alkoxy en C₁ à C₃, (alkoxy en C₁ à C₄)-carbonyle, alkyle en C₁ à C₄, qui peut lui-même être substitué par un radical chloro ou hydroxy, un groupe de formule -(CO)_{b}-NR⁴R⁵,
dans laquelle
b représente le nombre 0 ou 1,
R⁴ et R⁵ sont identiques ou différents et représentent indépendamment l'un de l'autre l'hydrogène, un radical acyle en C₁ à C₃, cycloacyle en C₄ à C₆, benzoyle ou un radical alkyle en C₁ à C₃ qui est éventuellement substitué par un radical amino, mono-(alkylamino en C₁ à C₃), di-(alkyle en C₁ à C₃)-amino,
ou bien
R⁴ et R⁵ forment conjointement avec l'atome d'azote un noyau morpholine, pipéridine ou N-méthylpipérazine,
et
un groupe de formule -NR⁶-SO₂-R⁷
dans laquelle
R⁶ est l'hydrogène, un radical alkyle en C₁ à C₃ ou acyle en C₁ à C₃,
et
R⁷ est un groupe phényle ou alkyle en C₁ à C₄,
A et E sont identiques ou différents et forment une liaison ou un groupe alkyle en C₁ à C₃,
D représente un atome d'oxygène ou un reste de formule -S(O)_{c}- ou -N(R⁹)-,
où
c représente le nombre 0, 1 ou 2,
R⁹ est l'hydrogène ou un radical alkyle en C₁ à C₃ ou acyle en C₁ à C₃,
G représente un reste phényle, naphtyle, pyrimidyle, pyridazinyle ou pyridyle comprenant deux liaisons, portant le cas échéant un ou plusieurs substituants identiques ou différents choisis dans le groupe des substituants :
hydroxy, trichlorométhyle, carboxyle, fluoro, chloro, bromo, alkyle en C₁ à C₃, hydroxyalkyle en C₁ à C₃, alkoxy en C₁ à C₃, (alkoxy en C₁ à C₃)-carbonyle, ainsi que des groupes de formules
-CO-O-(CH₂)_{d}-NR¹⁰R¹¹, -NR¹²-SO₂R¹³,
-(CH₂)_{c}-(CO)_{f}-NR¹⁴R¹⁵CH₂OH et -OR¹⁶,
dans lesquelles
d représente le nombre 1, 2 ou 3,
e et f sont identiques ou différents et représentent le nombre 0 ou 1,
R¹⁰ et R¹¹ représentent l'hydrogène ou le groupe méthyle,
R¹² est l'hydrogène,
R¹³ est un reste alkyle en C₁ à C₄,
R¹⁴ et R¹⁵ ont la définition indiquée ci-dessus pour R⁴ et R⁵ et y sont identiques ou en sont différents, ou forment un reste de formule -(CH₂)_{g}-NR¹⁷R¹⁸,
dans laquelle
g représente le nombre 1, 2 ou 3
et
R¹⁷ et R¹⁸ représentent l'hydrogène ou des restes méthyle,
ou bien
R¹⁴ et R¹⁵ forment conjointement avec l'azote un reste de formule 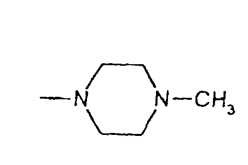
R¹⁶ est un reste phényle ou naphtyle,
L est un reste de formule
O-, -NH-, 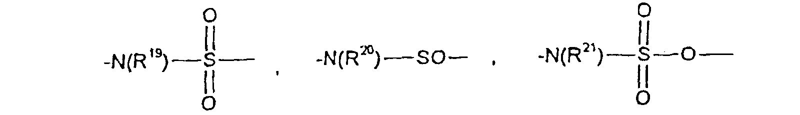 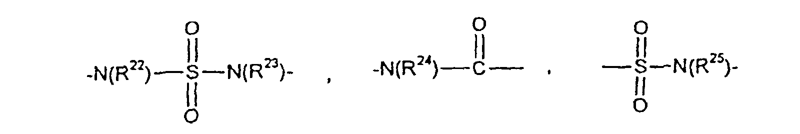 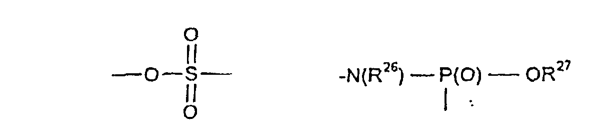
ou 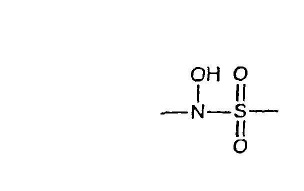 où le rattachement des restes à G s'effectue de façon cadrée à gauche,
et
où
R^{19,} R^{20,} R^{21,} R^{22,} R^{23,} R^{24,} R^{25,} R²⁶ et R²⁷ sont identiques ou différents et représentent l'hydrogène ou un reste méthyle ou éthyle,
ou bien
R¹⁹ est un reste de formule -SO₂R²
R² est un reste phényle, furyle ou pyridyle, ces restes portant éventuellement un ou plusieurs substituants identiques ou différents choisis dans le groupe des substituants :
fluoro, chloro, bromo ou trifluorométhyle,
ou bien
un reste de formule 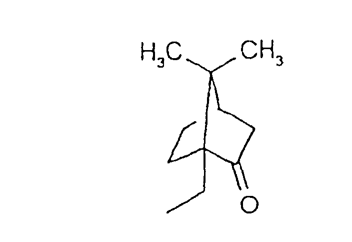 ou morpholine,
ou bien
un reste cyclopentyle ou cyclohexyle, ou bien
un reste alkyle en C₁ à C₈, alcényle en C₂ à C₈ ou alcynyle en C₂ à C₈, ces restes portant éventuellement un ou plusieurs substituants, identiques ou différents, qui sont choisis entre les substituants suivants :
fluoro, chloro, bromo, trifuorométhyle, hydroxy, azido, alkoxy en C₁ à C₆, perfluoralkoxy en C₁ à C₄, alkoxy en C₁ à C₄ à substituant trifluorométhyle, un reste de formule 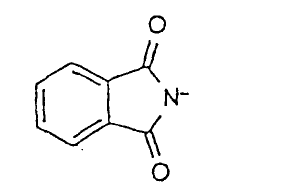 où
R²⁸ et R²⁹ représentent l'hydrogène ou le radical méthyle,
phényle, pyridyle et pyrimidyle portant le cas échéant un ou plusieurs substituants identiques ou différents choisis dans le groupe constitué de :
fluor, chlore, brome, nitro, hydroxy, alkyle en C₁ à C₃, alkoxy en C₁ à C₃ et un groupe de formule -NR³⁰R³¹,
où R³⁰ et R³¹ sont identiques ou différents et représentent l'hydrogène ou des restes méthyle ou méthylcarbonyle,
ou bien
L et R² forment ensemble un reste de formule 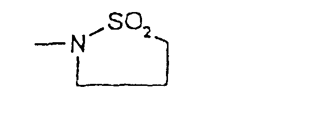 et des sels de ces composés, destinés à être utilisés comme médicament en médecine humaine et en médecine vétérinaire.

4. Composés de formule générale (I)
R¹-A-D-E-G-L-R (I)
dans laquelle R¹, A, D, E, G, L et R² ont les définitions indiquées dans les revendications 1 à 3,
à l'exception des composés de formule générale (I) dans lesquels
R¹ est un reste napht-1-yle, dont la position 3 est éventuellement substituée avec du chlore ou un radical alkyle en C₁ à C₄ et la position 4 est éventuellement substituée avec du chlore ou un radical phényle,
A et E représentent une liaison,
D est un atome d'oxygène,
G est un groupe 1,4-phénylène qui est éventuellement substitué par un radical alkyle en C₁ à C₄,
L est un atome d'oxygène, et
R² est un groupe méthyle,
à l'exception du m-bis-(1-naphtyloxy)-benzène.

5. Composés de formule (I) suivant la revendication 1 dans laquelle
R¹ est un reste naphtyle ou un reste de formule 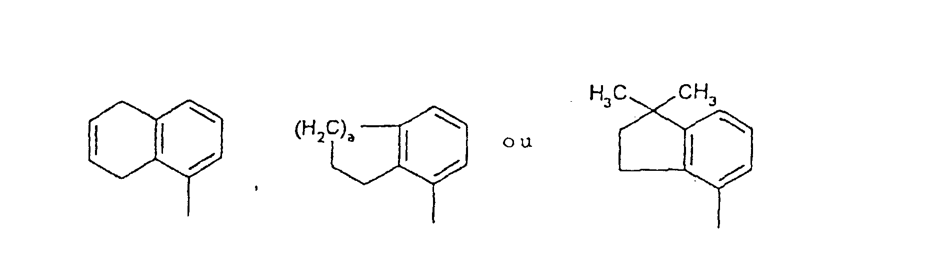 dans laquelle
a représente le nombre 1 ou 2,
tous les systèmes de noyaux et tous les restes énumérés ci-dessus portant éventuellement 1 à 3 substituants éventuellement géminés identiques ou différents qui sont choisis dans le groupe des substituants suivants :
halogéno, carboxyle, hydroxy, alkoxy en C₁ à C₆, (alkoxy en C₁ à C₆)-carbonyle, alkyle en C₁ à C₈ qui peut lui-même être substitué par un radical halogéno ou hydroxy,
un groupe de formule -(CO)_{b}-NR⁴R⁵,
dans laquelle
b représente le nombre 0 ou 1,
R⁴ et R⁵ sont identiques ou différents et représentent l'hydrogène ou un radical phényle ou alkyle en C₁ à C₆,
et
un groupe de formule -NR⁶-SO₂-R⁷
dans laquelle
R⁶ est l'hydrogène, un radical phényle ou alkyle en C₁ à C₆,
R⁷ est un radical phényle ou alkyle en C₁ à C₆,
A et E sont identiques ou différents et représentent une liaison ou un groupe alkylène en C₁ à C₄,
D est un atome d'oxygène ou représente un reste de formule -S(O)_{c}- ou -NH-,
où
c représente le nombre 0, 1 ou 2,
G est un reste aryle en C₆ à C₁₀ comprenant deux liaisons ou un hétérocycle aromatique pentagonal à heptagonal comprenant deux liaisons ayant jusqu'à 3 atomes de carbone de la série S, N et/ou O, qui portent éventuellement 1 à 3 substituants identiques ou différents choisis dans le groupe des substituants suivants :
hydroxy, carboxyle, halogéno, alkyle en C₁ à C₆, hydroxyalkyle en C₁ à C₆, alkoxy en C₁ à C₆, (alkoxy en C₁ à C₆)-carbonyle, de même que des groupes de formules
-CO-O-(CH₂)_{d}-NR¹⁰R¹¹, -NR¹²-SO₂R¹³ et -(CO)-NR¹⁴R¹⁵,
où
d représente le nombre 1, 2, 3 ou 4,
R¹⁰ et R¹¹ ont la définition indiquée ci-dessus pour R⁴ et R⁵ et y sont identiques ou en sont différents,
R¹² a la définition indiquée ci-dessus pour R⁶ et il est identique ou en est différent,
R¹³ a la définition indiquée ci-dessus pour R⁷ et il est identique ou en est différent,
R¹⁴ et R¹⁵ ont la définition indiquée ci-dessus pour R⁴ et R⁵ et y sont identiques ou en sont différents, ou forment conjointement avec l'atome d'azote un hétérocycle pentagonal ou hexagonal qui peut encore contenir le cas échéant un autre hétéro-atome de la série S et O ou un groupe de formule -NH-,
L représente un reste de formule 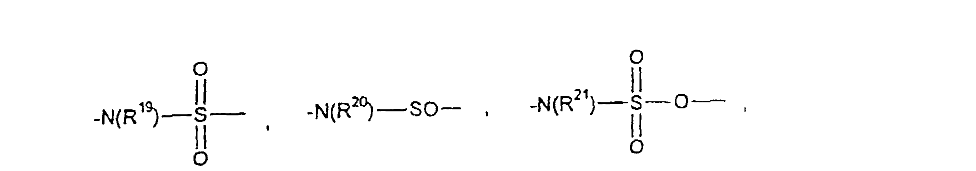 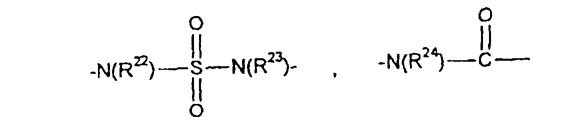 ou 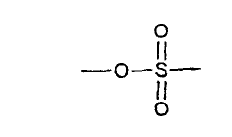 le rattachement des restes à G étant cadré du côté gauche,
et R^{19,} R^{20,} R^{21,} R^{22,} R²³ et R²⁴ sont identiques ou différents et représentent l'hydrogène ou des restes alkyle en C₁ à C₄,
R² est un reste phényle qui porte éventuellement un substitué halogéno, trifluorométhyle, nitro, amino ou alkyle en C₁ à C₆,
R² représente le reste de formule 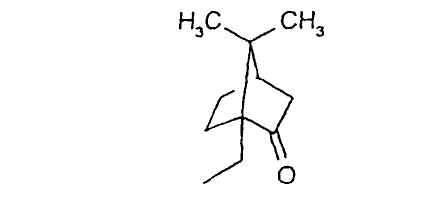 ou une morpholine, ou bien
un reste perfluoralkyle ayant jusqu'à 12 atomes de carbone, ou bien
un reste alkyle en C₁ à C₁₂ ou alcynyle en C₂ à C₁₂, ces restes portant éventuellement un substituant halogéno, trifluoréthyle, hydroxy, azido ou un reste de formule 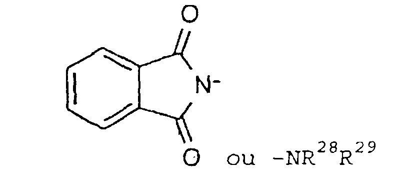 où R²⁸ et R²⁹ ont la définition indiquée pour R⁴ et R⁵ et y sont identiques ou en sont différents et/ou le cas échéant un substituant phényle ou un hétérocycle aromatique pentagonal ou hexagonal ayant jusqu'à 3 hétéro-atomes de la série S, N et/ou O, ces substituants pouvant eux-mêmes être substitués jusqu'à deux fois identiques ou différentes par un radical halogéno, nitro, hydroxy, alkyle en C₁ à C₆, alkoxy en C₁ à C₆ ou par un groupe de formule -NR³⁰R³¹,
dans laquelle R³⁰ et R³¹ sont identiques ou différents et représentent l'hydrogène, un radical alkyle en C₁ à C₆ ou acyle en C₁ à C₆,
L et R² forment ensemble un reste de formule 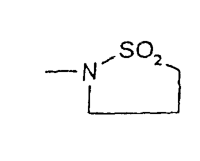
et leurs sels.

6. Composés de formule (I) suivant la revendication 1, dans laquelle
R¹ est un reste napht-1-yle, éventuellement substitué par un radical alkyle en C₁ à C₆ à substituant hydroxy, acylamino en C₁ à C₆, amino ou alkoxy en C₁ à C₆, un reste indane-4-yle substitué par un radical hydroxyalkyle en C₁ à C₆,
un reste de formule 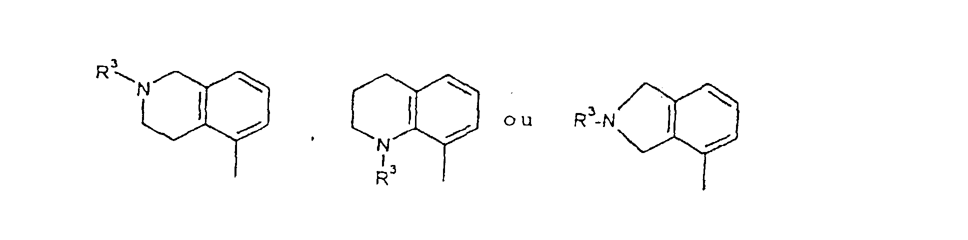 dans laquelle
R³ est un reste alkyle en C₁ à C₆,
E et A forment un liaison,
D est un atome d'oxygène,
G est un reste 1,3-phénylène, 1,4-phénylène ou 2,5-pyridylène, ces restes étant éventuellement substitués par un halogène,
L est un reste de formule -NH-SO₂- ou -O-SO₂-
et
R² représente un reste alkyle en C₁ à C₆ qui est éventuellement substitué par un radical chloro, trifluorométhyle, par un reste de formule -O-CH₂-CF₃ ou par un reste phényle ou un reste pyridyle qui peuvent être eux-mêmes substitués par du brome ou du chlore,
et leurs sels.

7. Composés de formules suivantes :

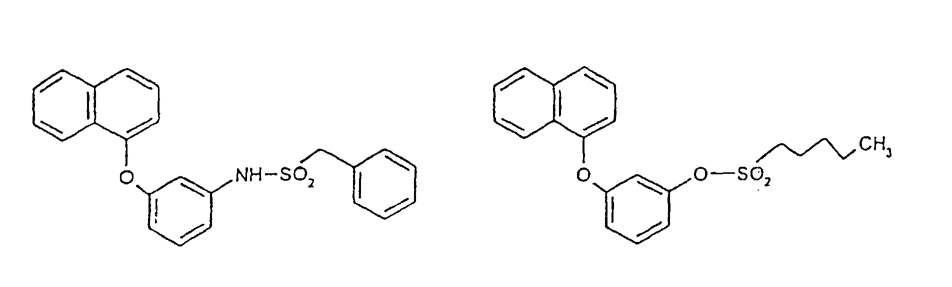

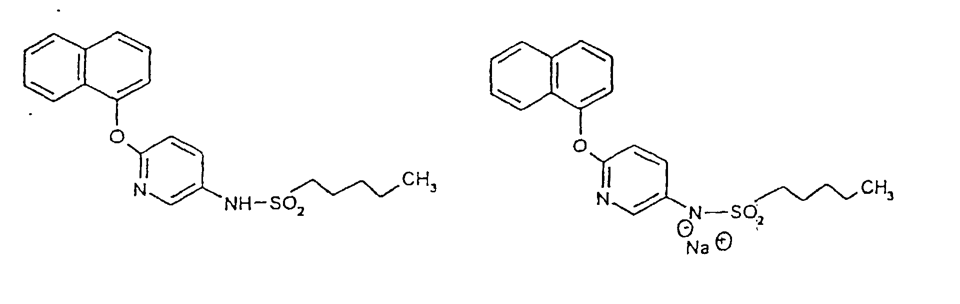

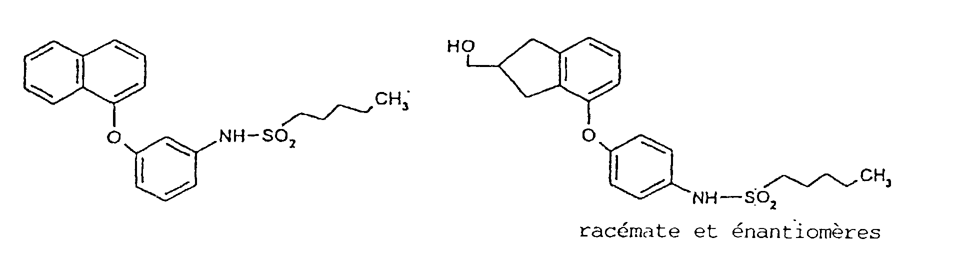

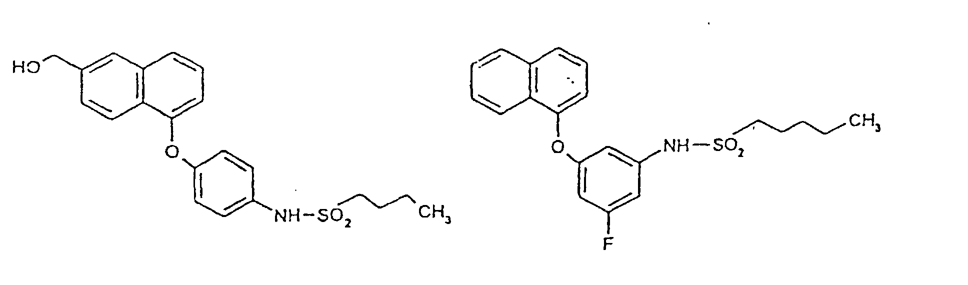

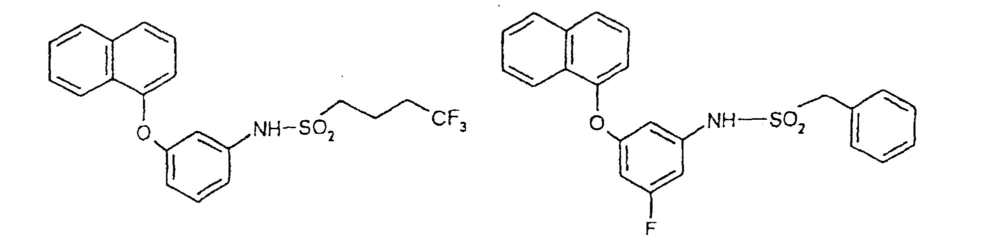

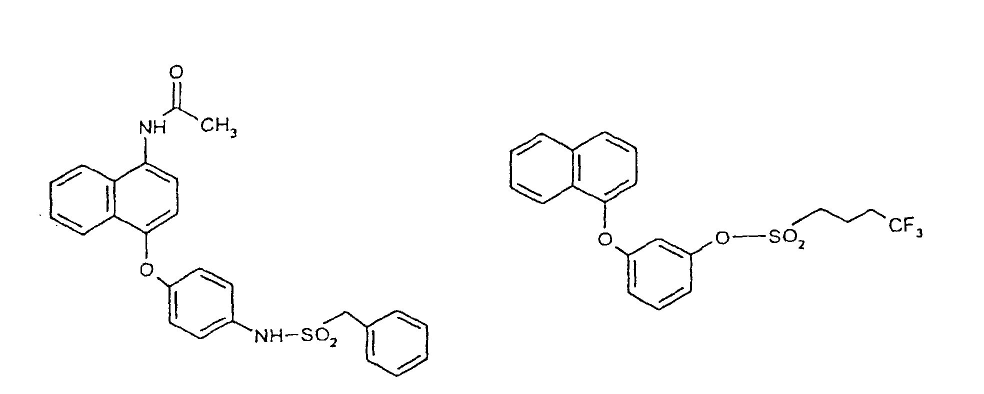

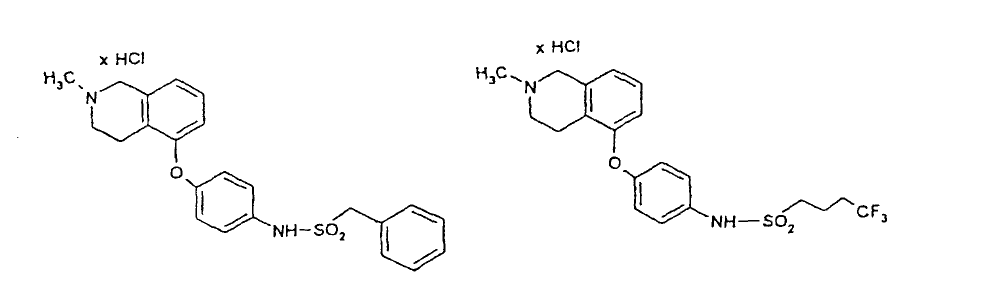

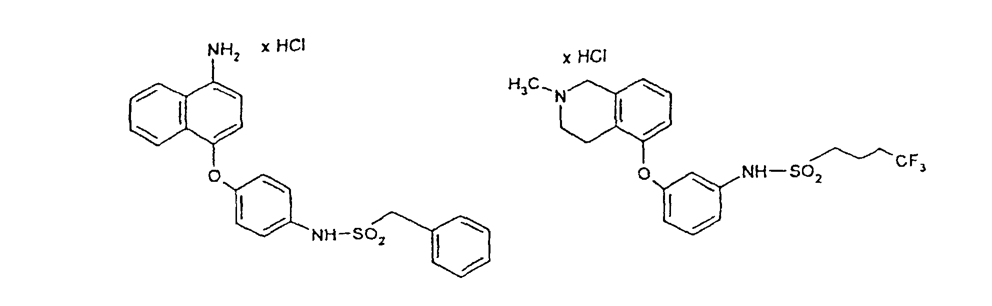

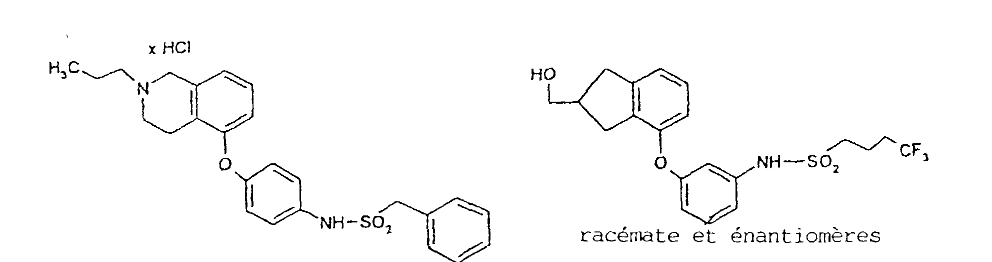

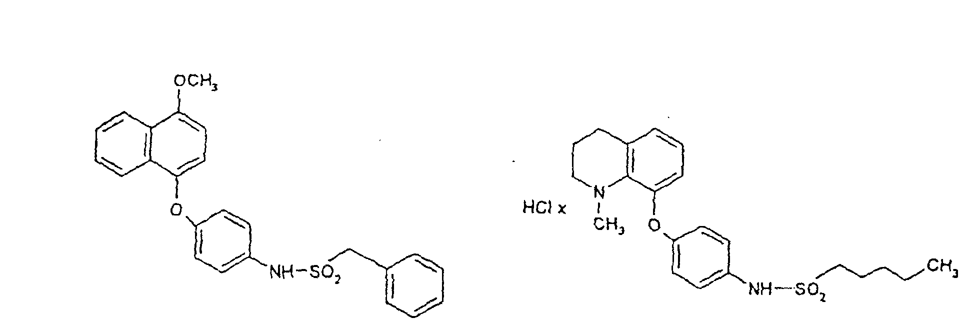

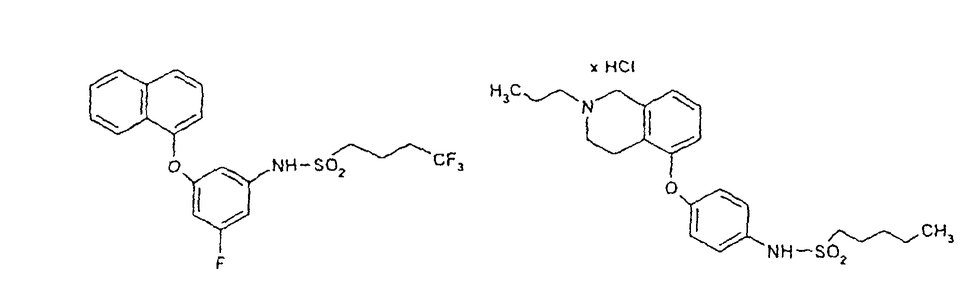

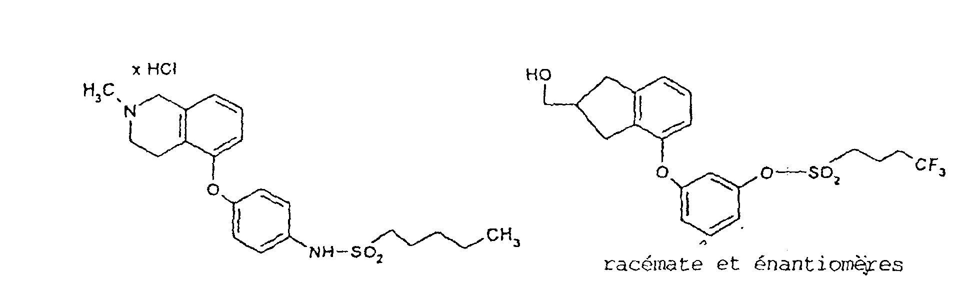

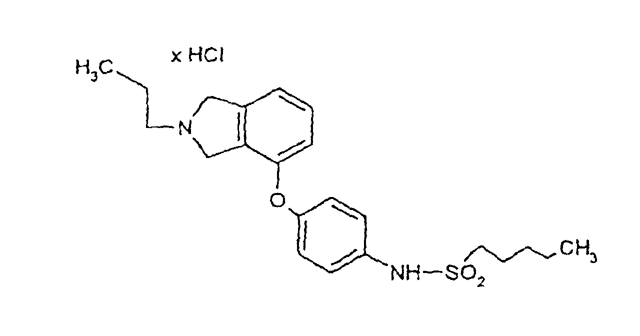

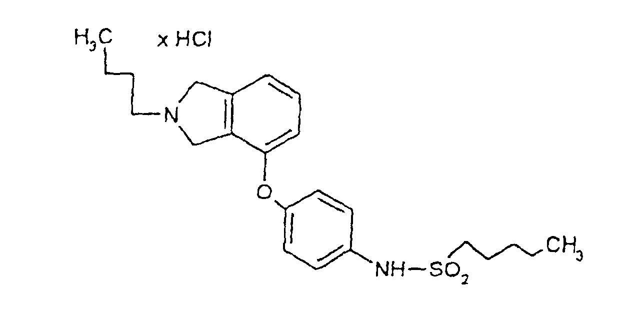

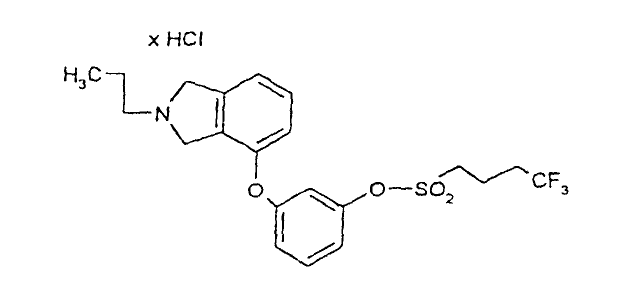

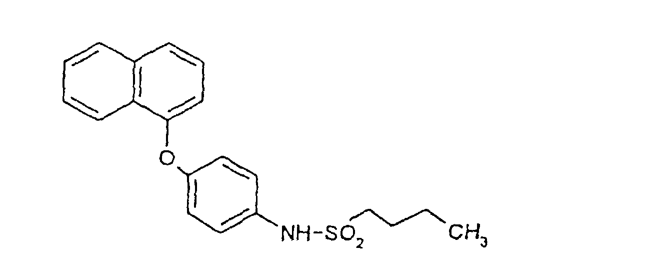

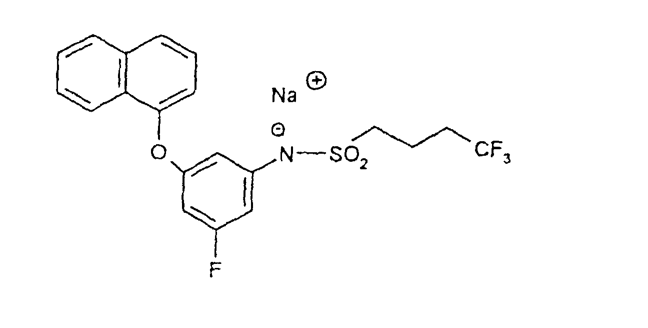

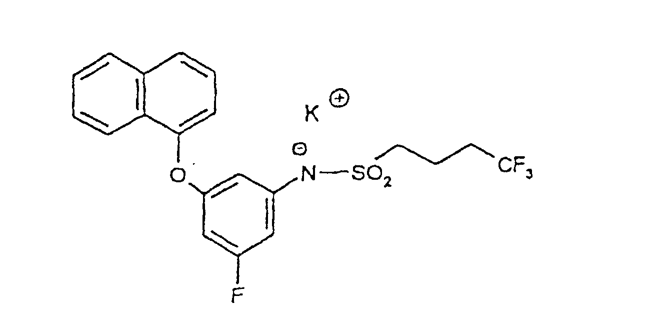

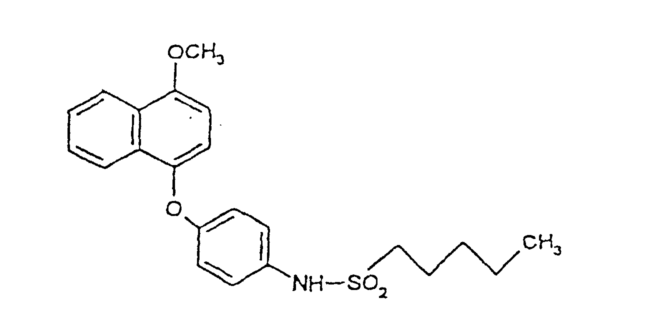

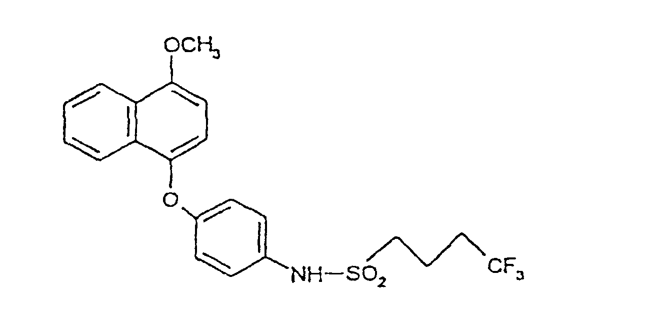

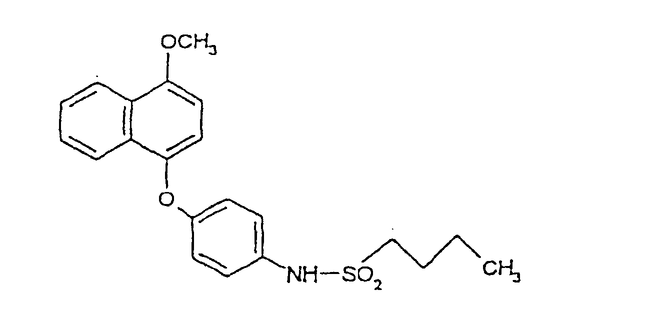

8. Procédé de production des composés de formule suivant la revendication 4,
dans lequel
[A] on fait réagir des composés de formule générale (II)
R¹-A-D-E-G-M-H (I)
dans laquelle
R¹, A, D, E et G ont la définition indiquée dans la revendication 1 et
M représente l'oxygène ou un reste -N(R³²)- et
R³² est l'hydrogène ou un radical alkyle en C₁ à C₄,
avec des composés de formule générale (III)
R³³-Q-R² (III)
dans laquelle
R² a la définition indiquée dans la revendication 1,
R³³ est un halogène, de préférence le chlore ou l'iode,
Q est un reste de formule -SO₂-, -SO-, -CO-, -P(O) (OR²⁷)- ou une liaison simple,
où
R²⁷ a la définition indiquée dans la revendication 1, pour obtenir des composés de formule générale (Ia)
R¹-A-D-E-G-M-Q-R² (Ia)
dans laquelle
R¹, A, D, E, G, M, Q et R² ont la définition indiquée ci-dessus,
dans des solvants inertes, le cas échéant en présence d'une base,
ou bien
[B] on fait réagir des composés de formule générale (II) tout d'abord avec un ester trialkylsilylique d'acide chlorosulfonique, de préférence l'ester triméthylsilylique d'acide chlorosulfonique, on ajoute un acide puis on fait réagir avec un agent de chloration, de préférence le pentachlorure de phosphore, pour obtenir un composé de formule générale (IV)
R¹-A-D-E-G-M-SO₂-Cl (IV)
dans laquelle R¹, A, D, E, G et M ont la définition indiquée ci-dessus, puis on fait réagir les composés obtenus avec des composés de formule générale (V)
H-T-R² (V)
dans laquelle
R² a la définition indiquée dans la revendication 1 et
T représente l'oxygène ou l'azote,
pour obtenir des composés de formule générale (Ib)
R¹-A-D-E-G-M-SO₂-T-R² (Ib)
dans laquelle
R¹, A, D, E, G, M, T et R² ont la définition indiquée ci-dessus,
dans des solvants inertes, éventuellement en présence de Bzl-Net⁺₃ + Cl⁻ et d'une base
ou bien
[C] on fait réagir des composés de formule générale (VI)
R¹-A-D'-H (VI)
dans laquelle
R¹ et A ont la définition indiquée ci-dessus et
D' représente l'oxygène, le soufre ou un groupe -N(R⁹)- et
R⁹ a la définition indiquée dans la revendication 1,
avec des composés de formule générale (VII)
R³⁴-E-G-SO₂-NH-R² (VII)
dans laquelle
E, G et R² ont la définition indiquée ci-dessus et
R³⁴ est un groupe partant, de préférence un halogène, notamment le fluor, le chlore ou le brome,
pour obtenir des composés de formule générale (Ic)
R¹-A-D'-E-G-SO₂-NH-R² (Ic)
dans laquelle
R¹, A, D', E, G et R² ont la définition indiquée ci-dessus,
ou bien
[D] on fait réagir des composés de formule générale (Id)
R³⁷-A-D-E-G-L-R² (Id)
dans laquelle
A, D, E, G, L et R² ont la définition indiquée ci-dessus et
R³⁷ est un reste de formule 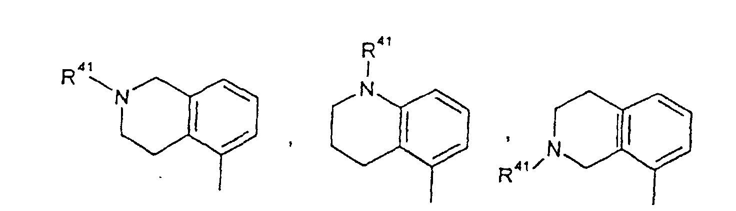 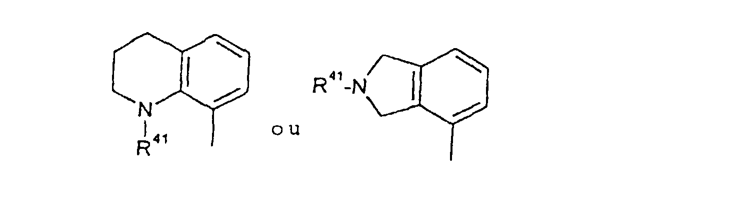 où
R⁴¹ est un groupe alkyle en C₁ à C₆
avec un ester d'acide chloroformique, de préférence l'ester 1-(1-chloro)-éthylique d'acide chloroformique ou l'ester méthylique d'acide chloroformique, puis avec des alcools, de préférence le méthanol, éventuellement en présence d'une base, pour obtenir des composés de formule générale (Ie)
R³⁸-A-D-E-G-L-R² (Ie)
dans laquelle
A, D, E, G, L et R² ont la définition indiquée ci-dessus et
R³⁸ représente un reste de formule 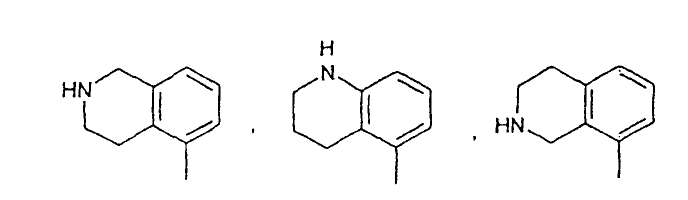 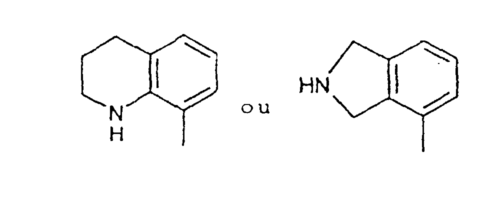
ou bien
[E] on fait réagir des composés de formule générale (Ie) avec des cétones en C₁ à C₆ ou des aldéhydes en C₁ à C₆ en présence d'un agent réducteur, de préférence le cyanoborohydrure de sodium, éventuellement en présence d'un acide pour obtenir des composés de formule générale (If)
R³⁹-A-D-E-G-L-R² (If)
dans laquelle
A, D, E, G, L et R² ont la définition indiquée ci-dessus et
R³⁹ est un groupe alcényle en C₃ à C₆ ou alkyle en C₁ à C₆,
ou bien
[F] on fait réagir des composés de formule générale (Ie) avec des composés de formule générale (VIII)
R³⁵-R² (VIII)
dans laquelle
R³ a la définition indiquée dans la revendication 1
R³⁵ est un groupe partant, de préférence un halogène
dans des solvants inertes, éventuellement en présence d'une base, pour obtenir des composés de formule générale (Ig)
R⁴⁰-A-D-E-G-L-R² (Ig)
dans laquelle
A, D, E, G, L et R² ont la définition indiquée ci-dessus et
R⁴⁰ représente un reste de formule 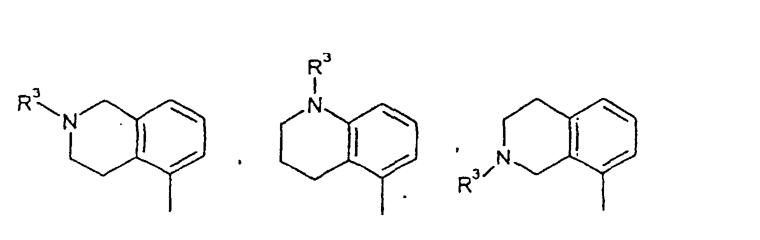 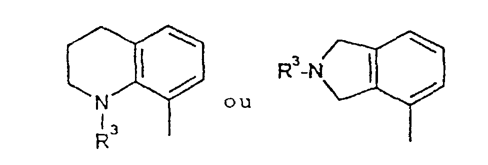
où
R³ a la définition indiquée ci-dessus,
ou bien
[G] on transforme des composés de formule générale (Ih) 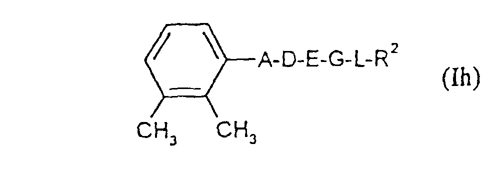 dans laquelle
A, D, E, G, L et R² ont la définition indiquée ci-dessus, par bromation radicalaire, par exemple avec le N-bromosuccinimide, dans un solvant inerte en composés de formule générale (Ii) 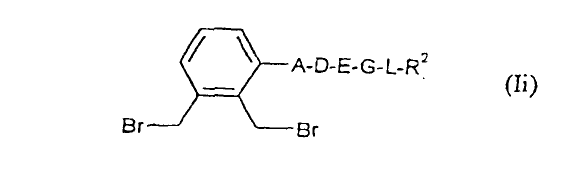 dans laquelle
A, D, E, G, L et R² ont la définition indiquée ci-dessus, que l'on fait ensuite réagir avec des composés de formule générale (IX) ou (X)
CH₂(CO₂R⁴²)₂ (IX)
H₂N-R³ (X)
formules dans lesquelles
R⁴² est un groupe alkyle en C₁ à C₆ et
R³ a la définition indiquée ci-dessus,
dans des solvants inertes, éventuellement en présence d'une base, pour obtenir des composés de formule générale (Ij)
R⁴³-A-D-E-G-L-R² (Ij)
dans laquelle
A, D, E, G, L et R² ont la définition indiquée ci-dessus et
R⁴³ représente un groupe 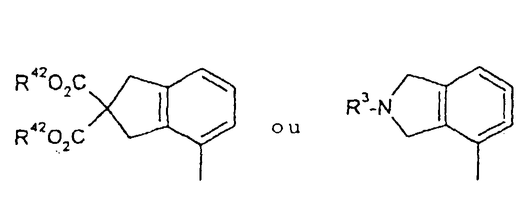
où
R⁴² et R³ ont la définition indiquée ci-dessus,
et le cas échéant, on l'introduit et l'on transforme en dérivés les substituants énumérés ci-dessus selon des modes opératoires classiques,
et au cas où D représente un groupe -SO- ou -SO₂-, on effectue une oxydation selon des modes opératoires classiques en partant des thioéthers correspondants (D = S),
et dans le cas des composés d'ammonium, on effectue l'alkylation en partant des amines correspondantes.

9. Composés de formule générale (II)
R¹-A-D-E-G-M-H (II)
dans laquelle
R¹ est un reste de formule 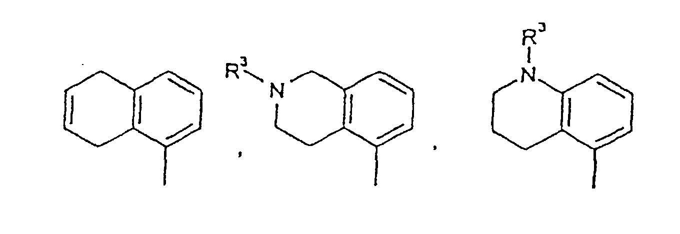 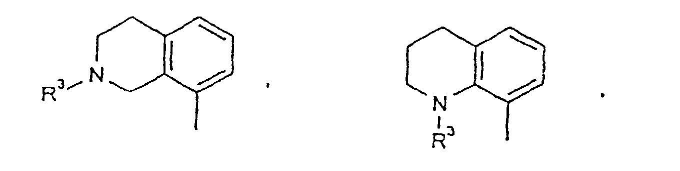 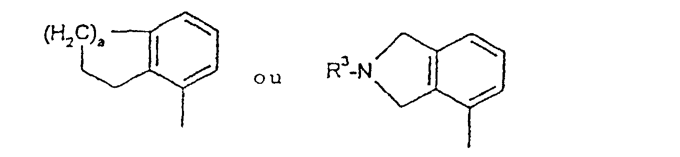 dans laquelle
a représente le nombre 1,
R³ est l'hydrogène, un groupe alcényle en C₂ à C₆, alkyle en C₁ à C₆ ou acyle en C₁ à C₆,
et où tous les systèmes cycliques et les restes énumérés ci-dessus sont éventuellement substitués comme indiqué dans la revendication 1, et
A, D, E, G et M ont les définitions indiquées dans les revendications 1 et 8.

10. Composés de formule générale (II) suivant la revendication 9, formule dans laquelle
R¹ représente un reste indane-4-yle substitué par un radical hydroxyalkyle en C₁ à C₆, ou bien
un reste de formule 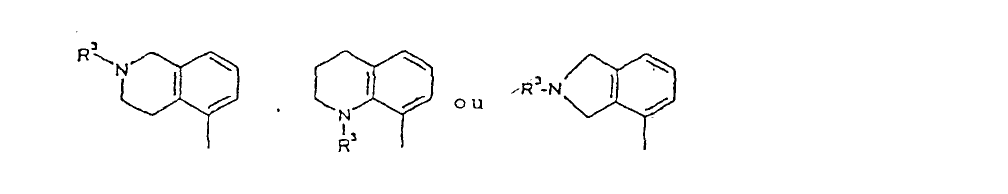 dans laquelle
R₃ est un radical alkyle en C₁ à C₆,
A, D, E et G ont les définitions indiquées dans la revendication 6 et
M a la définition indiquée dans la revendication 8.

11. Procédé de production de composés suivant la revendication 9, **caractérisé en ce que**
[A] on fait réagir des composés de formule générale (VI)
R¹-A-D'-H (VI)
dans laquelle
R¹, A et D' ont la définition indiquée dans la revendication 8,
avec des composés de formule générale (XI)
R⁴⁴-E-G-NO₂ (XI)
dans laquelle
E et G ont la définition indiquée dans la revendication 1 et
R⁴⁴ est un groupe partant, de préférence un halogène
dans des solvants inertes, éventuellement en présence d'une base puis on fait réagir les composés obtenus avec des agents réducteurs classiques, de préférence H₂/Pd/C dans un solvant inerte, ou bien avec l'hydrate d'hydrazine en présence de Pd/C, éventuellement avec hydrogénation simultanée des liaisons multiples carbone-à-carbone, pour obtenir des composés de formule générale (IIa)
R¹-A-D'-E-G-NH₂ (IIa)
dans laquelle
R¹, A, D', E et G ont la définition indiquée ci-dessus, ou bien
[B] on fait réagir des composés de formule générale (IIb)
R¹-A-D-E-G-NH₂ (IIb)
dans laquelle
R¹, A, D, E et G ont la définition indiquée ci-dessus, avec un agent de nitrosation, de préférence une solution aqueuse d'acide sulfurique et de nitrite de sodium, la réaction étant suivie d'un chauffage de préférence à une température de 60 à 100°C, pour obtenir des composés de formule générale (IIc)
R¹-A-D-E-G-OH (IIc)
dans laquelle
R¹, A, D, E et G ont la définition indiquée ci-dessus, ou bien
[C] on fait réagir des composés de formule générale (XII)
R¹-R³⁶ (XII)
dans laquelle
R¹ a la définition indiquée ci-dessus et
R³⁶ est un groupe partant, de préférence un halogène, notamment le brome, et
avec des composés de formule générale (XIII)
HO-G-O-R⁴⁵ (XIII)
dans laquelle
G a la définition indiquée ci-dessus et
R⁴⁵ est un groupe alkyle en C₁ à C₆, de préférence le groupe méthyle,
dans un solvant inerte, de préférence le diméthylformamide ou la pyridine, éventuellement en présence d'une base, de préférence le carbonate de calcium et le cas échéant en présence de sels de cuivre (I/II), de préférence l'oxyde de cuivre (II) ou d'iodure de cuivre (I), dans une plage de températures de 0°C à 200°C, de préférence de 80 à 150°C et sous pression normale, pour obtenir des composés de formule générale (Ik)
R¹-O-G-O-R⁴⁵ (Ik)
dans laquelle
R¹, G et R⁴⁵ ont la définition indiquée ci-dessus, puis on fait réagir un des composés obtenus en présence d'un acide, de préférence l'acide bromhydrique, pour obtenir des composés de formule générale (IId)
R¹-O-G-OH (IId)
ou bien
[D] on fait réagir des composés de formule générale (VI)
R¹-A-D'-H (VI)
dans laquelle
R¹, A et D' ont la définition indiquée dans la revendication 8,
avec des composés de formule générale (XIV)
R⁴⁶-E-G'-R⁴⁷ (XIV)
dans laquelle
R⁴⁶ a la définition indiquée pour R³⁶ et y est identique ou en est différent,
E a la définition mentionnée ci-dessus,
G' est un hétérocycle aromatique pentagonal à heptagonal à deux liaisons, ayant jusqu'à 3 hétéro-atomes de la série soufre, azote et/ou oxygène, qui peut être substitué le cas échéant avec un ou plusieurs substituants identiques ou différents tels que définis pour G dans la revendication 1 et
R⁴⁷ représente un halogène, de préférence le chlore ou le brome,
pour obtenir un composé de formule générale (XV)
R¹-A-D'-E-G'-R⁴⁷ (XV)
dans laquelle
R¹, A, D', E, G' et R⁴⁷ ont la définition indiquée ci-dessus, dans des solvants inertes, éventuellement en présence d'une base puis on transforme les composés obtenus avec l'amidure de potassium dans l'ammoniac liquide en les amines libres correspondantes de formule générale (IIe)
R¹-A-D'-E-G'-NH₂ (IIe)
dans laquelle
R¹, A, D', E et G' ont la définition indiquée ci-dessus.

12. Composés de formule
Cl -SO₂-CH₂-CH₂-CH₂-CF₃
ou
Cl -SO₂-CH₂-CH₂-CH₂-CF₂-CF₃,

13. Composition pharmaceutique qui comprend comme composant actif au moins un composé suivant l'une quelconque des revendications 1 à 7 en mélange avec au moins un support ou excipient essentiellement non toxique acceptable du point de vue pharmaceutique.

14. Composés suivant l'une quelconque des revendications 5 à 7, destinés à être utilisés comme médicament en médecine humaine et en médecine vétérinaire.

15. Utilisation des composés suivant l'une quelconque des revendications 1 à 7 pour la préparation d'un médicament destiné au traitement préventif et/ou curatif de maladies neurodégénératives.

16. Utilisation des composés suivant l'une quelconque des revendications 1 à 7 pour la préparation d'un médicament destiné au traitement préventif et/ou curatif d'ischémies cérébrales ou de traumatismes crâne / cerveau.

17. Utilisation des composés suivant l'une quelconque des revendications 1 à 7 pour la préparation d'un médicament destiné au traitement d'états douloureux, de vomissements, de nausées, du glaucome, de l'asthme, de l'anorexie, de convulsions, de rhumatismes, de sédation et de troubles moteurs.
